(19) Europäisches Patentamt / European Patent Office / Office européen des brevets

(11) EP 4 480 480 A2

(12) EUROPEAN PATENT APPLICATION

(43) Date of publication:
**25.12.2024 Bulletin 2024/52**

(21) Application number: **24196936.9**

(22) Date of filing: **27.07.2021**

(51) International Patent Classification (IPC):
***A61K 31/437*** *(2006.01)*

(52) Cooperative Patent Classification (CPC):
**C07D 471/04; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.07.2020 US 202063058220 P**

(62) Document number(s) of the earlier application(s) in accordance with Art. 76 EPC:
**21759022.3 / 4 188 929**

(71) Applicants:
- **Bayer AG**
  **51373 Leverkusen (DE)**
- **The Broad Institute Inc.**
  **Cambridge, MA 02142 (US)**
- **Dana-Farber Cancer Institute, Inc.**
  **Boston, MA 02215 (US)**

(72) Inventors:
- **SCHULZE, Volker**
  **16562 Hohen Neuendorf OT Bergfelde (DE)**
- **MENGEL, Anne**
  **13187 Berlin (DE)**
- **FARIA ALVARES DE LEMOS, Adelaide, Clara**
  **10823 Berlin (DE)**
- **RAUH, Ulrike**
  **13465 Berlin (DE)**
- **BÖMER, Ulf**
  **16548 Glienicke (DE)**
- **HILLIG, Roman**
  **12159 Berlin (DE)**
- **LECHNER, Christian**
  **10117 Berlin (DE)**
- **MORTIER, Jeremie, Xavier, G.**
  **12055 Berlin (DE)**
- **SÜLZLE, Detlev**
  **13465 Berlin (DE)**
- **KAULFUSS, Stefan**
  **10245 Berlin (DE)**
- **CORSELLO, Steven**
  **Boston, Massachusetts, 02215 (US)**
- **HANDING, Katarzyna**
  **Cambridge Massachusetts, 02142 (US)**
- **CALIMAN, Alisha**
  **Cambridge, Massachusetts, 02142 (US)**
- **MADEC, Amael**
  **Cambridge, Massachusetts, 02142 (US)**

(74) Representative: **HGF**
**HGF Limited**
**1 City Walk**
**Leeds LS11 9DX (GB)**

Remarks:
- This application was filed on 28-08-2024 as a divisional application to the application mentioned under INID code 62.
- Claims filed after the date of filing of the application (Rule 68(4) EPC).

(54) **SUBSTITUTED PYRROLO-PYRIDINONE DERIVATIVES AND THERAPEUTIC USES THEREOF**

(57) Compounds of formula (I)

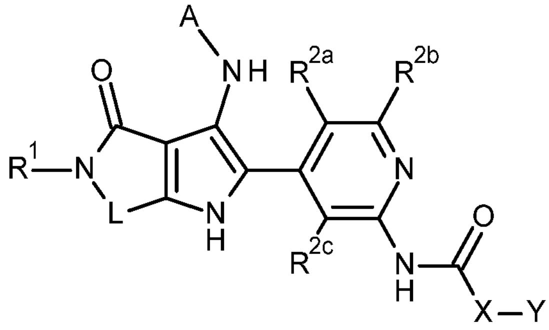

(I),



EP 4 480 480 A2

processes for their production and their use as pharmaceuticals.

**Figure 1:**

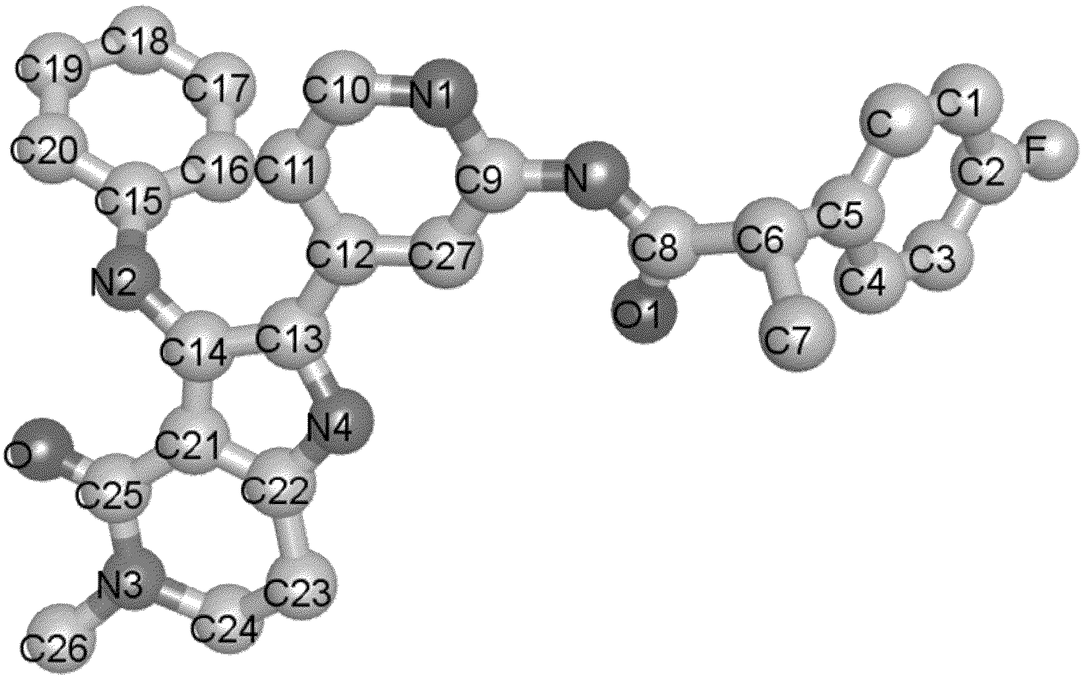

**Figure 1: Structure of Intermediate 42**

For clarity only the ligand atoms are shown. Carbon atom C6 unambiguously features the (*S*)-configuration.

## Description

## INTRODUCTION

The invention relates to substituted 3-amino-2-[2-(acylamino)pyridin-4-yl]-4H-pyrrolo[3,2-c]pyridin-4-one compounds, a process for their production and uses thereof.

## BACKGROUND OF THE INVENTION

One of the most fundamental characteristics of cancer cells is their ability to sustain chronic proliferation whereas in normal tissues the entry into and progression through the cell division cycle is tightly controlled to ensure a homeostasis of cell number and maintenance of normal tissue function. Loss of proliferation control was emphasized as one of the six hallmarks of cancer [Hanahan D and Weinberg RA, Cell 100, 57, 2000; Hanahan D and Weinberg RA, Cell 144, 646, 2011].

The members of the casein kinase 1 (CSNK1) family are highly conserved and are expressed in many eukaryotes ranging from yeast to humans. Mammalian CSNK1 isoforms (α, β, γ, δ, ε) and their splice variants are involved in diverse cellular processes including membrane trafficking, circadian rhythm, cell cycle progression, chromosome segregation, apoptosis and cellular differentiation. Mutations and deregulation of CSNK1 expression and activity have been linked to proliferative diseases such as cancer (Knippschild, Onkologie 2005;28:508-514).

CSNK1 substrates are enzymes, transcription factors, splice factors, cytoskeleton proteins, receptors, membrane-associated proteins and cell signaling proteins. Since recognition motifs for CSNK1 are found on most cellular proteins, more than 140 in vitro and in vivo substrates have been reported thus far (Knippschild et al., Front Oncol. 2014 May 19;4:96). Several known substrates, especially of the CSNK1α and δ isoforms, are involved in oncogenic signaling pathways as Wnt/β-catenin (β-catenin; dishevelled (DVL); adenomatous polyposis coli (APC); nuclear factor of activated Tcells, cytoplasmic 3 (NFATC3)), p53 (p53; p53/E3 ubiquitin-protein ligase Mdm2 (MDM2)), PI3K/AKT (forkhead box protein O1 (Foxo1)), death receptor signaling (Fas-associated death domain protein (FADD); and BH3-interactive domain death agonist (BID)) (Schittek and Sinnberg Molecular Cancer 2014, 13:231). A distinctive feature of CSNK1 family members is their exclusive need of ATP to phosphorylate their substrates and their independency of other co-factors.

CSNK1α plays a role in the mitotic spindle formation during cell division and in DNA repair mechanisms, and participates in RNA metabolism. Antibodies specific for CSNK1α block cell cycle progression during M phase in mouse oocytes, which indicates that CSNK1α is required for proper cell cycle progression in these cells. CSNK1α can be found at the centrosomes, microtubule asters and the kinetochore. Similarly, CSNK1α regulates apoptotic signaling pathways, however, there seems to be cell type-specific differences. CSNK1α has been shown to have an anti-apoptotic function in the extrinsic apoptosis pathway. Its inhibition increased Fas-induced apoptosis in Hela cells, whereas the overexpression of CSNK1α delayed cell death, caused by the phosphorylation of BID, prevented the caspase 8 dependent cleavage of BID. In addition, CSNK1α inhibits TRAIL induced apoptosis by modification of the TNF receptor or FADD at the death-inducing signaling complex (DISC). Therefore, downregulation of CSNK1α leads to an enhancement of TRAIL-induced cell death. Likewise, CSNK1α promotes cell survival by interacting with the retinoid X receptor (RXR). Downregulation of CSNK1α enhances the apoptotic effect of RXR agonists (Schittek and Sinnberg, Molecular Cancer 2014, 13:231).

Knockdown or downregulation of CSNK1α in the intestinal epithelium of mice, in human colon cancers or in leukemia cells triggers p53 activation. Similarly, one study showed that CSNK1α stably associates with MDM2, stimulates MDM2-p53 binding, and cooperates with MDM2 to inactivate p53. These data suggest that inhibition of CSNK1α activity increases p53 activity. The knockdown of CSNK1α induces p53 transcriptional activity by reducing the inhibitory effect of MDM2 for p53 since MDM2 phosphorylation is necessary for interaction with p53 (Schittek and Sinnberg, Molecular Cancer 2014, 13:231).

Ribosomal protein S6 (RPS6) is a critical component of the 40S ribosomal subunit that mediates translation initiation. RPS6 activity is regulated by phosphorylation by CSNK1α, which phosphorylates serine residue 247, enhancing the phosphorylation of upstream sites (Hutchinson et al., JBC, 2011, 286, 10, 8688). CSNK1α inhibition leads to dramatic reduction in RPS6 phosphorylation and activation of p53, resulting in selective elimination of solid tumor and AML cells. Pharmacological inhibition of CSNK1α in p53wt colon and lung carcinoma as well as in AML induces p53 accumulation along with apoptosis. Targeting of CSNK1α provides a potential approach to the therapeutic activation of p53 in AML, a disorder predominantly associated with non-mutated p53 (Jaras et al., J. Exp. Med. 2014, 211, 4, 605).

CSNK1α is an essential participant in the aberrant NF-kB activity required for ABC DLBCL subtype survival. CSNK1α knockdown is specifically lethal to ABC DLBCL cells (Bidere, Nature, 458, 5 March 2009). Pharmacological inhibition of CSNK1α will specifically kill ABC-DLBCL due to the blocking of the CARD1 1-Bcl-10-MALT1 complex (CBM complex).

Thus, pharmacological inhibition of CSNK1α represents a new approach for the treatment of proliferative disorders, including solid tumors such as carcinomas, sarcomas, leukaemias and lymphoid malignancies or other disorders,

associated with uncontrolled cellular proliferation.

Due to the fact that especially cancer disease, being expressed by uncontrolled proliferative cellular processes in tissues of different organs of the human- or animal body, is still not considered to be a controlled disease in that sufficient drug therapies do not already exist, there is a strong need to provide further new therapeutically useful drugs, preferably those inhibiting new targets and providing new therapeutic options.

Therefore, inhibitors of Casein kinase 1 alpha and/or delta represent valuable compounds as single agent therapies that in some instances, can complement other therapeutic options either as single agents or in combination with other drugs.

WO 2016/120196 discloses 4H-pyrrolo[3,2-c]pyridin-4-one derivatives, which may be useful as Bub1 kinase inhibitors.

## SUMMARY OF THE INVENTION

The invention provides compounds that inhibit Casein kinase 1 alpha and/or Casein kinase 1 delta.

It has now been found that compounds of the present invention have surprising and advantageous properties.

In particular, compounds of the present invention have surprisingly been found to effectively inhibit CSNK1A1. In certain embodiments, compounds of the present invention display an $IC_{50}$ of below 100 nM in a CSNK1A1 kinase assay in the presence of 1 $\mu$M ATP. Furthermore, in certain embodiments, compounds of the present invention additionally show low inhibition of wild type-EGFR kinase. In certain embodiments, compounds of the present invention are less potent than 600 nM in a wild type-EGFR kinase assay in the presence of 2 mM ATP. In certain embodiments, compounds of the present invention display an $IC_{50}$ below 100 nM in a CSNK1A1 kinase assay in the presence of 1 mM ATP and are less potent than 100 nM in a wild type-EGFR kinase assay in the presence of 2 mM ATP.

In general, reduced or no inhibition of other kinases and specifically reduced or no inhibition of wild type EGFR kinase, in particular in a high ATP assay (e.g. with a concentration of 2 mM ATP), is considered to be relevant in the clinical setting to avoid/reduce unwanted side effects

associated with the inhibition of said wild type EGFR kinase, such as, for example, skin rash and GI toxicity.

## BRIEF DESCRIPTION OF THE FIGURE

Figure 1 shows an electron density map of the compound of Intermediate 42. For clarity only the ligand atoms are shown. Carbon atom C6 unambiguously features the (S)-configuration.

## DESCRIPTION OF THE INVENTION

In accordance with a first aspect, the invention relates to compounds of formula (I),

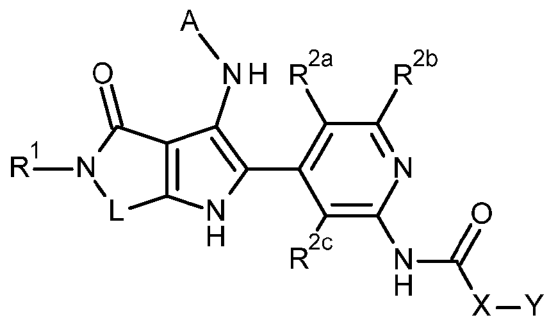

(I)

in which:

| | |
|---|---|
| $R^1$ | represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen; |
| $R^{2a}$ | represents hydrogen, hydroxy, halogen, cyano, $-NH_2$, -NHMe, $-NMe_2$, $C_1$-$C_2$-alkoxy, $C_1$-$C_2$-haloalkyl, or $C_1$-$C_2$-haloalkoxy; |

$R^{2b}$ represents hydrogen, hydroxy, halogen, cyano, $-NH_2$, $-NHR^9$, or-$NMe_2$;

$R^{2c}$ represents hydrogen, halogen, cyano, $-NMe_2$, $C_1$-$C_2$-alkoxy, or $C_1$-$C_2$-haloalkoxy;

L represents , wherein * represents the point of direct

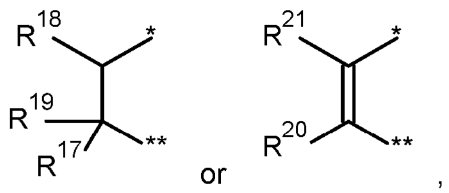

attachment to the adjacent N-atom and ** represents the point of direct attachment to the adjacent C-atom;

$R^{17}$ represents $C_1$-$C_3$-alkyl, $C_1$-$C_3$-alkoxy, $C_1$-$C_3$-haloalkyl or $C_3$-$C_6$-cycloalkyl-$C_1$-$C_4$-alkyl-;

$R^{18}$ represents hydrogen, $C_1$-$C_3$-alkyl, $C_1$-$C_3$-haloalkyl or $C_3$-$C_6$-cycloalkyl-$C_1$-$C_4$-alkyl-, or

$R^{17}$ and $R^{18}$, together with the carbon atoms to which they are attached, form a $C_3$-$C_6$-cycloalkyl ring;

$R^{19}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, $C_1$-$C_3$-alkyl, $C_1$-$C_3$-haloalkyl or $C_3$-$C_6$-cycloalkyl-$C_1$-$C_4$-alkyl-;

X represents methylene or ethylene, wherein said methylene and ethylene group are each independently optionally substituted, one or more times, with $R^3$;

Y represents phenyl or a heteroaryl group, wherein said phenyl and heteroaryl group are each independently optionally substituted, one or more times, with $R^4$;

$R^3$ represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, $C_3$-$C_4$-cycloalkyl, $-NHR^6$, $R^5O$-$C_1$-$C_3$-alkyl, $R^6R^7N$-$C_1$-$C_5$-alkyl, or $R^6R^7N$-X'-C($R^{10}$)($R^{11}$)-Ci-$C_2$-alkyl-;

X' represents methylene or ethylene;

$R^4$ represents hydroxy, halogen, cyano, $C_1$-$C_4$-alkyl, $C_1$-$C_4$-alkoxy, $C_1$-$C_4$-haloalkyl, $C_1$-$C_4$-haloalkoxy, $-NMe_2$, $-CO_2R^{14}$, $-CONR^{15}R^{16}$, or-$SO_2Me$;

$R^5$ represents hydrogen, $C_1$-$C_2$-alkyl, $C_1$-$C_2$-haloalkyl, or $C_3$-$C_4$-cycloalkyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;

A represents a group selected from:

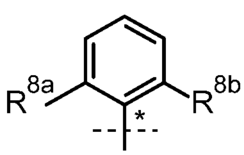, 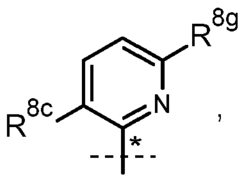, 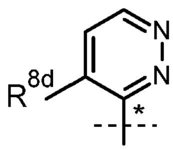, 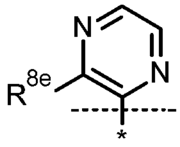,

and

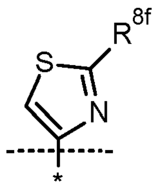

,

wherein * indicates the point of attachment of said group to the -NH- of formula (I);

$R^{8a}$ represents hydrogen, hydroxy, halogen, cyano, $C_1$-$C_2$-alkyl, or $C_1$-$C_2$-haloalkyl;

$R^{8b}$ represents hydrogen, hydroxy, halogen, cyano, $C_1$-$C_2$-alkyl, or $C_1$-$C_2$-haloalkyl;

$R^{8c}$ represents hydrogen, hydroxy, halogen, cyano, $C_1$-$C_2$-alkyl, or $C_1$-$C_2$-haloalkyl;

$R^{8d}$ represents hydrogen, hydroxy, halogen, cyano, $C_1$-$C_2$-alkyl, or $C_1$-$C_2$-haloalkyl;

$R^{8e}$ represents hydrogen, hydroxy, halogen, cyano, $C_1$-$C_2$-alkyl, or $C_1$-$C_2$-haloalkyl;

$R^{8f}$ represents hydrogen or $C_1$-$C_2$-alkyl;

$R^{8g}$ represents hydrogen or halogen;

$R^9$ represents $C_1$-$C_3$-alkyl or cyclopropyl;

$R^{9a}$ represents $C_1$-$C_3$-alkyl or cyclopropyl;

$R^{10}$ represents methyl, halogen, or hydroxy;

$R^{11}$ represents methyl, halogen, or hydroxy;

$R^{14}$ represents hydrogen, methyl or ethyl;

$R^{15}$ represents hydrogen, methyl, or ethyl;

$R^{16}$ represents hydrogen, methyl, ethyl, methoxyethyl, or dimethylaminoethyl, or

$R^{15}$ and $R^{16}$, together with the nitrogen atom to which they are attached, represent a 5- to 6-membered heterocyclic ring, wherein said heterocyclic ring optionally contains one or two further heteroatoms selected from N, O, S, or groups selected from C(=O), S(=O), and $SO_2$;

or an N-oxide, a salt, a tautomer or a stereoisomer of said compound, or a salt of said N-oxide, tautomer or stereoisomer.

In accordance with an embodiment of the first aspect, the present invention relates to compounds of formula (I) supra, wherein:

$R^1$ represents hydrogen or a group selected from $C_1$-$C_4$-alkyl and $C_3$-$C_6$-cycloalkyl, wherein said groups are each independently optionally substituted, one or more times, with halogen;

$R^{2a}$ represents hydrogen or halogen;

$R^{2b}$ represents hydrogen, halogen, $-NH_2$, or $-NHR^9$;

$R^{2c}$ represents hydrogen or halogen;

L represents wherein * represents the point of direct

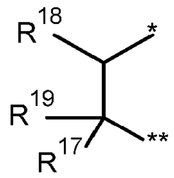
or
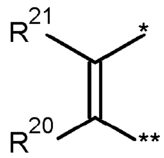
,

attachment to the adjacent N-atom and ** represents the point of direct attachment to the adjacent C-atom;

$R^{17}$ represents $C_1$-$C_3$-alkyl, $C_1$-$C_3$-haloalkyl or $C_3$-$C_6$-cycloalkyl-$C_1$-$C_4$-alkyl-;

$R^{18}$ represents hydrogen, $C_1$-$C_3$-alkyl, $C_1$-$C_3$-haloalkyl or $C_3$-$C_6$-cycloalkyl-$C_1$-$C_4$-alkyl-, or

$R^{17}$ and $R^{18}$, together with the carbon atoms to which they are attached, form a $C_3$-$C_6$-cycloalkyl ring;

$R^{19}$ represents hydrogen or $C_1$-$C_3$-alkyl;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, $C_1$-$C_3$-alkyl, or $C_1$-$C_3$-haloalkyl;

X represents methylene, wherein said methylene group is optionally substituted, one or more times, with $R^3$;

Y represents phenyl or a heteroaryl group, wherein said phenyl and heteroaryl group are each independently optionally substituted, one or more times, with $R^4$;

$R^3$ represents $C_1$-$C_3$-alkyl, $C_1$-$C_3$-haloalkyl, $R^5O$-$C_1$-$C_3$-alkyl, or $R^6R^7N$-$C_1$-$C_5$-alkyl;

$R^4$ represents halogen, $C_1$-$C_4$-alkyl, $C_1$-$C_4$-haloalkyl, $-OCF_3$, $-CO_2R^{14}$, $-CONR^{15}R^{16}$, or $-SO_2Me$;

$R^5$ represents hydrogen, methyl, or trifluoromethyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;

A represents a group selected from:

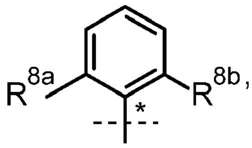
,
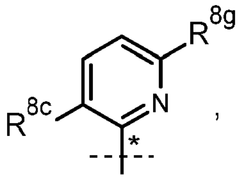
,
and
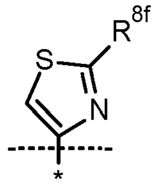
,

wherein * indicates the point of attachment of said group to the -NH- of formula (I);

$R^{8a}$ represents hydrogen, halogen, or $C_1$-$C_2$-alkyl;

$R^{8b}$ represents hydrogen or halogen;

$R^{8c}$ represents hydrogen or halogen;

$R^{8f}$ represents hydrogen or methyl;

$R^{8g}$ represents hydrogen or halogen;

$R^9$ represents $C_1$-$C_3$-alkyl or cyclopropyl;

$R^{9a}$ represents $C_1$-$C_3$-alkyl or cyclopropyl;

$R^{14}$ represents hydrogen, methyl or ethyl;

$R^{15}$ represents hydrogen or methyl;

$R^{16}$ represents methyl, ethyl, methoxyethyl, or dimethylaminoethyl, or

$R^{15}$ and $R^{16}$, together with the nitrogen atom to which they are attached, represent a 5- to 6-membered heterocyclic ring, wherein said heterocyclic ring optionally contains one further heteroatom selected from N and O;

or an N-oxide, a salt, a tautomer or a stereoisomer of said compound, or a salt of said N-oxide, tautomer or stereoisomer.

In accordance with an embodiment of the first aspect, the present invention relates to compounds of formula (I) supra, wherein:

$R^1$ represents hydrogen or $C_1$-$C_2$-alkyl, wherein the alkyl is optionally substituted, one or more times, with halogen;

$R^{2a}$ represents hydrogen or halogen;

$R^{2b}$ represents hydrogen, $-NH_2$ or $-NHR^9$;

$R^{2c}$ represents hydrogen or halogen;

L represents

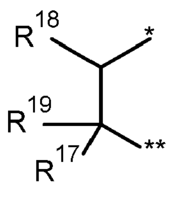
or
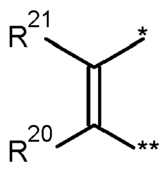
,

wherein * represents the point of direct attachment to the adjacent N-atom and ** represents the point

of direct attachment to the adjacent C-atom;

$R^{17}$ represents $C_1$-$C_3$-alkyl, $C_1$-$C_3$-haloalkyl or $C_3$-$C_4$-cycloalkyl-$C_1$-$C_3$-alkyl-;

$R^{18}$ represents hydrogen, $C_1$-$C_3$-alkyl, or $C_1$-$C_3$-haloalkyl, or

$R^{17}$ and $R^{18}$, together with the carbon atoms to which they are attached, form a $C_3$-$C_6$-cycloalkyl ring;

$R^{19}$ represents hydrogen or $C_1$-$C_3$-alkyl;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, $C_1$-$C_3$-alkyl, or $C_1$-$C_3$-haloalkyl;

X represents methylene, wherein said methylene group is optionally substituted, one or more times, with $R^3$;

Y represents phenyl or a pyridine group, wherein said phenyl and pyridine group are each independently optionally substituted, one or more times, with $R^4$;

$R^3$ represents $C_1$-$C_3$-alkyl, $C_1$-$C_3$-haloalkyl, $R^5O$-$C_1$-$C_2$-alkyl, or $R^6R^7N$-$C_1$-$C_5$-alkyl;

$R^4$ represents halogen, $C_1$-$C_4$-alkyl or $C_1$-$C_4$-haloalkyl;

$R^5$ represents hydrogen;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or

$R^6$ and $R^7$, together with the nitrogen atom to which they are attached, represent a 4- to 6-membered heterocyclic ring, wherein said heterocyclic ring is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;

A represents a group:

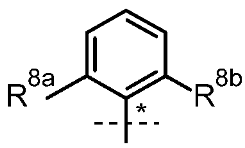,

and

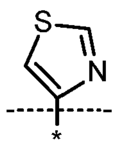,

wherein * indicates the point of attachment of said group to the -NH- of formula (I);

$R^{8a}$ represents hydrogen, halogen, or $C_1$-$C_2$-alkyl;

$R^{8b}$ represents hydrogen;

$R^9$ represents $C_1$-$C_3$-alkyl or cyclopropyl;

$R^{9a}$ represents $C_1$-$C_3$-alkyl or cyclopropyl;

or an N-oxide, a salt, a tautomer or a stereoisomer of said compound, or a salt of said N-oxide, tautomer or stereoisomer.

In accordance with an embodiment of the first aspect, the present invention relates to compounds of formula (I) supra, wherein:

$R^1$ represents hydrogen or $C_1$-$C_2$-alkyl;

$R^{2a}$ represents hydrogen;

$R^{2b}$ represents hydrogen;

$R^{2c}$ represents hydrogen;

L represents

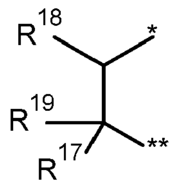 or 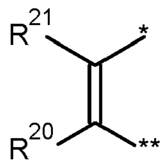 ,

wherein * represents the point of direct attachment to the adjacent N-atom and ** represents the point of direct attachment to the adjacent C-atom;

$R^{17}$ represents $C_1$-$C_3$-alkyl, $C_1$-$C_3$-haloalkyl or $C_3$-$C_4$-cycloalkyl-$C_1$-$C_3$-alkyl-;

$R^{18}$ represents hydrogen or $C_1$-$C_3$-alkyl, or

$R^{17}$ and $R^{18}$, together with the carbon atoms to which they are attached, form a $C_3$-$C_5$-cycloalkyl ring;

$R^{19}$ represents hydrogen;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, or $C_1$-$C_2$-alkyl;

X represents methylene, wherein said methylene group is optionally substituted, one or more times, with $R^3$;

Y represents phenyl, wherein said phenyl is independently optionally substituted, one or more times, with $R^4$;

$R^3$ represents $C_1$-$C_3$-alkyl, or $C_1$-$C_3$-haloalkyl;

$R^4$ represents halogen;

A represents a group:

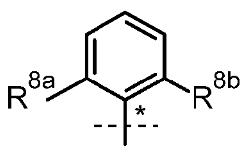 , and 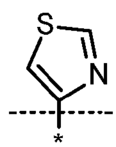 ,

wherein * indicates the point of attachment of said group to the -NH- of formula (I);

$R^{8a}$ represents hydrogen, halogen, or methyl;

$R^{8b}$ represents hydrogen;

or an N-oxide, a salt, a tautomer, or a stereoisomer of said compound, or a salt of said N-oxide, tautomer or stereoisomer.

In accordance with an embodiment of the first aspect, the present invention relates to compounds of formula (I),

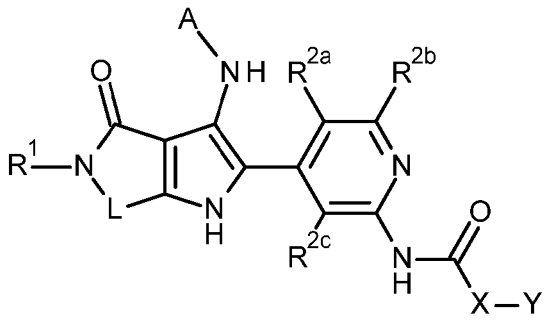

(I)

in which:

| | |
|---|---|
| $R^1$ | represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen; |
| $R^{2a}$ | represents hydrogen, hydroxy, halogen, cyano, $-NH_2$, -NHMe, $-NMe_2$, $C_1$-$C_2$-alkoxy, $C_1$-$C_2$-haloalkyl, or $C_1$-$C_2$-haloalkoxy; |
| $R^{2b}$ | represents hydrogen, hydroxy, halogen, cyano, $-NH_2$, $-NHR^9$, or $-NMe_2$; |
| $R^{2c}$ | represents hydrogen, halogen, cyano, $-NMe_2$, $C_1$-$C_2$-alkoxy, or $C_1$-$C_2$-haloalkoxy; |
| L | represents |

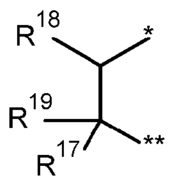

or

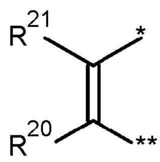

,

wherein * represents the point of direct attachment to the adjacent N-atom and ** represents the point of direct attachment to the adjacent C-atom;

| | |
|---|---|
| $R^{17}$ | represents $C_1$-$C_3$-alkyl, $C_1$-$C_3$-alkoxy, $C_1$-$C_3$-haloalkyl or $C_3$-$C_6$-cycloalkyl-$C_1$-$C_4$-alkyl-; |
| $R^{18}$ | represents hydrogen, $C_1$-$C_3$-alkyl, $C_1$-$C_3$-haloalkyl or $C_3$-$C_6$-cycloalkyl-$C_1$-$C_4$-alkyl-, or |
| $R^{17}$ and $R^{18}$, | together with the carbon atoms to which they are attached, form a $C_3$-$C_6$-cycloalkyl ring; |
| $R^{19}$ | represents hydrogen, $C_1$-$C_3$-alkyl, $C_1$-$C_3$-haloalkyl or $C_3$-$C_6$-cycloalkyl-$C_1$-$C_4$-alkyl-; |
| $R^{20}$ | represents hydrogen, $C_1$-$C_3$-alkyl, $C_1$-$C_3$-haloalkyl or $C_3$-$C_6$-cycloalkyl-$C_1$-$C_4$-alkyl-; |
| $R^{21}$ | represents hydrogen, $C_1$-$C_3$-alkyl, $C_1$-$C_3$-haloalkyl or $C_3$-$C_6$-cycloalkyl-$C_1$-$C_4$-alkyl-; |
| X | represents methylene or ethylene, wherein said methylene and ethylene group are each independently optionally substituted, one or more times, with $R^3$; |
| Y | represents phenyl or a heteroaryl group, wherein said phenyl and heteroaryl group are each independently optionally substituted, one or more times, with $R^4$; |
| $R^3$ | represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, |

$C_3$-$C_4$-cycloalkyl, -$NHR^6$, $R^5O$-$C_1$-$C_3$-alkyl, $R^6R^7N$-$C_1$-$C_5$-alkyl, or $R^6R^7N$-X'-C($R^{10}$)($R^{11}$)-$C_1$-$C_2$-alkyl-;

X' represents methylene or ethylene;

$R^4$ represents hydroxy, halogen, cyano, $C_1$-$C_4$-alkyl, $C_1$-$C_4$-alkoxy, $C_1$-$C_4$-haloalkyl, $C_1$-$C_4$-haloalkoxy, -$NMe_2$, -$CO_2R^{14}$, -$CONR^{15}R^{16}$, or -$SO_2Me$;

$R^5$ represents hydrogen, $C_1$-$C_2$-alkyl, $C_1$-$C_2$-haloalkyl, or $C_3$-$C_4$-cycloalkyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or -$CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, -$NH_2$, -$NHR^9$, -$NR^9R^{9a}$, or hydroxy;

A represents a group selected from:

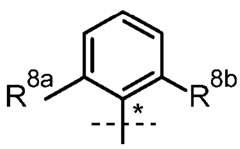, 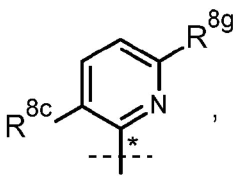, 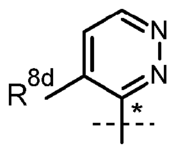 and 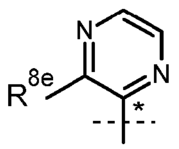,

wherein * indicates the point of attachment of said group to the -NH- of formula (I);

$R^{8a}$ represents hydrogen, hydroxy, halogen, cyano, $C_1$-$C_2$-alkyl, or $C_1$-$C_2$-haloalkyl;

$R^{8b}$ represents hydrogen, hydroxy, halogen, cyano, $C_1$-$C_2$-alkyl, or $C_1$-$C_2$-haloalkyl;

$R^{8c}$ represents hydrogen, hydroxy, halogen, cyano, $C_1$-$C_2$-alkyl, or $C_1$-$C_2$-haloalkyl;

$R^{8d}$ represents hydrogen, hydroxy, halogen, cyano, $C_1$-$C_2$-alkyl, or $C_1$-$C_2$-haloalkyl;

$R^{8e}$ represents hydrogen, hydroxy, halogen, cyano, $C_1$-$C_2$-alkyl, or $C_1$-$C_2$-haloalkyl;

$R^{8g}$ represents hydrogen or halogen;

$R^9$ represents $C_1$-$C_3$-alkyl or cyclopropyl;

$R^{9a}$ represents $C_1$-$C_3$-alkyl or cyclopropyl;

$R^{10}$ represents methyl, halogen, or hydroxy;

$R^{11}$ represents methyl, halogen, or hydroxy;

$R^{14}$ represents hydrogen, methyl or ethyl;

$R^{15}$ represents hydrogen, methyl, or ethyl;

$R^{16}$ represents hydrogen, methyl, ethyl, methoxyethyl, or dimethylaminoethyl, or

$R^{15}$ and $R^{16}$, together with the nitrogen atom to which they are attached, represent a 5- to 6-membered heterocyclic ring, wherein said heterocyclic ring optionally contains one or two further heteroatoms selected from N,

O, S, or groups selected from C(=O), S(=O), and $SO_2$;

or an N-oxide, a salt, a tautomer or a stereoisomer of said compound, or a salt of said N-oxide, tautomer or stereoisomer.

One aspect of the invention are compounds of formula (I) as described in the examples, as characterized by their names in the title, as claimed in claim 5, and/or their structures as well as the subcombinations of all residues specifically disclosed in the compounds of the examples.

Another aspect of the present invention are the intermediates used for their synthesis.

One special aspect of the invention are intermediates 1-3, or a salt thereof:

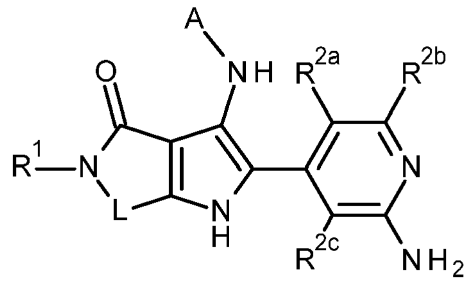

1-3

in which $R^1$, $R^{2a}$, $R^{2b}$, $R^{2c}$, A, and L are as defined herein for the compound of general formula (I).

Another aspect of the invention relates to the use of intermediates 1-3, or a salt thereof:

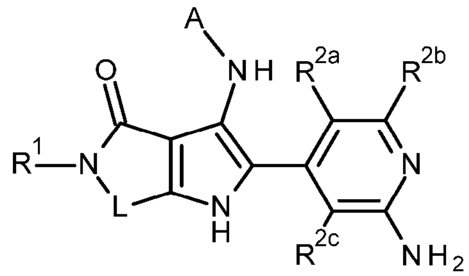

1-3

in which $R^1$, $R^{2a}$, $R^{2b}$, $R^{2c}$, A, and L are as defined herein for the compound of general formula (I), for preparing a compound of formula (I) as defined herein or an N-oxide, a salt, a tautomer or a stereoisomer of said compound, or a salt of said N-oxide, tautomer or stereoisomer.

Another aspect of the invention relates to the use of intermediates 6-1:

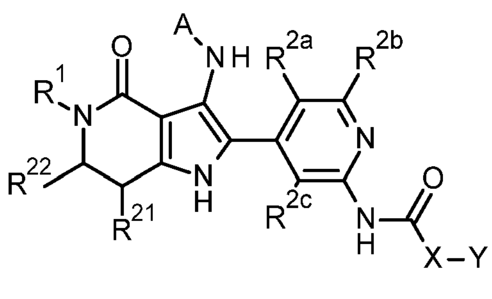

**6-1**

,

in which $R^1$, $R^{2a}$, $R^{2b}$, $R^{2c}$, $R^{21}$, $R^{22}$, A, X, and Y are as defined for the compound of general formula (I), for preparing a compound of formula (Ib) as defined herein or an N-oxide, a salt, a tautomer or a stereoisomer of said compound, or a salt of said N-oxide, tautomer or stereoisomer.

Another aspect of the invention relates to the use of any of the intermediates described herein for preparing a compound of formula (I) as defined herein or an N-oxide, a salt, a tautomer or a stereoisomer of said compound, or a

salt of said N-oxide, tautomer or stereoisomer.

In further embodiments of the above-mentioned aspects, the invention relates to compounds of formula (I), wherein:

$R^1$ represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen.

In further embodiments of the above-mentioned aspects, the invention relates to compounds of formula (I), wherein:

$R^1$ represents hydrogen or a group selected from $C_1$-$C_4$-alkyl and $C_3$-$C_6$-cycloalkyl, wherein said groups are each independently optionally substituted, one or more times, with halogen.

In further embodiments of the above-mentioned aspects, the invention relates to compounds of formula (I), wherein:

$R^1$ represents hydrogen or $C_1$-$C_2$-alkyl, wherein said alkyl is independently optionally substituted, one or more times, with halogen.

In further embodiments of the above-mentioned aspects, the invention relates to compounds of formula (I), wherein:

$R^1$ represents hydrogen or $C_1$-$C_2$-alkyl.

In further embodiments of the above-mentioned aspects, the invention relates to compounds of formula (I), wherein:

$R^{2a}$ represents hydrogen, hydroxy, halogen, cyano, $-NH_2$, -NHMe, $-NMe_2$, $C_1$-$C_2$-alkoxy, $C_1$-$C_2$-haloalkyl, or $C_1$-$C_2$-haloalkoxy.

In further embodiments of the above-mentioned aspects, the invention relates to compounds of formula (I), wherein:

$R^{2a}$ represents hydrogen or halogen.

In further embodiments of the above-mentioned aspects, the invention relates to compounds of formula (I), wherein:

$R^{2a}$ represents hydrogen.

In further embodiments of the above-mentioned aspects, the invention relates to compounds of formula (I), wherein:

$R^{2b}$ represents hydrogen, hydroxy, halogen, cyano, $-NH_2$, $-NHR^9$, or $-NMe_2$.

In further embodiments of the above-mentioned aspects, the invention relates to compounds of formula (I), wherein:

$R^{2b}$ represents hydrogen, halogen, $-NH_2$, or $-NHR^9$.

In further embodiments of the above-mentioned aspects, the invention relates to compounds of formula (I), wherein:

$R^{2b}$ represents hydrogen, $-NH_2$, or $-NHR^9$.

In further embodiments of the above-mentioned aspects, the invention relates to compounds of formula (I), wherein:

$R^{2b}$ represents hydrogen.

In further embodiments of the above-mentioned aspects, the invention relates to compounds of formula (I), wherein:

$R^{2c}$ represents hydrogen, halogen, cyano, $-NMe_2$, $C_1$-$C_2$-alkoxy, or $C_1$-$C_2$-haloalkoxy.

In further embodiments of the above-mentioned aspects, the invention relates to compounds of formula (I), wherein:

$R^{2c}$ represents hydrogen or halogen.

In further embodiments of the above-mentioned aspects, the invention relates to compounds of formula (I), wherein:

$R^{2c}$ represents hydrogen.

In further embodiments of the above-mentioned aspects, the invention relates to compounds of formula (I), wherein:

L represents

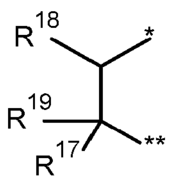 or 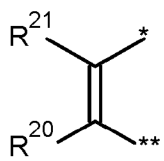 ,

wherein * represents the point of direct attachment to the adjacent N-atom and ** represents the point of direct attachment to the adjacent C-atom.

In further embodiments of the above-mentioned aspects, the invention relates to compounds of formula (I), wherein:

L represents

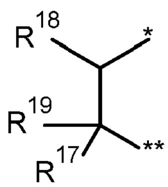 ,

wherein * represents the point of direct attachment to the adjacent N-atom and ** represents the point of direct attachment to the adjacent C-atom.

In further embodiments of the above-mentioned aspects, the invention relates to compounds of formula (I), wherein:

L represents

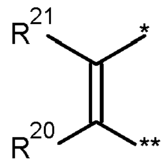

,

wherein * represents the point of direct attachment to the adjacent N-atom and ** represents the point of direct attachment to the adjacent C-atom.

In further embodiments of the above-mentioned aspects, the invention relates to compounds of formula (I), wherein:

$R^{17}$ represents $C_1$-$C_3$-alkyl, $C_1$-$C_3$-alkoxy, $C_1$-$C_3$-haloalkyl or $C_3$-$C_6$-cycloalkyl-$C_1$-$C_4$-alkyl-.

In further embodiments of the above-mentioned aspects, the invention relates to compounds of formula (I), wherein:

$R^{17}$ represents $C_1$-$C_3$-alkyl, $C_1$-$C_3$-haloalkyl or $C_3$-$C_6$-cycloalkyl-$C_1$-$C_4$-alkyl-.

In further embodiments of the above-mentioned aspects, the invention relates to compounds of formula (I), wherein:

$R^{17}$ represents $C_1$-$C_3$-alkyl, $C_1$-$C_3$-haloalkyl or $C_3$-$C_4$-cycloalkyl-$C_1$-$C_3$-alkyl-.

In further embodiments of the above-mentioned aspects, the invention relates to compounds of formula (I), wherein:

$R^{18}$ represents hydrogen, $C_1$-$C_3$-alkyl, $C_1$-$C_3$-haloalkyl or $C_3$-$C_6$-cycloalkyl-$C_1$-$C_4$-alkyl-.

In further embodiments of the above-mentioned aspects, the invention relates to compounds of formula (I), wherein:

$R^{18}$ represents hydrogen, $C_1$-$C_3$-alkyl, or $C_1$-$C_3$-haloalkyl.

In further embodiments of the above-mentioned aspects, the invention relates to compounds of formula (I), wherein:

$R^{18}$ represents hydrogen or $C_1$-$C_3$-alkyl.

In further embodiments of the above-mentioned aspects, the invention relates to compounds of formula (I), wherein:
$R^{17}$ and $R^{18}$, together with the carbon atoms to which they are attached, form a $C_3$-$C_6$-cycloalkyl ring.

In further embodiments of the above-mentioned aspects, the invention relates to compounds of formula (I), wherein:
$R^{17}$ and $R^{18}$, together with the carbon atoms to which they are attached, form a $C_3$-$C_5$-cycloalkyl ring.

In further embodiments of the above-mentioned aspects, the invention relates to compounds of formula (I), wherein:

$R^{19}$ represents hydrogen, $C_1$-$C_3$-alkyl, $C_1$-$C_3$-haloalkyl or $C_3$-$C_6$-cycloalkyl-$C_1$-$C_4$-alkyl-.

In further embodiments of the above-mentioned aspects, the invention relates to compounds of formula (I), wherein:

$R^{19}$ represents hydrogen or $C_1$-$C_3$-alkyl.

In further embodiments of the above-mentioned aspects, the invention relates to compounds of formula (I),

wherein:

$R^{19}$ represents hydrogen.

In further embodiments of the above-mentioned aspects, the invention relates to compounds of formula (I), wherein:

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-.

In further embodiments of the above-mentioned aspects, the invention relates to compounds of formula (I), wherein:

$R^{21}$ represents hydrogen, $C_1$-$C_3$-alkyl, $C_1$-$C_3$-haloalkyl or $C_3$-$C_6$-cycloalkyl-$C_1$-$C_4$-alkyl-.

In further embodiments of the above-mentioned aspects, the invention relates to compounds of formula (I), wherein:

$R^{21}$ represents hydrogen, $C_1$-$C_3$-alkyl, or $C_1$-$C_3$-haloalkyl.

In further embodiments of the above-mentioned aspects, the invention relates to compounds of formula (I), wherein:

$R^{21}$ represents hydrogen, or $C_1$-$C_2$-alkyl.

In further embodiments of the above-mentioned aspects, the invention relates to compounds of formula (I), wherein:

X represents methylene or ethylene,

wherein said methylene and ethylene group are each independently optionally substituted, one or more times, with $R^3$.
In further embodiments of the above-mentioned aspects, the invention relates to compounds of formula (I), wherein:

X represents methylene,

wherein said methylene group is optionally substituted, one or more times, with $R^3$.
In further embodiments of the above-mentioned aspects, the invention relates to compounds of formula (I), wherein:

Y represents phenyl or a heteroaryl group,

wherein said phenyl and heteroaryl group are each independently optionally substituted, one or more times, with $R^4$.
In further embodiments of the above-mentioned aspects, the invention relates to compounds of formula (I), wherein:

Y represents phenyl or a pyridine group,

wherein said phenyl and pyridine group are each independently optionally substituted, one or more times, with $R^4$.
In further embodiments of the above-mentioned aspects, the invention relates to compounds of formula (I), wherein:

Y represents phenyl,

wherein said phenyl is independently optionally substituted, one or more times, with $R^4$.
In further embodiments of the above-mentioned aspects, the invention relates to compounds of formula (I), wherein:

$R^3$ represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, $C_3$-$C_4$-cy-

cloalkyl, $-NHR^6$, $R^5O-C_1-C_3$-alkyl, $R^6R^7N-C_1-C_5$-alkyl, or $R^6R^7N-X'-C(R^{10})(R^{11})-C_1-C_2$-alkyl-.

In further embodiments of the above-mentioned aspects, the invention relates to compounds of formula (I), wherein:

$R^3$ represents $C_1-C_3$-alkyl, $C_1-C_3$-haloalkyl, $R^5O-C_1-C_3$-alkyl, or $R^6R^7N-C_1-C_5$-alkyl.

In further embodiments of the above-mentioned aspects, the invention relates to compounds of formula (I), wherein:

$R^3$ represents $C_1-C_3$-alkyl, $C_1-C_3$-haloalkyl, $R^5O-C_1-C_2$-alkyl, or $R^6R^7N-C_1-C_5$-alkyl.

In further embodiments of the above-mentioned aspects, the invention relates to compounds of formula (I), wherein:

$R^3$ represents $C_1-C_3$-alkyl or $C_1-C_3$-haloalkyl.

In further embodiments of the above-mentioned aspects, the invention relates to compounds of formula (I), wherein:

X' represents methylene or ethylene.

In further embodiments of the above-mentioned aspects, the invention relates to compounds of formula (I), wherein:

X' represents methylene.

In further embodiments of the above-mentioned aspects, the invention relates to compounds of formula (I), wherein:

X' represents ethylene.

In further embodiments of the above-mentioned aspects, the invention relates to compounds of formula (I), wherein:

$R^4$ represents hydroxy, halogen, cyano, $C_1-C_4$-alkyl, $C_1-C_4$-alkoxy, $C_1-C_4$-haloalkyl, $C_1-C_4$-haloalkoxy, $-NMe_2$, $-CO_2R^{14}$, $-CONR^{15}R^{16}$, or $-SO_2Me$.

In further embodiments of the above-mentioned aspects, the invention relates to compounds of formula (I), wherein:

$R^4$ represents halogen, $C_1-C_4$-alkyl, $C_1-C_4$-haloalkyl, $-OCF_3$, $-CO_2R^{14}$, $-CONR^{15}R^{16}$, or $-SO_2Me$.

In further embodiments of the above-mentioned aspects, the invention relates to compounds of formula (I), wherein:

$R^4$ represents halogen, $C_1-C_4$-alkyl or $C_1-C_4$-haloalkyl.

In further embodiments of the above-mentioned aspects, the invention relates to compounds of formula (I), wherein:

$R^4$ represents halogen.

In further embodiments of the above-mentioned aspects, the invention relates to compounds of formula (I), wherein:

$R^5$ represents hydrogen, $C_1-C_2$-alkyl, $C_1-C_2$-haloalkyl, or $C_3-C_4$-cycloalkyl.

In further embodiments of the above-mentioned aspects, the invention relates to compounds of formula (I), wherein:

$R^5$ represents hydrogen, methyl, or trifluoromethyl.

In further embodiments of the above-mentioned aspects, the invention relates to compounds of formula (I), wherein:

$R^5$ represents hydrogen.

In further embodiments of the above-mentioned aspects, the invention relates to compounds of formula (I), wherein:

$R^6$ represents hydrogen, $C_1$-$C_2$-alkyl, $C_1$-$C_2$-haloalkyl, or $C_3$-$C_4$-cycloalkyl.

In further embodiments of the above-mentioned aspects, the invention relates to compounds of formula (I), wherein:

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl.

In further embodiments of the above-mentioned aspects, the invention relates to compounds of formula (I), wherein:

$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy.

In further embodiments of the above-mentioned aspects, the invention relates to compounds of formula (I), wherein:

$R^6$ and $R^7$, together with the nitrogen atom to which they are attached, represent a 4- to 7-membered heterocyclic ring, wherein said heterocyclic ring optionally contains one or more further heteroatoms selected from N, O, S, and is independently optionally substituted, one or more times, with halogen, $C_1$-$C_3$-alkyl, $C_3$-cycloalkyl-$C_1$-$C_2$-alkyl-, - $NH_2$, $-NHR^9$, $-NR^9R^{9a}$, or hydroxy.

In further embodiments of the above-mentioned aspects, the invention relates to compounds of formula (I), wherein:

$R^6$ and $R^7$, together with the nitrogen atom to which they are attached, represent a 4- to 6-membered heterocyclic ring, wherein said heterocyclic ring is independently optionally substituted, one or more times, with halogen, $C_1$-$C_3$-alkyl, $C_3$-cycloalkyl-$C_1$-$C_2$-alkyl-, - $NH_2$, $-NHR^9$, $-NR^9R^{9a}$, or hydroxy.

In further embodiments of the above-mentioned aspects, the invention relates to compounds of formula (I), wherein:

A represents a group selected from:

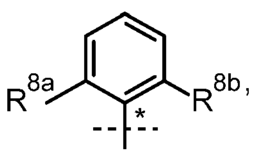 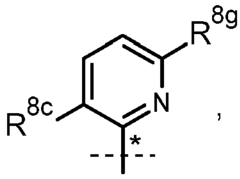 , 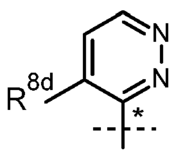 , 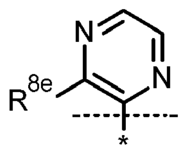 ,

and

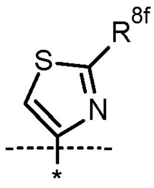

,

wherein * indicates the point of attachment of said group to the -NH- of formula (I);

In further embodiments of the above-mentioned aspects, the invention relates to compounds of formula (I), wherein:

A represents a group selected from:

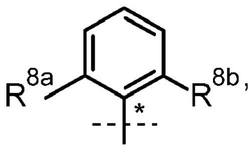

,

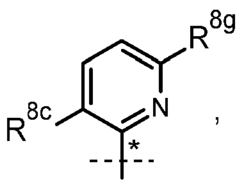

and

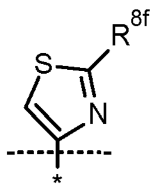

,

wherein * indicates the point of attachment of said group to the -NH- of formula (I);

In further embodiments of the above-mentioned aspects, the invention relates to compounds of formula (I), wherein:

A represents a group:

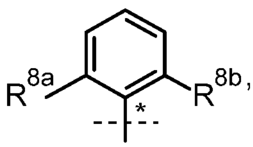

and

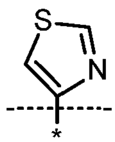

,

wherein * indicates the point of attachment of said group to the -NH- of formula (I);

In further embodiments of the above-mentioned aspects, the invention relates to compounds of formula (I), wherein:

A represents a group selected from:

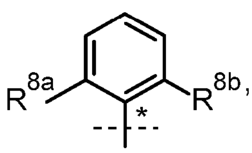

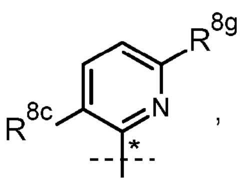

,

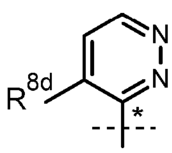

and

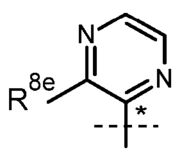

,

wherein * indicates the point of attachment of said group to the -NH- of formula (I).

In further embodiments of the above-mentioned aspects, the invention relates to compounds of formula (I), wherein:

A represents a group selected from:

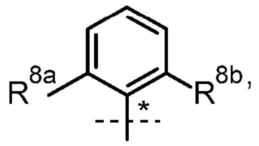

and

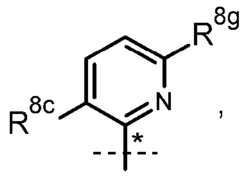

,

wherein * indicates the point of attachment of said group to the -NH- of formula (I).

In further embodiments of the above-mentioned aspects, the invention relates to compounds of formula (I), wherein:

A represents a group:

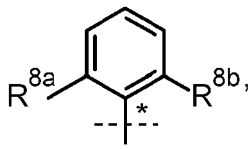

wherein * indicates the point of attachment of said group to the -NH- of formula (I).

In further embodiments of the above-mentioned aspects, the invention relates to compounds of formula (I), wherein:

$R^{8a}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8a}$ represents hydrogen, halogen, or $C_1$-$C_2$-alkyl.

In further embodiments of the above-mentioned aspects, the invention relates to compounds of formula (I), wherein:

$R^{8a}$ represents hydrogen, halogen, or methyl.

In further embodiments of the above-mentioned aspects, the invention relates to compounds of formula (I), wherein:

$R^{8b}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8b}$ represents hydrogen or halogen.

In further embodiments of the above-mentioned aspects, the invention relates to compounds of formula (I), wherein:

$R^{8b}$ represents hydrogen.

In further embodiments of the above-mentioned aspects, the invention relates to compounds of formula (I), wherein:

$R^{8c}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I),

wherein:

$R^{8c}$ represents hydrogen or halogen.

In further embodiments of the above-mentioned aspects, the invention relates to compounds of formula (I), wherein:

$R^{8d}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8e}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8f}$ represents hydrogen or $C_1$-$C_2$-alkyl;

In further embodiments of the above-mentioned aspects, the invention relates to compounds of formula (I), wherein:

$R^{8f}$ represents hydrogen or methyl;

In further embodiments of the above-mentioned aspects, the invention relates to compounds of formula (I), wherein:

$R^{8g}$ represents hydrogen or halogen.

In further embodiments of the above-mentioned aspects, the invention relates to compounds of formula (I), wherein:

$R^9$ represents $C_1$-$C_3$-alkyl or cyclopropyl.

In further embodiments of the above-mentioned aspects, the invention relates to compounds of formula (I), wherein:

$R^{9a}$ represents $C_1$-$C_3$-alkyl or cyclopropyl.

In further embodiments of the above-mentioned aspects, the invention relates to compounds of formula (I), wherein:

$R^{10}$ represents methyl, halogen, or hydroxy.

In further embodiments of the above-mentioned aspects, the invention relates to compounds of formula (I), wherein:

$R^{11}$ represents methyl, halogen, or hydroxy.

In further embodiments of the above-mentioned aspects, the invention relates to compounds of formula (I), wherein:

$R^{14}$ represents hydrogen, methyl or ethyl.

In further embodiments of the above-mentioned aspects, the invention relates to compounds of formula (I), wherein:

$R^{15}$ represents hydrogen, methyl, or ethyl.

In further embodiments of the above-mentioned aspects, the invention relates to compounds of formula (I), wherein:

$R^{15}$ represents hydrogen or methyl.

In further embodiments of the above-mentioned aspects, the invention relates to compounds of formula (I), wherein:

$R^{16}$ represents hydrogen, methyl, ethyl, methoxyethyl, or dimethylaminoethyl.

In further embodiments of the above-mentioned aspects, the invention relates to compounds of formula (I), wherein:

$R^{16}$ represents methyl, ethyl, methoxyethyl, or dimethylaminoethyl.

In further embodiments of the above-mentioned aspects, the invention relates to compounds of formula (I), wherein:

$R^{15}$ and $R^{16}$, together with the nitrogen atom to which they are attached, represent a 5- to 6-membered heterocyclic ring, wherein said heterocyclic ring optionally contains one or two further heteroatoms selected from N, O, S, or groups selected from C(=O), S(=O), and $SO_2$.

A further aspect of the invention are compounds of formula (I), which are present as their salts.

It is to be understood that the present invention relates to any sub-combination within any embodiment or aspect of the present invention of compounds of general formula (I), supra.

More particularly still, the present invention covers compounds of general formula (I) which are disclosed in the Example section of this text, infra.

In accordance with another aspect, the present invention covers methods of preparing compounds of the present invention, said methods comprising the steps as described in the Experimental Section herein.

In other embodiments of the invention, compounds are defined according to the claims as disclosed in the Claims section wherein the definitions are limited according to the preferred or more preferred definitions as disclosed below or specifically disclosed residues of the exemplified compounds and subcombinations thereof.

In certain embodiments, compounds of the present invention have surprising and advantageous properties.

In particular, compounds of the present invention have surprisingly been found to effectively inhibit Casein kinase 1 alpha and/or delta and may therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses. In particular, the disclosed compounds can be used for treatment or prophylaxis of diseases in which the uncontrolled cell growth, proliferation and/or survival, inappropriate cellular immune responses, or inappropriate cellular inflammatory responses is mediated by Casein kinase 1 alpha and/or delta, such as, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

**Definitions**

Constituents which are optionally substituted as stated herein, may be substituted, unless otherwise noted, one or more times, independently of one another at any possible position. When any variable occurs more than one time in any constituent, each definition is independent. For example, when in which $R^2$, $R^3$, $R^4$, $R^5$, $R^6$, $R^7$, and/or $R^8$, occur more than one time in any compound of formula (I) each definition of $R^2$, $R^3$, $R^4$, $R^5$, $R^6$, $R^7$, and $R^8$ is independent.

Should a constituent be composed of more than one part, e.g. $C_3$-$C_6$-cycloalkyl-$C_1$-$C_4$-alkyl-, unless stated otherwise, the position of a possible substituent can be at any of these parts at any suitable position (e.g. in the case of a substituted $C_3$-$C_6$-cycloalkyl-$C_1$-$C_4$-alkyl- group, substituents may be present at the $C_3$-$C_6$-cycloalkyl part of the group, at the $C_1$-$C_4$-alkyl part of the group or both). A hyphen at the beginning or at the end of the constituent marks the point of attachment to the rest of the molecule. Should a ring be substituted the substituent could be at any suitable position of the ring, also on a ring nitrogen atom if suitable.

The term "comprising" when used in the specification includes "consisting of".

If it is referred to "as mentioned above" or "mentioned above", *"supra"* within the description it is referred to any of the disclosures made within the specification in any of the preceding pages, or above on the same page.

If it is referred to "as mentioned herein", "described herein", "provided herein" or "stated herein" within the description it is referred to any of the disclosures made within the specification in any of the preceding or subsequent pages.

"suitable" within the sense of the invention means chemically possible to be made by methods within the knowledge of a skilled person.

The terms as mentioned in the present text have preferably the following meanings:

The term "halogen atom", "halo-" or "Hal-" is to be understood as meaning a fluorine, chlorine, bromine or iodine atom, preferably a fluorine, chlorine or bromine atom.

The term "$C_1$-$C_6$-alkyl" is to be understood as meaning a linear or branched, saturated, monovalent hydrocarbon group having 1, 2, 3, 4, 5, or 6 carbon atoms, e.g. a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl group, or an isomer thereof. Preferably, said group has 1, 2, 3 or 4 carbon atoms ("$C_1$-$C_4$-alkyl"), e.g. a methyl, ethyl, propyl, butyl, iso-propyl, iso-butyl, sec-butyl, tert-butyl group, more preferably 1, 2 or 3 carbon atoms ("$C_1$-$C_3$-alkyl"), e.g. a methyl, ethyl, n-propyl or iso-propyl group.

The term "$C_1$-$C_4$-haloalkyl" is to be understood as meaning a linear or branched, saturated, monovalent hydrocarbon group in which the term "$C_1$-$C_4$-alkyl" is defined *supra,* and in which one or more hydrogen atoms are replaced by a halogen atom, identically or differently, *i.e.* one halogen atom being independent from another. Preferably, said halogen atom is F. Said $C_1$-$C_4$-haloalkyl group is, for example, $-CF_3$, $-CHF_2$, $-CH_2F$, $-CF_2CF_3$, $-CH_2CH_2F$, $-CH_2CHF_2$, $-CH_2CF_3$, $-CH_2CH_2CF_3$, or $-CH(CH_2F)_2$. Preferably, said group has 1, 2, or 3 carbon atoms ("$C_1$-$C_3$-haloalkyl"). More Preferably, said group has 1, or 2 carbon atoms ("$C_1$-$C_2$-haloalkyl").

The term "$C_1$-$C_5$-hydroxyalkyl" means a linear or branched, saturated, monovalent hydrocarbon group in which the term "$C_1$-$C_5$-alkyl" is defined *supra,* and in which 1 or 2 hydrogen atoms are replaced with a hydroxy group, e.g. a hydroxymethyl, 1-hydroxyethyl, 2-hydroxyethyl, 1,2-dihydroxyethyl, 3-hydroxypropyl, 2-hydroxypropyl, 1-hydroxypropyl, 1-hydroxypropan-2-yl, 2-hydroxypropan-2-yl, 2,3-dihydroxypropyl, 1,3-dihydroxypropan-2-yl, 3-hydroxy-2-methyl-propyl, 2-hydroxy-2-methyl-propyl or 1-hydroxy-2-methyl-propyl group.

The term "$C_1$-$C_4$-alkoxy" is to be understood as meaning a linear or branched, saturated, monovalent, hydrocarbon group of formula -O-alkyl, in which the term "alkyl" is defined *supra,* e.g. a methoxy, ethoxy, n-propoxy, iso-propoxy, n-butoxy, iso-butoxy, tert-butoxy or sec-butoxy group, or an isomer thereof.

The term "$C_1$-$C_4$-haloalkoxy" is to be understood as meaning a linear or branched, saturated, monovalent $C_1$-$C_4$-alkoxy group, as defined *supra,* in which one or more of the hydrogen atoms are replaced, identically or differently, by a halogen atom. Preferably, said halogen atom is F. Said $C_1$-$C_4$-haloalkoxy group is, for example, $-OCF_3$, $-OCHF_2$, $-OCH_2F$, $-OCF_2CF_3$, or - $OCH_2CF_3$. Preferably, said group has 1, 2, or 3 carbon atoms ("$C_1$-$C_3$-haloalkoxy"). More particularly, said group has 1, or 2 carbon atoms ("$C_1$-$C_2$-haloalkoxy").

The term "$C_3$-$C_6$-cycloalkyl" is to be understood as meaning a saturated, monovalent, mono- or bicyclic hydrocarbon ring which contains 3, 4, 5 or 6 carbon atoms ("$C_3$-$C_6$-cycloalkyl"). Said $C_3$-$C_6$-cycloalkyl group is for example, a monocyclic hydrocarbon ring, e.g. a cyclopropyl, cyclobutyl, cyclopentyl or cyclohexyl, or a bicyclic hydrocarbon ring.

The term "halocyclopropyl" is to be understood as meaning a cyclopropyl group in which one or more hydrogen atoms are replaced by a halogen atom, identically or differently, *i.e.* one halogen atom being independent from another. Particularly, said halogen atom is F.

The term "4- to 7-membered heterocyclic ring" mean a monocyclic, saturated heterocycle with 4, 5, 6 or 7 ring atoms in total, wherein said heterocyclic ring contains one N atom and optionally one or more heteroatoms from the series N, O and S, or groups from the series C(=O), S(=O), and $SO_2$.

Said heterocyclic ring, without being limited thereto, can be a 4-membered ring, such as azetidinyl, oxetanyl or thietanyl, for example; or a 5-membered ring, such as tetrahydrofuranyl, 1,3-dioxolanyl, thiolanyl, pyrrolidinyl, imidazolidinyl, pyrazolidinyl, 1,1-dioxidothiolanyl, 1,2-oxazolidinyl, 1,3-oxazolidinyl or 1,3-thiazolidinyl, for example; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl or 1,2-oxazinanyl, for example, or a 7-membered ring, such as azepanyl, 1,4-diazepanyl or 1,4-oxazepanyl, for example.

The term "heteroaryl" means a monovalent, monocyclic, bicyclic or tricyclic aromatic ring having 5, 6, 8, 9, 10, 11, 12, 13 or 14 ring atoms (a "5- to 14-membered heteroaryl" group), preferably 5, 6, 9 or 10 ring atoms, which contains at least one ring heteroatom and optionally one, two or three further ring heteroatoms from the series: N, O and/or S, and which is bound via a ring carbon atom or optionally via a ring nitrogen atom (if allowed by valency).

Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl, pyridazinyl, pyrimidinyl, pyrazinyl or triazinyl; or a tricyclic heteroaryl group, such as, for example, carbazolyl, acridinyl or phenazinyl; or a 9-membered heteroaryl group, such as, for example, benzofuranyl, benzothienyl, benzoxazolyl, benzisoxazolyl, benzimidazolyl, benzothiazolyl, benzotriazolyl, indazolyl, indolyl, isoindolyl, indolizinyl or purinyl; or a 10-membered heteroaryl group, such as, for example, quinolinyl, quinazolinyl, isoquinolinyl, cinnolinyl, phthalazinyl, quinoxalinyl or pteridinyl.

In general, and unless otherwise mentioned, the heteroaryl or heteroarylene groups include all possible isomeric forms thereof, e.g.: tautomers and positional isomers with respect to the point of linkage to the rest of the molecule. Thus, for some illustrative non-restricting examples, the term pyridinyl includes pyridin-2-yl, pyridin-3-yl and pyridin-4-yl; or the term thienyl includes thien-2-yl and thien-3-yl.

The term "$C_1$-$C_6$", as used throughout this text, e.g. in the context of the definition of "$C_1$-$C_6$-alkyl" is to be understood as meaning an alkyl group having a finite number of carbon atoms of 1 to 6, *i.e.* 1, 2, 3, 4, 5 or 6 carbon atoms. It is to be understood further that said term "$C_1$-$C_6$" is to be interpreted as any sub-range comprised therein, e.g. $C_1$-$C_6$ , $C_2$-$C_6$ , $C_3$-$C_6$ , $C_1$-$C_2$ , $C_1$-$C_3$ , particularly $C_1$-$C_2$, $C_1$-$C_3$, $C_1$-$C_4$,

The term "$C_1$-$C_4$", as used throughout this text, e.g. in the context of the definition of "$C_1$-$C_4$-alkyl", "$C_1$-$C_4$-haloalkyl", "$C_1$-$C_4$-alkoxy", or "$C_1$-$C_4$-haloalkoxy" is to be understood as meaning an alkyl group having a finite number of carbon atoms of 1 to 4, *i.e.* 1, 2, 3 or 4 carbon atoms. It is to be understood further that said term "$C_1$-$C_4$" is to be interpreted as any sub-range comprised therein, e.g. $C_1$-$C_4$, $C_2$-$C_4$, $C_3$-$C_4$, $C_1$-$C_2$, $C_1$-C, preferably $C_1$-$C_2$, $C_1$-$C_3$, $C_1$-$C_4$, in the case of "$C_1$-$C_4$-haloalkyl" or "$C_1$-$C_4$-haloalkoxy" even more preferably $C_1$-$C_2$.

Further, as used herein, the term "$C_3$-$C_6$", as used throughout this text, e.g. in the context of the definition of "$C_3$-$C_6$-cycloalkyl", is to be understood as meaning a cycloalkyl group having a finite number of carbon atoms of 3 to 6, *i.e.* 3, 4, 5 or 6 carbon atoms. It is to be understood further that said term "$C_3$-$C_6$" is to be interpreted as any sub-range comprised therein, e.g. $C_3$-$C_6$, $C_4$-$C_5$ , $C_3$-$C_5$ , $C_3$-$C_4$ , $C_4$-$C_6$, $C_5$-$C_6$ ; preferably $C_3$-$C_6$.

The term "substituted" means that one or more hydrogens on the designated atom is replaced with a selection from the indicated group, provided that the substituted atom's normal valency under the existing circumstances is not exceeded, and that the substitution results in a stable compound. Combinations of substituents and/or variables are permissible only if such combinations result in stable compounds.

The term "optionally substituted" means optional substitution with the specified groups, radicals or moieties.

Ring system substituent means a substituent attached to an aromatic or nonaromatic ring system which, for example, replaces an available hydrogen on the ring system.

As used herein, the term "one or more", e.g. in the definition of the substituents of the compounds of the general formulae of the present invention, is understood as meaning "one, two, three, four or five, preferably one, two, three or four, more particularly one, two or three, even more preferably one or two".

The compounds of general formula (I) may exist as isotopic variants. The invention therefore includes one or more isotopic variant(s) of the compounds of general formula (I), particularly deuterium-containing compounds of general formula (I).

The term "Isotopic variant" of a compound or a reagent is defined as a compound exhibiting an unnatural proportion of one or more of the isotopes of the elements that constitute such a compound.

The term "Isotopic variant of the compound of general formula (I)" is defined as a compound of general formula (I) exhibiting an unnatural proportion of one or more of the isotopes of the elements that constitute such a compound.

The expression "unnatural proportion" is to be understood as meaning a proportion of such isotope which is higher than its natural abundance. The natural abundances of isotopes to be applied in this context are described in "Isotopic Compositions of the Elements 1997", Pure Appl. Chem., 70(1), 217-235, 1998.

Examples of such isotopes include stable and radioactive isotopes of hydrogen, carbon, nitrogen, oxygen, phosphorus, sulfur, fluorine, chlorine, bromine and iodine, such as $^{2}H$ (deuterium), $^{3}H$ (tritium), $^{11}C$, $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$ and $^{131}I$, respectively. Accordingly, recitation of "hydrogen" or "H" should be understood to encompass $^{1}H$ (protium), $^{2}H$ (deuterium), and $^{3}H$ (tritium) unless otherwise specified.

With respect to the treatment and/or prophylaxis of the disorders specified herein, isotopic variant(s) of the compounds of general formula (I) preferably contain elevated levels of deuterium ("deuterium-containing compounds of general formula (I)"). Isotopic variants of the compounds of general formula (I) in which one or more radioactive isotopes, such as $^{3}H$ or $^{14}C$, are incorporated are useful e.g. in drug and/or substrate tissue distribution studies. These isotopes are particularly preferred for the ease of their incorporation and detectability. Positron emitting isotopes such as $^{18}F$ or $^{11}C$ may be incorporated into a compound of general formula (I). These isotopic variants of the compounds of general formula (I) are useful for in vivo imaging applications. Deuterium-containing and $^{13}C$-containing compounds of general formula (I) can be used in mass spectrometry analyses (H. J. Leis et al., Curr. Org. Chem., 1998, 2, 131) in the context of preclinical or clinical studies.

Isotopic variants of the compounds of general formula (I) can generally be prepared by methods known to a person skilled in the art, such as those described in the schemes and/or examples herein, e.g., by substituting a reagent

for an isotopic variant of said reagent, preferably for a deuterium-containing reagent. Depending on the desired sites of deuteration, in some cases deuterium from $D_2O$ can be incorporated either directly into the compounds or into reagents that are useful for synthesizing such compounds (Esaki et al., Tetrahedron, 2006, 62, 10954; Esaki et al., Chem. Eur. J., 2007, 13, 4052). Deuterium gas is also a useful reagent for incorporating deuterium into molecules. Catalytic deuteration of olefinic bonds (H. J. Leis et al., Curr. Org. Chem., 1998, 2, 131; J. R. Morandi et al., J. Org. Chem., 1969, 34 (6), 1889) and acetylenic bonds (N. H. Khan, J. Am. Chem. Soc., 1952, 74 (12), 3018; S. Chandrasekhar et al., Tetrahedron, 2011, 52, 3865) is a rapid route for incorporation of deuterium. Metal catalysts (i.e. e.g., Pd, Pt, and Rh) in the presence of deuterium gas can be used to directly exchange deuterium for hydrogen in functional groups containing hydrocarbons (J. G. Atkinson et al., US Patent 3966781). A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA. Further information on the state of the art with respect to deuterium-hydrogen exchange is given for example in Hanzlik et al., J. Org. Chem. 55, 3992-3997, 1990; R. P. Hanzlik et al., Biochem. Biophys. Res. Commun. 160, 844, 1989; P. J. Reider et al., J. Org. Chem. 52, 3326-3334, 1987; M. Jarman et al., Carcinogenesis 16(4), 683-688, 1993; J. Atzrodt et al., Angew. Chem., Int. Ed. 2007, 46, 7744; K. Matoishi et al., J. Chem. Soc, Chem. Commun. 2000, 1519-1520; K. Kassahun et al., WO2012/112363.

The term "deuterium-containing compound of general formula (I)" is defined as a compound of general formula (I), in which one or more protium ($^1H$) atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).

The selective incorporation of one or more deuterium atom(s) into a compound of general formula (I) may alter the physicochemical properties (such as for example acidity [A. Streitwieser et al., J. Am. Chem. Soc., 1963, 85, 2759; C. L. Perrin, et al., J. Am. Chem. Soc., 2007, 129, 4490], basicity [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (D. J. Kushner et al., Can. J. Physiol. Pharmacol., 1999, 77, 79; A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula (I). In some cases deuterium substitution reduces or eliminates the formation of an undesired or toxic metabolite and/or enhances the formation of a desired metabolite (e.g. Nevirapine: A. M. Sharma et al., Chem. Res.Toxicol., 2013, 26, 410; Uetrecht et al., Chemical Research in Toxicology, 2008, 21, 9, 1862; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels, i.e., reduced peak-trough variation. This could result in lower side effects and/or enhanced efficacy, depending on the particular compound's pharmacokinetic/ pharmacodynamic relationship. Indiplon (A. J. Morales et al., Abstract 285, The 15th North American Meeting of the International Society of Xenobiotics, San Diego, CA, October 12-16, 2008), ML-337 (C. J. Wenthur et al., J. Med. Chem., 2013, 56, 5208), and Odanacatib (K. Kassahun et al., WO2012/112363) are examples of this deuterium effect. Still other cases have been reported in which reduced rates of metabolism result in an increase in exposure of the drug without changing the rate of systemic clearance (e.g. Rofecoxib: F. Schneider et al., Arzneim. Forsch. Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.

A compound of general formula (I) may have multiple potential sites of vulnerabillity to metabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium-containing compound(s) of general formula (I) is/are attached to a carbon atom and/or is/are located at those positions of the compound of general formula (I), which are sites of attack for metabolizing enzymes such as e.g. cytochrome $P_{450}$.

Where the plural form of the word compounds, salts, polymorphs, hydrates, solvates and the like, is used herein, this is taken to include also a single compound, salt, polymorph, isomer, hydrate, solvate or the like.

By "stable compound' or "stable structure" is meant a compound that is sufficiently robust to survive isolation to a useful degree of purity from a reaction mixture, and is capable of being subjected to further chemical transformation or, preferably, formulation into an efficacious therapeutic agent.

The compounds of this invention may contain one or more asymmetric centres, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds, (i.e., atropisomers).

Substituents on a non-planar ring may also be present in either cis or trans configurations. It is intended that all such configurations (including enantiomers and diastereomers), are included within the scope of the present invention.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

Separated optical isomers can be obtained by resolution of the racemic mixtures according to conventional processes, for example, by the formation of diastereoisomeric salts using an

optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., chiral HPLC columns), with or without conventional derivatisation, optimally chosen to maximise the separation of the enantiomers. Suitable chiral HPLC columns are manufactured by Daicel, e.g., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials or optically active catalysts.

In order to distinguish different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, e.g. R- or S- isomers, or E- or Z-isomers, in any ratio. Isolation of a single stereoisomer, e.g. a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1H tautomer, or a 2H tautomer, or even a mixture in any amount of the two tautomers, or a triazole moiety for example can exist as a 1H tautomer, a 2H tautomer, or a 4H tautomer, or even a mixture in any amount of said 1H, 2H and 4H tautomers, namely:

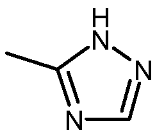

1H-tautomer

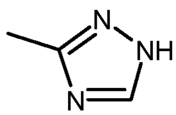

2H-tautomer

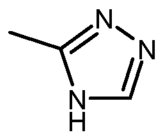

4H-tautomer

Specifically, the compound of formula (I) may exist, at least as the following tautomers:

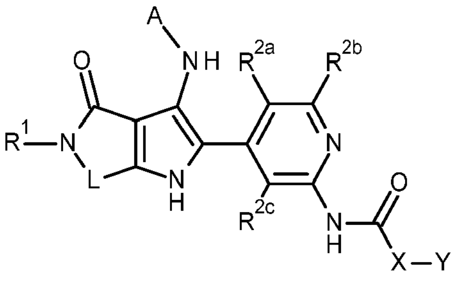

,

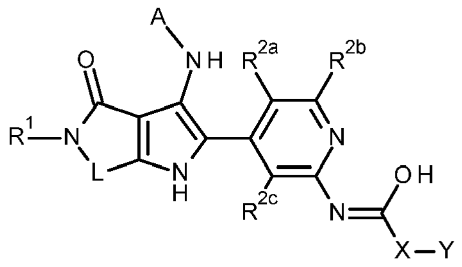

,

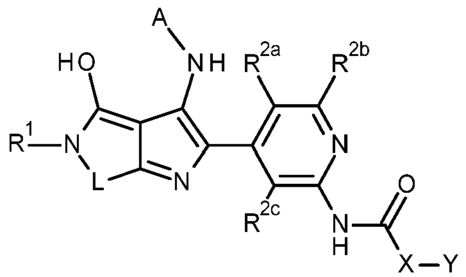

, and

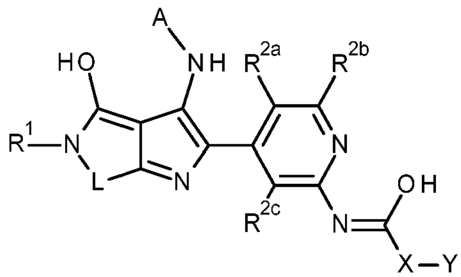

More specifically, when $R^1$ is hydrogen, the compound of formula (I) may exist, at least as the following tautomers:

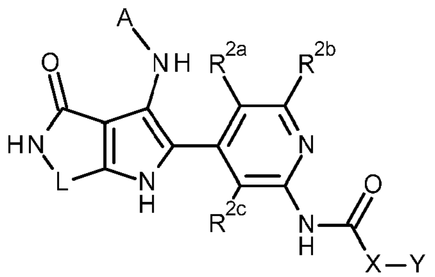

,

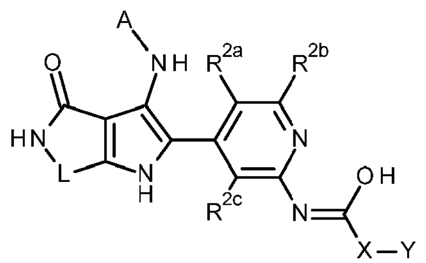

,

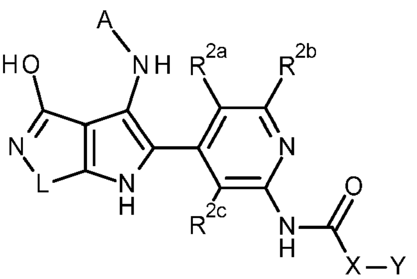

, and

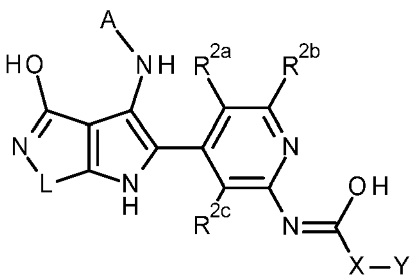

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also relates to useful forms of the compounds as disclosed herein, such as metabolites, hydrates, solvates, prodrugs, salts, in particular pharmaceutically acceptable salts, and co-precipitates.

The compounds of the present invention can exist as a hydrate, or as a solvate, wherein the compounds of the present invention contain polar solvents, preferably water, methanol or ethanol for example as structural element of the crystal lattice of the compounds. The amount of polar solvents, in particular water, may exist in a stoichiometric or non-stoichiometric ratio. In the case of stoichiometric solvates, e.g. a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in free form, e.g. as a free base, or as a free acid,

or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalenedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and stearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention can be prepared by reacting the compounds of the invention with the appropriate base via any of a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae such as "hydrochloride", "trifluoroacetate", "sodium salt", or "x HCl", "x $CF_3COOH$", "x $Na^+$", for example, are to be understood as not a stoichiometric specification, but simply as a salt form.

This applies analogously to cases in which synthesis intermediates or example compounds or salts thereof have been obtained, by the preparation and/or purification processes described, as solvates, such as hydrates with (if defined) unknown stoichiometric composition.

The salts include water-insoluble and, particularly, water-soluble salts.

Furthermore, derivatives of the compounds of formula (I) and the salts thereof which are converted into a compound of formula (I) or a salt thereof in a biological system (bioprecursors or pro-drugs) are covered by the invention. Said biological system is e.g. a mammalian organism, particularly a human subject. The bioprecursor is, for example, converted into the compound of formula (I) or a salt thereof by metabolic processes.

As used herein, the term *"in vivo* hydrolysable ester" is understood as meaning an *in vivo* hydrolysable ester of a compound of the present invention containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, $C_1$-$C_6$ alkoxymethyl esters, *e.g.* methoxymethyl, $C_1$-$C_6$ alkanoyloxymethyl esters, e.g. pivaloyloxymethyl, phthalidyl esters, $C_3$-$C_8$ cycloalkoxy-carbonyloxy-$C_1$-$C_6$ alkyl esters, e.g. 1-cyclohexylcarbonyloxyethyl, 1,3-dioxolen-2-onylmethyl esters, *e.g.* 5-methyl-1,3-dioxolen-2-onylmethyl, and $C_1$-$C_6$-alkoxycarbonyloxyethyl esters, *e.g.* 1-methoxycarbonyloxyethyl, and may be formed at any carboxy group in the compounds of this invention.

An *in vivo* hydrolysable ester of a compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups

for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present invention covers all such esters.

Furthermore, the present invention includes all possible crystalline forms, or polymorphs, of the compounds of the present invention, either as single polymorphs, or as a mixture of more than one polymorphs, in any ratio.

In the context of the properties of the compounds of the present invention the term "pharmacokinetic profile" means one single parameter or a combination thereof including permeability, bioavailability, exposure, and pharmacodynamic parameters such as duration, or magnitude of pharmacological effect, as measured in a suitable experiment. Compounds with improved pharmacokinetic profiles can, for example, be used in lower doses to achieve the same effect, may achieve a longer duration of action, or a may achieve a combination of both effects.

The term "combination" in the present invention is used as known to persons skilled in the art and may be present as a fixed combination, a non-fixed combination or kit-of-parts.

A "fixed combination" in the present invention is used as known to persons skilled in the art and is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A non-fixed combination or "kit-of-parts" in the present invention is used as known to persons skilled in the art and is defined as a combination wherein the said first active ingredient and the said second active ingredient are present in more than one unit. One example of a non-fixed combination or kit-of-parts is a combination wherein the said first active ingredient and the said second active ingredient are present separately. The components of the non-fixed combination or kit-of-parts may be administered separately, sequentially, simultaneously, concurrently or chronologically staggered. Any such combination of a compound of formula (I) of the present invention with an anti-cancer agent as defined below is an embodiment of the invention.

The term "(chemotherapeutic) anti-cancer agents" includes but is not limited to:

131I-chTNT, abarelix, abiraterone, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, aprepitant, arcitumomab, arglabin, arsenic trioxide, asparaginase, atezolizumab, axitinib, azacitidine, basiliximab, belotecan, bendamustine, besilesomab, belinostat, bevacizumab, bexarotene, bicalutamide, bisantrene, bleomycin, blinatumomab, bortezomib, buserelin, bosutinib, brentuximab vedotin, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, eculizumab, edrecolomab, elliptinium acetate, elotuzumab, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (123I), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, necitumumab, nedaplatin, nelarabine, neridronic acid, netupitant/palonosetron, nivolumab, pentetreotide, nilotinib, nilutamide, nimorazole, nimotuzumab, nimustine, nintedanib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, rolapitant, romidepsin, romiplostim, romurtide, rucaparib, samarium (153Sm) lexidronam, sargramostim, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

**General Procedures**

The compounds according to the invention can be prepared according to the following Schemes 1 through 6.

The schemes and procedures described below illustrate synthetic routes to the compounds of general formula (I) of the invention and are not intended to be limiting. It is obvious to the person skilled in the art that the order of transformations as exemplified in the schemes can be modified in various ways. The order of transformations exemplified in the schemes is therefore not intended to be limiting. In addition, interconversion of any of the substituents $R^1$, $R^{2a}$, $R^{2b}$, $R^{2c}$, $R^{17}$, $R^{18}$, $R^{19}$, $R^{20}$, $R^{21}$, A, X, Y, Z, $Z^2$, $Z^3$ and PG can be achieved before and/or after the exemplified transformations. These modifications can be such as the introduction of protecting groups, cleavage of protecting groups, reduction or oxidation of functional groups, dehydrogenation, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art.

Specific examples are described in the subsequent paragraphs.

## Scheme 1

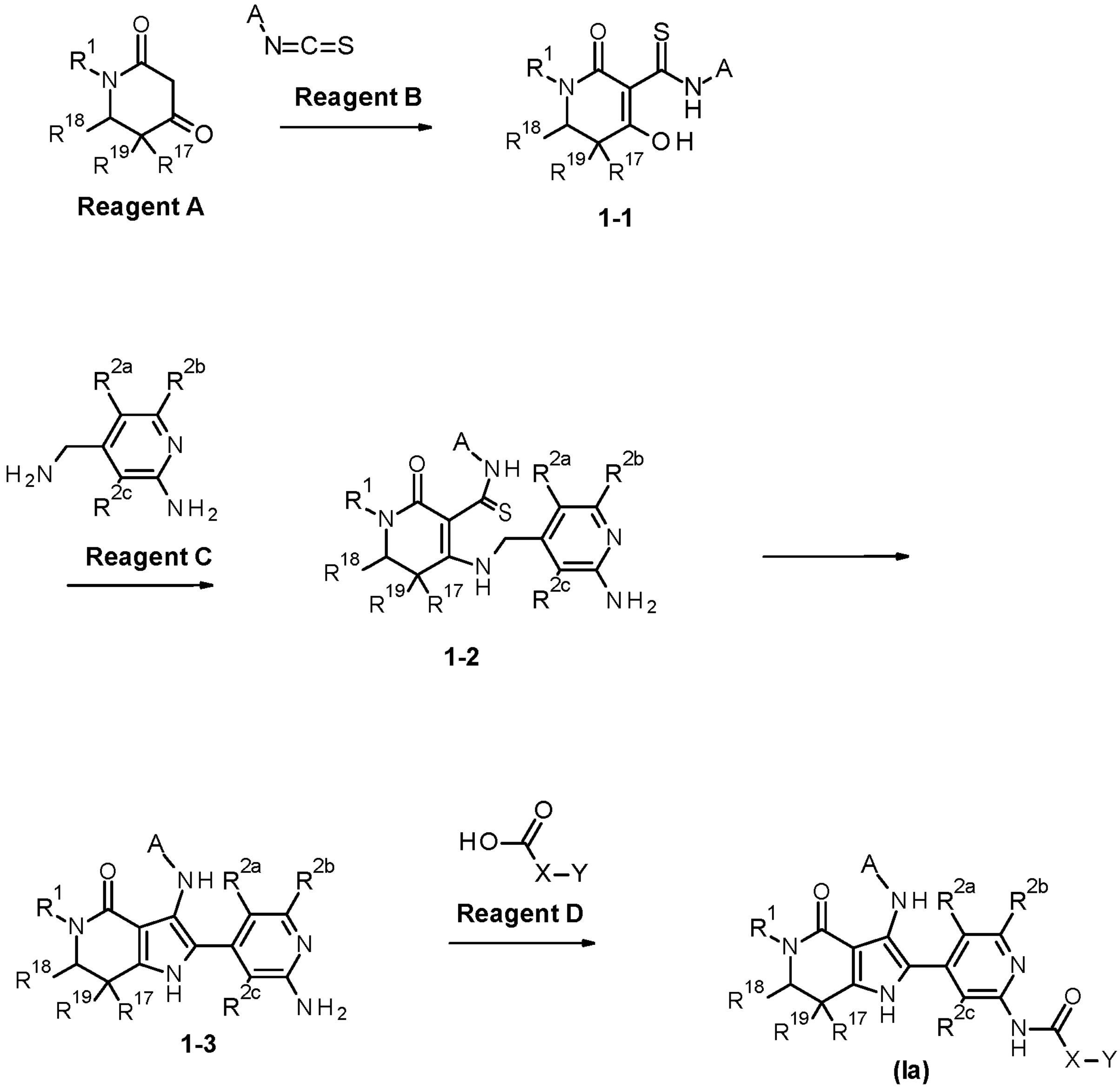


*Scheme* 1: Route for the preparation of compounds of general formula (Ia), wherein $R^1$, $R^{2a}$, $R^{2b}$, $R^{2c}$, $R^{17}$, $R^{18}$, $R^{19}$, A, X and Y have the meaning as given for general formula (I), supra. In addition, interconversion of any of the substituents, $R^1$, $R^{2a}$, $R^{2b}$, $R^{2c}$, $R^{17}$, $R^{18}$, $R^{19}$, A, X and Y can be achieved before and/or after the exemplified transformation.

Reagent A, reagent B, reagent C and reagent D are either commercially available or can be prepared according to procedures available from the public domain, as understandable to the person skilled in the art. Specific examples are described in the subsequent paragraphs.

A suitably substituted piperidine-2,4-dione of general formula (reagent A), such as, for example, N-methylpiperidine-2,4-dione, can be reacted with a suitably substituted isothiocyanate (reagent B), such as, for example, phenylisothiocyanate, in a suitable solvent system, such as, for example, acetonitrile or THF, in the presence of a suitable base, such as, for example, triethylamine or DBU, at temperatures ranging from -78°C to +100°C. Preferably the reaction is carried out at 0°C or +100°C to furnish compounds of general formula 1-1. Similar reactions have been performed in the literature (D. E. Worrall, J. Am. Chem. Soc., 1940, 62, 675).

Intermediates of general formula 1-1 can be converted to Intermediates of general formula 1-2 by reaction with a suitable amine, such as, for example 4-(aminomethyl)pyridin-2-amine, in a suitable solvent system, such as, for example, ethanol and ethyl acetate or N,N-dimethylacetamide, at a temperature between room temperature and the boiling point of the respective solvents, preferably the reaction is carried out at the boiling point of the respective solvents,

whereby the water formed in the reaction can be removed from the reaction by methods known to those skilled in the art, such as, for example, azeotropic removal of water (Dean-Stark conditions) or with molecular sieves, to furnish intermediates of general formula 1-2. Alternatively the reaction can be carried out in a sealed tube or in a microwave oven at a temperature between room temperature and the boiling point of the respective solvents or at a temperature slightly above the boiling point of the respective solvents.

Intermediates of general formula 1-2 are reacted with a base and/or oxidizing reagent, preferably an oxidizing agent, such as, for example hydrogen peroxide, SIBX (stabilized iodoxybenzoic acid or mCPBA, in a suitable solvent system, such as, for example, methanol, in a temperature range from -30°C to the boiling point of the respective solvent which may contain an acid like for example TFA. Preferably the reaction is carried out at the boiling point of the respective solvent, to furnish intermediates of general formula 1-3.

Intermediates of general formula 1-3 can be subjected to a peptide coupling by reaction with a carboxylic acid of formula Y-X-(=O)OH, in which X and Y are as defined for compounds of general formula (I), in the presence of a peptide coupling reagent, selected from HATU (O-(7-azabenzotriazol-1-yl)-N,N,N',N'-tetramethyluronium hexafluorophosphate), TBTU (O-(benzotriazol-1-yl)-N,N,N',N'-tetramethyluronium tetrafluoroborate), PyBOP (benzotriazol-1-yl-oxytripyrrolidinophosphonium hexafluorophosphate), or T3P (2,4,6-tripropyl-1,3,5,2,4,6-trioxatriphosphinane 2,4,6-trioxide), all of them being well known to the person skilled in the art and all of them being commercially available, in the presence of a base such as a tertiary aliphatic amine of the formula $N(C_1\text{-}C_4\text{-alkyl})_3$, or sodium bicarbonate, or potassium carbonate, in an appropriate solvent such as N,N-dimethylformamide, N,N-dimethylacetamide, dimethylsulfoxide, THF, dichloromethane or N-methyl pyrrolidin-2-one in a temperature range from 0°C to the boiling point of the respective solvent to furnish compounds of general formula (I). Specific examples are described in the Experimental Section.

**Scheme 2**

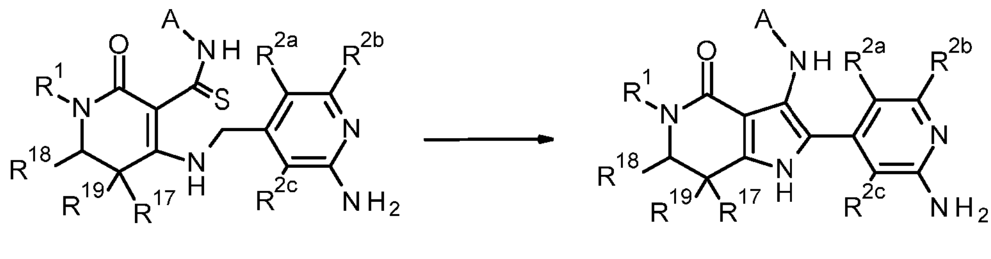

**1-2** **1-3**

*Scheme 2:* Route for the preparation of compounds of general formula 1-3, wherein $R^1$, $R^{2a}$, $R^{2b}$, $R^{2c}$, $R^{17}$, $R^{18}$, $R^{19}$ and A have the meaning as given for general formula (I), supra.

Intermediates of general formula 1-2 are reacted under dehydrogenation conditions, such reactions are known (J. H. Hutchinson, et al., J. Med. Chem., 1996, 39, 4583 - 4591, N. L. Subasinghe, et al., Bioorg. Med. Chem. Lett., 2013, 23, 1063 - 1069, C. F. Jones, et al., Synlett, 2010, 654 - 658, M. Noguchi, et al., Bull. Chem. Soc. Japan, 1986, 59, 1355 - 1362). These conditions can be carried out using, for example, metal catalysis such as, for example, palladium on charcoal, in a suitable solvent system, such as, for example, dimethylacetamide, in a temperature range from 0°C to 200°C of the respective solvent, preferably at elevated temperatures, to furnish compounds of general formula 1-3.

**Scheme 3**

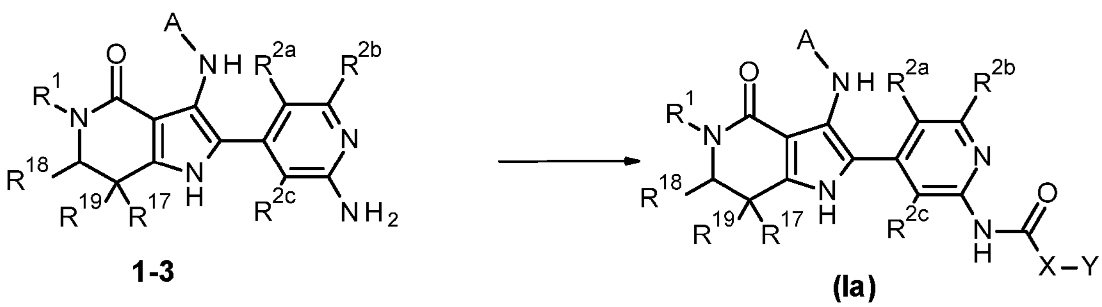

**1-3** **(Ia)**

*Scheme 3:* Route for the preparation of compounds of general formula (I), wherein $R^1$, $R^{2a}$, $R^{2b}$, $R^{2c}$, $R^{17}$, $R^{18}$, $R^{19}$, A, X and Y have the meaning as given for general formula (I), supra. In addition, interconversion of any of the

substituents, $R^1$, $R^{2a}$, $R^{2b}$, $R^{2c}$, $R^{17}$, $R^{18}$, $R^{19}$, A, X and Y can be achieved before and/or after the exemplified transformation.

Intermediates of general formula 1-3 are reacted with an acylating reagent, an acylating agent which can be generated *in situ,* to furnish compounds of general formula (I). These types of reactions are well-known (selected literature examples are: S. Miwatashi, et al., J. Med. Chem., 2005, 48, 5966 - 5979; J. Zhao, et al., Bioorg. Med. Chem. Lett., 2014, 24,. 2802 - 2806; M. P. Hay, et al., J. Med. Chem., 2010, 53, 787 - 797; J. M. Keith, et al., Med. Chem. Lett, 2012, 3, 823 - 827; J. Liang, et al., Eur. J. Med. Chem., 2013, 67, 175 - 187).

Not-limiting examples of these types of reagents are:

i) carboxylic acids with dehydrating reagents typically used in amide bond formation, such as, for example (HBTU, HATU, PyBOP, BOP, T3P, EDC, DIC, DCC)
ii) acid fluorides, acid chlorides or acid bromides, preferably in the presence of a base
iii) acid anhydrides of the general formulae

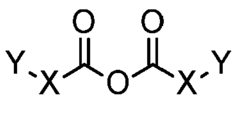

or

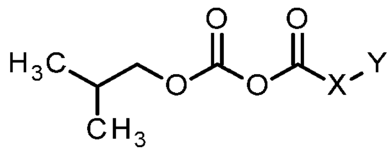

preferably in the presence of a base

Said acylating agent can be in a protected form, containing a protecting group like Boc for example leading to a protected form of compounds of general formula **(I)** which furnishes compounds of general formula **(I)** after an additional deprotection step. Further protecting groups are well known to the person skilled in the art. A detailed description for the use of a large number of proven protective groups is found, for example, in T. W. Greene, Protective Groups in Organic Synthesis, John Wiley & Sons, 1999, 3rd Ed., or in P. Kocienski, Protecting Groups, Thieme Medical Publishers, 2000.

## Scheme 4

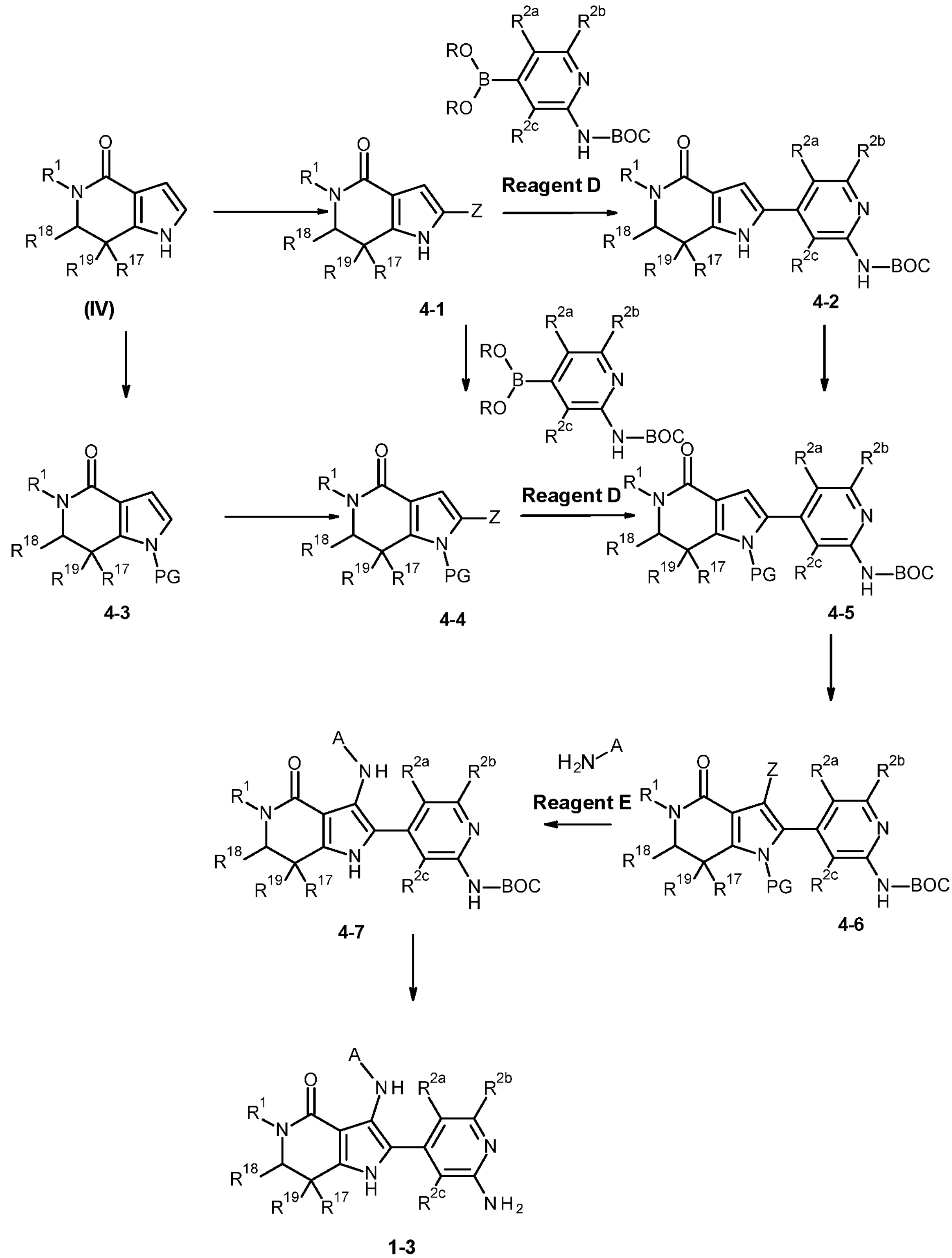


*Scheme 4:* Route for the preparation of compounds of general formula **(I),** wherein $R^1$, $R^{2a}$, $R^{2b}$, $R^{2c}$, $R^{17}$, $R^{18}$, $R^{19}$ and A have the meaning as given for general formula (I), supra. BOC represents a Boc-protecting group. PG is a suitable protecting group like for example SEM, Tosyl, paramethoxy-benzyl or Boc. In addition, interconversion of any of the substituents, $R^1$, $R^{2a}$, $R^{2b}$, $R^{2c}$, $R^{17}$, $R^{18}$, $R^{19}$ and A can be achieved before and/or after the exemplified transformation. The R in reagent D can be hydrogen to represent boronic acids or alkyl groups to represent boronic esters, optionally both R groups can be attached to each other to represent, for example, pincacol boronic esters. The substituent Z in the intermediates of general formulae **4-1, 4-4** and **4-6** can be a suitable leaving group, such as, for example, Cl, Br, I, aryl sulfonates such as for example *p*-toluene sulfonate, or alkyl sulfonates such as for example methane sulfonate or trifluoromethane sulfonate.

These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wutts in Protective Groups in Organic Synthesis, 3rd edition, Wiley 1999). Specific examples are described in the subsequent paragraphs.

Intermediates of general formula **(IV)** are commercially available or are reported in the public domain, see the teachings of, for example, Menichincheri et al., WO2014/72220 A1; Clark et al., J. Heterocyclic Chem., 1993, 30, 829-831; Clark et al., J. Med. Chem., 1993, 36, 2645-2657; Schneller et al., J. Med. Chem., 1978, 21, 990 - 993.

Intermediates of general formula **(IV)** or intermediates of the formula **4-3** can be reacted to introduce a substituent Z, which is preferably a halide, such reactions are known to those skilled in the art (see Menichincheri et al., WO2014/72220 A1 (introduction of bromide and iodide); Smith et al., Bioorg. Med. Chem. Lett., 2007, 17, 673 - 678 (introduction of bromide) Cee et al., WO2014/22752 A1 (introduction of bromide)) to furnish intermediates of the formula **4-1** from formula **(IV)** or intermediates of the formula **4-4** from formula **4-3.**

Intermediates of general formula (IV), of formula **4-1** or of formula **4-2** can be reacted to introduce the PG group, such as, for example, a Tosyl-group or a SEM-group via alkylation under basic conditions (Marchionni et al., WO2009/40399 A1, also see for example T.W. Greene and P.G.M. Wutts in Protective Groups in Organic Synthesis, 3rd edition, Wiley 1999) or a tert-butoxycarbonyl (Boc) group (Kim et al., WO2013/62344 A1; Voss et al., WO2015/22073 A1) to furnish intermediates of the formula **4-3** from general formula (IV) or intermediates of the formula **4-4** from formula **4-1** or intermediates of the formula **4-5** from formula **4-2.**

Intermediates of general formula **4-1** or intermediates of the formula **4-4** can be reacted with reagent D to introduce the substituted pyridinyl substituent, using metal-catalyzed reactions, such as, for example, the Suzuki reaction. Such reactions are known to those skilled in the art (WO2007/39740 A2; Cee et al., WO2014/22752 A1; Smith et al., Bioorg. Med. Chem. Lett., 2007, 17, 673 - 678) and can be used to furnish intermediates of the formula **4-2** from general formula **4-1** or intermediates of the formula **4-5** from formula **4-4.**

Intermediates of general formula **4-5** can be reacted with a suitable halogenating reagent, such as, for example, copper(I) bromide and *N*-bromosuccinimide, preferably *N*-bromosuccinimide, in a suitable solvent system, such as, for example, acetonitrile, in a temperature range from 0°C to the boiling point of the respective solvent, preferably the reaction is carried out at room temperature, to furnish general formula 4-6. Similar examples for the bromination of pyrroles have been previously published using lactams (Aiello, E. et al., J. Heterocyclic Chem., 1982, 19, 977 - 979; Duranti, A. et al., Bioorg. Med. Chem., 2003, 11, 3965 - 3973).

Intermediates of general formula 4-6 can be reacted with a suitable primary amines, such as, for example, primary aromatic amines and primary amines, preferably primary aromatic amines, such as, for example aniline or 3-aminothiophene, in the presence of a base, such as, for example, lithium bis(trimethylsilyl)amide (LHMDS), in the presence of a catalyst, such as, for example a suitable ligand, preferably 2-(di-tert-butylphosphino)-2',4',6'-triisopropyl-3,6-dimethoxy-1,1'-biphenyl (tBuBrettPhos) and in the presence of a pre-catalyst, such as, for example a palladium pre-catalyst, preferably chloro[2-(dicyclohexylphosphino)-3,6-dimethoxy-2',4',6'-triisopropyl-1,1'-biphenyl][2-(2-aminoethyl)phenyl]palladium(II) (BrettPhos-PreCat MTBE ether adduct) in a suitable solvent system, such as, for example, tetrahydrofuran (THF), in a temperature range from 0°C to the 200°C, preferably the reaction is carried out at 80°C, to furnish compounds of general formula **4-7.**

Intermediates of general formula **4-7** can be subjected to reaction conditions for the removal of protecting groups to furnish intermediates of general formula **1-3,** such as for example acidic conditions for the removal of a Boc group and TBAF for the removal of a SEM group. A comprehensive overview of conditions for possible protecting groups is outlined for example in T.W. Greene and P.G.M. Wutts in Protective Groups in Organic Synthesis, 3rd edition, Wiley 1999). In general, the PG group can be removed first or the Boc group can be removed first or both protecting groups could be selected in a way that they can be removed simultaneously.

## Scheme 5

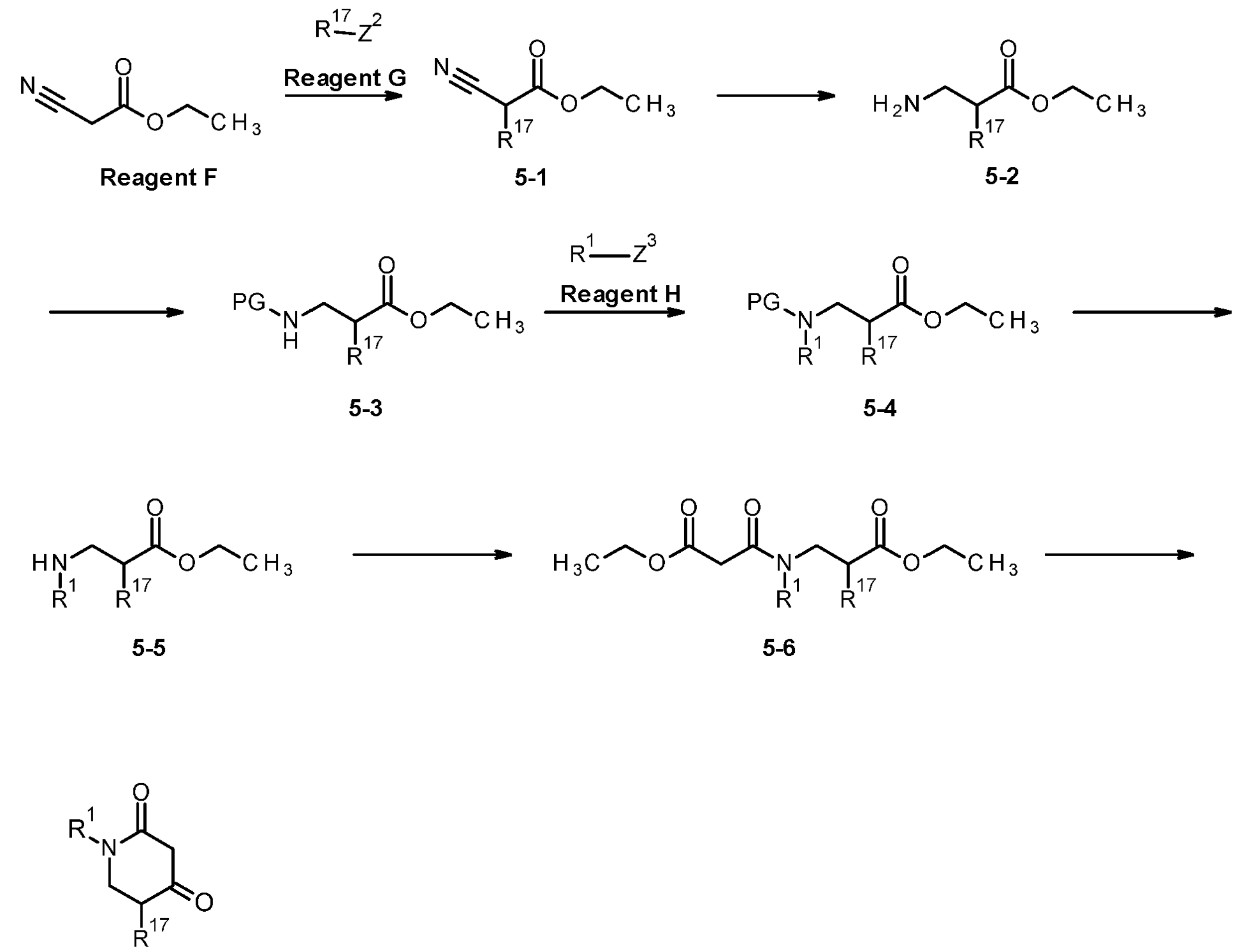


Reagent F

5-1

5-2

5-3

5-4

5-5

5-6

Reagent A-a

*Scheme 5:* Route for the preparation of reagent of general formula (A-a), wherein $R^1$ and $R^{17}$ have the meaning as given for general formula (I), supra. In addition, interconversion of any of the substituents, $R^1$ and $R^{17}$ can be achieved before and/or after the exemplified transformation. Reagent F, G, and reagent H are either commercially available or can be prepared according to procedures available from the public domain, as understandable to the person skilled in the art. Specific examples are described in the subsequent paragraphs.

Ethyl cyanoacetate (Reagent F) can be reacted with a suitably substituted reagent GI, such as, for example, 2,2,2-trifluoroethyl trifluoromethanesulfonate, in the presence of a strong base, such as, for example, lithium bis(trimethylsilyl)amide, in a suitable solvent system, such as, for example, THF, at temperatures ranging from -70 °C to rt. Preferably the reaction is carried out at -70°C to furnish intermediate of general formula **5-1.**

An intermediate of general formula **5-1** can be reduced by hydrogenation in the presence of a catalyst, such as, for example platinum(IV) oxide, in a suitable solvent system, such as, for example, methanol, at temperatures ranging from 0°C to rt. Preferably the reaction is carried out at rt to furnish compounds of general formula **5-2.**

Intermediates of general formula **5-2** can be reacted to introduce the PG group, such as, for example, a Tosyl-group or a SEM-group via alkylation under basic conditions (Marchionni et al., WO2009/40399 A1, also see for example T.W. Greene and P.G.M. Wutts in Protective Groups in Organic Synthesis, 3rd edition, Wiley 1999) or a tert-butoxycarbonyl (Boc) group (Kim et al., WO2013/62344 A1; Voss et al., WO2015/22073 A1) or a Benzyloxycarbonyl (Z) group to furnish intermediates of the formula **5-3.**

An intermediate of general formula **5-3** can be alkylated with reagent K, such as, for example iodomethane, in the presence of a base, such as, for example, sodium hydride in a suitable solvent system, such as, for example, THF, at temperatures ranging from 0°C to rt. Preferably the reaction is carried out at 0 °C to furnish compounds of general formula **5-4.**

Intermediates of general formula **5-4** can be subjected to reaction conditions for the removal of protecting

groups to furnish intermediates of general formula **5-5,** such as for example acidic conditions for the removal of a Boc group and TBAF for the removal of a SEM group and hydrogynation for the removal of Z group. A comprehensive overview of conditions for possible protecting groups is outlined for example in T.W. Greene and P.G.M. Wutts in Protective Groups in Organic Synthesis, 3rd edition, Wiley 1999).

An intermediate of general formula **5-5** can build an amide bond by the addition of ethyl 3-chloro-3-oxopropanoate, in the presence of a base, such as, for example, N,N-Diisopropylethylamine and 4-dimethylaminopyridine, in a suitable solvent system, such as, for example, dichloromethane, at temperatures ranging from 0°C to rt. Preferably the reaction is carried out below 15 °C to furnish compounds of general formula **5-6.**

An intermediate of general formula **5-6** can cyclize and decarboxylate by the addition of a base, such as, for example, sodium ethoxide in a suitable solvent system, such as, for example, ethanol, at temperatures ranging from 0°C to rt. Preferably the reaction is carried out at 0°C and after workup dissolved in a sutiable solvent system, such as, for example acetonitrile and water, at temperatures ranging from rt to reflux. Preferably the reaction is carried out at reflux to furnish reagent of general formula **A-a.**

## Scheme 6

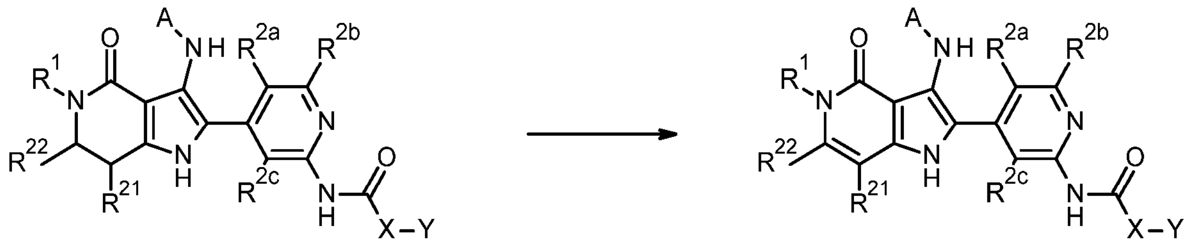

**6-1** **(Ib)**

*Scheme 6:* Route for the preparation of compounds of general formula (Ib), wherein $R^1$, $R^{2a}$, $R^{2b}$, $R^{2c}$, $R^{21}$, $R^{22}$, A, X and Y have the meaning as given for general formula (I), supra. In addition, interconversion of any of the substituents, $R^1$, $R^{2a}$, $R^{2b}$, $R^{2c}$, $R^{21}$, $R^{22}$, A, X and Y can be achieved before and/or after the exemplified transformation.

Intermediates of general formula 6-1 can be oxidized to general fomula (Ib) by reaction with an oxidizing agent, such as, for example, 2,3-dichloro-5,6-dicyano-1,4-benzochinone, in a suitable solvent system, such as, for example, 1,4-dioxane, at temperatures ranging from rt to +80°C. Preferably the reaction is carried out at 80°C to furnish compounds of general formula **(1b).** Similar reactions have been performed in the literature (Hart et al.,WO2016/100166).

It is known to the person skilled in the art that, if there are a number of reactive centers on a starting or intermediate compound, it may be necessary to block one or more reactive centers temporarily by protective groups in order to allow a reaction to proceed specifically at the desired reaction center. A detailed description for the use of a large number of proven protective groups is found, for example, in T. W. Greene, Protective Groups in Organic Synthesis, John Wiley & Sons, 1999, 3rd Ed., or in P. Kocienski, Protecting Groups, Thieme Medical Publishers, 2000.

The compounds according to the invention are isolated and purified in a manner known per se, e.g. by distilling off the solvent *in vacuo* and recrystallizing the residue obtained from a suitable solvent or subjecting it to one of the customary purification methods, such as chromatography on a suitable support material. Furthermore, reverse phase preparative HPLC may be applied. The compounds of the present invention which possess a sufficiently basic or acidic functionality, may result as a salt, such as, in the case of a compound of the present invention which is sufficiently basic, a trifluoroacetate or formate salt for example, or, in the case of a compound of the present invention which is sufficiently acidic, an ammonium salt for example. Salts of this type can either be transformed into its free base or free acid form, respectively, by various methods known to the person skilled in the art, or be used as salts in subsequent biological assays. Additionally, the drying process during the isolation of the compounds of the present invention may not fully remove traces of cosolvents, especially such as formic acid or trifluoroacetic acid, to give solvates or inclusion complexes. The person skilled in the art will recognise which solvates or inclusion complexes are acceptable to be used in subsequent biological assays. It is to be understood that the specific form (e.g. salt, free base, free acid, solvate, inclusion complex) of a compound of the present invention as isolated and described herein is not necessarily the only form in which said compound can be applied to a biological assay in order to quantify the specific biological activity.

Salts of the compounds of formula (I) according to the invention can be obtained by dissolving the free compound in a suitable solvent (for example a ketone such as acetone, methylethylketone or methylisobutylketone, an ether such as diethyl ether, tetrahydrofuran or dioxane, a chlorinated hydrocarbon such as methylene chloride or chloroform, or a low molecular weight aliphatic alcohol such as methanol, ethanol or isopropanol) which contains the desired acid or base, or to which the desired acid or base is then added. The acid or base can be employed in salt preparation, depending

on whether a mono- or polybasic acid or base is concerned and depending on which salt is desired, in an equimolar ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art. Especially preferred are hydrochlorides and the process used in the example section.

Pure diastereomers and pure enantiomers of the compounds and salts according to the invention can be obtained e.g. by asymmetric synthesis, by using chiral starting compounds or optically active catalysts in synthesis or by separating enantiomeric and diasteriomeric mixtures obtained in synthesis.

Enantiomeric and diastereomeric mixtures can be separated into the pure enantiomers and pure diastereomers by methods known to the person skilled in the art. Preferably, diastereomeric mixtures are separated by crystallization, in particular fractional crystallization, or chromatography. Enantiomeric mixtures can be separated e.g. by forming diastereomers with a chiral auxiliary agent, resolving the diastereomers obtained and removing the chiral auxiliary agent. As chiral auxiliary agents, for example, chiral acids can be used to separate enantiomeric bases such as e.g. mandelic acid and chiral bases can be used to separate enantiomeric acids by formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be separated using chiral separating columns in chromatography. Another suitable method for the isolation of enantiomers is the enzymatic separation.

One preferred aspect of the invention is the process for the preparation of the compounds of claims 1 to 5 according to the examples as well as the intermediates used for their preparation.

The intermediates used for the synthesis of the compounds of claims of formula (I) as described herein, as well as their use for the synthesis of the compounds of formula (I), are one further aspect of the present invention. Preferred intermediates are the Intermediate Examples as disclosed herein.

Optionally, compounds of the formula (I) can be converted into their salts, or, optionally, salts of the compounds of the formula (I) can be converted into the free compounds. Corresponding processes are customary for the skilled person.

**Commercial utility**

As mentioned supra, the compounds of the present invention have surprisingly been found to effectively inhibit Casein kinase 1 alpha and/or delta finally resulting in cell death e.g. apoptosis and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses. In particular, the disclosed compounds can be used for the treatment or prophylaxis of diseases in which the uncontrolled cell growth, proliferation and/or survival, inappropriate cellular immune responses, or inappropriate cellular inflammatory responses are mediated by Casein kinase 1 alpha and/or delta, such as, for example, benign and malignant neoplasia, more specifically haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof, especially haematological tumours, solid tumours, and/or metastases of breast, bladder, bone, brain, central and peripheral nervous system, cervix, colon, endocrine glands (e.g. thyroid and adrenal cortex), endocrine tumours, endometrium, esophagus, gastrointestinal tumours, germ cells, kidney, liver, lung, larynx and hypopharynx, mesothelioma, ovary, pancreas, prostate, rectum, renal, small intestine, soft tissue, stomach, skin, testis, ureter, vagina and vulva as well as malignant neoplasias including primary tumors in said organs and corresponding secondary tumors in distant organs ("tumor metastases"). Haematological tumors can e.g be exemplified by aggressive and indolent forms of leukemia and lymphoma, namely non-Hodgkins lymphoma, chronic and acute myeloid leukemia (CML / AML), acute lymphoblastic leukemia (ALL), Hodgkins lymphoma, multiple myeloma and T-cell lymphoma. Also included are myelodysplastic syndrome, plasma cell neoplasia, paraneoplastic syndromes, and cancers of unknown primary site as well as AIDS related malignancies.

The term "inappropriate" within the context of the present invention, in particular in the context of "inappropriate cellular immune responses, or inappropriate cellular inflammatory responses", as used herein, is to be understood as preferably meaning a response which is associated with, responsible for, or results in, the pathology of said diseases.

Preferably, the use is in the treatment or prophylaxis of diseases, especially the treatment, wherein the diseases

are haematological tumours, solid tumours and/or metastases thereof. Another aspect is the use of a compound of formula (I) for the prophylaxis and/or treatment of cervical tumours, lung tumours (such as lung carcinoma), colon tumours (such as colorectal carcinoma), or lymphoma (such as diffuse large B-cell lymphoma) and/or metastases thereof, especially preferred for the treatment thereof.

Another aspect of the present invention is the use of a compound of formula (I) or a stereoisomer, a tautomer, an N-oxide, a hydrate, a solvate, or a salt thereof, particularly a pharmaceutically acceptable salt thereof, or a mixture of same, as described herein, in the manufacture of a medicament for the treatment or prophylaxis of a disease, wherein such disease is a hyperproliferative disorder or a disorder responsive to induction of cell death e.g. apoptosis. In an embodiment the disease is a haematological tumour, a solid tumour and/or metastases thereof. In another embodiment the disease is is a cervical tumour, a lung tumour (such as lung carcinoma), a colon tumour (such as colorectal carcinoma), or a lymphoma (such as diffuse large B-cell lymphoma and/or metastases thereof.

Methods of treating hyper-proliferative disorders

The present invention relates to a method for using the compounds of the present invention and compositions thereof, to treat mammalian hyper-proliferative disorders. Compounds can be utilized to inhibit, block, reduce, decrease, etc., cell proliferation and/or cell division, and/or produce cell death e.g. apoptosis. This method comprises administering to a mammal in need thereof, including a human, an amount of a compound of this invention, or a pharmaceutically acceptable salt, isomer, polymorph, metabolite, hydrate, solvate or ester thereof; etc. which is effective to treat the disorder. Hyper-proliferative disorders include but are not limited, e.g., psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypothalamic glioma, cerebellar and cerebral astrocytoma, medulloblastoma, ependymoma, as well as neuroectodermal and pineal tumour.

Tumours of the male reproductive organs include, but are not limited to prostate and testicular cancer. Tumours of the female reproductive organs include, but are not limited to endometrial, cervical, ovarian, vaginal, and vulvar cancer, as well as sarcoma of the uterus.

Tumours of the digestive tract include, but are not limited to anal, colon, colorectal, oesophageal, gallbladder, gastric, pancreatic, rectal, small-intestine, and salivary gland cancers.

Tumours of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi’s sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell. Lymphomas include, but are not limited to AIDS-related lymphoma, non-Hodgkin’s lymphoma, cutaneous T-cell lymphoma, Burkitt lymphoma, Hodgkin’s disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, e.g., the management or care of a subject for the purpose of combating, alleviating, reducing, relieving, improving the condition of, *etc.,* of a disease or disorder, such as cancer.

Methods of treating kinase disorders

The present invention also provides methods for the treatment of disorders associated with aberrant mitogen extracellular kinase activity, including, but not limited to stroke, heart failure, hepatomegaly, cardiomegaly, diabetes, Alzheimer's disease, cystic fibrosis, symptoms of xenograft rejections, septic shock or asthma.
Effective amounts of compounds of the present invention can be used to treat such disorders, including those diseases (*e.g.*, cancer) mentioned in the Background section above. Nonetheless, such cancers and other diseases can be treated with compounds of the present invention, regardless of the mechanism of action and/or the relationship between the kinase and the disorder.
The phrase "aberrant kinase activity" or "aberrant tyrosine kinase activity," includes any abnormal expression or activity of the gene encoding the kinase or of the polypeptide it encodes. Examples of such aberrant activity, include, but are not limited to, over-expression of the gene or polypeptide; gene amplification; mutations which produce constitutively-active or hyperactive kinase activity; gene mutations, deletions, substitutions, additions, etc.
The present invention also provides for methods of inhibiting a kinase activity, especially of mitogen extracellular kinase, comprising administering an effective amount of a compound of the present invention, including salts, polymorphs, metabolites, hydrates, solvates, prodrugs (*e.g.*: esters) thereof, and diastereoisomeric forms thereof. Kinase activity can be inhibited in cells (*e.g., in vitro*), or in the cells of a mammalian subject, especially a human patient in need of treatment.

Methods of treating angiogenic disorders

The present invention also provides methods of treating disorders and diseases associated with excessive and/or abnormal angiogenesis.
Inappropriate and ectopic expression of angiogenesis can be deleterious to an organism. A number of pathological conditions are associated with the growth of extraneous blood vessels. These include, e.g., diabetic retinopathy, ischemic retinal-vein occlusion, and retinopathy of prematurity [Aiello et al. New Engl. J. Med. 1994, 331, 1480 ; Peer et al. Lab. Invest. 1995, 72, 638], age-related macular degeneration [AMD ; see, Lopez et al. Invest. Opththalmol. Vis. Sci. 1996, 37, 855], neovascular glaucoma, psoriasis, retrolental fibroplasias, angiofibroma, inflammation, rheumatoid arthritis (RA), restenosis, in-stent restenosis, vascular graft restenosis, etc. In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, compounds of the present invention can be utilized to treat and/or prevent any of the aforementioned angiogenesis disorders, e.g., by inhibiting and/or reducing blood vessel formation; by inhibiting, blocking, reducing, decreasing, etc. endothelial cell proliferation or other types involved in angiogenesis, as well as causing cell death, e.g., apoptosis, of such cell types.
Preferably, the diseases of said method are haematological tumours, solid tumour and/or metastases thereof.
The compounds of the present invention can be used in particular in therapy and prevention, e.g., prophylaxis, especially in therapy of tumour growth and metastases, especially in solid tumours of all indications and stages with or without pre-treatment of the tumour growth.

**Pharmaceutical compositions of the compounds of the invention**

This invention also relates to pharmaceutical compositions containing one or more compounds of the present invention. These compositions can be utilised to achieve the desired pharmacological effect by administration to a patient in need thereof. A patient, for the purpose of this invention, is a mammal, including a human, in need of treatment for the particular condition or disease.
Therefore, the present invention includes pharmaceutical compositions that are comprised of a pharmaceutically acceptable carrier or auxiliary and a pharmaceutically effective amount of a compound, or salt thereof, of the present invention.
Another aspect of the invention is a pharmaceutical composition comprising a pharmaceutically effective amount of a compound of formula (I) and a pharmaceutically acceptable auxiliary for the treatment of a disease mentioned *supra,* especially for the treatment of haematological tumours, solid tumours and/or metastases thereof.
A pharmaceutically acceptable carrier or auxiliary is preferably a carrier that is non-toxic and innocuous to a patient at concentrations consistent with effective activity of the active ingredient so that any side effects ascribable to the carrier do not vitiate the beneficial effects of the active ingredient. Carriers and auxiliaries are all kinds of additives assisting to the composition to be suitable for administration.
A pharmaceutically effective amount of compound is preferably that amount which produces a result or exerts the intended influence on the particular condition being treated.
The compounds of the present invention can be administered with pharmaceutically-acceptable carriers or

auxiliaries well known in the art using any effective conventional dosage unit forms, including immediate, slow and timed release preparations, orally, parenterally, topically, nasally, ophthalmically, optically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatine type containing auxiliaries, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatine, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, colouring agents, and flavouring agents such as peppermint, oil of wintergreen, or cherry flavouring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavouring and colouring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavouring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more colouring agents; one or more flavouring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavouring and colouring agents.

The compounds of this invention may also be administered parenterally, that is, subcutaneously, intravenously, intraocularly, intrasynovially, intramuscularly, or interperitoneally, as injectable dosages of the compound in preferably a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates ; anionic detergents, for example, alkyl, aryl, and olefin sulfonates, alkyl, olefin, ether, and monoglyceride sulfates, and sulfosuccinates ; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of

the active ingredient in solution. Preservatives and buffers may also be used advantageously. In order to minimise or eliminate irritation at the site of injection, such compositions may contain a non-ionic surfactant having a hydrophile-lipophile balance (HLB) preferably of from about 12 to about 17. The quantity of surfactant in such formulation preferably ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia ; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Controlled release formulations for parenteral administration include liposomal, polymeric microsphere and polymeric gel formulations that are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for administration, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized.

Such ingredients and procedures include those described in the following references, each of which is incorporated herein by reference: Powell, M.F. et al., "Compendium of Excipients for Parenteral Formulations" PDA Journal of Pharmaceutical Science & Technology 1998, 52(5), 238-311 ; Strickley, R.G "Parenteral Formulations of Small Molecule Therapeutics Marketed in the United States (1999)-Part-1" PDA Journal of Pharmaceutical Science & Technology 1999, 53(6), 324-349 ; and Nema, S. et al., "Excipients and Their Use in Injectable Products" PDA Journal of Pharmaceutical Science & Technology 1997, 51(4), 166-171.

Commonly used pharmaceutical ingredients that can be used as appropriate to formulate the composition for its intended route of administration include:

acidifying agents (examples include, but are not limited to acetic acid, citric acid, fumaric acid, hydrochloric acid, nitric acid);

alkalinizing agents (examples include, but are not limited to ammonia solution, ammonium carbonate, diethanolamine, monoethanolamine, potassium hydroxide, sodium borate, sodium carbonate, sodium hydroxide, triethanolamine, trolamine);

adsorbents (examples include, but are not limited to powdered cellulose and activated charcoal);

aerosol propellants (examples include, but are not limited to carbon dioxide, $CCl_2F_2$, $F_2ClC\text{-}CClF_2$ and $CClF_3$);

air displacement agents - examples include, but are not limited to nitrogen and argon;

antifungal preservatives (examples include, but are not limited to benzoic acid, butylparaben, ethylparaben, methylparaben, propylparaben, sodium benzoate);

antimicrobial preservatives (examples include, but are not limited to benzalkonium chloride, benzethonium chloride, benzyl alcohol, cetylpyridinium chloride, chlorobutanol, phenol, phenylethyl alcohol, phenylmercuric nitrate and thimerosal);

antioxidants (examples include, but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);

binding materials (examples include, but are not limited to block polymers, natural and synthetic rubber, polyacrylates, polyurethanes, silicones, polysiloxanes and styrene-butadiene copolymers);

buffering agents (examples include, but are not limited to potassium metaphosphate, dipotassium phosphate, sodium acetate, sodium citrate anhydrous and sodium citrate dihydrate);

carrying agents (examples include, but are not limited to acacia syrup, aromatic syrup, aromatic elixir, cherry syrup, cocoa syrup, orange syrup, corn oil, mineral oil, peanut oil, sesame oil, bacteriostatic sodium chloride injection and bacteriostatic water for injection);

chelating agents (examples include, but are not limited to edetate disodium and edetic acid);

colourants (examples include, but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);

clarifying agents (examples include, but are not limited to bentonite);

emulsifying agents (examples include, but are not limited to acacia, cetomacrogol, cetyl alcohol, glyceryl monostearate, lecithin, sorbitan monooleate, polyoxyethylene 50 monostearate);

encapsulating agents (examples, include but are not limited to gelatin and cellulose acetate phthalate);

flavourants (examples include, but are not limited to anise oil, cinnamon oil, cocoa, menthol, orange oil, peppermint oil and vanillin);

humectants (examples include, but are not limited to glycerol, propylene glycol and sorbitol);

levigating agents (examples include, but are not limited to mineral oil and glycerin);

oils (examples include, but are not limited to arachis oil, mineral oil, olive oil, peanut oil, sesame oil and vegetable oil);

ointment bases (examples include, but are not limited to lanolin, hydrophilic ointment, polyethylene glycol ointment, petrolatum, hydrophilic petrolatum, white ointment, yellow ointment, and rose water ointment);

penetration enhancers (transdermal delivery) (examples include, but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas);

plasticizers (examples include, but are not limited to diethyl phthalate and glycerol);

solvents (examples include, but are not limited to ethanol, corn oil, cottonseed oil, glycerol, isopropanol, mineral oil, oleic acid, peanut oil, purified water, water for injection, sterile water for injection and sterile water for irrigation);

stiffening agents (examples include, but are not limited to cetyl alcohol, cetyl esters wax, microcrystalline wax, paraffin, stearyl alcohol, white wax and yellow wax);

suppository bases (examples include, but are not limited to cocoa butter and polyethylene glycols (mixtures));

surfactants (examples include, but are not limited to benzalkonium chloride, nonoxynol 10, oxtoxynol 9, polysorbate 80, sodium lauryl sulfate and sorbitan mono-palmitate);

suspending agents (examples include, but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);

sweetening agents (examples include, but are not limited to aspartame, dextrose, glycerol, mannitol, propylene glycol, saccharin sodium, sorbitol and sucrose);

tablet anti-adherents (examples include, but are not limited to magnesium stearate and talc);

tablet binders (examples include, but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);

tablet and capsule diluents (examples include, but are not limited to dibasic calcium phosphate, kaolin, lactose, mannitol, microcrystalline cellulose, powdered cellulose, precipitated calcium carbonate, sodium carbonate, sodium phosphate, sorbitol and starch);

tablet coating agents (examples include, but are not limited to liquid glucose, hydroxyethyl cellulose, hydroxypropyl cellulose, hydroxypropyl methylcellulose, methylcellulose, ethylcellulose, cellulose acetate phthalate and shellac);

tablet direct compression excipients (examples include, but are not limited to dibasic calcium phosphate);

tablet disintegrants (examples include, but are not limited to alginic acid, carboxymethylcellulose calcium, microcrystalline cellulose, polacrillin potassium, cross-linked polyvinylpyrrolidone, sodium alginate, sodium starch glycollate and starch);

tablet glidants (examples include, but are not limited to colloidal silica, corn starch and talc);

tablet lubricants (examples include, but are not limited to calcium stearate, magnesium stearate, mineral oil, stearic acid and zinc stearate);

tablet/capsule opaquants (examples include, but are not limited to titanium dioxide);

tablet polishing agents (examples include, but are not limited to carnuba wax and white wax);

thickening agents (examples include, but are not limited to beeswax, cetyl alcohol and paraffin);

tonicity agents (examples include, but are not limited to dextrose and sodium chloride);

viscosity increasing agents (examples include, but are not limited to alginic acid, bentonite, carbomers, carboxymethylcellulose sodium, methylcellulose, polyvinyl pyrrolidone, sodium alginate and tragacanth); and

wetting agents (examples include, but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:

Sterile i.v. solution: A 5 mg/mL solution of the desired compound of this invention can be made using sterile, injectable water, and the pH is adjusted if necessary. The solution is diluted for administration to 1 - 2 mg/mL with sterile 5% dextrose and is administered as an i.v. infusion over about 60 minutes.

Lyophilised powder for i.v. administration: A sterile preparation can be prepared with (i) 100 - 1000 mg of the desired compound of this invention as a lyophilised powder, (ii) 32- 327 mg/mL sodium citrate, and (iii) 300 - 3000 mg

Dextran 40. The formulation is reconstituted with sterile, injectable saline or dextrose 5% to a concentration of 10 to 20 mg/mL, which is further diluted with saline or dextrose 5% to 0.2 - 0.4 mg/mL, and is administered either IV bolus or by IV infusion over 15 - 60 minutes.

Intramuscular suspension: The following solution or suspension can be prepared, for intramuscular injection:

50 mg/mL of the desired, water-insoluble compound of this invention
5 mg/mL sodium carboxymethylcellulose
4 mg/mL TWEEN 80
9 mg/mL sodium chloride
9 mg/mL benzyl alcohol

Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.

Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

Tablets: A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg of starch, and 98.8 mg of lactose. Appropriate aqueous and non-aqueous coatings may be applied to increase palatability, improve elegance and stability or delay absorption.

Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

**Dose and administration**

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed,

the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

**Combination Therapies**

The compounds of this invention can be administered as the sole pharmaceutical agent or in combination with one or more other pharmaceutical agents where the combination causes no unacceptable adverse effects. Those combined pharmaceutical agents can be other agents having antiproliferative effects such as for example for the treatment of haematological tumours, solid tumours and/or metastases thereof and/or agents for the treatment of undesired side effects. The present invention relates also to such combinations.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, especially (chemotherapeutic) anti-cancer agents as defined supra. The combination can be a non-fixed combination or a fixed-dose combination as the case may be.

Methods of testing for a particular pharmacological or pharmaceutical property are well known to persons skilled in the art.

The example testing experiments described herein serve to illustrate the present invention and the invention is not limited to the examples given.

As will be appreciated by persons skilled in the art, the invention is not limited to the particular embodiments described herein, but covers all modifications of said embodiments that are within the spirit and scope of the invention as defined by the appended claims.

The following examples illustrate the invention in greater detail, without restricting it. Further compounds according to the invention, of which the preparation is not explicitly described, can be prepared in an analogous way.

The compounds, which are mentioned in the examples and the salts thereof represent preferred embodiments of the invention as well as a claim covering all subcombinations of the residues of the compound of formula (I) as disclosed by the specific examples.

The term "according to" within the experimental section is used in the sense that the procedure referred to is to be used "analogously to".

**EXPERIMENTAL PART**

Table 1 lists the abbreviations used in this paragraph and in the Intermediates and Examples sections as far as they are not explained within the text body.

**Table 1**

| Abbreviation | Meaning |
|---|---|
| AcOH | acetic acid (ethanoic acid) |
| aq. | aqueous |
| Boc | t-butoxycarbonyl |
| BOP | (benzotriazol-1-yloxy)tris(dimethylamino)phosphonium hexafluorophosphate |
| br | broad |
| brine | saturated aqueous sodium chloride solution |
| CI | chemical ionisation |
| $Cs_2CO_3$ | caesium carbonate |
| d | doublet |
| DAD | diode array detector |
| DBU | 1,8-diazabicyclo(5.4.0)undec-7-ene |
| DCC | N,N'-dicyclohexylcarbodiimide |

(continued)

| Abbreviation | Meaning |
|---|---|
| DCM | dichloromethane |
| dd | double-doublet |
| DIC | N,N'-diisopropylcarbodiimide |
| DIPEA | diisopropylethylamine |
| DMA | *N*,*N*-dimethylacetamide |
| DMF | *N*,*N*-dimethylformamide |
| DMSO | dimethyl sulfoxide |
| dt | double-triplet |
| EDC | 1-ethyl-3-(3-dimethylaminopropyl)carbodiimide |
| ELSD | Evaporative Light Scattering Detector |
| EtOAc | ethyl acetate |
| EtOH | ethanol |
| eq. | equivalent |
| ESI | electrospray (ES) ionisation |
| h | hour |
| HATU Or 2-(7-Azabenzotriazol-1-yl)-N, N, N', N'-tetramethyluronium hexafluorophosphate | 1-[bis(dimethylamino)methylene]-1H-1,2,3-triazolo[4,5-b]pyridinium 3-oxid hexafluorophosphate |
| HBTU | (o-benzotriazole-10yl)-N,N,N',N,-tetramethyluronium hexafluorophosphate |
| HCl | hydrochloric acid |
| HPLC | high performance liquid chromatography |
| $K_2CO_3$ | potassium carbonate |
| LC-MS | liquid chromatography mass spectrometry |
| m | multiplet |
| mCPBA | meta-Chloroperbenzoic acid |
| min | minute |
| MeCN | acetonitrile |
| MeOH | methanol |
| MS | mass spectrometry |
| MTBE | methyl-tert-butyl ether |
| NaCl | sodium chloride |
| $NaHCO_3$ | sodium hydrogen carbonate or sodium bicarbonate |
| NMR | nuclear magnetic resonance spectroscopy: chemical shifts ($\delta$) are given in ppm. The chemical shifts were corrected by setting the DMSO signal to 2.50 ppm unless otherwise stated. |
| $Na_2SO_4$ | sodium sulfate |
| PDA | Photo Diode Array |

(continued)

| Abbreviation | Meaning |
|---|---|
| Pd/C | palladium on activated charcoal |
| PyBOP | (benzotriazol-1-yloxy)tripyrrolidinophosphonium hexafluorophosphate |
| q | quartet |
| r.t. or rt or RT | room temperature |
| Rt | retention time (as measured either with HPLC or UPLC) in minutes |
| s | singlet |
| sat. | saturated |
| SFC | supercritical fluid chromatography |
| SIBX | stabilized 2-iodoxybenzoic acid |
| SM | starting material |
| SQD | Single-Quadrupole-Detector |
| T3P | propylphosphonic anhydride |
| t | triplet |
| td | triple-doublet |
| TEA | triethylamine |
| TFA | trifluoroacetic acid |
| THF | tetrahydrofuran |
| UPLC | ultra performance liquid chromatography |

Other abbreviations have their meanings customary per se to the skilled person.

The various aspects of the invention described in this application are illustrated by the following examples which are not meant to limit the invention in any way.

**Specific Experimental Descriptions**

NMR peak forms in the following specific experimental descriptions are stated as they appear in the spectra, possible higher order effects have not been considered.

Reactions employing microwave irradiation may be run with a Biotage Initiator® microwave oven optionally equipped with a robotic unit. The reported reaction times employing microwave heating are intended to be understood as fixed reaction times after reaching the indicated reaction temperature.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example pre-packed silica gel cartridges, *e.g.* from Separtis such as Isolute® Flash silica gel or Isolute® Flash $NH_2$ silica gel in combination with a Isolera® autopurifier (Biotage) and eluents such as gradients of e.g. hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art,

or be used as salts in subsequent biological assays. It is to be understood that the specific form (*e.g.* salt, free base etc) of a compound of the present invention as isolated as described herein is not necessarily the only form in which said compound can be applied to a biological assay in order to quantify the specific biological activity.

The percentage yields reported in the following examples are based on the starting component that was used in the lowest molar amount. Air and moisture sensitive liquids and solutions were transferred via syringe or cannula and introduced into reaction vessels through rubber septa. Commercial grade reagents and solvents were used without further purification. The term "concentrated in vacuo" refers to the use of a Buchi rotary evaporator at a minimum pressure of approximately 15 mm of Hg. All temperatures are reported uncorrected in degrees Celsius (°C).

In order that this invention may be better understood, the following examples are set forth. These examples are for the purpose of illustration only, and are not to be construed as limiting the scope of the invention in any manner. All publications mentioned herein are incorporated by reference in their entirety.

**Analytical conditions**

**UPLC-MS Standard Procedures**

UPLC-MS-data given in the subsequent specific experimental descriptions refer (unless otherwise noted) to the following conditions:

**Method 1:**

| | |
|---|---|
| ***System:*** | Waters Acquity UPLC-MS: Binary Solvent Manager, Sample Manager/Organizer, Column Manager, PDA, ELSD, SQD 3001 |
| ***Column:*** | Acquity BEH C18 1.7 50×2.1mm |
| ***Solvent:*** | A = water + 0.1% vol. formic acid (99%) |
| | B = acetonitrile |
| ***Gradient:*** | 0-1.6 min 1-99% B, 1.6-2.0 min 99% B |
| ***Flow:*** | 0.8 mL/min |
| ***Temperature:*** | 60 °C |
| ***Injection:*** | 2.0 μL |
| ***Detection:*** | DAD scan range 210-400 nm |
| | MS ESI+, ESI-, scan range 160-1000 m/z |
| | ELSD |

**Method 2:**

| | |
|---|---|
| ***System:*** | Waters Acquity UPLC-MS: Binary Solvent Manager, Sample Manager/Organizer, Column Manager, PDA, ELSD, SQD 3001 |
| ***Column:*** | Acquity BEH C18 1.7 50×2.1mm |
| ***Solvent:*** | A = water + 0.2% vol. ammonia (32%) |
| | B = acetonitrile |
| ***Gradient:*** | 0-1.6 min 1-99% B, 1.6-2.0 min 99% B |
| ***Flow:*** | 0.8 mL/min |
| ***Temperature:*** | 60 °C |
| ***Injection:*** | 2.0 μL |

(continued)

| | |
|---|---|
| ***Detection:*** | DAD scan range 210-400 nm |
| | MS ESI+, ESI-, scan range 160-1000 m/z |
| | ELSD |

**Method 3:**

| | |
|---|---|
| ***System:*** | SHIMADZU LCMS-2020 SingleQuad |
| ***Column:*** | Chromolith@Flash RP-18E 25-2 MM |
| ***Solvent:*** | A = water + 0.0375 vol % trifluoroacetic acid |
| | B = acetonitrile + 0.01875 vol % trifluoroacetic acid |
| ***Gradient:*** | 0-0.8 min, 5-95% B, 0.8-1.2 min 95% B |
| ***Flow:*** | 1.5 mL/min |
| ***Temperature:*** | 50 °C |
| ***Detection:*** | PDA: 220 nm & 254 nm |

**Flash column chromatography conditions**

"Purification by (flash) column chromatography" as stated in the subsequent specific experimental descriptions refers to the use of a Biotage Isolera purification system. For technical specifications see "Biotage product catalogue" on www.biotage.com.

Chemical names were generated using the ACD/Name software from ACD/Labs. In some cases generally accepted names of commercially available reagents were used in place of ACD/Name generated names.

Optical rotations were measured using a JASCO P2000 Polarimeter. Typical, a solution of the compound with a concentration of 1 mg/mL to 15 mg/mL was used for the measurement. The specific rotation $[\alpha]_D$ was calculated according to the following formula:

$$[\alpha]\mathrm{D} = \frac{\propto}{\beta \times d}$$

In this equation, $\alpha$ is the measured rotation in degrees; d is the path length in decimetres and $\beta$ is the concentration in g/mL.

**EXPERIMENTAL SECTION - INTERMEDIATES**

**Intermediate 1**

tert-butyl 3-ethyl-4-hydroxy-6-oxo-5-(phenylcarbamothioyl)-3,6-dihydropyridine-1(2H)-carboxylate (Racemate)

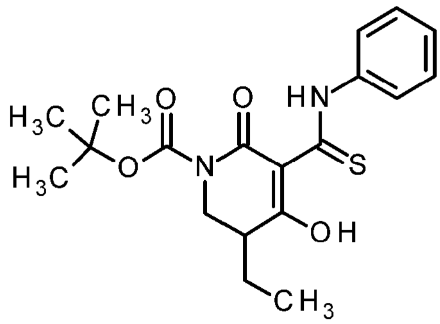

Tert-butyl 5-ethyl-2,4-dioxopiperidine-1-carboxylate (CAS 845267-80-3, 1.70 g, 7.05 mmol) and isothiocyanatobenzene (580 μL, 7.0 mmol) were dissolved 68 mL acetonitrile and were cooled down to 0°C. DBU (1.7 mL, 11 mmol) was added and the reaction mixture was stirred over night at room temperature. The reaction mixture was cooled down to 0 °C and the pH was adjusted to pH 3 by the addition of hydrochloric acid (4 M). Water was added and the aqueous layer was extracted with ethyl acetate. The combined organic layers were extracted with brine filtered through a silicone coated filter and were concentrated under red. Pressure. The crude product was purified by flash chromatography (Biotage 50g silica ultra column, Gradient: 0-80% hexane / ethyl acetate) to provide the target compound in 98% purity: 2.23 g.

LC-MS (Method 1): $R_t$ = 1.57 min; MS (ESIpos): m/z = 378 $[M+H]^+$

$^1$H-NMR (400MHz, DMSO-d6): δ [ppm]= 0.98 (t, 3H), 1.48 (s, 9H), 1.51 - 1.61 (m, 1H), 1.75 (ddd, 1H), 2.60 - 2.70 (m, 1H), 3.70 - 3.79 (m, 1H), 3.81 - 3.91 (m, 1H), 7.25 - 7.37 (m, 1H), 7.39 - 7.50 (m, 2H), 7.55 (d, 2H), 12.82 (br s, 1H), 15.45 (br s, 1H).

**Intermediate 2**

tert-butyl 4-{[(2-aminopyridin-4-yl)methyl]amino}-3-ethyl-6-oxo-5-(phenylcarbamothioyl)-3,6-dihydropyridine-1(2H)-carboxylate (Racemate)

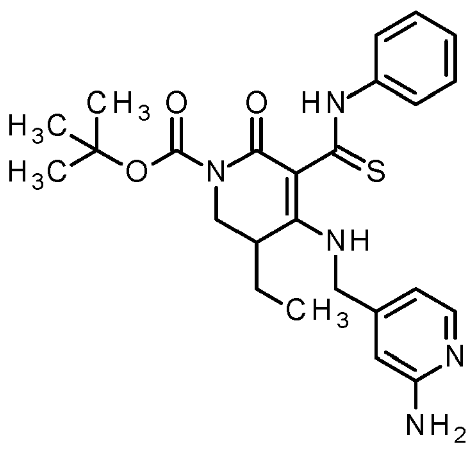

Tert-butyl 3-ethyl-4-hydroxy-6-oxo-5-(phenylcarbamothioyl)-3,6-dihydropyridine-1(2H)-carboxylate (Racemate) (see Intermediate 1, 1.97 g) and 4-(aminomethyl)pyridin-2-amine (1.16 g, 9.43 mmol) were combined and it was stirred at 100°C under argon atmosphere for 3 h without further solvent addition. The reaction mixture was cooled down to room temperature and turned very thick. It was treated with dichloromethane and methanol and sonicated for 20 min. No precipitate was formed. It was combined with a further batch starting from 100 mg of Intermediate 1 under the same conditions. The crude product was purified by flash chromatography twice (25g column, silica ULTRA; dichloromethane / ethanol 0%-20%) and (25 g column, silica ultra; dichloromethane / ethanol 0%-5%) to provide the target compound in 88% purity: 55 mg.

LC-MS (Method 2): $R_t$ = 1.28 min; MS (ESIpos): m/z = 482 $[M+H]^+$

$^1$H-NMR (400MHz, DMSO-d6): δ [ppm]= 0.96 (t, 3H), 1.40 - 1.57 (m, 11H), 2.75 - 2.93 (m, 1H), 3.46 (br d, 1H), 4.03 (br d, 1H), 4.55 (br s, 2H), 6.00 (s, 2H), 6.38 (s, 1H), 6.44 - 6.50 (m, 1H), 7.17 - 7.27 (m, 1H), 7.38 (br t, 2H), 7.52 (br d, 2H), 7.87 (d, 1H), 12.27 - 12.56 (m, 1H), 13.10 (br s, 1H).

**Intermediate 3**

4-{[(2-aminopyridin-4-yl)methyl]amino}-5-ethyl-2-oxo-N-phenyl-1,2,5,6-tetrahydropyridine-3-carbothioamide (Racemate)

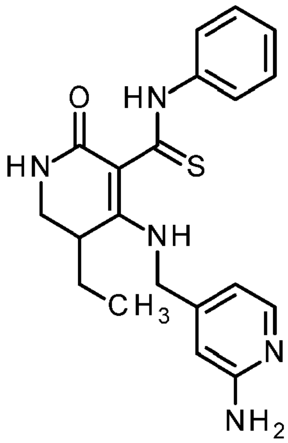

From the previous reaction (see Intermediate 2) it was possible to isolate the following side product: 89 mg in 78% purity, which was used without further purification.
LC-MS (Method 2): $R_t$ = 0.98 min; MS (ESIpos): m/z = 382 $[M+H]^+$

**Intermediate 4**

2-(2-aminopyridin-4-yl)-3-anilino-7-ethyl-1,5,6,7-tetrahydro-4H-pyrrolo[3,2-c]pyridin-4-one (Racemate)

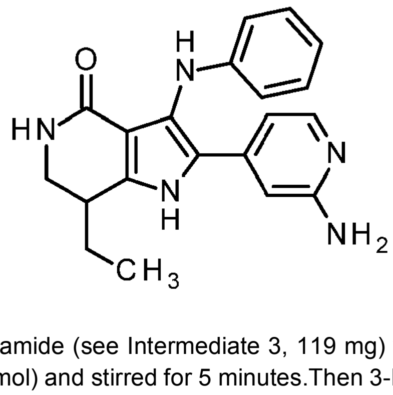

Tetrahydropyridine-3-carbothioamide (see Intermediate 3, 119 mg) was suspended in 2.7 mL methanol and treated with trifluoric acid (48 μL, 620 μmol) and stirred for 5 minutes.Then 3-Bralcloroperoxybenzoic acid (154 mg, 70 % purity, 624 μmol) was added and it was stirred at 90°C under argon atmosphere in a sealed vessel for 2 hours. Aqueous saturated sodium hydrogencarbonate solution was added to the reaction mixture and the methanol was removed under vacuo. The residue was diluted with dichloromethane, was stirred for a few minutes, filtered through a silicone coated filter and concentrated under reduced pressure The crude product was combined with the crude product starting from 340 mg of Intermediate 2 under the same conditions and purified by flash chromatography (11g column, aminophase; dichloromethane / ethanol 0%-5%) to provide the target compound in 75% purity: 62 mg.

LC-MS (Method 2): $R_t$ = 0.83 min; MS (ESIpos): m/z = 349 $[M+H]^+$

$^1$H-NMR (400MHz, DMSO-d6): δ [ppm]= 1.05 (t, 3H), 1.50 - 1.64 (m, 1H), 1.73 - 1.87 (m, 1H), 2.80 (dd, 1H), 3.12 - 3.22 (m, 1H), 3.47 - 3.53 (m, 1H), 5.66 (s, 2H), 6.52 - 6.64 (m, 4H), 6.69 (dd, 1H), 6.97 - 7.06 (m, 3H), 7.20 (s, 1H), 7.72 (d, 1H), 11.39 (s, 1H).

**Intermediate 5**

4-hydroxy-1,5-dimethyl-2-oxo-N-phenyl-1,2,5,6-tetrahydropyridine-3-carbothioamide (Racemate)

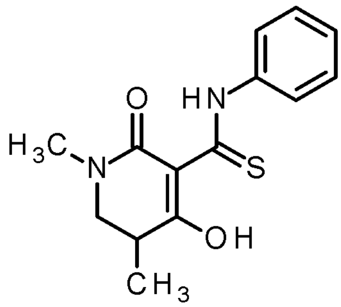

1,5-Dimethylpiperidine-2,4-dione (Racemte) (181 mg, 1.28 mmol, CAS-RN:[115497-23-9]) was suspended in 1.7 mL acetonitrile and treated with isothiocyanatobenzene (150 µl, 1.3 mmol). The mixture was cooled down to 0°C and treated slowly with DBU (310 µl, 2.1 mmol). It was stirred at room temperature over night. The reaction mixture was poured into a mixture of ice and hydrochloric acid (4 M). The aqueous layer was extracted with dichloromethane three times.

The collected organic layers were with water and brine, filtered through a silicone coated filter and concentrated under reduced pressure to provide the crude product in 97% purity, which was used without further purification: 291 mg

LC-MS (Method 2): $R_t$ = 0.55 min; MS (ESIpos): m/z = 277 $[M+H]^+$

$^1$H-NMR (400 MHz, DMSO-d6) δ [ppm] = 1.05 - 1.24 (m, 3H), 2.86 - 2.95 (m, 1H), 2.97 - 3.16 (m, 3H), 3.21 (dd, 1H), 3.59 (dd, 1H), 7.22 - 7.53 (m, 5H), 14.59 (s, 1H), 16.61 - 17.33 (m, 1H).

**Intermediate 6**

4-{[(2-aminopyridin-4-yl)methyl]amino}-1,5-dimethyl-2-oxo-N-phenyl-1,2,5,6-tetrahydropyridine-3-carbothioamide (Racemate)

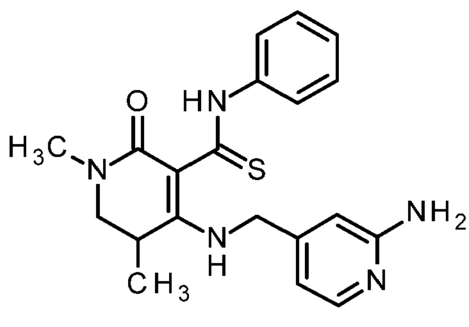

4-Hydroxy-1,5-dimethyl-2-oxo-N-phenyl-1,2,5,6-tetrahydropyridine-3-carbothioamide (Racemate) (see Intermediate 5, 291 mg) and 4-(aminomethyl)pyridin-2-amine (195 mg, 1.58 mmol) were suspended in 1.6 mL dry 1,2-dimethoxyethane in a sealed vessel. At 0°C N,O-Bis-(trimethylsiliyl)-acetamide (520 µl, 2.1 mmol; CAS-RN:[10416-59-8]) was added drop wise. The reaction mixture was stirred at 80°C in a sealed vessel under nitrogen atmosphere over night.

The reaction mixture was diluted with ethyl acetate and water. The aqueous layer was extracted with ethyl acetate three times. The combined organic layers were washed with water and brine, filtered through a silicone coated filter and concentrated under reduced pressure. The crude product was suspended in dichloromethane and diluted with MTBE. The undissolved precipitate was filtered off, to provide the target compound in 88% purity: 99 mg.

$^1$H-NMR (400 MHz, DMSO-d6) δ [ppm] = 1.09 - 1.17 (m, 3H), 2.93 - 3.03 (m, 4H), 3.10 (dd, 1H), 3.55 (dd, 1H), 4.44 - 4.68 (m, 2H), 5.25 - 6.08 (m, 2H), 6.38 (s, 1H), 6.45 (dd, 1H), 7.16 - 7.23 (m, 1H), 7.30 - 7.47 (m, 4H), 7.87 (d, 1H), 13.69 (br s, 1H), 14.70 (s, 1H).

**Intermediate 7**

2-(2-aminopyridin-4-yl)-3-anilino-5,7-dimethyl-1,5,6,7-tetrahydro-4H-pyrrolo[3,2-c]pyridin-4-one (Racemate)

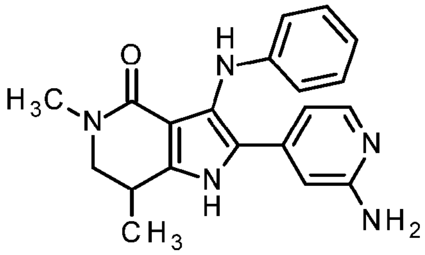

4-{[(2-Aminopyridin-4-yl)methyl]amino}-1,5-dimethyl-2-oxo-N-phenyl-1,2,5,6-tetrahydropyridine-3-carbothioamide (Racemate) (see Intermediate 6, 272 mg) was suspended in 3.4 mL methanol and treated with hydrogen peroxide (150 μl, 30 % purity, 1.4 mmol). The reaction mixture was stirred at 90 °C for 2 hours in a microwave reactor under nitrogen atmosphere. To the reaction saturated aqueous sodium thiosulfate-solution, half concentrated aqueous potassium carbonate solution and dichloromethane was added. It was stirred for a few minutes, filtered through a silicone coated filter and concentrated under reduced pressure. The crude product was combined with another batch starting from 4-{[(2-Aminopyridin-4-yl)methyl]amino}-1,5-dimethyl-2-oxo-N-phenyl-1,2,5,6-tetrahydropyridine-3-carbothioamide (Racemate) (see Intermediate 6, 99 mg) and purified by flash chromatography (28g column, aminophase; dichloromethane / ethanol 0%-100%) to provide the target compound in 82% purity: 98 mg.

$^1$H-NMR (400 MHz, DMSO-d6) δ [ppm] = 1.29 (d, 3H), 2.86 (s, 3H), 3.07 - 3.27 (m, 2H), 3.58 (dd, 1H), 5.67 (s, 2H), 6.47 - 6.63 (m, 4H), 6.70 (dd, 1H), 6.97 - 7.08 (m, 2H), 7.25 (s, 1H), 7.73 (d, 1H), 11.36 (s, 1H). -contains ethanol.

**Intermediate 8**

ethyl-2-cyano-4,4,4-trifluorobutanoate (Racemate)

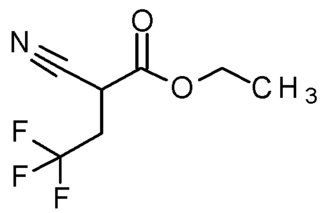

To a solution of ethyl cyanoacetate (CAS-RN:[105-56-6], 75.0 g, 663 mmol) in tetrahydrofuran (700 mL) was added lithium bis(trimethylsilyl)amide (CAS-RN:[4039-32-1], 663 mL, 1.0 M in tetrahydrofuran, 663 mmol) drop-wise at -70 °C. After addition, the mixture was stirred at -70 °C for 1 hour, a solution of 2,2,2-trifluoroethyl trifluoromethanesulfonate (CAS-RN:[6226-25-1], 154 g, 663 mmol) in tetrahydrofuran (100 mL) was added drop-wise and the resulting mixture was stirred at -70 °C for another 0.5 hour. Then the mixture was warmed to room temperature and stirred for 14 hours. Hydrochloric acid solution (1 M in water) was added and the mixture was extracted with ethyl acetate. The combined organic phase was dried over anhydrous sodium sulfate, filtered and concentrated to give a residue. The residue was purified by silica gel chromatography (petroleum ether: ethyl acetate = 100: 1 to 50: 1) to give 42 g ethyl-2-cyano-4,4,4-trifluorobutanoate as a yellow oil.

$^1H$ NMR (400 MHz, $CDCl_3$): δ [ppm] = 4.34 (q, 2H), 3.78 (dd, 1H), 2.92-2.87 (m, 1H), 2.79-2.75 (m, 1H), 1.36 (t, 1H).

**Intermediate 9**

ethyl-2-(aminomethyl)-4,4,4-trifluorobutanoate hydrogen chloride salt (Racemate)

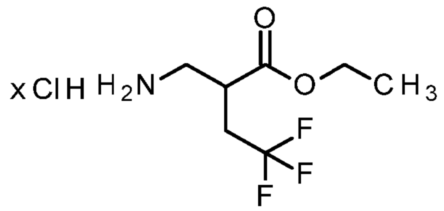

The reactions were performed as two batches in parallel: A mixture of ethyl-2-cyano-4,4,4-trifluorobutanoate (see Intermediate 8, 21.0 g) and Platinum(IV) oxide (2.44 g, 10.8 mmol) in methanol (300 mL) and hydrogen chloride (11 mL, 12 M in water, 130 mmol) was stirred at room temperature for 16 hours under hydrogen (15 psi). The mixture (combined with two other batches starting both from 21 g) was filtered and the filtrate was concentrated by evaporation in vacuum. Water and ethyl acetate were added and the mixture was separated. The aqueous phase was dried by lyophilization to give 45 g ethyl-2-(aminomethyl)-4,4,4-trifluorobutanoate hydrogen chloride salt (Racemate) as a white solid.

$^1H$ NMR (400 MHz, DMSO-$d_6$): δ [ppm] = 8.40 (brs, 2H), 4.18-4.12 (m, 2H), 3.10-3.00 (m, 3H), 2.82-2.68 (m, 2H), 1.21 (t, 3H).

**Intermediate 10**

ethyl-2-({[(benzyloxy)carbonyl]amino}methyl)-4,4,4-trifluorobutanoate (Racemate)

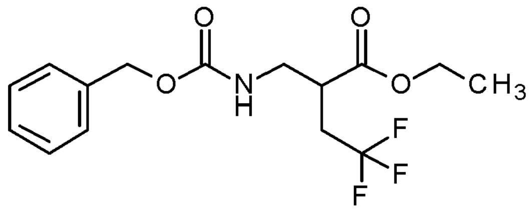

A mixture of ethyl-2-(aminomethyl)-4,4,4-trifluorobutanoate·hydrogen chloride salt (Racemate) (see Intermediate 9, 44.8 g), benzyl carbonochloridate (41 mL, 290 mmol) and sodium hydrogen carbonate (63.9 g, 760 mmol) in water (400 mL) and dichloromethane (400 mL) was stirred at room temperature for 2 hours. Water was added and the mixture was extracted with dichloromethane. The combined organic phase was dried over anhydrous sodium sulfate, filtered and concentrated to give a residue. The residue was purified by column chromatography (300-400 mesh, petroleum ether: ethyl acetate = 20: 1 then 10: 1) to give 63 g ethyl-2-({[(benzyloxy)carbonyl]amino}methyl)-4,4,4-trifluorobutanoate (Racemate) as yellow oil.

$^1H$ NMR (400 MHz, $CDCl_3$): δ [ppm] = 7.39-7.35 (m, 5H), 5.11 (s, 2H), 4.71 (d, 1H), 4.25-4.10 (m, 2H), 3.51-3.44 (m, 2H), 2.95-2.92 (m, 1H), 2.61-2.52 (m, 1H), 2.37-2.29 (m, 1H), 1.27 (t, 3H).

**Intermediate 11**

ethyl-2-({[(benzyloxy)carbonyl](methyl)amino}methyl)-4,4,4-trifluorobutanoate (Racemate)

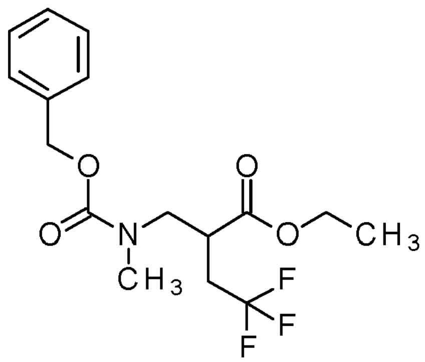

To a solution of ethyl-2-({[(benzyloxy)carbonyl]amino}methyl)-4,4,4-trifluorobutanoate (Racemate) (see Intermediate 10, 10.0 g) in tetrahydrofuran (120 mL) was added sodium hydride (1.44 g, 60% purity, contained mineral oil) in portions at 0 °C. The mixture was stirred at 0 °C for 1 hour, iodomethane (2.8 mL, 45 mmol) was added drop-wise. After addition, the resulting mixture was stirred at 0 °C for 0.5 hour. Then the mixture was warmed to room temperature and stirred for 3 hours. Saturated ammonium chloride aqueous solution was added and the mixture was extracted with ethyl acetate. The organic phase was washed with brine, dried over anhydrous sodium sulfate, filtered and concentrated to give a residue. The residue was purified by silica gel chromatography (200-300mesh, petroleum ether: ethyl acetate = 20: 1 to 10: 1) to give 4.70 g ethyl-2-({[(benzyloxy)carbonyl](methyl)amino}methyl)-4,4,4-trifluorobutanoate (Racemate) as yellow oil.

$^1H$ NMR (400 MHz, $CDCl_3$): $\delta$ [ppm] = 7.29-7.28 (m, 5H), 5.06-5.03 (m, 2H), 4.09-4.02 (m, 2H), 3.70-3.43 (m, 2H), 2.95 (s, 3H), 2.75-2.53 (m, 2H), 2.25-2.09 (m, 1H), 1.25 (t, 3H).

**Intermediate 12**

ethyl-4,4,4-trifluoro-2-[(methylamino)methyl]butanoate hydrogen chloride salt (Racemate)

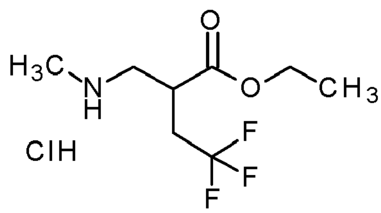

To a solution of ethyl-2-({[(benzyloxy)carbonyl](methyl)amino}methyl)-4,4,4-trifluorobutanoate (Racemate) (see Intermediate 11, 4.70 g) and hydrochloric acid (1.4 mL, 12 M in water, 16 mmol) in methanol (50 mL) was added palladium on carbon (500 mg, 10 % purity, contained 50% water, 470 $\mu$mol) at room temperature. The reaction mixture was stirred at room temperature for 16 hours under hydrogen atmosphere (15 psi). The mixture was filtered and the filtrate was concentrated by evaporation in vacuum to give 3.20 g ethyl-4,4,4-trifluoro-2-[(methylamino)methyl]butanoate-hydrogen chloride salt (Racemate). The residue was used for next step directly without further purification.

**Intermediate 13**

ethyl-2-{[(3-ethoxy-3-oxopropanoyl)(methyl)amino]methyl}-4,4,4-trifluorobutanoate (Racemate)

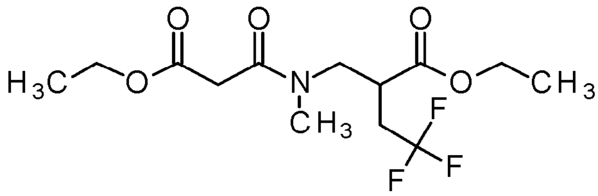

N,N-diisopropylethylamine (6.7 mL, 38 mmol) and 4-dimethylaminopyridine (157 mg, 1.28 mmol) were added to a solution of 4,4,4-trifluoro-2-[(methylamino)methyl]butanoate-hydrogen chloride salt (Racemate) (see Intermediate 12, 3.20 g) in dichloromethane (50 mL) at 0°C and ethyl-3-chloro-3-oxopropanoate (1.5 mL, 12 mmol) was added to the mixture slowly below 15°C. The mixture was stirred at room temperature for 16 hours. The mixture was adjusted to pH~3 by addtion hydrochloric acid solution (2 M in water). Then the mixture was extracted with dichloromethane and the combined organic layer was washed with brine, dried over anhydrous sodium sulfate, filtered and concentrated by evaporation in vacuum to give the target compound: 3.30 g. The residue was used for next step directly without further purification.

## Intermediate 14

1-methyl-5-(2,2,2-trifluoroethyl)piperidine-2,4-dione (Racemate)

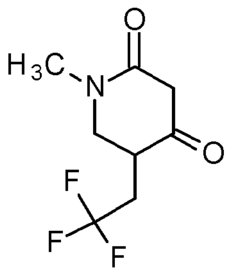

To a solution of ethyl-2-{[(3-ethoxy-3-oxopropanoyl)(methyl)amino]methyl}-4,4,4-trifluorobutanoate (Racemate) (see Intermediate 13, 3.30 g) in ethanol (20 mL) was added sodium ethoxide (CAS-RN:[141-52-6], 823 mg) at 0-5 °C. Then the mixture was stirred at room temperature for 4 hours. The mixture was treated with aqueous hydrogen chloride solution (2 M in water), the aqueous phase was extracted with ethyl acetate. The combined organic phase was washed with brine, dried over anhydrous sodium sulfate, filtered and concentrated to give a residue. Then the residuewas dissolved in acetonitrile (20 mL) and water (1 mL), and the resulting mixture was stirred at reflux for 16 hours. The mixture was concentrated by evaporation in vacuum to give 1.40 g 1-methyl-5-(2,2,2-trifluoroethyl)piperidine-2,4-dione (Racemate) in 76 % purity. The residue was used for next step directly and no futher purification.

LC-MS (Method 3): $R_t$ = 0.391 min; MS (ESIpos): m/z = 210.1 $[M+H]^+$.

## Intermediate 15

4-hydroxy-1-methyl-2-oxo-N-phenyl-5-(2,2,2-trifluoroethyl)-1,2,5,6-tetrahydropyridine-3-carbothioamide (Racemate)

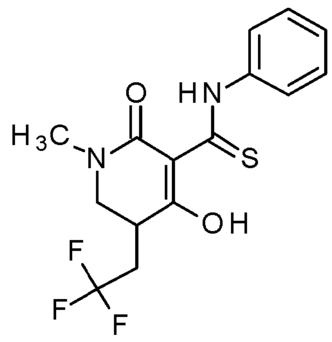

To a solution of 1-methyl-5-(2,2,2-trifluoroethyl)piperidine-2,4-dione (Racemate) (see Intermediate 14, 1.40 g) and isothiocyanatobenzene (688 mg, 5.09 mmol) in acetonitrile (15 mL) was added 1,8-diazabicyclo(5.4.0)undec-7-ene(DBU) (1.2 mL, 8.1 mmol) drop-wise slowly below 5 °C. Then the mixture was stirred at room temperature for 5 hours. The mixture was treated with hydrogen chloride solution (2 M in water), the aqueous phase was extracted with ethyl acetate. The combined organic phase was dried over anhydrous sodium sulfate, filtered and concentrated to give a residue. The residue was purified by silica gel chromatography (100-200 mesh, petroleum ether: ethyl acetate = 50: 1 to 20: 1) to give 1.45 g 4-hydroxy-1-methyl-2-oxo-N-phenyl-5-(2,2,2-trifluoroethyl)-1,2,5,6-tetrahydropyridine-3-carbothioamide (Racemate) in 83 % purity as yellow oil.

$^{1}H$ NMR (400 MHz, $CDCl_3$): $\delta$ [ppm] = 14.21 (s, 1H), 13.99 (s, 1H), 7.49-7.38 (m, 4H), 7.34-7.28 (m, 1H), 3.70-3.63 (m, 1H), 3.47-3.35 (m, 1H), 3.21 (s, 1H) , 3.10 (s, 3H) , 2.83-2.68 (m, 1H), 2.46-2.32 (m, 1H).

## Intermediate 16

4-{[(2-aminopyridin-4-yl)methyl]amino}-1-methyl-2-oxo-N-phenyl-5-(2,2,2-trifluoroethyl)-1,2,5,6-tetrahydropyridine-3-carbothioamide (Racemate)

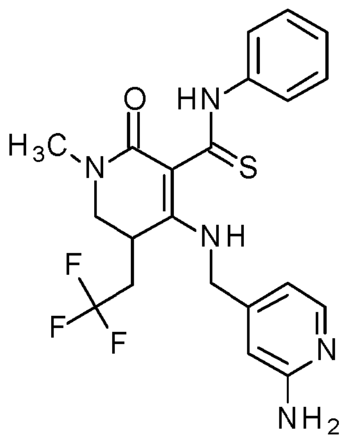

A mixture of 4-hydroxy-1-methyl-2-oxo-N-phenyl-5-(2,2,2-trifluoroethyl)-1,2,5,6-tetrahydropyridine-3-carbothioamide (Racemate) (see Intermediate 15, 1.35 g), 4-(aminomethyl)pyridin-2-amine (CAS-RN:[199296-51-0], 577 mg) and trimethylsilyl (1Z)-N-(trimethylsilyl)ethanimidate (2.20 g, 10.8 mmol) was stirred at 80 °C for 16 hours. The mixture (combined with another batch starting from 100 mg) was concentrated by evaporation in vacuum. The residue was purified by column chromatography (1000 mesh, petroleum ether: ethyl acetate = 10: 1 then 2: 1) to give the target compound in 88 % purity as a white solid: 1.1 g.

LC-MS (Method 3): $R_t$ = 0.804 min; MS (ESIpos): m/z = 450.1 $[M+H]^+$.

**Intermediate 17**

2-(2-aminopyridin-4-yl)-3-anilino-5-methyl-7-(2,2,2-trifluoroethyl)-1,5,6,7-tetrahydro-4H-pyrrolo[3,2-c]pyridin-4-one (Racemate)

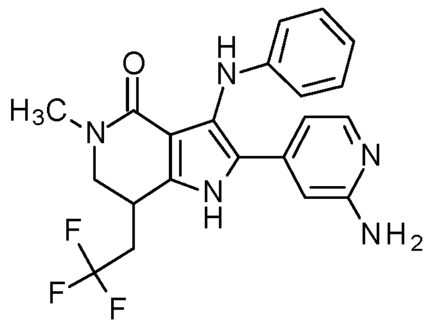

To a solution of 4-{[(2-aminopyridin-4-yl)methyl]amino}-1-methyl-2-oxo-N-phenyl-5-(2,2,2-trifluoroethyl)-1,2,5,6-tetrahydropyridine-3-carbothioamide (Racemate) (see Intermediate 16, 900 mg) in methanol (20 mL) was added trifluoroacetic acid (154 μl, 2.00 mmol) and and stirred for 5 minutes. Then meta-Chloroperoxybenzoic acid (432 mg, 80 % purity, 2.00 mmol) was added at room temperature and the mixture was stirred at 100 °C for 2 hours under nitrogen. The mixture was cooled to room temperature and poured into saturated sodium thiosulfate solution: Saturated sodium bicarbonate solution = 1: 1 and the mixture was extracted with ethyl acetate. The combined organic phase was washed with brine, dried over anhydrous sodium sulfate, filtered and concentrated in vacuum. The residue (combined with another batch starting from 100 mg) was purified by silica gel chromatography (petroleum ether: ethyl acetate= 10: 1 to 0: 1) to give a crude product. The crude product was purified by silica gel chromatography (petroleum ether: (ethyl acetate: methanol = 10: 1) = 5: 1 to 1: 1 to give 452 mg 2-(2-aminopyridin-4-yl)-3-anilino-5-methyl-7-(2,2,2-trifluoroethyl)-1,5,6,7-tetrahydro-4H-pyrrolo[3,2-c]pyridin-4-one (Racemate) in 98% purity as a yellow solid.

LC-MS (Method 3): $R_t$ = 1.205 min; MS (ESIpos): m/z = 416.2 $[M+H]^+$.

$^1$H NMR (400 MHz, DMSO-$d_6$): δ [ppm] = 11.68 (s, 1H), 7.77 (d, 1H), 7.41 (s, 1H), 7.07-7.03 (m, 2H), 6.85-6.83 (m, 1H), 6.68-6.64 (m, 2H), 6.58 (d, 2H), 6.47 (brs, 1H), 3.74-3.71 (m, 1H), 3.50-3.40 (m, 3H), 2.89 (s, 3H), 2.70-2.66 (m, 1H).

**Intermediate 18**

ethyl-2-cyano-3-cyclopropylpropanoate (Racemate)

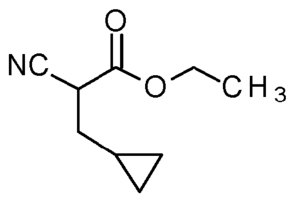

To a solution of ethyl cyanoacetate (CAS-RN:[105-56-6], 100.0 g, 884 mmol) and (bromomethyl)cyclopropane (CAS-RN:[7051-34-5], 131.0 g, 972 mmol) in acetonitrile (2000 mL) was added potassium carbonate (367 g, 2.65 mol) and sodium iodate (17.5 g, 88.4 mmol). Then the mixture was stirred at 60 °C for 14 hours. Aqueous hydrochloric acid solution (1 M) was added until pH was adjust to 3, and the mixture was extracted with ethyl acetate. The combined organic phase was dried over anhydrous sodium sulfate, filtered and concentrated to give a residue. The residue was purified by silica gel chromatography (200-300 mesh, petroleum ether: ethyl acetate = 100: 1 to 50: 1) to give 100 g ethyl-2-cyano-3-cyclopropylpropanoate (Racemate) as yellow oil.

$^1H$ NMR (400 MHz, $CDCl_3$): $\delta$ [ppm] = 4.26 (q, 2H), 3.57 (t, 1H), 1.94-1.88 (m, 1H), 1.85-1.82 (m, 1H), 1.32 (t, 3H), 0.93-0.84 (m, 1H), 0.60-0.54 (m, 2H), 0.25-0.16 (m, 2H).

**Intermediate 19**

ethyl-2-(aminomethyl)-3-cyclopropylpropanoate-hydrogen chloride salt (Racemate)

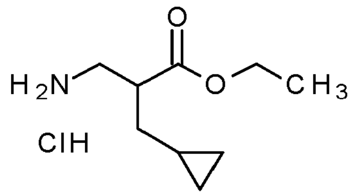

This reaction was performed parallel for 4 batched: A mixture of ethyl-2-cyano-3-cyclopropylpropanoate (Racemate)(see Intermediate 18, 17.5 g) and Platinum(IV) oxide (1.75 g, 7.7 mmol) and hydrochloric acid (12 M, 8 mL) in ethanol (530 mL) was stirred at room temperature for 16 hours under hydrogen (15 psi). The 4 batches of mixture were combined together, filtered and the filtrate was concentrated by evaporation in vacuum. The residue (65.0 g) was used to next step directly.

**Intermediate 20**

ethyl-2-({[(benzyloxy)carbonyl]amino}methyl)-3-cyclopropylpropanoate (Racemate)

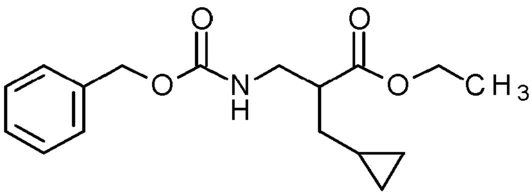

A mixture of ethyl-2-(aminomethyl)-3-cyclopropylpropanoate-hydrogen chloride salt (Racemate) (see Intermediate 19, 65.0 g), benzyl carbonochloridate (13153.4 g, 313 mmol) and sodium hydrogen carbonate (105.0 g, 1.25 mol) in water (650 mL) and dichloromethane (650 mL) was stirred at room temperature for 2 hours. The organic phase was dried over anhydrous sodium sulfate, filtered and concentrated to give a residue (combined with another batch starting from 35 g). 10 g of crude product was used next directly. The remained residue (90 g) was purified by preparative HPLC (Column: Phenomenex luna C18 (250*70mm,10 um); Mobile phase: water(0.225% FA)-CAN; Gradient B%: 45%-73%,140ml/min; 97%, 21min; Instrument: ACSWH-PREP-HPLC-I) and then extrated with ethyl acetate and concentrated to give 44.0 g ethyl-3-{[(benzyloxy)carbonyl]amino}-2-(cyclopropylmethyl)propanoate (Racemate) as yellow oil.

LC-MS (Method 3): $R_t$ = 0.956 min; MS (ESIpos): m/z = 306.2 $[M+H]^+$.

$^1H$ NMR (400 MHz, $CDCl_3$): $\delta$ [ppm] = 7.32-7.25 (m, 5H), 5.27 (s, 1H), 5.06 (s, 2H), 4.12 (q, 2H), 3.46-3.44 (m, 1H), 3.39-3.33 (m, 1H), 2.72-2.68 (m, 1H), 1.46 (t, 2H), 1.22 (t, 3H), 0.71-0.65 (m, 1H), 0.44-0.38 (m, 2H), 0.04-0.01 (m, 2H).

**Intermediate 21**

ethyl-2-({[(benzyloxy)carbonyl](methyl)amino}methyl)-3-cyclopropylpropanoate (Racemate)

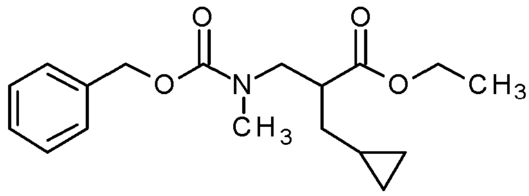

To a solution of ethyl-2-({[(benzyloxy)carbonyl]amino}methyl)-3-cyclopropylpropanoate (see Intermediate 20, 44.0 g) in tetrahydrofuran (500 mL) was drop-wise added sodium hydride (6.92 g, 60% purity, contained mineral oil, 173 mmol) at 0 °C. The mixture was stirred at 0 °C for 1 hour, iodomethane (30.7 g, 216 mmol) was drop-wise added and the resulting mixture was stirred at 0 °C for 0.5 hour. Then the mixture was stirred at room temperature for 3 hours. The mixture was quenched by pouring into cold ammonium chloride solution, extracted with ethyl acetate. The organic phase was washed with brine, dried over anhydrous sodium sulfate, filtered and concentrated to give a residue. The residue was purified by silica gel chromatography (200-300 mesh, petroleum ether: ethyl acetate = 20: 1 to 10: 1) to give 46.0 g ethyl-3-{[(benzyloxy)carbonyl](methyl)amino}-2-(cyclopropylmethyl)propanoate as a yellow oil.

LC-MS (Method 3): $R_t$ = 0.813 min; MS (ESIpos): m/z = 319.9 $[M+H]^+$.

$^1H$ NMR (400 MHz, $CDCl_3$): δ [ppm] = 7.37-7.30 (m, 5H), 5.14-5.09 (m, 2H), 4.16-4.10 (m, 2H), 3.59-3.54 (m, 1H), 3.43-3.37 (m, 1H), 2.93 (s, 3H), 1.54-1.48 (m, 1H), 1.27 (t, 3H), 0.78-0.55 (m, 1H), 0.45-0.40 (m, 2H), 0.05-0.01 (m, 2H).

**Intermediate 22**

ethyl-2-(cyclopropylmethyl)-3-(methylamino)propanoate-hydrogen chloride salt (Racemate)

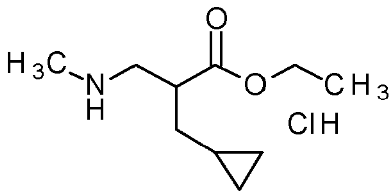

To a solution of ethyl-2-({[(benzyloxy)carbonyl](methyl)amino}methyl)-3-cyclopropylpropanoate (Racemate) (see Intermediate 21, 46.0 g) and hydrochloric acid (12 M) (14 mL, 172 mmol) in ethanol (380 mL) was added palladium on carbon (2.0 g, 10% putiry, contained 50% water) and palladium hydroxide on carbon (2.0 g, contained 50% water) at room temperature (20 °C). The reaction mixture was stirred at room temperature (20 °C) for 20 hours under hydrogen atmosphere (15 psi). The mixture was filtered and the filtrate was concentrated by evaporation in vacuum. The residue (31.0 g) was used to next step directly.

**Intermediate 23**

ethyl-2-(cyclopropylmethyl)-3-[(3-ethoxy-3-oxopropanoyl)(methyl)amino]propanoate (Racemate)

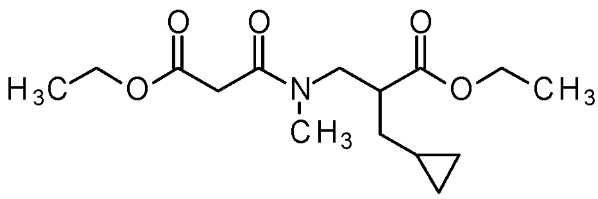

N,N-Diisopropylethylamine (73 mL, 420 mmol) and 4-Dimethylaminopyridine (1.71 g, 14.0 mmol) were added at 0 °C to a solution of ethyl-2-(cyclopropylmethyl)-3-(methylamino)propanoate-hydrogen chloride salt (see Intermediate 22, 31.0 g) in dichloromethane. And ethyl-3-chloro-3-oxopropanoate (CAS-RN:[37517-81-0], 18.9 g, 126 mmol) was added to the mixture slowly at below 15°C. The mixture was stirred at room temperature for 16 hours. The mixture was adjusted to pH~3 with aqueous hydrochloric acid solution (2 M). Then the mixture was extracted with dichloromethane and the combined organic layer was washed with brine, dried over anhydrous sodium sulfate, filtered and concentrated by evaporation in vacuum. The residue (18.0 g) was used for next step directly and no purification.
LC-MS (Method 3): $R_t$ = 0.880 min; MS (ESIpos): m/z = 300.2 $[M+H]^+$.

## Intermediate 24

5-(cyclopropylmethyl)-1-methylpiperidine-2,4-dione (Racemate)

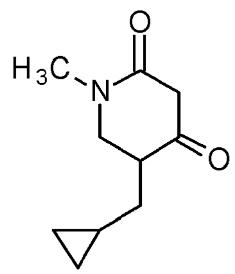

To a solution of ethyl-2-(cyclopropylmethyl)-3-[(3-ethoxy-3-oxopropanoyl)(methyl)-amino]propanoate (Racemate) (see Intermediate 23, 60.0 g) in ethanol (600 mL) was added sodium ethoxide (16.4 g, 241 mmol) at 0-5 °C. Then the mixture was stirred at room temperature for 4 hours. The mixture was treated with aqueous hydrogen chloride solution (2 M), the aqueous phase was extracted with ethyl acetate. The combined organic phase was washed with brine, dried over anhydrous sodium sulfate, filtered and concentrated to give a residue. Then the residue dissolved in acetonitrile (600 mL) and water (30 mL), and the resulting mixture was heated at reflux for 16 hours. The residue was purified by column chromatography (300-400 mesh, petroleum ether: ethyl acetate = 30: 1 then 1: 1) to give 25 g 5-(cyclopropylmethyl)-1-methylpiperidine-2,4-dione (Racemate) in 95 % purity as yellow oil.

LC-MS (Method 3): $R_t$ = 0.489 min; MS (ESIpos): m/z = 182 $[M+H]^+$
$^1$H NMR (400 MHz, $CDCl_3$): $\delta$ [ppm] = 3.66-3.61(m, 1H), 3.49-3.39 (m, 1H), 3.32-3.29 (m, 2H), 3.07 (s, 3H), 2.75-2.70 (m, 1H), 1.76-7-1.71 (m, 1H), 1.36-1.30 (m, 1H), 0.73-0.69 (m, 1H), 0.54-0.49 (m, 2H), 0.10-0.06 (m, 2H).

## Intermediate 25

5-(cyclopropylmethyl)-4-hydroxy-1-methyl-2-oxo-N-phenyl-1,2,5,6-tetrahydropyridine-3-carbothioamide (Racemate)

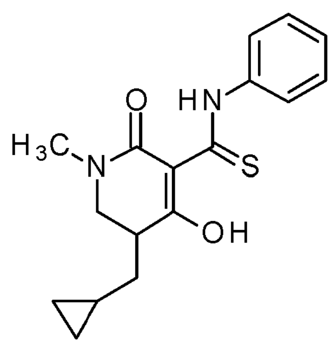

To a solution of 5-(cyclopropylmethyl)-1-methylpiperidine-2,4-dione (Racemate) (see Intermediate 24, 4.00 g) and isothiocyanatobenzene (2.98 g, 22.1 mmol) in acetonitrile (40 mL) was added 1,8-diazabicyclo(5.4.0)undec-7-ene(DBU) (4.9 mL, 33 mmol) slowly dropwise below 5 °C. Then the mixture was stirred at room temperature for 5 hours. The mixture was treated with aqueous hydrogen chloride solution (2 M) until pH was adjust to 3, the aqueous phase was extracted with ethyl acetate. The combined organic phase was dried over anhydrous sodium sulfate, filtered and concentrated to give a residue. The residue was purified by silica gel chromatography (300-400 mesh, petroleum ether: ethyl acetate = 50: 1 to 20: 1) to give 6.5 g of the target compound as yellow oil.

$^1H$ NMR (400 MHz, DMSO-$d_6$): δ [ppm] = 14.30 (s, 1H), 7.48 (d, 2H), 7.41 (t, 2H), 7.28 (s, 1H), 3.68 (dd, 1H), 3.37 (dd, 1H), 3.08 (s, 3H), 2.75-2.71 (m, 1H), 1.78-1.72 (m, 1H), 1.58-1.50 (m, 1H), 0.79-0.75 (m, 1H), 0.60-0.58 (m, 1H), 0.54-0.50 (m, 1H), 0.15-0.12 (m, 2H).

## Intermediate 26

4-{[(2-aminopyridin-4-yl)methyl]amino}-5-(cyclopropylmethyl)-1-methyl-2-oxo-N-phenyl-1,2,5,6-tetrahydropyridine-3-carbothioamide (Racemate)

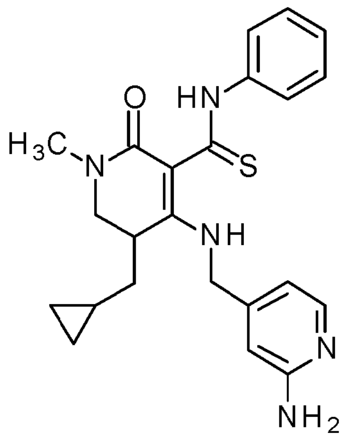

5-(cyclopropylmethyl)-4-hydroxy-1-methyl-2-oxo-N-phenyl-1,2,5,6-tetrahydropyridine-3-carbothioamide (Racemate) (see Intermediate 25, 2.50 g) was dried over by azeotropic rotation with toluene twice. 4-(aminomethyl)pyridin-2-amine (CAS-RN:[199296-51-0], 1.26 g, 10.3 mmol) was dried over by azeotropic rotation with toluene twice. A mixture of 5-(cyclopropylmethyl)-4-hydroxy-1-methyl-2-oxo-N-phenyl-1,2,5,6-tetrahydropyridine-3-carbothioamide (see Intermediate 25, 2.50 g), 4-(aminomethyl)pyridin-2-amine (CAS-RN:[199296-51-0], 1.26 g, 10.3 mmol), 4A molecular sieve and trimethylsilyl (1Z)-N-(trimethylsilyl)ethanimidate (4.82 g, 23.7 mmol) was stirred at 80 °C for 16 hours under nitrogen. The mixture was filtered and the filtrate was concentrated by evaporation in vauum. The residue was purified by silica gel chromatography (200-300 mesh, petroleum ether: ethyl acetate = 10: 1 to 0: 1) to give 3.0 g 4-{[(2-aminopyridin-4-yl)methyl]amino}-5-(cyclopropylmethyl)-1-methyl-2-oxo-N-phenyl-1,2,5,6-tetrahydropyridine-3-carbothioamide as yellow oil.

$^1H$ NMR (400 MHz, DMSO-$d_6$): δ [ppm] = 14.68 (s, 1H), 13.76 (s, 1H), 7.88 (d, 1H), 7.42 (d, 2H), 7.35 (t, 2H), 7.20-7.16 (m, 1H), 6.46 (dd, 1.2 Hz, 1H), 6.39 (s, 1H), 6.02 (s, 2H), 4.61-4.57 (m, 2H), 3.54-3.49 (m, 1H), 3.40-3.37 (m, 1H), 2.98 (s, 3H), 2.95-2.92 (m, 1H), 1.49-1.32 (m, 2H), 0.84-0.77 (m, 1H), 0.56-0.38 (m, 2H), 0.12-0.01 (m, 2H).

## Intermediate 27

2-(2-aminopyridin-4-yl)-3-anilino-7-(cyclopropylmethyl)-5-methyl-1,5,6,7-tetrahydro-4H-pyrrolo[3,2-c]pyridin-4-one (Racemate)

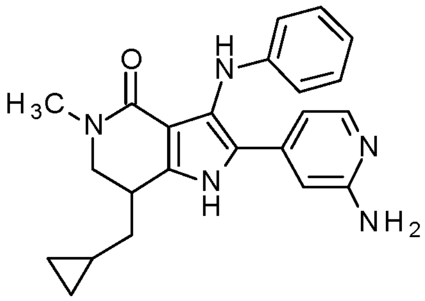

To a solution of 4-{[(2-aminopyridin-4-yl)methyl]amino}-5-(cyclopropylmethyl)-1-methyl-2-oxoN-phenyl-1,2,5,6-tetrahydropyridine-3-carbothioamide (Racemate) (see Intermediate 26, 3.00 g) in methanol (30 mL) was added trifluoroacetic acid (550 μl, 7.1 mmol). The mixture was stirred at room temperature for 5 minutes. Then meta-Chloroperoxybenzoic acid (550 μl, 7.1 mmol) was added at room temperature. The mixture was stirred at 100 °C for 2 hours. The Mixture was combined with two other batches starting from 0.70 g and 1.00 g. Saturated sodium hydrogencarbonate solution was added until moist potassium iodide test strip tured white. And then the methanol was removed under vacuo. The crude product triturated with dichloromethane for 1 hour, filtered. The filter cake was collected. The residue was then triturated with acetonitrile for 1 hour at room temperature. Filtered, the filter cake was collected. The cake was diluited in dichloromethane, then satureted sodium bicarbonate was added until pH was adjust to 10. The mixture was extracted with dichloromethane. The organic phase was washed with brine, dried over anhydrous sodium sulfate, filtered and concentrated to give the target compound in 95% purity as grey solid: 1.02 g.

LC-MS (Method 3): $R_t$ = 0.819 min; MS (ESIpos): m/z = 388.2 $[M+H]^+$.

$^1$H NMR (400 MHz, MeOD): δ [ppm] = 7.70 (d, 1H), 7.05 (t, 2H), 6.83 (dd, 1H), 6.70-6.66 (m, 2H), 6.62 (d, 2H), 3.87-3.83 (m, 1H), 3.57-3.52 (m, 1H), 3.15-3.09 (m, 1H), 3.00 (s, 3H), 1.65-1.61 (m, 2H), 0.81-0.78 (m, 1H), 0.55-0.46 (m, 2H), 0.08-0.01 (m, 2H).

## Intermediate 28

rac-(1R*,5S*)-6-azabicyclo[3.2.0]heptan-7-one

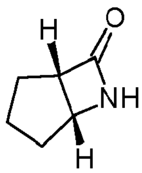

Sulfurisocyanatidoyl chloride (CAS-RN:[ 1189-71-5], 93.5 g, 660 mmol, 0.90 eq) was added drop-wise in 1 hour to neat cyclopentene (CAS-RN:[142-29-0], 50.0 g, 734 mmol, 1.00 eq) at -70 °C. After the addition was complete, the mixture was warmed to 25 °C over a period of 1 hour and then stirred at 25 °C for 12 hour. The reaction mixture was added drop-wise to a stirring suspension of ice water (1.7 L), $Na_2SO_3$ (140 g), and $NaHCO_3$ (500 g) over a period of 20 min. The mixture was warmed to 23 °C and stirred at that temperature for 20 min, after which dichloromethane (300 mL) was added and stirring was continued for an additional 5 min. The solids were collected by vacuum filtration, rinsed sequentially with water (800 mL) and dichloromethane (800 mL). The organic layer was separated from the filtrate and the aqueous layer was extracted with dichloromethane (250 mL × 3). The combined organic phases were washed with water (100 mL × 3), brine (100 mL × 3), dried with anhydrous $Na_2SO_4$, filtered and concentrated under reduced pressure to give the title compound as yellow oil, and used to next step directly.

$^1$H-NMR (400 MHz, $CDCl_3$) δ [ppm] δ 6.25 (s, 1H), 4.02-4.00 (m, 1H), 3.47-3.44 (m, 1H), 2.00-1.97 (m, 1H), 1.84-1.73 (m, 3H), 1.39-1.34 (m, 2H).

**Intermediate 29**

Ethyl rac-(1R*,2S*)-2-aminocyclopentanecarboxylate

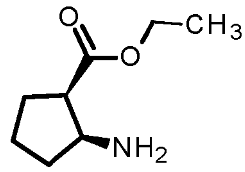

$SOCl_2$ (131 g, 1.10 mol, 80 mL, 1.36 eq) was added drop-wise to EtOH (500 mL) at-30 °C, then rac (1R*,5S*)-6-azabicyclo[3.2.0]heptan-7-one (see Intermediate 28, 90.0 g, 809 mmol, 1.00 *eq*) in EtOH (200 mL) was added to the mixture at 0 °C and the mixture was stirred at 25 °C for 12 h. The reaction mixture was concentrated under reduced pressure to remove EtOH. The residue was adjusted to pH = 8-9 with $NaHCO_3$ and extracted with Ethyl acetate 600 mL (200 mL × 3). The combined organic layers were washed with brine 300 mL, dried over $Na_2SO_4$, filtered and concentrated under reduced pressure to give the title compound (40.0 g, 254 mmol, 31.4% yield) as a brown oil, and used directly in the next step.

$^1$H-NMR (400 MHz, $CDCl_3$) δ [ppm]: 4.19-4.13 (m, 2H), 3.67-3.62 (m, 1H), 3.30 (s, 2H), 2.85-2.79 (m, 1H), 2.05-2.04 (m, 1H), 1.94-1.89 (m, 3H), 1.88-1.64 (m, 2H), 1.26-1.24 (m, 3H).

**Intermediate 30**

Ethyl rac-(1R*,2S*)-2-[(2,4-dimethoxybenzyl)amino]cyclopentanecarboxylate

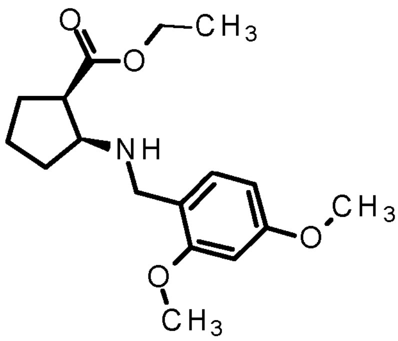

To a solution of ethyl rac-(1R*,2S*)-2-aminocyclopentanecarboxylate (see Intermediate 29, 40.0 g, 254 mmol, 1.00 eq) in THF (200 mL) was added Sodium triacetoxyborohydride (107 g, 508 mmol, 2.00 *eq*) and AcOH (15.9 g, 266 mmol, 1.05 *eq*) and 2,4-dimethoxybenzaldehyde (42.2 g, 254 mmol, 1.00 eq) at 0 °C. The mixture was stirred at 50 °C for 12 h. The reaction mixture was partitioned between water 500 mL and Ethyl acetate 600 mL. The organic phase was separated, washed with sodium hydrogencarbonate solution 600 mL (200 mL × 3) and brine 300 mL, dried over sodium sulfate, filtered and concentrated under reduced pressure to give the title compound (20.0 g, 65.1 mmol, 25.5% yield) as a brown oil.

LC-MS (Method: MS instrument type: SHIMADZU LCMS-2020, Column: Kinetex EVO C18 2.1 × 30mm, 5um, mobile phase A: 0.025% $NH_3$•$H_2O$ in Water (v/v) , B: Acetonitrile, gradient: 0.0 min 5% B→0.8 min 95% B→1.2 min 95% B→1.21 min 5% B→1.55 min 5%B, flow rate: 1.5 mL/min, oven temperature: 40°C; PDA detection: 220 nm & 254 nm.): $R_t$ = 1.091 min; MS (ESIpos): m/z = 308.3 [M+H]$^+$

**Intermediate 31**

Ethyl rac-(1R*,2S*)-2-[(2,4-dimethoxybenzyl)(3-ethoxy-3-oxopropanoyl)amino]cyclopentane-carboxylate

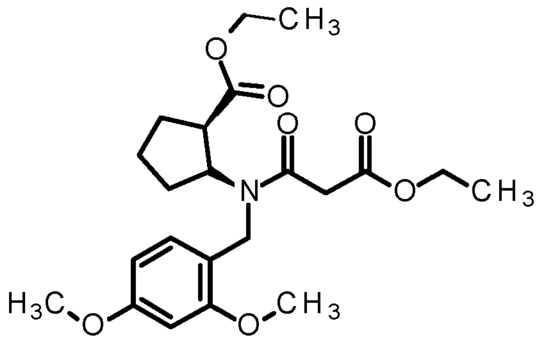

To a solution of ethyl rac-(1R*,2S*)-2-[(2,4-dimethoxybenzyl)amino]cyclopentanecarboxylate (see Intermediate 30, 20.0 g, 65.0 mmol, 1.00 eq) in DCM (100 mL) was added TEA (13.2 g, 130 mmol, 2.00 *eq*) and ethyl 3-chloro-3-oxopropanoate (10.7 g, 71.5 mmol, 1.10 eq) at 0 °C. The mixture was stirred at 25 °C for 12 hrs. The reaction mixture was quenched by addition $H_2O$ 100 mL at 25 °C, and extracted with DCM 150 mL (50 mL × 3). The combined organic layers were washed with brine 100 mL, dried over $Na_2SO_4$, filtered and concentrated under reduced pressure to give a residue. The residue was purified by flash silica gel chromatography (ISCO®; 80 g SepaFlash® Silica Flash Column, Eluent of 0-100% Ethyl acetate/Petroleum ether gradient @ 100 mL/min) (TLC Petroleum ether/Ethyl acetate = 1/1 Product $R_f$ = 0.5) to give the title compound (12.0 g, 28.4 mmol, 43.7% yield) as a yellow oil.

$^1$H-NMR (400 MHz, $CDCl_3$) δ [ppm]: 7.08-6.92 (m, 1H), 6.46-6.40 (m, 2H), 4.88-4.85 (m, 1H), 4.42-4.41 (m, 1H), 4.18-4.12 (m, 5H), 3.82-3.77 (m, 6H), 3.37-3.22 (m, 2H), 2.03-1.92 (m, 1H), 1.88-1.83 (m, 4H), 1.32-1.30 (m, 1H), 1.27-1.24 (m, 7H).

## Intermediate 32

Ethyl rac-(4aR*,7aS*)-1-(2,4-dimethoxybenzyl)-2,4-dioxooctahydro-1H-cyclopenta[b]pyridine-3-carboxylate

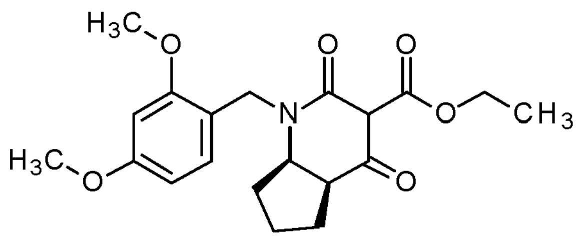

To a solution of ethyl rac-(1R*,2S*)-2-[(2,4-dimethoxybenzyl)(3-ethoxy-3-oxopropanoyl)amino]cyclopentanecarboxylate (see Intermediate 31, 16.0 g, 37.9 mmol, 1.00 eq) in DMF (300 mL) was added $Cs_2CO_3$ (24.7 g, 75.9 mmol, 2.00 eq). The mixture was stirred at 80 °C for 2 h. The reaction mixture was filtered and concentrated under reduced pressure to give the title compound (15.0 g, crude) as a yellow liquid (in DMF), and used to next step directly.

LC-MS (Method: MS instrument type: SHIMADZU LC-20AB, Column: Kinetex C18 LC Column 4.6X50mm,5um, mobile phase A: 0.025% NH3 H2O in water (v/v) , B: Acetonitrile (v/v), gradient: 0.00 min 10% B→2.40 min 80% B→3.30 min 80% B→3.31 min 10%B→4.00 min 10%B, flow rate: 1.5 mL/min, oven temperature: 50 °C; UV detection: 220 nm & 215 nm & 254 nm): $R_t$ = 0.780 min; MS (ESIpos): m/z = 367.3 $[M+H]^+$

## Intermediate 33

rac-(4aR*,7aS*)-1-(2,4-dimethoxybenzyl)tetrahydro-1H-cyclopenta[b]pyridine-2,4(3H,4aH)-dione

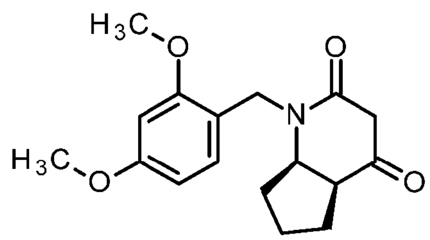

To a solution of ethyl rac-(4aR*,7aS*)-1-(2,4-dimethoxybenzyl)-2,4-dioxooctahydro-1H-cyclopenta[b]pyridine-3-carboxylate (see Intermediate 32, 15.0 g, 39.9 mmol, 1.00 eq) in DMF (200 mL) was added aq. HCl (1 M, 39.9 mL, 1 eq). The mixture was stirred at 90 °C for 1 h. The reaction mixture was partitioned between water 500 mL and ethyl acetate 800 mL. The organic phase was separated, washed with brine 600 mL (300 mL × 2), dried over $Na_2SO_4$, filtered and concentrated under reduced pressure to give a residue. The crude product was purified by reversed-phase HPLC (0.1% HCl condition). The residue was purified by flash silica gel chromatography (ISCO®; 120 g SepaFlash® Silica Flash Column, Eluent of 0-100% Ethyl acetate/Petroleum ether gradient @ 100 mL/min) to give the title compound (11.0 g, 35.3 mmol, 88.3% yield, 97.4% purity) as a yellow oil.

LC-MS (Method: MS instrument type: SHIMADZU LCMS-2020;, Column: Kinetex EVO C18 30*2.1mm,5um, mobile phase A: 0.0375% TFA in water (v/v), B: 0.01875% TFA in Acetonitrile (v/v), gradient: 0.00 min 5% B→ 0.80 min 95% B→1.20 min 95% B→1.21 min 5% B→1.55 min 5% B, flow rate: 1.5 mL/min, oven temperature: 50°C; UV detection: 220 nm & 254 nm): $R_t$ = 0.862 min; MS (ESIpos): m/z = 304.2 $[M+H]^+$

$^1$H-NMR (400 MHz, $CDCl_3$) δ [ppm]: 7.21-7.18 (m, 1H), 6.48-6.43 (m, 2H), 5.02 (d, *J*=14.4 Hz, 1H), 4.36 (d, J=14.8 Hz, 1H), 3.86-3.84 (m, 1H), 3.82 (s, 3H), 3.81 (s, 3H), 3.49-3.39 (m, 2H), 2.88-2.85 (m, 1H), 2.23-2.22 (m, 1H), 2.21-2.05 (m, 1H), 1.79-1.73 (m, 2H), 1.29-1.26 (m, 1H), 1.26-1.25 (m, 1H).

**Intermediate 34**

rac-(4aR*,7aS*)-1-[(2,4-dimethoxyphenyl)methyl]-4-hydroxy-2-oxo-N-phenyl-2,4a,5,6,7,7a-hexahydro-1H-cyclopenta[b]pyridine-3-carbothioamide

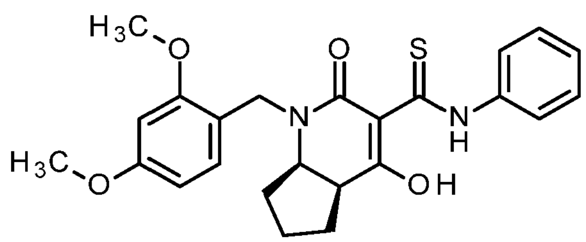

rac-(4aR*,7aS*)-1-(2,4-dimethoxybenzyl)tetrahydro-1H-cyclopenta[b]pyridine-2,4(3H,4aH)-dione (see Intermediate 33, 2.00 g) and isothiocyanatobenzene (CAS-RN:[103-72-0], 790 μL, 6.6 mmol;) were dissolved acetonitrile (8.2 mL) and were cooled down to 0 °C. 1,8-Diazabicyclo[5.4.0]undec-7-ene (CAS-RN:[6674-22-2], 1.6 mL, 11 mmol;) was added and the reaction mixture was stirred over night at room temperature. The reaction mixture was poured into a mixture of ice and hydrochloric acid (10 mL, 4 M). The aqueous layer was extracted with ethyl acetate three times. The combined organic layers were washed with brine, dried over anhydrous sodium sulfate, filtered and concentrated to give a residue. The crude product was purified by flash chromatography (Biotage 100 g silica ultra column, gradient: hexane / ethyl acetate 20-60% ethyl acetate) to provide the target compound: 2.64 g.

LC-MS (Method 2): $R_t$ = 1.09 min; MS (ESIpos): m/z = 439 $[M+H]^+$

$^1$H-NMR (400 MHz, DMSO-$d_6$) δ [ppm]: 1.155 (3.71), 1.173 (8.19), 1.190 (4.20), 1.501 (0.59), 1.520 (0.60), 1.548 (0.78), 1.563 (0.86), 1.584 (0.69), 1.603 (0.60), 1.624 (0.43), 1.941 (0.61), 1.960 (0.55), 1.988 (14.92), 2.035 (0.45), 2.050 (0.42), 2.092 (0.44), 2.107 (0.52), 2.124 (0.41), 2.332 (0.40), 2.518 (1.84), 2.523 (1.26), 3.276 (0.59), 3.288 (0.62), 3.309 (0.41), 3.656 (0.60), 3.746 (16.00), 3.765 (1.69), 3.789 (0.83), 3.803 (13.37), 3.818 (1.40), 4.000 (1.10),

4.017 (3.25), 4.035 (3.15), 4.053 (0.99), 4.290 (1.19), 4.328 (1.36), 4.770 (1.53), 4.808 (1.32), 5.758 (10.51), 6.482 (1.07), 6.488 (1.23), 6.503 (1.12), 6.509 (1.34), 6.575 (2.34), 6.581 (2.11), 7.098 (1.79), 7.119 (1.68), 7.293 (0.51), 7.299 (0.63), 7.306 (0.74), 7.313 (0.72), 7.321 (0.60), 7.328 (0.51), 7.401 (0.92), 7.418 (0.84), 7.439 (3.07), 7.447 (3.30), 7.453 (6.67), 14.813 (1.79), 16.816 (4.30).

**Intermediate 35**

rac-(4aR*,7aS*)-4-{[(2-aminopyridin-4-yl)methyl]amino}-1-[(2,4-dimethoxyphenyl)methyl]-2-oxo-N-phenyl-2,4a,5,6,7,7a-hexahydro-1H-cyclopenta[b]pyridine-3-carbothioamide

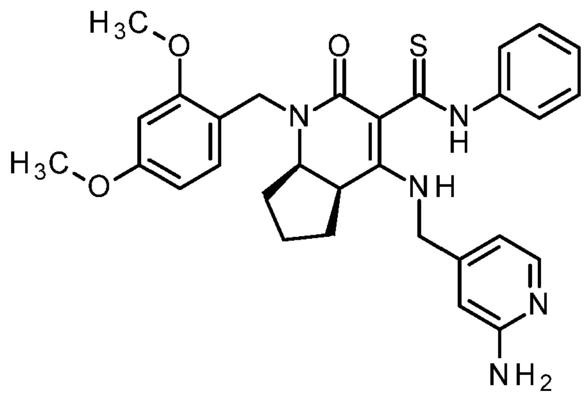

4-(aminomethyl)pyridin-2-amine (CAS-RN:[199296-51-0], 1.05 g) was dried by azeotropic evaporation with toluene/trichloromethane. To a mixture of rac-(4aR*,7aS*)-1-(2,4-dimethoxybenzyl)tetrahydro-1H-cyclopenta[b]pyridine-2,4(3H,4aH)-dione (see Intermediate 34, 2.50 g), 4-(aminomethyl)pyridin-2-amine (CAS-RN:[199296-51-0], 1.05 g) in 1,2-dimethoxyethane (8.0 mL) trimethylsilyl N-(trimethylsilyl)ethanimidate (2.8 mL, 11 mmol) was added at 0 °C. Then the mixture was stirred at 80 °C in a microwave tube under argon over night. The mixture was diluted in ethyl acetate and washed with brine three times. The organic layer was dried using a waterresistant filter and concentrated under reduced pressure. The crude product was purified by flash chromatography (28 g column, aminophase and 50 g silica ULTRA, gradient: dichloromethane / ethanol 0%-5% ethanol) to give 1.71 g of the target compound.

LC-MS (Method 1): $R_t$ = 1.11 min; MS (ESIneg): m/z = 542 $[M-H]^-$

$^1$H-NMR (400 MHz, DMSO-$d_6$) $\delta$ [ppm]: -0.002 (4.89), 0.002 (0.42), 0.010 (12.11), 1.613 (0.84), 1.627 (0.77), 2.066 (0.50), 2.518 (0.77), 2.523 (0.52), 3.233 (0.68), 3.330 (13.15), 3.423 (0.50), 3.734 (16.00), 3.740 (1.67), 3.746 (0.43), 3.769 (0.70), 3.779 (0.71), 3.789 (1.61), 3.807 (0.41), 4.404 (0.54), 4.444 (0.69), 4.573 (0.61), 4.589 (0.72), 4.595 (0.79), 4.612 (1.25), 4.653 (0.71), 5.292 (0.69), 5.758 (2.27), 6.000 (2.28), 6.387 (1.58), 6.451 (0.99), 6.455 (0.90), 6.464 (1.00), 6.468 (0.92), 6.483 (0.77), 6.488 (0.73), 6.504 (0.80), 6.509 (0.79), 6.554 (2.06), 6.560 (1.76), 7.000 (0.89), 7.021 (0.80), 7.168 (0.50), 7.186 (1.09), 7.201 (0.40), 7.204 (0.68), 7.207 (0.40), 7.336 (1.17), 7.341 (0.53), 7.348 (0.50), 7.357 (2.05), 7.367 (0.42), 7.371 (0.55), 7.376 (1.65), 7.423 (1.78), 7.441 (1.40), 7.863 (1.47), 7.875 (1.40), 13.886 (0.40), 14.926 (0.94).

**Intermediate 36**

rac-(5aR*,8aS*)-2-(2-aminopyridin-4-yl)-3-anilino-5-[(2,4-dimethoxyphenyl)methyl]-5,5a,6,7,8,8a-hexahydrocyclopenta[b]pyrrolo[2,3-d]pyridin-4(1H)-one

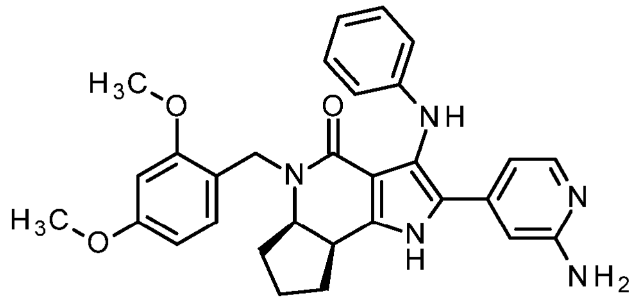

rac-(4aR*,7aS*)-4-{[(2-aminopyridin-4-yl)methyl]amino}-1-[(2,4-dimethoxyphenyl)methyl]-2-oxo-N-phenyl-2,4a,5,6,7,7a-hexahydro-1H-cyclopenta[b]pyridine-3-carbothioamide (see Intermediate 35, 1.00 g) was suspended in 10 mL methanol and treated with trifluoroacetic acid (140 µL, 1.8 mmol) and hydrogen peroxide (380 µL, 30 % purity, 3.7 mmol). The reaction mixture was stirred at 100 °C for 2 hours in a microwave tube. To the reaction mixture saturated aqueous sodium thiosulfate-solution, concentrated aqueous potassium carbonate solution and ethyl acetate was added. The aqueous layer was extracted with ethyl acetate three times. The combined organic layers were dried using a waterresistant filter and concentrated under reduced pressure The crude product was purified by flash chromatography (50 g column, silica ULTRA; dichloromethane / methanol 0%-50%) to provide the target compound: 327 mg.

LC-MS (Method 2): $R_t$ = 1.17 min; MS (ESIpos): m/z = 510 $[M+H]^+$

$^1$H-NMR (400 MHz, DMSO-$d_6$) δ [ppm]: 1.035 (2.11), 1.053 (4.32), 1.070 (1.94), 1.466 (0.40), 1.486 (0.57), 1.502 (0.83), 1.523 (0.79), 1.539 (0.60), 1.982 (0.45), 2.000 (0.52), 2.014 (0.56), 2.047 (0.45), 2.518 (1.38), 2.522 (0.95), 3.422 (0.92), 3.435 (0.96), 3.440 (1.01), 3.452 (1.23), 3.457 (0.47), 3.463 (0.63), 3.470 (0.81), 3.726 (16.00), 3.737 (1.26), 3.782 (12.15), 3.805 (0.77), 3.825 (0.89), 4.081 (1.08), 4.119 (1.13), 4.343 (0.57), 4.355 (1.09), 4.369 (0.53), 4.849 (1.14), 4.887 (1.04), 5.651 (2.47), 5.758 (3.80), 6.447 (1.07), 6.453 (1.19), 6.468 (1.10), 6.474 (1.26), 6.547 (2.95), 6.552 (3.88), 6.571 (2.41), 6.573 (2.05), 6.582 (1.98), 6.584 (2.00), 6.597 (0.52), 6.600 (0.74), 6.618 (1.37), 6.637 (0.72), 6.688 (1.37), 6.692 (1.23), 6.702 (1.28), 6.706 (1.24), 7.006 (1.70), 7.024 (2.13), 7.027 (2.08), 7.035 (1.92), 7.041 (0.68), 7.045 (1.38), 7.057 (1.59), 7.393 (2.56), 7.717 (1.74), 7.730 (1.64), 11.361 (1.43).

## Intermediate 37

(2RS)-N-(4-{(cis)-3-anilino-5-[(2,4-dimethoxyphenyl)methyl]-4-oxo-1,4,5,5a,6,7,8,8a-octahydrocyclopenta[b]pyrrolo[2,3-d]pyridin-2-yl}pyridin-2-yl)-4,4-difluoro-2-(4-fluorophenyl)butanamide (Mixture of 4 Stereoisomers)

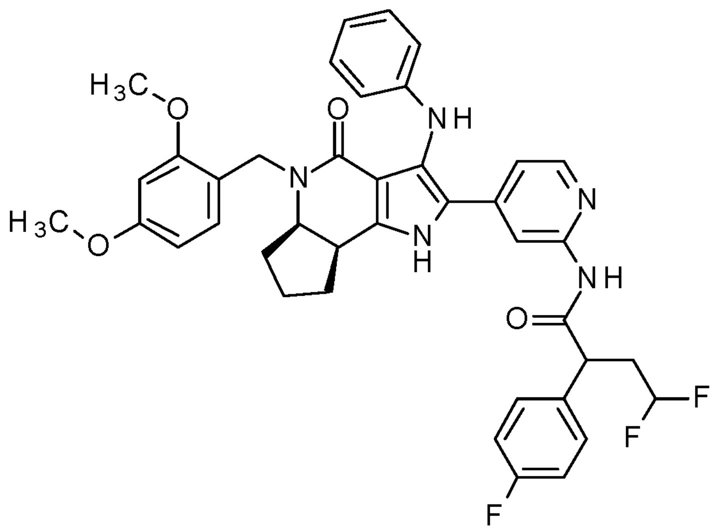

rac-(5aR*,8aS*)-2-(2-aminopyridin-4-yl)-3-anilino-5-[(2,4-dimethoxyphenyl)methyl]-5,5a,6,7,8,8a-hexahydrocyclopenta[b]pyrrolo[2,3-d]pyridin-4(1H)-one (see Intermediate 36, 406 mg), 4,4-difluoro-2-(4-fluorophenyl)butanoic acid

(Racemate) (CAS-RN:[1538957-14-0], 435 mg, 1.99 mmol), N,N-diisopropylethylamine (830 μL, 4.8 mmol) and PYBOP (CAS-RN:[128625-52-5], 1.24 g, 2.39 mmol) were dissolved in 1.3 mL DMA and stirred at room temperature under argon atmosphere for two days. To the reaction mixture aqueous saturated sodium hydrogencarbonate solution, water and ethyl acetate was added. The aqueous layer was extracted with ethyl acetate two times. The combined organic layers were washed with brine three times, dried using a waterresistant filter and concentrated under reduced pressure. The crude product was purified by flash chromatography (25 g silica ULTRA column and 28 g column aminophase; gradient: dichloromethane / ethanol 0%-25% ethanol) to provide the target compound: 429 mg.

LC-MS (Method 2): $R_t$ = 1.50 min; MS (ESIpos): m/z = 711 $[M+H]^+$

$^1$H-NMR (400 MHz, DMSO-d6) δ [ppm]: 1.481 (0.50), 1.501 (0.81), 1.511 (0.87), 1.517 (0.94), 1.529 (0.87), 1.546 (0.60), 1.993 (0.51), 2.005 (0.44), 2.011 (0.54), 2.023 (0.41), 2.078 (0.40), 2.176 (0.56), 2.184 (0.56), 2.204 (0.49), 2.211 (0.50), 2.218 (0.47), 2.327 (0.45), 2.518 (1.52), 2.523 (0.99), 2.664 (0.54), 2.669 (0.65), 2.673 (0.54), 3.476 (0.52), 3.485 (0.64), 3.495 (0.53), 3.726 (16.00), 3.785 (14.03), 3.825 (0.78), 3.845 (0.58), 3.850 (0.51), 4.102 (1.21), 4.140 (1.74), 4.857 (1.23), 4.895 (1.13), 5.919 (0.44), 5.951 (0.45), 6.450 (1.16), 6.456 (1.29), 6.471 (1.18), 6.477 (1.39), 6.535 (2.31), 6.551 (3.73), 6.556 (4.35), 6.591 (0.59), 6.594 (0.57), 6.609 (1.16), 6.612 (1.10), 6.627 (0.64), 6.630 (0.61), 6.984 (1.78), 7.003 (2.33), 7.004 (2.27), 7.023 (1.44), 7.041 (2.01), 7.062 (1.82), 7.148 (1.38), 7.152 (1.43), 7.162 (2.26), 7.166 (1.74), 7.183 (2.60), 7.204 (1.46), 7.411 (0.85), 7.416 (0.42), 7.427 (1.43), 7.433 (1.22), 7.441 (1.26), 7.447 (1.30), 7.463 (0.76), 7.561 (1.56), 7.568 (1.54), 8.025 (1.86), 8.038 (1.76), 8.165 (1.68), 10.614 (1.70), 11.601 (1.56).

**Intermediate 38**

4-hydroxy-1-methyl-2-oxo-N-phenyl-1,2,5, 6-tetrahydropyridine-3-carbothioam ide

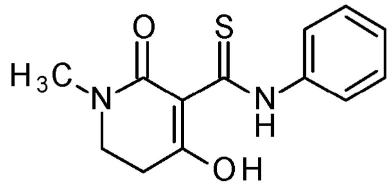

To an ice-cooled mixture of 1-methylpiperidine-2,4-dione (CAS-RN:[118263-97-1], 5.00 g, 39.3 mmol) and isothiocyanatobenzene (CAS-RN:[103-72-0], 4.7 mL, 39 mmol) in acetonitrile (53 mL) was slowly added DBU (9.4 mL, 63 mmol) and the mixture was stirred at room temperature for 16 h. The reaction was poured into ice-water containing concentrated hydrochloric acid (10 mL). The mixture was extracted with ethyl acetate. The organic phase was washed with saturated sodium chloride solution, dried (sodium sulfate), filtered and the solvent was removed in vacuum. Silicagel chromatography (20 - 60 % ethyl acetate / hexane) gave 9.84 g of the title compound as a crude product, that was used without further purification.

LC-MS (Method 1): $R_t$ = 1.23 min; MS (ESIneg): m/z = 253.3 $[M-H]^-$
$^1$H-NMR (400 MHz, DMSO-$d_6$) δ [ppm]: 1.985 (0.63), 1.987 (0.66), 2.667 (0.49), 2.803 (2.63), 2.820 (4.96), 2.838 (2.74), 2.962 (16.00), 3.099 (0.63), 3.332 (9.71), 3.333 (10.46), 3.451 (2.85), 3.469 (5.14), 3.487 (2.64), 5.756 (1.37), 5.758 (1.41), 7.290 (1.30), 7.304 (1.86), 7.309 (1.89), 7.318 (1.34), 7.418 (1.98), 7.440 (7.89), 7.452 (12.48), 14.570 (2.62), 16.485 (4.87).

**Intermediate 39**

4-{[(2-aminopyridin-4-yl)methyl]amino}-1-methyl-2-oxo-N-phenyl-1,2,5,6-tetrahydropyridine-3-carbothioamide

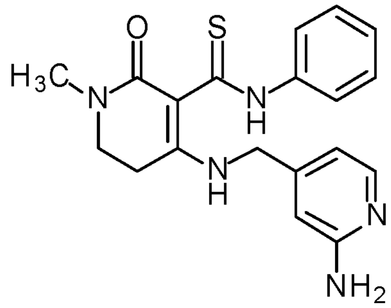

A stirred solution of 4-hydroxy-1-methyl-2-oxo-N-phenyl-1,2,5,6-tetrahydropyridine-3-carbothioamide (see Intermediate 38, 10.6 g, 40.6 mmol) and 4-(aminomethyl)pyridin-2-amine (CAS-RN:[199296-51-0], 27.0 g, 74 % purity, 162 mmol) in DMA (110 mL) distributed to 8 20-mL-microwave tubes was heated to 130° C for 1 h. A half-saturated solution of potassium carbonate was added and the mixture was extracted with ethyl acetate. The organic phase was washed with half-saturated sodium chloride solution, dried (sodium sulfate), filtered and the solvent was removed in vacuum. Silicagel chromatography gave a solid that was triturated with a dichloromethane to give 4.10 g of the title compound as a crude product, that was used without further purification.

LC-MS (Method 1): $R_t$ = 0.83 min; MS (ESIpos): m/z = 368.6 $[M+H]^+$
$^1$H-NMR (400 MHz, DMSO-$d_6$) $\delta$ [ppm]: 1.154 (0.48), 1.172 (1.01), 1.190 (0.51), 1.988 (1.85), 2.518 (0.93), 2.523 (0.61), 2.716 (1.34), 2.734 (2.54), 2.750 (1.45), 2.935 (13.17), 3.304 (1.58), 3.321 (2.91), 3.333 (16.00), 4.017 (0.40), 4.557 (2.74), 4.572 (2.70), 6.003 (3.82), 6.332 (2.92), 6.402 (1.62), 6.405 (1.48), 6.416 (1.64), 6.419 (1.51), 7.166 (0.75), 7.184 (1.76), 7.203 (1.12), 7.339 (1.92), 7.359 (3.50), 7.373 (0.83), 7.378 (2.63), 7.425 (3.50), 7.443 (2.24), 7.859 (2.41), 7.872 (2.33), 13.631 (0.80), 14.697 (1.88).

**Intermediate 40**

2-(2-aminopyridin-4-yl)-3-anilino-5-methyl-1,5,6,7-tetrahydro-4H-pyrrolo[3,2-c]pyridin-4-one

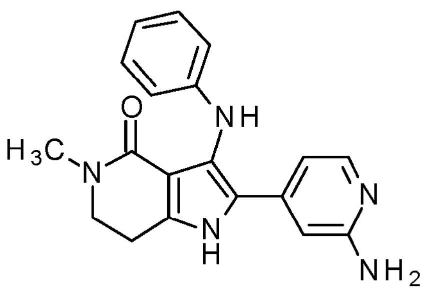

A stirred solution of 4-{[(2-aminopyridin-4-yl)methyl]amino}-1-methyl-2-oxo-N-phenyl-1,2,5,6-tetrahydropyridine-3-carbothioamide (see Intermediate 39, 1.10 g, 2.99 mmol) and hydrogen peroxide (30 % in water, 610 μL, 6.0 mmol) in methanol (14 mL) was heated in a microwave tube to 90° C for 2 h. An aqueous solution of disodium sulfurothioate and a half-saturated solution of potassium carbonate were added and the mixture was extracted with ethyl acetate. The organic phase was washed with half-saturated sodium chloride solution, dried (sodium sulfate), filtered and the solvent was removed in vacuum. Aminophase-silicagel chromatography followed by silicagel chromatography gave 535 mg of the title compound.

LC-MS (Method 1): $R_t$ = 0.73 min; MS (ESIpos): m/z = 334.5 $[M+H]^+$

$^1$H-NMR (400 MHz, DMSO-$d_6$) $\delta$ [ppm]: 2.518 (1.58), 2.523 (1.01), 2.853 (16.00), 2.882 (1.57), 2.899 (3.32), 2.916 (1.67), 3.377 (0.59), 3.507 (1.84), 3.524 (3.76), 3.542 (1.64), 5.657 (3.93), 5.759 (7.76), 6.547 (5.28), 6.549 (6.04), 6.568 (3.28), 6.570 (2.79), 6.595 (0.62), 6.597 (1.01), 6.600 (0.63), 6.616 (1.96), 6.634 (1.08), 6.670 (1.91), 6.674 (1.83), 6.684 (1.88), 6.688 (1.78), 7.003 (2.43), 7.022 (3.07), 7.025 (3.05), 7.043 (2.03), 7.223 (3.78), 7.720 (2.48), 7.733 (2.39), 11.520 (1.76).

**Intermediate 41**

N-[4-(3-anilino-5-methyl-4-oxo-4,5,6,7-tetrahydro-1H-pyrrolo[3,2-c]pyridin-2-yl)pyridin-2-yl]-2-(4-fluorophenyl)propanamide (Racemate)

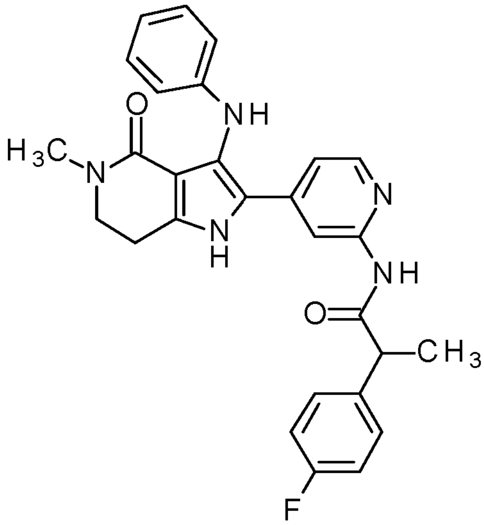

To a stirred solution of 2-(2-aminopyridin-4-yl)-3-anilino-5-methyl-1,5,6,7-tetrahydro-4H-pyrrolo[3,2-c]pyridin-4-one (see Intermediate 40, 10.0 g, 30.0 mmol) in DMA (200 mL) was added N,N-diisopropylethylamine (31 mL, 180 mmol), racemic 2-(4-fluorophenyl)propanoic acid (10.1 g, 60.0 mmol) and PyBOP (46.8 g, 90.0 mmol). The mixture was stirred at room temperature for 24 h. An aqueous solution of sodium bicarbonate was added, the mixture was stirred for 15 minutes and the mixture was extracted with ethyl acetate. The organic phase was washed with saturated sodium chloride solution, dried (sodium sulfate), filtered and the solvent was removed in vacuum. Silicagel chromatography gave a solid that was triturated with a dichloromethane and then with ethanol to give 11.9 g of the title compound.

LC-MS (Method 1): $R_t$ = 1.04 min; MS (ESIneg): m/z = 482.5 $[M-H]^-$

$^1$H-NMR (400 MHz, DMSO-$d_6$) δ [ppm] 1.39 (d, 3 H), 2.86 (s, 3 H), 2.92 (t, 2 H), 3.54 (t, 2 H), 4.01 (q, 1 H), 6.52 - 6.58 (m, 2 H), 6.61 (t, 1 H), 7.01 (dd, 2 H), 7.06 - 7.24 (m, 3 H), 7.31 - 7.50 (m, 3 H), 8.02 (d, 1 H), 8.20 (d, 1 H), 10.50 (s, 1 H), 11.74 (s, 1 H).

**Intermediate 42**

(2S)-N-[4-(3-anilino-5-methyl-4-oxo-4,5,6,7-tetrahydro-1H-pyrrolo[3,2-c]pyridin-2-yl)pyridin-2-yl]-2-(4-fluorophenyl)propanamide (Enantiomer 1)

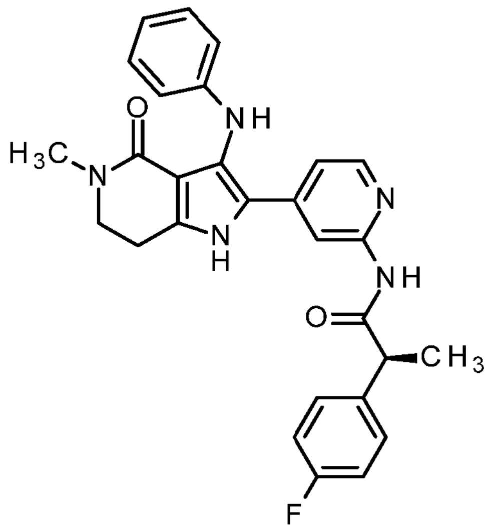

Racemic N-[4-(3-anilino-5-methyl-4-oxo-4,5,6,7-tetrahydro-1H-pyrrolo[3,2-c]pyridin-2-yl)pyridin-2-yl]-2-(4-fluorophenyl)propanamide (see Intermediate 41, 2.22 g, 4.58 mmol) was separated into the single enantiomers (see Intermediate 42 and Intermediate 43) via preparative, chiral HPLC.
Instrument: Labomatic HD5000, Labocord-5000; Gilson GX-241, Labcol Vario 4000,
Column: Chiralpak IA 5μ 250x30mm;
Eluent: Methanol + 0.1 Vol-% diethylamine (99%) / Ethanol 50:50%;
Flow: 40.0 mL / min;
Detection: UV: 254 nm
Injected solution: 2210 mg racemic N-[4-(3-anilino-5-methyl-4-oxo-4,5,6,7-tetrahydro-1H-pyrrolo[3,2-c]pyridin-2-yl)pyridin-2-yl]-2-(4-fluorophenyl)propanamide dissolved in 22 mL DMSO Injected volume: 22 x 1 mL.
Analytical Chiral HPLC (method Intermediate 42):
Instrument: Agilent 1260 HPLC; Column: Chiralpak IA 3μ 100x4.6; Eluent A: Methanol + 0.1% diethylamin; Eluent B: Ethanol; isokratic: 50%A+50%B; Flow: 1.4 mL/min; Temperature: 25°C; UV: 254 nm;

| | Retention time in min | e.e. [%] | yield | Optical rotation $[\alpha]_D$ |
|---|---|---|---|---|
| Intermediate 42 Enantiomer 1 Peak 1; (2S)-Enantiomer | 5.6-7.7 | > 99.8% | 1.07g | + 165.8° (from solution in DMSO, c = 2.2 mg/mL) |
| Intermediate 43 Enantiomer 2 Peak 2; | 9.0 - 14.3 | 98.8% | 996 mg | - 178.4° |
| (2R)-Enantiomer | | | | (from solution in DMSO, c = 13.2 mg/mL) |

Isolated Enantiomer 1: 1.07g
Analytical Chiral HPLC (method see Intermediate 42): $R_t$ = 2.20 min (e.e.: > 99.8 %)
$[a]_D$ = + 165,8° (from solution in DMSO, c = 2.2 mg/mL)
LC-MS (Method 1): $R_t$ = 1.07 min; MS (ESIneg): m/z = 482.5 $[M-H]^-$
$^1$H-NMR (400 MHz, DMSO-$d_6$) δ [ppm]: 1.376 (6.33), 1.394 (6.55), 2.518 (1.71), 2.523 (1.11), 2.863 (16.00), 2.903 (1.59), 2.920 (3.45), 2.938 (1.75), 3.385 (0.46), 3.518 (1.95), 3.535 (3.99), 3.553 (1.72), 3.998 (1.36), 4.015 (1.33), 5.758 (14.38), 6.538 (3.29), 6.558 (3.46), 6.560 (2.90), 6.594 (1.01), 6.613 (2.12), 6.631 (1.14), 6.988 (2.61), 7.007 (3.30), 7.009 (3.27), 7.027 (2.05), 7.103 (2.07), 7.108 (2.00), 7.117 (1.96), 7.121 (2.10), 7.133 (2.41), 7.138 (0.85), 7.149 (0.97), 7.155 (5.04), 7.161 (1.08), 7.172 (0.85), 7.177 (2.73), 7.369 (4.17), 7.390 (2.44), 7.395 (1.05), 7.404 (2.70), 7.412 (2.39), 7.421 (0.95), 7.426 (2.10), 8.009 (2.77), 8.024 (2.63), 8.201 (2.63), 10.498 (3.03), 11.740 (1.69).

**Intermediate 43**

(2R)-N-[4-(3-anilino-5-methyl-4-oxo-4,5,6,7-tetrahydro-1H-pyrrolo[3,2-c]pyridin-2-yl)pyridin-2-yl]-2-(4-fluorophenyl)propanamide (Enantiomer 2)

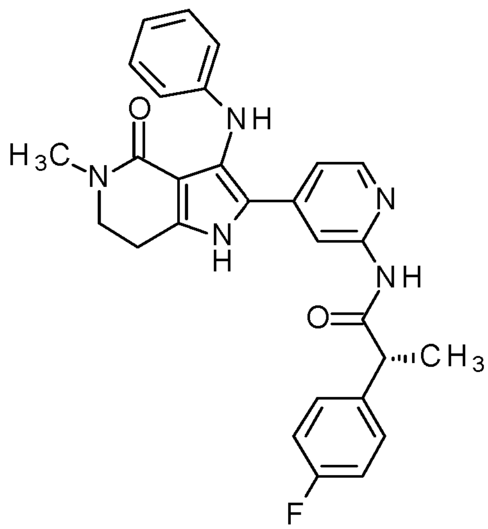

Obtained Enantiomer 2: 996 mg

Analytical Chiral HPLC (method see Intermediate 42): $R_t$ = 4.16 min (e.e.: 98.8 %)

$[a]_D$ = - 178.4° (from solution in DMSO, c = 2.1 mg/mL)

LC-MS (Method 1): $R_t$ = 1.04 min; MS (ESIneg): m/z = 482.4 $[M-H]^-$

$^1$H-NMR (400 MHz, DMSO-$d_6$) δ [ppm]: 1.036 (0.61), 1.054 (1.28), 1.071 (0.59), 1.378 (6.48), 1.395 (6.62), 2.518 (0.61), 2.539 (1.18), 2.863 (16.00), 2.903 (1.69), 2.921 (3.61), 2.938 (1.86), 3.518 (2.02), 3.535 (4.13), 3.552 (1.81), 3.983 (0.42), 4.000 (1.43), 4.017 (1.41), 4.034 (0.40), 5.758 (4.10), 6.541 (3.48), 6.560 (3.64), 6.595 (1.04), 6.613 (2.16), 6.632 (1.17), 6.989 (2.54), 7.008 (3.44), 7.010 (3.47), 7.029 (2.11), 7.106 (1.98), 7.110 (1.99), 7.120 (1.97), 7.124 (2.18), 7.133 (2.33), 7.138 (0.88), 7.150 (1.07), 7.155 (4.90), 7.161 (1.10), 7.173 (0.89), 7.178 (2.76), 7.372 (4.38), 7.384 (0.43), 7.392 (2.49), 7.397 (1.15), 7.405 (2.76), 7.414 (2.52), 7.423 (0.98), 7.428 (2.17), 8.012 (2.93), 8.025 (2.78), 8.204 (2.86), 10.500 (3.25), 11.742 (1.78).

**Intermediate 44**

N-[4-(3-anilino-5-methyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate)

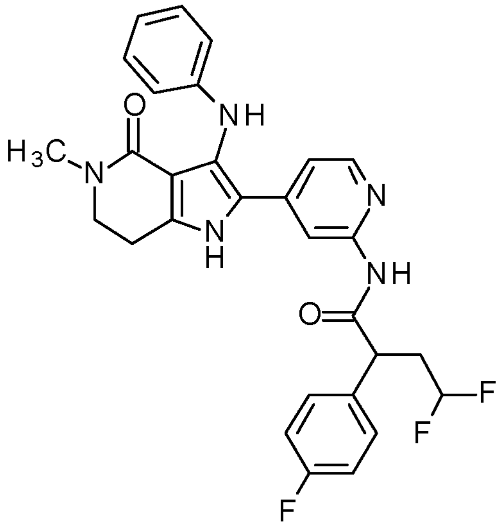

To a stirred solution of 2-(2-aminopyridin-4-yl)-3-anilino-5-methyl-1,5,6,7-tetrahydro-4H-5 pyrrolo[3,2-c]pyridin-4-one (see Intermediate 40, 200 mg) in DMA (1.0 mL) was added N,N-diisopropylethylamine (CAS-RN:[7087-68-5], 630 μl, 3.6 mmol), (rac)-4,4-difluoro-2-(4fluorophenyl)butanoic acid (CAS-RN:[1538957-14-0], 327 mg, 1.50 mmol) and Py-BOP (CAS-RN:[128625-52-5], 937 mg, 1.80 mmol). The mixture was stirred at room temperature for 16 h. An aqueous solution of sodium bicarbonate was added, the mixture was stirred for 5 minutes and the mixture was 10 extracted with ethyl acetate. The organic phase was washed with saturated sodium chloride solution, dried (sodium sulfate), filtered and the solvent was removed in vacuum. Silicagel chromatography followed by aminophase-silicagel chromatography gave a solid that was triturated with a mixture of dichloromethane and hexane to give 198 mg (56 % yield) of the title compound.

LC-MS (Method 2): $R_t$ = 1.24 min; MS (ESIpos): m/z = 534 $[M+H]^+$

$^1$H NMR (400 MHz, DMSO-$d_6$) δ [ppm]: 2.10 - 2.29 (m, 1 H), 2.58 - 2.78 (m, 1 H), 2.87 (s, 3 H), 2.93 (t, 2 H), 3.54 (t, 2 H), 4.14 (dd, 1 H), 5.93 (tt, 1 H), 6.54 (dd, 2 H), 6.61 (t, 1 H), 7.01 (dd, 2 H), 7.13 (dd, 1 H), 7.15 - 7.22 (m, 2 H), 7.39 (s, 1 H), 7.41 - 7.47 (m, 2 H), 8.03 (d, 1 H), 8.16 (s, 1 H), 10.63 (s, 1 H), 11.74 (s, 1 H).

## Intermediate 45

ethyl-3-[(3-ethoxy-3-oxopropanoyl)(methyl)amino]butanoate (Racemate)

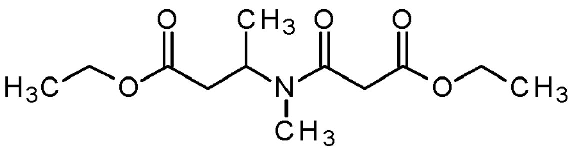

A solution of ethyl-3-(methylamino)butanoate (CAS-RN:[68384-70-3], 13.5 g, 87.4 mmol) in DMC (116 mL) placed in a three neck round bottom flask, cooled to 0 °C, N,N-diisopropylethylamine (CAS-RN:[7087-68-5], 17 mL, 1.1 eq.) and 4-dimethylaminopyridine (CAS-RN:[1122-58-3], 1.07 g, 0.1 eq.) was added. Then ethyl 3-chloro-3-oxopropanoate (12 mL, 1.1eq.) was slowly added so that the temperature of the reaction was kept below 15°C, stirred for 0.5 h at 0 °C and for 18 h at room temperature. Then HCl (2 M, 55 mL) was added, stirred for 15 min, extracted with dichloromethane, dried over sodium sulfate, concentrated under reduced pressure and purified by column chromatography (340 g Si-Biotage, gradient: ethyl acetate/hexane) 50-100% One obtained the title compound (4.6 g).

LC-MS (Method 2): $R_t$ = 0.86 min; MS (ESIpos): m/z = 260 $[M+H]^+$

$^1$H NMR (DMSO-$d_6$) δ [ppm]: 4.81 (sxm, 1H), 4.05-4.11 (m, 4H), 3.47 (d, 1H), 3.41 (d, 1H), 2.77 (s, 3H), 2.47 (d, 2H), 1.17-1.21 (m, 6H), 1.06 (d, 3H).

**Intermediate 46**

ethyl 4-hydroxy-1,2-dimethyl-6-oxo-2,3-dihydropyridine-5-carboxylate (Racemate)

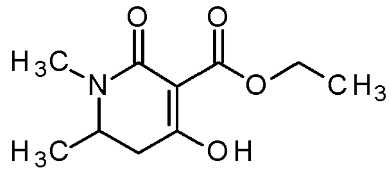

A solution of sodium ethylate (7.3 mL, 21% purity, 1.1eq.) was treated at 0°C with a solution of ethyl 3-[(3-ethoxy-3-oxopropanoyl)(methyl)amino]butanoate (Racemate) (see Intermediate 45, 4.60 g, 17.7mmol) in ethanol (35 mL), in a dropwise manner so that the temperature was kept below 5°C and stirred for 18 h at room temperature. Then the reaction mixture was adjusted to pH4 by the addition of HCl (2 M in water) extracted with dichloromethane dried, concentrated under reduced pressure and purified by chromatography (Si-Biotage 100 g, gradient: ethyl acetate / hexane 20-100%). One obtained the title compound (2.78 g).

LC-MS (Method 1): $R_t$ = 0.76 min; MS (ESIpos): m/z = 214 $[M+H]^+$
$^1$H-NMR (DMSO-$d_6$) $\delta$ [ppm]: 12.51 (br s, 1H), 4.18 (q, 2H), 3.60 (br s, 1H), 2.99 (br s, 2H), 2.82 (br s, 3H), 1.22 (m, 3H), 1.12 (t, 3H).

**Intermediate 47**

1,6-dimethylpiperidine-2,4-dione (Racemate)

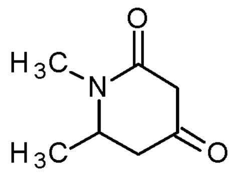

A solution of ethyl 4-hydroxy-1,2-dimethyl-6-oxo-2,3-dihydropyridine-5-carboxylate (Racemate) (see Intermediate 46, 2.77 g) in dichloromethane (20 mL) was treated with HCl (CAS-RN:[7647-01-0], 20 mL, 2.0 M), stirred for 15 min at room temperature. Then the reaction mixture was extracted with dichloromethane, the organic phases were dried, concentrated under reduced pressure, solved in acetonitrile (30 mL) and water (4 mL), stirred for 2 h at reflux and concentrated under vacuum. One obtained the title compound (1.72g).

LC-MS (Method 1): $R_t$ = 0.45 min; MS (ESIneg): m/z = 140 $[M-H]^-$

$^1$H-NMR (DMSO-$d_6$) $\delta$ [ppm]: 3.78 (qd, 1H), 3.28 (d, 1H), 3.21 (d, 1H), 2.92 (s, 3H), 2.91 (m, 1H), 2.38 (m, 1H), 1.14 (d, 3H); keto- (78%) & enol-tautomer (22%): 10.33 (s, 1H), 4.86 (d, 1H), 3.56 (qd, 1H), 2.70-2.78 (m, 4H), 1.98-2.05 (m, 1H), 1.10 (d, 3H).

**Intermediate 48**

N-(2-fluorophenyl)-4-hydroxy-1,6-dimethyl-2-oxo-1,2,5,6-tetrahydropyridine-3-carbothioamide (Racemate)

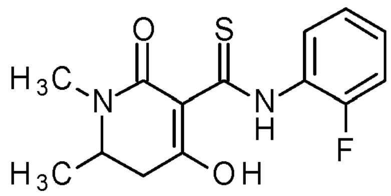

A solution of 1,6-dimethylpiperidine-2,4-dione (Racemate) (see Intermediate 47, 1.10g, 7.79mmol) and 1-fluoro-2-isothiocyanatobenzene (1.19 g, 1 eq.) in acetonitrile (20 mL) cooled with ice was treated with DBU (1.9 mL, 1.6 eq.), stirred for 16h at room temperature. Then the reaction was quenched in ice/water/HCl (4 mL, 4M) extracted with ethyl acetate, washed with brine, dried over sodium sulfat and concentrated under reduced pressure. After purification by Si-Biotage column (100 g, gradient: ethyl acetate / hexane, 20-40%) one obtained the title compound: 1.82 g.

LC-MS (Method 2): $R_t$ = 0.57 min; MS (ESIpos): m/z = 295 $[M+H]^+$

$^1$H-NMR (400 MHz, DMSO-$d_6$) δ [ppm]: 1.192 (5.13), 1.200 (1.43), 1.209 (5.29), 1.217 (1.26), 2.459 (1.00), 2.465 (1.05), 2.518 (1.50), 2.523 (1.00), 2.961 (16.00), 3.122 (2.49), 3.285 (0.74), 3.302 (0.84), 3.345 (0.89), 3.699 (0.40), 3.705 (0.46), 3.716 (0.61), 3.722 (0.62), 3.733 (0.43), 7.245 (0.46), 7.251 (0.64), 7.260 (0.43), 7.266 (0.82), 7.270 (0.72), 7.280 (0.42), 7.282 (0.48), 7.287 (0.68), 7.339 (0.43), 7.348 (1.09), 7.353 (0.98), 7.358 (0.93), 7.362 (0.70), 7.374 (1.71), 7.379 (1.54), 7.389 (0.49), 7.393 (0.46), 7.659 (0.51), 7.663 (0.53), 7.679 (0.97), 7.682 (0.94), 7.698 (0.48), 7.702 (0.46), 14.521 (1.28), 16.455 (4.39), 17.128 (1.02) tautomers (5 : 1).

**Intermediate 49**

4-{[(2-aminopyridin-4-yl)methyl]amino}-N-(2-fluorophenyl)-1,6-dimethyl-2-oxo-1,2,5,6-tetrahydropyridine-3-carbothioamide (Racemate)

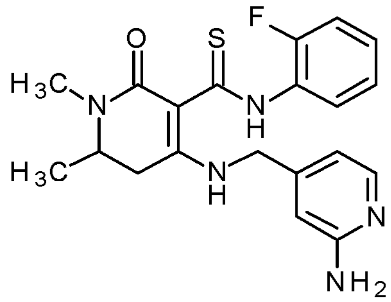

A mixture of of N-(2-fluorophenyl)-4-hydroxy-1,6-dimethyl-2-oxo-1,2,5,6-tetrahydropyridine-3-carbothioamide (Racemate) (see Intermediate 48, 0.200 g) and 4-(aminomethyl)pyridin-2-amine (0.165g) was heated to 120°C in a microwave for 2h. The crude product was taken-up in dichloromethane / methanol (4mL / 1mL) and purified by chromatography on silicagel (50 g, a) hexane / ethyl acetate 0-60%; b) ethyl acetate / methanol) gave 0.149 g of the title compound as a beige solid.

The formation of a less polar by-product: 3-(1,3-benzothiazol-2-yl)-4-hydroxy-1,6-dimethyl-5,6-dihydropyridin-2(1H)-one (as tautomers, 3 : 2) was observed under those conditions.

LC-MS (Method 2): $R_t$ = 1.08 min; MS (ESIpos): m/z = 400 $[M+H]^+$

$^1$H-NMR (400 MHz, DMSO-$d_6$) δ [ppm]: 0.798 (0.42), 0.815 (0.45), 0.822 (0.45), 0.905 (0.51), 1.061 (5.07), 1.078 (5.12), 2.518 (1.03), 2.523 (0.67), 2.680 (0.74), 2.687 (0.73), 2.722 (1.06), 2.729 (1.04), 2.868 (0.79), 2.883 (0.93), 2.916 (16.00), 3.584 (0.43), 3.591 (0.48), 3.600 (0.68), 3.607 (0.65), 3.616 (0.48), 4.584 (2.45), 4.598 (2.45), 5.758 (6.09), 6.006 (3.57), 6.340 (2.51), 6.401 (1.56), 6.404 (1.40), 6.414 (1.56), 6.418 (1.42), 7.163 (0.54), 7.174 (0.66), 7.185 (0.97), 7.190 (0.45), 7.195 (0.80), 7.205 (1.01), 7.247 (2.18), 7.256 (1.63), 7.259 (1.59), 7.263 (1.11), 7.270 (1.31), 7.275 (1.59), 7.278 (1.58), 7.663 (0.79), 7.682 (1.29), 7.684 (1.28), 7.702 (0.72), 7.858 (2.32), 7.871 (2.25), 13.820 (0.45), 13.834 (0.84), 13.848 (0.43), 14.697 (2.43).

**Intermediate 50**

2-(2-aminopyridin-4-yl)-3-(2-fluoroanilino)-5,6-dimethyl-1,5,6,7-tetrahydro-4H-pyrrolo[3,2-c]pyridin-4-one (Racemate)

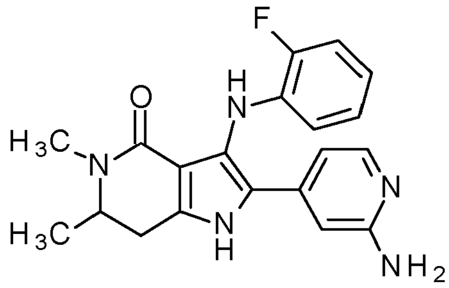

A suspension of 4-{[(2-aminopyridin-4-yl)methyl]amino}-N-(2-fluorophenyl)-1,6-dimethyl-2-oxo-1,2,5,6-tetrahydropyridine-3-carbothioamide (Racemate) (see Intermediate 49, 0.5g) in methanol (11 mL) was treated with hydrogen peroxide (0.26 mL, 30 % purity) stirred 2h at 90°C in the microwave, further hydrogen peroxide (0.12mL, 30 % purity) was added and stirred for 75 min at 90°C. Then the mixture was concentrated under reduced pressure, solved in dichloromethane / methanol (4 mL / 1 mL) and purified by chromatography (NH-column, 55 g, ethyl acetate / ethanol 0 - 25%). One obtained the title compound: 0.261 g.

LC-MS (Method 2): $R_t$ = 0.92 min; MS (ESIpos): m/z = 366 $[M+H]^+$

$^1$H-NMR (400 MHz, DMSO-$d_6$) δ [ppm]: 1.151 (5.41), 1.154 (6.76), 1.168 (5.49), 1.172 (10.61), 1.190 (4.64), 1.988 (16.00), 2.327 (0.43), 2.518 (1.93), 2.522 (1.20), 2.620 (0.96), 2.625 (0.98), 2.661 (1.24), 2.667 (1.30), 2.673 (0.48), 2.851 (14.15), 3.179 (0.78), 3.195 (0.91), 3.220 (0.78), 3.237 (0.75), 3.782 (0.49), 3.788 (0.56), 3.798 (0.78), 3.804 (0.77), 3.815 (0.56), 3.821 (0.48), 4.000 (1.25), 4.017 (3.79), 4.035 (3.76), 4.053 (1.21), 5.705 (3.76), 6.282 (0.65), 6.286 (0.71), 6.306 (1.30), 6.325 (0.71), 6.329 (0.69), 6.501 (2.64), 6.503 (2.74), 6.634 (0.48), 6.642 (2.44), 6.646 (2.32), 6.656 (2.43), 6.660 (2.34), 6.672 (0.51), 6.676 (0.46), 6.770 (0.91), 6.788 (1.38), 6.805 (0.61), 7.078 (0.81), 7.081 (0.83), 7.098 (0.80), 7.102 (0.81), 7.108 (0.85), 7.112 (0.82), 7.128 (0.76), 7.131 (0.71), 7.261 (1.96), 7.265 (1.95), 7.754 (2.35), 7.768 (2.25), 11.575 (1.75).

**Intermediate 51**

(+)-2-(2-aminopyridin-4-yl)-3-(2-fluoroanilino)-5,6-dimethyl-1,5,6,7-tetrahydro-4H-pyrrolo[3,2-c]pyridin-4-one (Enantiomer 1)

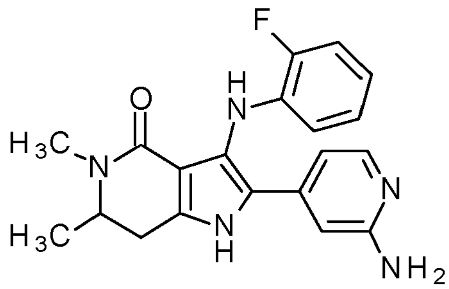

The racemic compound (see Intermediate 50, 680 mg) was separated into enantiomers by preparative chiral HPLC to give enantiomer 1 (see Intermediate 51, 260 mg, 100 % ee) and enantiomer 2 (see Intermediate 52, 307 mg, 98.8 % ee).

Preparative chiral HPLC method:

Instrument: PrepCon Labomatic HPLC; Column: YMC Cellulose SC 5 μ, 250x30; eluent A: hexane + 0.1 vol % diethylamine; eluent B: ethanol + 0.1 vol % diethylamine; isocratic: 80%A+20%B; flow: 50 mL/min; temperature: 25°C; UV: 254 nm

Analytical chiral HPLC method:

Instrument: Waters Alliance 2695; Column: YMC Cellulose SC 3 μ, 100x4.6; eluent A: hexane + 0.1 vol % diethylamine; eluent B: ethanol; isocratic: 80%A+20%B; flow: 1.4 mL/min; temperature: 25°C; UV: 254 nm

Analytical Chiral HPLC (method see Intermediate 51): $R_t$ = 3.42 min.

$[\alpha]_D$ = +50.1° (from solution in DMSO, c = 2.1 mg/mL)

$^1$H-NMR (400 MHz, DMSO-$d_6$): δ [ppm] = 11.58 (s, 1H), 7.76 (d, 1H), 7.26 (d, 1H), 7.11 (ddd, 1H), 6.83-6.76 (m, 1H), 6.69-6.61 (m, 2H), 6.50 (d, 1H), 6.35-6.27 (m, 1H), 5.71 (s, 2H), 3.80 (quind, 1H), 3.21 (dd, 1H), 2.85 (s, 3H), 2.64 (dd, 1H), 1.16 (d, 3H)

## Intermediate 52

(-)-2-(2-aminopyridin-4-yl)-3-(2-fluoroanilino)-5,6-dimethyl-1,5,6,7-tetrahydro-4H-pyrrolo[3,2-c]pyridin-4-one (Enantiomer 2)

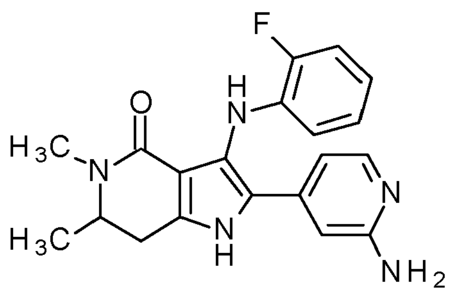

For the preparation of the racemic title compound see Intermediate 50. Separation of enantiomers by preparative chiral HPLC (method see Intermediate 51) gave the title compound (307 mg).

Analytical Chiral HPLC (method see Intermediate 51): $R_t$ = 4.87 min.

$[a]_D$ = -52.5° (from solution in DMSO, c = 2.0 mg/mL)

$^1$H-NMR (400 MHz, DMSO-$d_6$) δ [ppm]: 1.138 (0.43), 1.151 (5.54), 1.168 (5.57), 1.233 (0.45), 2.075 (0.50), 2.518 (1.59), 2.523 (1.07), 2.539 (3.32), 2.620 (0.97), 2.626 (1.01), 2.661 (1.26), 2.667 (1.34), 2.673 (0.45), 2.728 (1.25), 2.851 (16.00), 2.865 (0.46), 2.888 (1.56), 3.179 (0.82), 3.195 (0.95), 3.220 (0.83), 3.237 (0.79), 3.377 (0.44), 3.782 (0.50), 3.788 (0.56), 3.798 (0.80), 3.804 (0.79), 3.815 (0.57), 3.821 (0.49), 5.707 (3.80), 6.283 (0.68), 6.287 (0.75), 6.306 (1.34), 6.325 (0.73), 6.329 (0.73), 6.502 (2.67), 6.504 (2.78), 6.634 (0.45), 6.642 (2.45), 6.646 (2.28), 6.656 (2.58), 6.660 (2.41), 6.672 (0.57), 6.676 (0.50), 6.770 (0.97), 6.789 (1.43), 6.806 (0.63), 6.808 (0.62), 7.078 (0.84), 7.082 (0.86), 7.098 (0.85), 7.102 (0.83), 7.109 (0.90), 7.112 (0.89), 7.128 (0.79), 7.132 (0.75), 7.261 (2.00), 7.266 (2.03), 7.754 (2.39), 7.768 (2.31), 11.576 (1.82).

## Intermediate 53

(+)-(2RS)-4,4-difluoro-N-{4-[(6R or S)-3-(2-fluoroanilino)-5,6-dimethyl-4-oxo-4,5,6,7-tetrahydro-1H-pyrrolo[3,2-c]pyridin-2-yl]pyridin-2-yl}-2-(4-fluorophenyl)butanamide (2 Diastereoisomers #1)

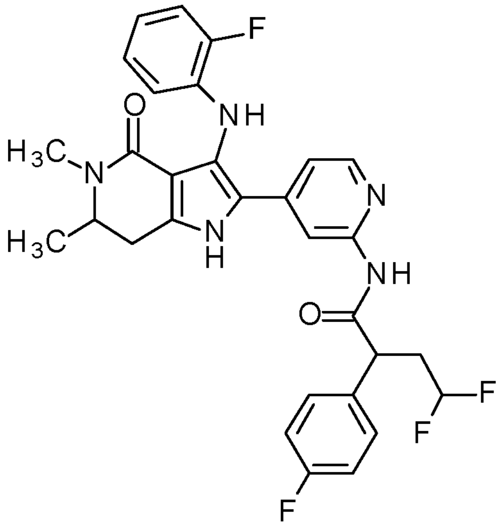

A solution of (+)-2-(2-aminopyridin-4-yl)-3-(2-fluoroanilino)-5,6-dimethyl-1,5,6,7-tetrahydro-4H-pyrrolo[3,2-c]pyridin-4-one (Enantiomer 1) (see Intermediate 51, 40 mg) in DMA (2.33 mL) was treated with N,N-diisopropylethylamine (CAS-RN:[7087-685], 0.20 mL, 6 eq), 4,4-difluoro-2-(4-fluorophenyl)butanoic acid (Racemate) (CAS-RN:[1538957-14-0], 47.8 mg, 2 eq), and benzotriazol-1-yloxytripyrrolidinophosphonium hexafluorophosphate (CAS-RN:[128625-52-5], 0.285 g, 5 eq) and stirred for 18 h at room temperature. Then a sodium bicarbonate solution in water was added, and the mixture was extracted with ethyl acetate. The organic phase was washed with saturated sodium chloride solution, dried (sodium sulfate), filtered, concentrated under reduced pressure, and purified by column chromatography (column silica gel, 50g, gradient dichloromethane/ethanol, 0-10 %) to give 42 mg of the title compound.

$[\alpha]_D$ = +29.9° (from solution in DMSO, c = 2.1 mg/mL)

LC-MS (Method 2): $R_t$ = 1.28 min; MS (ESIpos): m/z = 566 $[M+H]^+$

$^1$H-NMR (400 MHz, DMSO-$d_6$) $\delta$ [ppm]: 1.220 (6.58), 1.238 (12.52), 1.255 (4.54), 1.298 (0.81), 2.053 (16.00), 2.388 (0.71), 2.392 (1.01), 2.396 (0.72), 2.583 (3.72), 2.588 (2.56), 2.725 (1.26), 2.729 (1.53), 2.734 (1.45), 2.738 (0.98), 2.743 (0.54), 2.767 (1.03), 2.928 (10.26), 3.262 (0.47), 3.278 (0.54), 3.303 (0.45), 3.316 (0.44), 3.866 (0.44), 3.871 (0.49), 3.881 (0.69), 3.887 (0.71), 3.898 (0.49), 3.903 (0.44), 4.065 (1.16), 4.082 (3.45), 4.100 (3.35), 4.118 (1.06), 4.180 (0.45), 4.199 (0.56), 4.215 (0.44), 5.983 (0.47), 6.018 (0.50), 6.270 (0.57), 6.274 (0.62), 6.294 (1.14), 6.313 (0.64), 6.317 (0.62), 6.694 (0.59), 6.709 (0.62), 6.724 (0.42), 6.770 (0.49), 6.775 (0.49), 6.778 (0.49), 6.790 (0.71), 6.795 (0.69), 7.150 (0.40), 7.153 (0.45), 7.157 (0.47), 7.161 (0.49), 7.171 (1.56), 7.173 (1.67), 7.183 (1.68), 7.187 (1.72), 7.199 (0.44), 7.203 (0.45), 7.207 (0.42), 7.211 (0.40), 7.225 (1.60), 7.247 (3.42), 7.269 (1.90), 7.436 (1.19), 7.441 (1.26), 7.448 (1.38), 7.463 (1.09), 7.471 (1.08), 7.479 (1.30), 7.484 (1.21), 7.492 (1.13), 7.500 (0.98), 7.514 (0.86), 8.129 (2.09), 8.143 (1.92), 8.214 (1.35), 10.698 (1.28), 10.711 (1.30), 11.860 (1.04), 11.871 (1.06).

**Intermediate 54**

4-hydroxy-1-methyl-N-(2-methylphenyl)-2-oxo-5-(2,2,2-trifluoroethyl)-1,2,5,6-tetrahydropyridine-3-carbothioamide (Racemate)

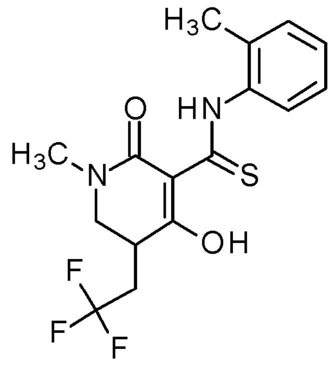

To a solution of 1-methyl-5-(2,2,2-trifluoroethyl)piperidine-2,4-dione (Racemate) (see Intermediate 14, 2.0 g) and 1-isothiocyanato-2-methylbenzene (1.43 g, 9.56 mmol) in acetonitrile (40 mL) was added 1,8-diazabicyclo(5.4.0)undec-7-ene (DBU) slowly dropwise below 5 °C. Then the mixture was stirred at room temperature for 5 hours. The reactions mixture was adjusted to pH~3 with hydrochloric acid (1 M in water). The mixture was extracted with ethyl acetate, the combined organic phase was washed with brine, dried over anhydrous sodium sulfuric, filtered and concentrated in vacuum. The residue was purified by silica gel chromatography (petroleum ether/ethyl acetate = 5:1 then 1:1) to give 3.20 g 4-hydroxy-1-methyl-N-(2-methylphenyl)-2-oxo-5-(2,2,2-trifluoroethyl)-1,2,5,6-tetrahydropyridine-3-carbothioamide (Racemate) as a yellow oil.

LC-MS (Method 3): $R_t$ = 0.937 min; MS (ESIpos): m/z = 359.0 $[M+H]^+$.

**Intermediate 55**

4-{[(2-aminopyridin-4-yl)methyl]amino}-1-methyl-N-(2-methylphenyl)-2-oxo-5-(2,2,2-trifluoroethyl)-1,2,5,6-tetrahydropyridine-3-carbothioamide (Racemate)

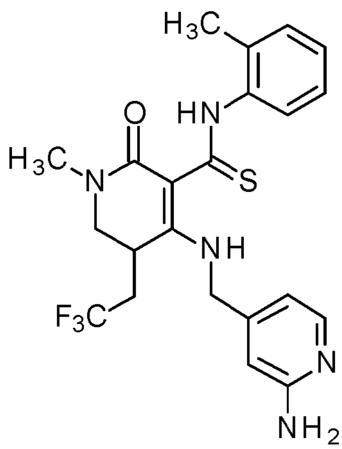

4-hydroxy-1-methyl-N-(2-methylphenyl)-2-oxo-5-(2,2,2-trifluoroethyl)-1,2,5,6-tetrahydropyridine-3-carbothioamide (Racemate) (see Intermediate 54, 3.20 g) was dried over by azeotropic rotation with toluene twice. 4-(aminomethyl)pyridin-2-amine (CAS-RN:[199296-51-0], 1.43 g) was dried over by azeotropic rotation with toluene twice. A mixture of 4-hydroxy-1-methyl-N-(2-methylphenyl)-2-oxo-5-(2,2,2-trifluoroethyl)-1,2,5,6-tetrahydropyridine-3-carbothioamide (see Intermediate 54, 3.20 g), 4-(aminomethyl)pyridin-2-amine (CAS-RN:[199296-51-0], 1.43 g), 4A molecular sieve and trimethylsilyl (1Z)-N-(trimethylsilyl)ethanimidate (5.45 g, 26.8 mmol) in 1,2-dimethoxyethane (30 mL) was stirred at 80 °C for 16 hours under nitrogen. The mixture was filtered and the filtrate was concentrated by evaporation in vauum. The residue was purified by silica gel chromatography (200-300mesh, petroleum ether: ethyl acetate = 10: 1, 5:1 then 0: 1) to give 3.70 g 4-{[(2-aminopyridin-4-yl)methyl]amino}-1-methyl-N-(2-methylphenyl)-2-oxo-5-(2,2,2-trifluoroethyl)-1,2,5,6-tetrahydropyridine-3-carbothioamide (Racemate) as yellow solid

LC-MS (Method 3): $R_t$ = 0.753 min; MS (ESIpos): m/z = 464.0 $[M+H]^+$.

**Intermediate 56**

2-(2-aminopyridin-4-yl)-5-methyl-3-(2-methylanilino)-7-(2,2,2-trifluoroethyl)-1,5,6,7-tetrahydro-4H-pyrrolo[3,2-c]pyridin-4-one (Racemate)

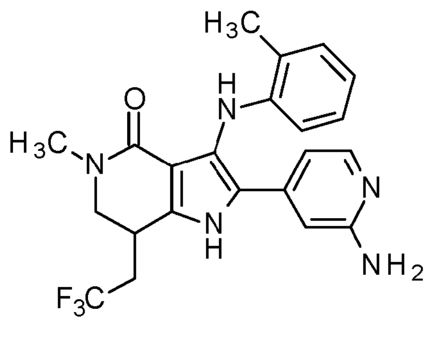

To a solution of 4-{[(2-aminopyridin-4-yl)methyl]amino}-1-methyl-N-(2-methylphenyl)-2-oxo-5-(2,2,2-trifluoroethyl)-1,2,5,6-tetrahydropyridine-3-carbothioamide (Racemate) (see Intermediate 55, 3.70 g) in methanol (40 mL) was added trifluoroacetic acid (615 μL, 7.98 mmol). The mixture was stirred at room temperature for 5 minutes. Then meta-Chloroperoxybenzoic acid (1.45 g, 80% purity, 8.38 mmol) was added at room temperature. The mixture was stirred at 100 °C for 2 hours. Saturated sodium hydrogencarbonate solution was added until moist potassium iodide test strip tured white. Then satureted sodium bicarbonate was added until pH was adjust to 10. The mixture was extrated wirh ethyl acetate. The organic phase was washed with brine, dried over anhydrous sodium sulfate, filtered and concentrated to give a residue. The residue was purified by column chromatography on silica gel (200-300 mesh, petroleum ether: ethyl acetate = 10: 1, then 5: 1, then 2: 1, then 1: 1, then 1: 2) to give 1.00 g 2-(2-aminopyridin-4-yl)-5-methyl-3-(2-methylanilino)-7-(2,2,2-trifluoroethyl)-1,5,6,7-tetrahydro-4H-pyrrolo[3,2-c]pyridin-4-one (Racemate) as yellow solid.

$^1$H-NMR (400 MHz, DMSO-$d_6$): δ [ppm] = 11.72 (s, 1H), 7.86-7.81 (m, 2H), 7.72 (d, 1H), 7.44 (d, 1H), 7.37 (t, 1H), 7.08-7.05 (m, 2H), 6.80 (t, 1H), 6.66-6.58 (m, 3H), 6.02 (d, 1H), 5.76 (s, 1 H), 3.72 (d, 1H), 3.01-2.96 (m, 1H), 2.90 (s, 3H), 2.68-2.67 (m, 1H), 2.32 (s, 3H).

**Intermediate 57**

2-(4-fluorophenyl)-N-{4-[5-methyl-3-(2-methylanilino)-4-oxo-7-(2,2,2-trifluoroethyl)-4,5,6,7-tetrahydro-1H-pyrrolo[3,2-c]pyridin-2-yl]pyridin-2-yl}acetamide (Racemate)

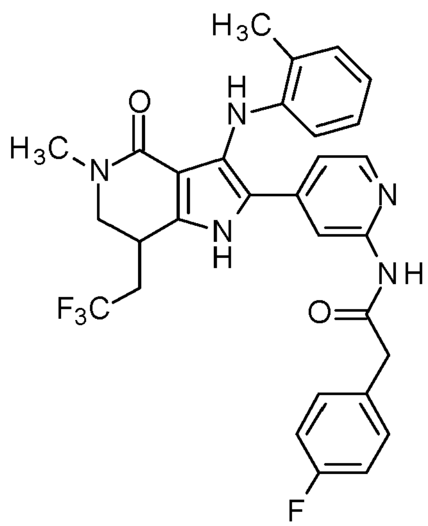

To a solution of (4-fluorophenyl)acetic acid (538 mg, 3.49 mmol) in N,N-dimethylacetamide (20 mL) was added N,N-diisopropylethylamine (1.2 mL, 7.0 mmol) and 2-(7-Azabenzotriazol-1-yl)-N,N,N',N'-tetramethyluronium hexafluorophosphate (HATU) (885 mg, 2.33 mmol). Then 2-(2-aminopyridin-4-yl)-5-methyl-3-(2-methylanilino)-7-(2,2,2-trifluoroethyl)-1,5,6,7-tetrahydro-4H-pyrrolo[3,2-c]pyridin-4-one (Racemate) (see Intermediate 56, 500 mg) was added to the mixture. The mixture was stirred at room temperature for 16 hours. The mixture was diluted in dichloromethane, then washed with saturated sodium bicarbonate, then followed by 1M sodium hydroxide. The organic phase was washed with brine, dried over anhydrous sodium sulfate, filtered and concentrated to give a residue. The residue was purified by flash silica gel chromatography (petroleum ether: ethyl acetate = 10: 1 to 0: 1) to give 300 mg 2-(4-fluorophenyl)-N-{4-[5-methyl-3-(2-methylanilino)-4-oxo-7-(2,2,2-trifluoroethyl)-4,5,6,7-tetrahydro-1H-pyrrolo[3,2-c]pyridin-2-yl]pyridin-2-yl}acetamide (Racemate) as yellow oil.

LC-MS (Method 3): $R_t$ = 0.899 min; MS (ESIpos): m/z = 566.3 $[M+H]^+$.

**Intermediate 58**

ethyl-2-cyano-4,4-difluorobutanoate (Racemate)

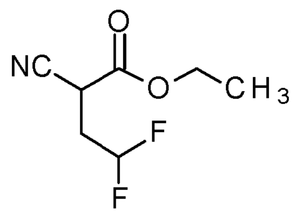

To a solution of ethyl cyanoacetate (CAS-RN:[105-56-6], 52.8 g, 467 mmol) in tetrahydrofuran (1000 mL) was drop-wise added lithium bis(trimethylsilyl)amide (467 mL, 1.0 M, 467mmol) at -70 °C. The mixture was stirred at -70 °C for 1 hour, a solution of 2,2-difluoroethyl trifluoromethanesulfonate ((CAS-RN:[74427-22-8], 100 g, 467 mmol) in tetrahydrofuran (500 mL) was drop-wise added and and the resulting mixture was stirred at room temperature for 16 hours. Aqueous hydrochloric acid solution (1 M) was added and extracted with ethyl acetate. The combined organic phase was dried over anhydrous sodium sulfate, filtered and concentrated to give a residue. The residue (combined with two other batches starting from 21.1 g and 52.8 g) was purified by silica gel chromatography (100-200 mesh, petroleum ether: ethyl acetate = 1: 0 then 100: 1) to give 107 g ethyl-2-cyano-4,4-difluorobutanoate (Racemate) as yellow oil.

$^1$H-NMR (400 MHz, $CDCl_3$): δ [ppm] = 6.22-5.93 (m, 1H), 4.37-4.27 (m, 2H), 3.73 (t, 1H), 2.63-2.40 (m, 2H), 1.35 (t, 3H).

**Intermediate 59**

ethyl-2-(aminomethyl)-4,4-difluorobutanoate hydrogen chloride salt (Racemate)

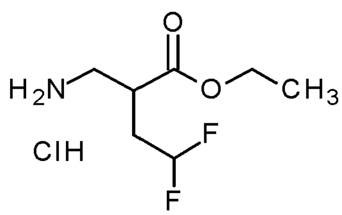

A mixture of ethyl-2-cyano-4,4-difluorobutanoate (Racemate) (see Intermediate 58, 20.0 g) and bis(oxido)platinum (2.56 g, 11.3 mmol) in methanol (400 mL) and hydrochloric acid (11 mL, 12 M, 140 mmol) was stirred at room temperature for 16 hours under hydrogen (15 psi). The combined mixture (combined with four other batches starting each from 20 g) was filtered through a pad of celiet. The filtrate was concentrated and the combined product (100 g) was used to next step directly.

**Intermediate 60**

ethyl-2-{[(tert-butoxycarbonyl)amino]methyl}-4,4-difluorobutanoate (Racemate)

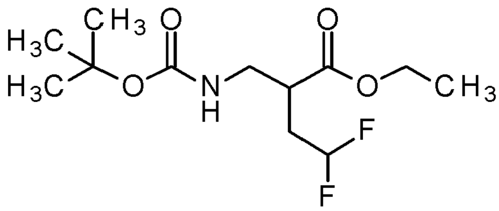

A mixture of ethyl-2-(aminomethyl)-4,4-difluorobutanoate hydrogen chloride salt (Racemate) (see Intermediate 59, 52.5 g), di-tert-butyl dicarbonate (63.2 g, 289 mmol) and N,N-diisopropylethylamine (130 mL, 720 mmol) in dichloromethane (520 mL) was stirred at room temperature for 2 hours. Water was added and the mixture was extracted with dichloromethane. The organic phase was washed with hydrochloric acid solutiondried (1M) twice. The organic phase was dried over anhydrous sodium sulfate, filtered and concentrated to give a residue. The residue was purified by silica gel chromatography (100-200 mesh, petroleum ether: ethyl acetate = 1: 0 then 100: 1) to give 75.0 g ethyl-2-{[(tert-butoxycarbonyl)amino]methyl}-4,4-difluorobutanoate (Racemate) as yellow oil.

$^1$H-NMR (400 MHz, $CDCl_3$): $\delta$ [ppm] = 6.11-5.83 (m, 1H), 4.88 (s, 1H), 4.22-4.17 (m, 2H), 3.42-3.36 (m, 2H), 2.84-2.80 (m, 1H), 2.28-2.20 (m, 1H), 2.01-2.00 (m, 1H), 1.44 (s, 9H), 1.30-1.26 (m, 3H).

**Intermediate 61**

ethyl-2-{[(tert-butoxycarbonyl)(methyl)amino]methyl}-4,4-difluorobutanoate (Racemate)

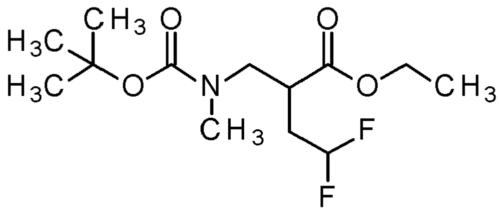

To a solution of ethyl-2-{[(tert-butoxycarbonyl)amino]methyl}-4,4-difluorobutanoate (Racemate) (see Intermediate 60, 73.0 g) in tetrahydrofuran (500 mL) was add drop-wise to a solution of sodium hydride (12.5 g, 60 % purity, 311 mmol, contained mineral oil) in tetrahydrofuran (500 mL) at 0 °C. The mixture was stirred at 0 °C for 1 hour. Iodomethane (73.7 g, 519 mmol) was drop-wise added and the resulting mixture was stirred at 0 °C for 0.5 hour. Then the mixture was stirred at 40 °C for 3 hours. Saturated ammonium chloride aqueous solution was added and the mixture was extracted with ethyl acetate. The organic phase was washed with brine, dried over anhydrous sodium sulfate, filtered and concentrated to give a residue. The residue (combined with another batch starting from 5 g) was purified by silica gel chromatography (200-300mesh, petroleum ether: ethyl acetate = 100: 1 then 10: 1) to give 40 g ethyl-2-{[(tert-butoxycarbonyl)(methyl)amino]methyl}-4,4-difluorobutanoate (Racemate) as yellow oil.

$^1$H-NMR (400 MHz, $CDCl_3$): $\delta$ [ppm] = 6.09-5.77 (m, 1H), 4.20-4.16 (m, 2H), 3.50-3.36 (m, 2H), 2.98-2.94 (m, 0.5H), 2.86 (s, 3H), 2.80-2.73 (m, 0.5H), 2.34-2.17 (m, 1H), 2.00-1.82 (m, 1H), 1.46 (s, 9H), 1.30-1.26 (m, 3H).

**Intermediate 62**

ethyl-4,4-difluoro-2-[(methylamino)methyl]butanoate hydrogen chloride salt (Racemate)

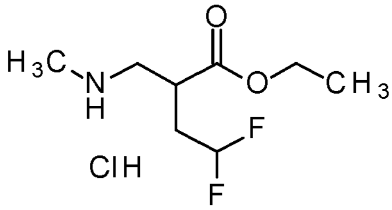

To a solution of ethyl-2-{[(tert-butoxycarbonyl)(methyl)amino]methyl}-4,4-difluorobutanoate (Racemate) (see Intermediate 61, 35.0 g) in 1,4-dioxane (150 mL) was added hydrochloric acid (150 mL, 4 M in dioxane) at room temperature. The reaction mixture was stirred at room temperature for 2 hours. The mixture was cocnentrated by evaporation in reduced pressure to give 27 g ethyl-4,4-difluoro-2-[(methylamino)methyl]butanoate hydrochloride salt (Racemate) as a yellow oil.

$^{1}$H-NMR (400 MHz, DMSO-$d_6$): δ [ppm] = 9.26 (s, 1H), 9.07 (s, 1H), 6.35-6.05 (m, 1H), 4.17-4.10 (m, 2H), 3.41 (s, 3H), 3.21-3.17 (m, 1H), 3.09-3.04 (m, 2H), 2.31-2.22 (m, 2H), 1.21(t, 3H).

## Intermediate 63

ethyl-2-{[(3-ethoxy-3-oxopropanoyl)(methyl)amino]methyl}-4,4-difluorobutanoate (Racemate)

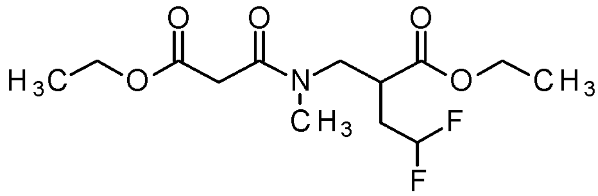

N,N-diisopropylethylamine (41 mL, 230 mmol) was added at 0°C to a solution of 4,4-difluoro-2-[(methylamino)methyl]butanoate hydrogen chloride salt (Racemate) (see Intermediate 62, 27.0 g) in dichloromethane (277 mL). And ethyl-3-chloro-3-oxopropanoate (17.5 g, 117 mmol) was added to the mixture slowly at below 15°C. The mixture was stirred at room temperature for 16 hours. The mixture (combined with another batch starting from 3.9 g) was adjusted to pH~3 with aqueous hydrochloric acid solution (2 M). Then the mixture was extracted with dichloromethane and the combined organic layer was washed with brine, dried over anhydrous sodium sulfate, filtered and concentrated by evaporation in vacuum. The residue was purified by column chromatography (100-200 mesh, petroleum ether: ethyl acetate = 20: 1 then 5: 1) to give 30 g ethyl-2-{[(3-ethoxy-3-oxopropanoyl)(methyl)amino]methyl}-4,4-difluorobutanoate (Racemate) as yellow oil.

## Intermediate 64

5-(2,2-difluoroethyl)-1-methylpiperidine-2,4-dione (Racemate)

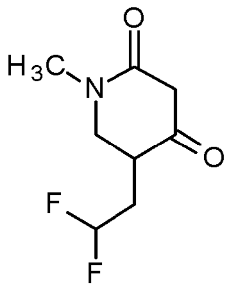

To a solution of ethyl-2-{[(3-ethoxy-3-oxopropanoyl)(methyl)amino]methyl}-4,4-difluorobutanoate (Racemate)

(see Intermediate 63, 30.0 g) in ethanol (300 mL) was added sodium ethoxide (CAS-RN:[141-52-6], 7.92 g) at 0-5 °C. Then the mixture was stirred at room temperature for 4 hours. The mixture was treated with aqueous hydrogen chloride solution (2 M) until pH was adjust to 3, the aqueous phase was extracted with ethyl acetate. The combined organic phase was washed with brine, dried over anhydrous sodium sulfate, filtered and concentrated to give a residue. Then the residue was dissolved in acetonitrile (300 mL) and water (15 mL) at room temperature, and the resulting mixture was heated at reflux for 16 hours. The mixture was concentrated to dryness. The residue was purified by flash column chromatography (petroleum ether: ethyl acetate = 30: 1 to 1: 1) to give 4.0 g 5-(2,2-difluoroethyl)-1-methylpiperidine-2,4-dione (Racemate) as yellow oil.

LC-MS (Method: Instrument: SHIMADZU LCMS-2020 SingleQuad; Column: Chromolith@Flash RP-18E 25-2 MM; eluent A: water + 0.0375 vol % trifluoroacetic acid, eluent B: acetonitrile + 0.01875 vol % trifluoroacetic acid; gradient: 0-0.8 min 0-60% B, 0.8-1.2 min 60% B; flow 1.5 mL/min; temperature: 50 °C; PDA: 220 nm & 254 nm.): $R_t$ = 0.627 min; MS (ESIpos): m/z = 192.1 $[M+H]^+$.

$^1$H-NMR (400 MHz, $CDCl_3$): $\delta$ [ppm] = 6.20-5.91 (m, 1H), 3.63-3.58 (m, 1H), 3.44-3.29 (m, 3H), 3.07 (s, 3H), 2.99-2.93 (m, 1H), 2.51-2.43 (m, 1H), 1.89-1.77 (m, 1H).

**Intermediate 65**

5-(2,2-difluoroethyl)-4-hydroxy-1-methyl-2-oxo-N-phenyl-1,2,5,6-tetrahydropyridine-3-carbothioamide (Racemate)

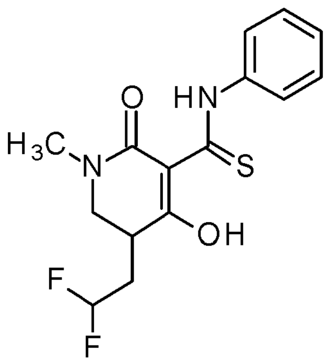

To a solution of 5-(2,2-difluoroethyl)-1-methylpiperidine-2,4-dione (Racemate) (see Intermediate 64, 3.50 g) and isothiocyanatobenzene (2.47 g, 18.3 mmol) in acetonitrile (40 mL) was added 1,8-diazabicyclo(5.4.0)undec-7-ene(DBU) (4.1 mL, 27 mmol) slowly dropwise below 5 °C. Then the mixture was stirred at room temperature for 5 hours. The combined mixture was concentrated by evaporation in vacuum. The residue was purified by flash chromatography (80 g, ethyl acetate in petroleum ether = 0% to 8%) to give 3.0 g 5-(2,2-difluoroethyl)-4-hydroxy-1-methyl-2-oxo-N-phenyl-1,2,5,6-tetrahydropyridine-3-carbothioamide (Racemate) as a yellow oil.

LC-MS (Method 3): $R_t$ = 0.873 min; MS (ESIpos): m/z = 326.9 $[M+H]^+$,

$^1$H-NMR (400 MHz, DMSO-$d_6$): $\delta$ [ppm] = 14.23 (s, 1H), 7.5-7.41 (m, 5H), 7.30-7.28 (m, 1H), 6.24-5.94 (m, 1H), 3.36-3.32 (m, 1H), 3.20 (s, 1H), 3.09 (s, 3H), 3.06-3.00 (m, 1H), 2.44-2.35 (m, 1H), 2.14-2.07 (m, 1H).

**Intermediate 66**

4-{[(2-aminopyridin-4-yl)methyl]amino}-5-(2,2-difluoroethyl)-1-methyl-2-oxo-N-phenyl-1,2,5,6-tetrahydropyridine-3-carbothioamide (Racemate)

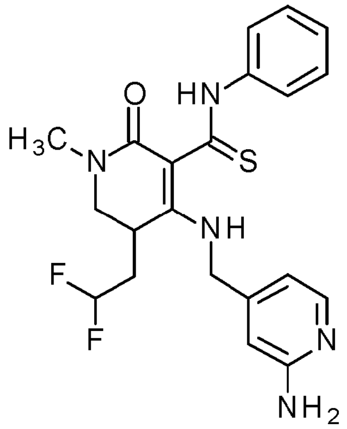

5-(2,2-difluoroethyl)-4-hydroxy-1-methyl-2-oxo-N-phenyl-1,2,5,6-tetrahydropyridine-3-carbothioamide (Racemate) (see Intermediate 65, 3.00 g) was dried over by azeotropic rotation with toluene twice. 3-(aminomethyl)aniline (CAS-RN:[4403-70-7], 1.26 g, 10.3 mmol) was dried over by azeotropic rotation with toluene twice. The mixture of 5-(2,2-difluoroethyl)-4-hydroxy-1-methyl-2-oxo-N-phenyl-1,2,5,6-tetrahydropyridine-3-carbothioamide (Racemate) (see Intermediate 65, 3.00 g) and 3-(aminomethyl)aniline (CAS-RN:[4403-70-7], 1.26 g, 10.3 mmol) was dried over by azeotropic rotation with toluene twice. A mixture of 5-(2,2-difluoroethyl)-4-hydroxy-1-methyl-2-oxo-N-phenyl-1,2,5,6-tetrahydropyridine-3-carbothioamide (Racemate) (see Intermediate 65, 3.00 g), 3-(aminomethyl)aniline (CAS-RN:[4403-70-7], 1.26 g, 10.3 mmol), 4A molecular sieve (4.68 g) and trimethylsilyl (1Z)-N-(trimethylsilyl)ethanimidate (5.25 g, 25.8 mmol) in 1,2-dimethoxyethane (28 mL) was stirred at 80 °C for 16 hours under nitrogen. The mixture was filtered and the filtrate was concentrated by evaporation in vauum to give a residue. The residue was purified by flash silica gel chromatography (40 g, ethyl acetate in petroleum ether = 0% to 20%) to give 2.8 g 4-{[(2-aminopyridin-4-yl)methyl]amino}-5-(2,2-difluoroethyl)-1-methyl-2-oxo-N-phenyl-1,2,5,6-tetrahydropyridine-3-carbothioamide (Racemate) as yellow oil.

## Intermediate 67

2-(2-aminopyridin-4-yl)-3-anilino-7-(2,2-difluoroethyl)-5-methyl-1,5,6,7-tetrahydro-4H-pyrrolo[3,2-c]pyridin-4-one (Racemate)

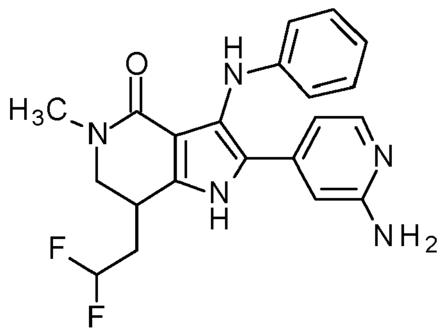

To a solution of 4-{[(2-aminopyridin-4-yl)methyl]amino}-5-(2,2-difluoroethyl)-1-methyl-2-oxo-N-phenyl-1,2,5,6-tetrahydropyridine-3-carbothioamide (Racemate) (see Intermediate 66, 2.60 g) in methanol (30 mL) was added trifluoroacetic acid (460 µL, 6.0 mmol) and stirred for 5 minutes at room temperature. Then meta-chloroperoxybenzoic acid (1.30 g, 80 % purity, 6.03 mmol) was added at room temperature and the mixture was stirred at 100 °C for 2 hours under nitrogen. The mixture was cooled to room temperature and poured into a solution (saturated sodium thiosulfate solution: saturated sodium bicarbonate solution=1: 1) and the mixture was extracted with ethyl acetate. The combined organic phase was washed with brine, dried over anhydrous sodium sulfate, filtered and concentrated in vacuum. The residue was purified by flash chromatography ((ethyl acetate: methanol = 10: 1) in petroleum ether) = 0% to 60% to give 189.4 mg 2-(2-aminopyridin-4-yl)-3-anilino-7-(2,2-difluoroethyl)-5-methyl-1,5,6,7-tetrahydro-4H-pyrrolo[3,2-c]pyridin-4-one (Racemate) as a yellow solid.

LC-MS (Method 3): $R_t$ = 0.80 min; MS (ESIpos): m/z = 398.2 $[M+H]^+$

$^1H$ NMR (400 MHz, DMSO-$d_6$ + $D_2O$): δ [ppm] = 7.66 (d, 1H), 7.04-7.00 (m, 2H), 6.75 (dd, 1H), 6.66-6.62 (m, 2H), 6.53-6.51 (m, 2H), 6.28-6.00 (m, 1H), 3.28-3.68 (m, 1H), 3.40-3.35 (m, 1H), 3.28-3.25 (m, 1H), 2.85 (s, 3H), 2.31-2.12 (m, 2H).

**Intermediate 68**

1,3-thiazol-4-amine-hydrogen chloride

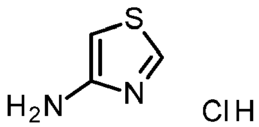

A mixture of tert-butyl 1,3-thiazol-4-ylcarbamate (5.00 g, 25.0 mmol) in a solution of hydrochloric acid in dioxane (50 ml, 4.0 M) was stirred at room temperature for 16 hours under nitrogen. The mixture was concentrated by evaporation in vacuum to give 1,3-thiazol-4-amine hydrochloride (4.00 g, crude) as a yellow solid. The residue was used directly as a crude product without further purification.

**Intermediate 69**

4-isothiocyanato-1,3-thiazole

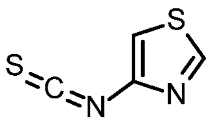

To a mixture of 1,3-thiazol-4-amine hydrochloride (5.00 g, 36.6 mmol, see Intermediate 68) in dichloromethane (100 ml) and saturated sodium bicarbonate solution (100 ml) was added dropwise carbonothioyl dichloride (5.6 ml, 73 mmol). The mixture was stirred at room temperature for 10 minutes. The mixture was separated and the water phase was extracted with dichloromethane. The combined organic phases were washed with saturated sodium chloride solution, dried (sodium sulfate), filtered and concentrated to give 4-isothiocyanato-1,3-thiazole (3.00 g, 58 % yield) as a black solid, which was used directly as a crude product without further purification.

**Intermediate 70**

(5RS)-4-hydroxy-1-methyl-2-oxo-N-(1,3-thiazol-4-yl)-5-(2,2,2-trifluoroethyl)-1,2,5,6-tetrahydropyridine-3-carbothioamide

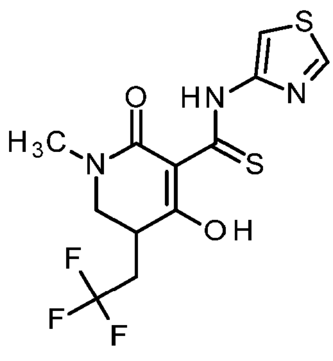

To a solution of (5RS)-1-methyl-5-(2,2,2-trifluoroethyl)piperidine-2,4-dione (2.00 g, 93 % purity, 8.85 mmol, see Intermediate 14) and 4-isothiocyanato-1,3-thiazole (2.52 g, 17.7 mmol, see Intermediate 69) in acetonitrile was added 1,8-diazabicyclo(5.4.0)undec-7-ene (DBU) (2.1 ml, 14 mmol) dropwise below 5 °C. Then the mixture was stirred at room temperature for 16 hours. The reaction mixture was concentrated by evaporation in vacuum. The residue was purified by silica gel chromatography (100-200 mesh, petroleum ether: ethyl acetate = 1: 0, then 20: 1) to give the target compound (1.50 g, 48 % yield) as yellow oil.

LC-MS (Method 3): $R_t$ = 0.978 min; MS (ESIpos): m/z = 352.1 [M+H]+.

$^1$H-NMR (400 MHz, $CDCl_3$): δ [ppm] = 15.47 (s, 1H), 8.70 (d, *J* = 2.0 Hz, 1H), 8.44 (d, *J* = 2.0 Hz, 1H), 3.70-3.62 (m, 1H), 3.38-3.33 (m, 1H), 3.23 (s, 1H), 3.11 (s, 3H), 2.80-2.67 (m, 1H), 2.44-2.35 (m, 1H).

## Intermediate 71

(5RS)-4-{[(2-aminopyridin-4-yl)methyl]amino}-1-methyl-2-oxo-N-(1,3-thiazol-4-yl)-5-(2,2,2-trifluoroethyl)-1,2,5,6-tetrahydropyridine-3-carbothioamide

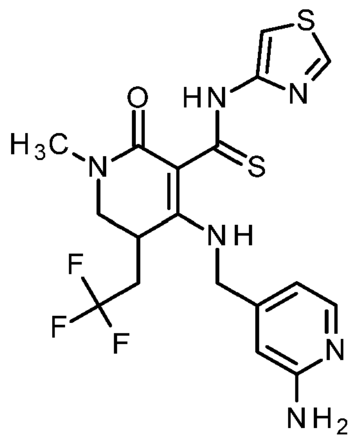

(5RS)-4-hydroxy-1-methyl-2-oxo-N-(1,3-thiazol-4-yl)-5-(2,2,2-trifluoroethyl)-1,2,5,6-tetrahydropyridine-3-carbothioamide (1.50 g, 4.27 mmol, see Intermediate 70) was dried by azeotropic evaporation with toluene twice. 4-(Aminomethyl)pyridin-2-amine (526 mg, 4.27 mmol) was dried by azeotropic evaporation with toluene twice. Both (5RS)-4-hydroxy-1-methyl-2-oxoN-(1,3-thiazol-4-yl)-5-(2,2,2-trifluoroethyl)-1,2,5,6-tetrahydropyridine-3-carbothioamide (1.50 g, 4.27 mmol, see Intermediate 70) and 4-(aminomethyl)pyridin-2-amine (526 mg, 4.27 mmol) were combined and the mixture was dried again by azeotropic evaporation with toluene twice. 1,2-Dimethoxyethane (20 ml), 4 A molecular sieve (5 g), and trimethylsilyl (1Z)-N-(trimethylsilyl)ethanimidate (2.61 g, 12.8 mmol) were added and the mixture was stirred at 80 °C for 16 hours under nitrogen. The mixture was filtered and the filtrate was concentrated by evaporation in vauum. The residue was purified by silica gel chromatography (40 g, 100-200 mesh, ethyl acetate in petroleum ether: = 0% to 30%) to give the target compound (1.20 g, crude) as a yellow solid as a crude product which was used directly without further purification

LC-MS (Method 3): $R_t$ = 0.780 min; MS (ESIpos): m/z = 457.3 [M+H]+.

**Intermediate 72**

(7RS)-2-(2-aminopyridin-4-yl)-5-methyl-3-(1,3-thiazol-4-ylamino)-7-(2,2,2-trifluoroethyl)-1,5,6,7-tetrahydro-4H-pyrrolo[3,2-c]pyridin-4-one

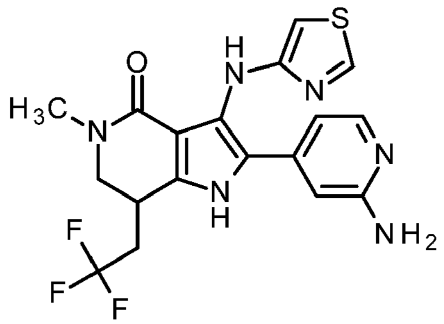

To a solution of (5RS)-4-{[(2-aminopyridin-4-yl)methyl]amino}-1-methyl-2-oxo-N-(1,3-thiazol-4-yl)-5-(2,2,2-trifluoroethyl)-1,2,5,6-tetrahydropyridine-3-carbothioamide (800 mg, 1.75 mmol, see Intermediate 71) in methanol (20 ml) was added trifluoroacetic acid (140 µl, 1.8 mmol) and the mixture was stirred for 5 minutes. Then meta-chloroperoxybenzoic acid (378 mg, 80 % purity, 1.75 mmol) was added at room temperature and the mixture was stirred at 100 °C for 2 hours under nitrogen. The mixture was cooled to room temperature and poured into a mixture of saturated sodium thiosulfate solution and saturated sodium bicarbonate solution (1: 1) and the mixture was extracted with ethyl acetate. The combined organic phases were washed with brine, dried (sodium sulfate), filtered and concentrated in vacuum. The residue was purified by silica gel chromatography (petroleum ether: (ethyl acetate: methanol = 10: 1) = 5: 1, then 1: 1 to give the target compound 248 mg (97 % purity, 32 % yield) as a yellow solid.

LC-MS (Method 3): $R_t$ = 0.753 min; MS (ESIpos): m/z = 423.2 $[M+H]^+$.

$^1$H-NMR (400 MHz, DMSO-$d_6$): δ [ppm] = 8.74 (d, *J*= 2.4 Hz, 1H), 7.74 (d, *J*= 5.6 Hz, 1H), 6.65 (dd, *J*= 4 Hz, 1.6Hz, 1H), 6.50 (s, 1H), 5.48 (d, *J*= 2 Hz, 1H), 3.42-3.40 (m, 2H), 2.89 (s, 3H), 2.84-2.78 (m, 1H), 2.66-2.58 (m, 2H).

**Intermediate 73**

(5RS)-5-(cyclopropylmethyl)-4-hydroxy-1-methyl-2-oxo-N-(1,3-thiazol-4-yl)-1,2,5,6-tetrahydropyridine-3-carbothioamide

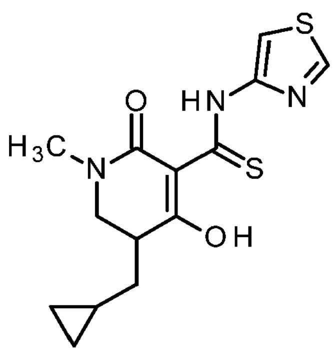

To a solution of (5RS)-5-(cyclopropylmethyl)-1-methylpiperidine-2,4-dione (2.00 g, 11.0 mmol, see Intermediate 24) and 4-isothiocyanato-1,3-thiazole (1.57 g, 11.0 mmol, see Intermediate 69) in acetonitrile (20 ml) was added 1,8-diazabicyclo(5.4.0)undec-7-ene (DBU) (2.47 ml, 16.6 mmol) dropwise below 5 °C. Then the mixture was stirred at room temperature for 5 hours. The mixture was treated with aqueous hydrogen chloride solution (2 M) until the pH was adjust to 3 and the aqueous phase was extracted with ethyl acetate. The combined organic phases were dried over anhydrous sodium sulfate, filtered and concentrated in vacuum to give a residue. The residue was purified by flash silica gel chromatography (petroleum ether: ethyl acetate = 50: 1 to 20: 1) to give the title compound (1.50 g, 80 % purity, 34 % yield) as yellow oil.

LC-MS (Method 3): $R_t$ = 0.921 min; MS (ESIpos): m/z = 324.0 $[M+H]^+$.

**Intermediate 74**

(5RS)-4-{[(2-aminopyridin-4-yl)methyl]amino}-5-(cyclopropylmethyl)-1-methyl-2-oxo-N-(1,3-thiazol-4-yl)-1,2,5,6-tetrahydropyridine-3-carbothioamide

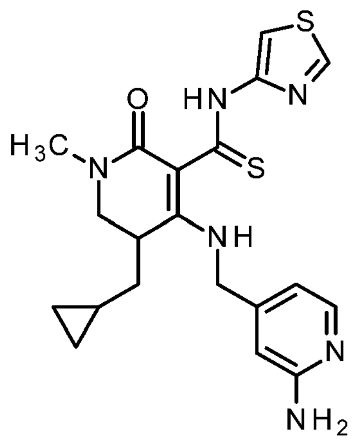

(5RS)-5-(cyclopropylmethyl)-4-hydroxy-1-methyl-2-oxo-N-(1,3-thiazol-4-yl)-1,2,5,6-tetrahydropyridine-3-carbothioamide (1.50 g, 80 % purity, 3.71 mmol, see Intermediate 73) was dried by azeotropic evaporation with toluene twice. 4-(Aminomethyl)pyridin-2-amine (594 mg, 4.82 mmol) was dried by azeotropic evaporation with toluene twice. Both, (5RS)-5-(cyclopropylmethyl)-4-hydroxy-1-methyl-2-oxo-N-(1,3-thiazol-4-yl)-1,2,5,6-tetrahydropyridine-3-carbothioamide (1.50 g, 80 % purity, 3.71 mmol, see Intermediate 73) and 4-(aminomethyl)pyridin-2-amine (594 mg, 4.82 mmol) were combined, 4 A molecular sieve (2 g), trimethylsilyl (1Z)-N-(trimethylsilyl)ethanimidate (2.26 g, 11.1 mmol), and 1,2-dimethoxyethane (10 ml) were added and the mixture was stirred at 80 °C for 16 hours under nitrogen. The mixture was filtered and the filtrate was concentrated by evaporation in vauum to give a residue. The residue was purified by flash silica gel chromatography (petroleum ether: ethyl acetate = 10: 1 to 0: 1) to give (the title compound (1.00 g, 63 % yield) as yellow oil.

LC-MS (Method 3): $R_t$ = 0.820 min; MS (ESIpos): m/z = 429.2 $[M+H]^+$.

**Intermediate 75**

(7RS)-2-(2-aminopyridin-4-yl)-7-(cyclopropylmethyl)-5-methyl-3-(1,3-thiazol-4-ylamino)-1,5,6,7-tetrahydro-4H-pyrrolo[3,2-c]pyridin-4-one

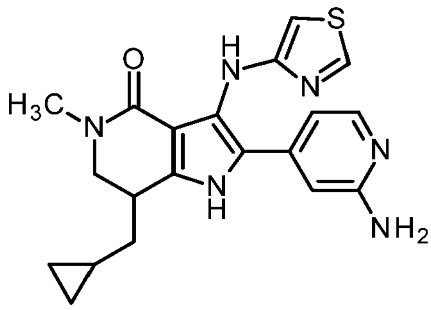

To a solution of (5RS)-4-{[(2-aminopyridin-4-yl)methyl]amino}-5-(cyclopropylmethyl)-1-methyl-2-oxo-N-(1,3-thiazol-4-yl)-1,2,5,6-tetrahydropyridine-3-carbothioamide (1.00 g, 2.33 mmol, see Intermediate 74) in methanol (20 ml) was added trifluoroacetic acid (180 µl, 2.33 mmol). The mixture was stirred at room temperature for 5 minutes. Then meta-chloroperoxybenzoic acid (528.5 mg, 80% purity, 2.45 mmol) was added at room temperature. The mixture was stirred at 100 °C for 2 hours. A mixture of saturated sodium thiosulfate solution and saturated sodium bicarbonate solution (1: 1) was added until a moist potassium iodide test strip tured white. Then the methanol was removed under vaccuo. The residue was triturated with acetonitrile for 1 hour at room temperature, the mixture was filtered and the filter cake was collected. The filter cake was suspended in dichloromethane, then saturated sodium bicarbonate solution was added until the pH was adjusted to 10. It was stirred for 10 minutes and the mixture was extracted with dichloromethane. The organic phase was washed with brine, dried over anhydrous sodium sulfate, filtered and concentrated in vacuum to give the title compound (200 mg, 22 % yield) as a yellow solid as a crude product that was used without further purification.

LC-MS (Method 3): $R_t$ = 0.774 min; MS (ESIpos): m/z = 395.2 $[M+H]^+$.
$^1$H-NMR (400 MHz, DMSO-$d_6$): δ [ppm] = 11.36 (s, 1 H), 8.77 (d, *J*= 2.0 Hz, 1H), 7.95 (s, 1H), 7.76 (d, J= 5.2 Hz, 1H), 6.64 (d, J= 5.2 Hz, 1H), 6.47 (s, 1H), 5.71 (s, 2H), 5.46 (d, *J*= 2.0 Hz, 1H), 3.75-3.71 (m, 1H), 3.44-3.41 (m, 1H), 3.11-3.05 (m, 1H), 2.90 (s, 3H), 1.58-1.55 (m, 2H), 0.81-0.75 (m, 1H), 0.55-0.37 (m, 2H), 0.09-0.01 (m, 2H).

EXPERIMENTAL SECTION -EXAMPLES

**Example** 1

N-{4-[3-anilino-7-ethyl-4-oxo-4,5,6,7-tetrahydro-1H-pyrrolo[3,2-c]pyridin-2-yl]pyridin-2-yl}-2-(4-fluorophenyl)acetamide (Racemate)

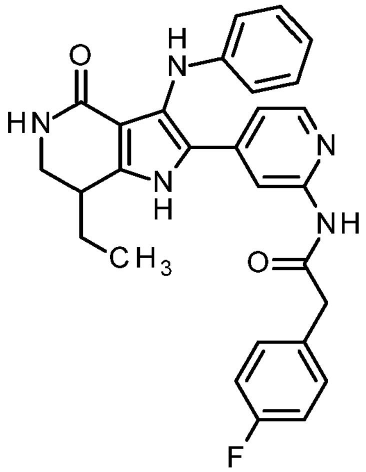

2-(2-Aminopyridin-4-yl)-3-anilino-7-ethyl-1,5,6,7-tetrahydro-4H-pyrrolo[3,2-c]pyridin-4-one (Racemate) (see Intermediate 4, 60.0 mg), (4-fluorophenyl)acetic acid (53.2 mg, 345 µmol), PyBOP (449 mg, 864 µmol) and N,N-diisopropylethylamine (180 µL, 1.0 mmol) were dissolved in 1.2 mL N,N-dimethylacetamide and were stirred at room temperature over night under argon atmosphere. Furhter (53.2 mg, 345 μmol), PyBOP (225 mg, 432 μmol) and N,N-diisopropylethylamine (180 μl, 1.0 mmol) were added and it was stirred at room temperature for 4 h. Aqueous saturated sodium hydrogencarbonate solution was added to the reaction mixture. It was diluted with dichloromethane and water. The aqueous layer was extracted with dichloromethane three times. The combined organic layers were washed with saturated ammonium chloride solution once with water three times and with brine once, filtered through a silicone coated filter and concentrated under reduced pressure. The crude product was purified by flash chromatography (28g column, aminophase; hexane / ethyl acetate 0%-100% // ethyl acetate / ethanol 0%-20%) and HPLC under basic conditions to provide the target compound with 93% purity: 28 mg.

LC-MS (Method 2): $R_t$ = 1.13 min; MS (ESIpos): m/z = 485 $[M+H]^+$
$^1$H-NMR (400MHz, DMSO-d6): δ [ppm]= 0.93 (t, 3H), 1.46 - 1.64 (m, 1H), 1.75 (ddd, 1H), 2.81 (br dd, 1H), 3.18 (dt, 1H), 3.46 - 3.56 (m, 1H), 3.69 (s, 2H), 6.54 (d, 2H), 6.61 (t, 1H), 6.96 - 7.09 (m, 3H), 7.11 - 7.22 (m, 3H), 7.30 - 7.41 (m, 3H), 8.05 (d, 1H), 8.20 (s, 1H), 10.56 (s, 1H), 11.62 (s, 1H). - contains ethanol.

## Example 2

N-{4-[3-anilino-7-ethyl-4-oxo-4,5,6,7-tetrahydro-1H-pyrrolo[3,2-c]pyridin-2-yl]pyridin-2-yl}-2-(4-fluorophenyl)acetamide (Enantiomer 1)

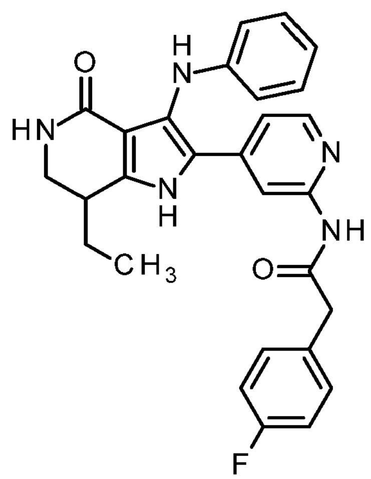

The racemic compound (see Example 1, 30 mg) was separated into enantiomers by preparative chiral HPLC to give enantiomer 1 (12 mg, 100 %ee, see Example 2) and enantiomer 2 (14 mg, 96.1 % ee, see Example 3).

Preparative chiral HPLC method:

Instrument: Sepiatec: Prep SFC100; Column: Chiralpak IA 5 μ 250x30 mm; eluent A: carbon dioxide; eluent B: 2-propanol + 0.4 vol % diethylamin; isocratic: 35%B; flow: 100 mL/min; temperature: 40 °C; BPR: 150 bar; UV: 254 nm.

Analytical chiral HPLC method:

Instrument: Agilent: 1260, Aurora SFC-Modul; Column: Chiralpak IA 5 μ 100x4.6 mm; eluent A: carbon dioxide; eluent B: ethanol + 0.2 vol % aqueous ammonia (32%); isocratic: 35%B; flow: 4 mL/min; temperature: 37.5°C; BPR: 100bar; UV: 254 nm.

Analytical Chiral HPLC (method Example 1): $R_t$ = 3.54 min.

$^1$H-NMR (400 MHz, DMSO-d6) δ [ppm] = 0.93 (t, 3H), 1.47 - 1.63 (m, 1H), 1.75 (ddd, 1H), 2.81 (br dd, 1H), 3.18 (dt, 1H), 3.46 - 3.56 (m, 1H), 3.69 (s, 2H), 6.54 (d, 2H), 6.60 (t, 1H), 6.95 - 7.08 (m, 3H), 7.12 - 7.20 (m, 3H), 7.30 - 7.41 (m, 3H), 8.05 (d, 1H), 8.20 (s, 1H), 10.56 (s, 1H), 11.62 (s, 1H).

**Example 3**

N-{4-[3-anilino-7-ethyl-4-oxo-4,5,6,7-tetrahydro-1H-pyrrolo[3,2-c]pyridin-2-yl]pyridin-2-yl}-2-(4-fluorophenyl)acetamide (Enantiomer 2)

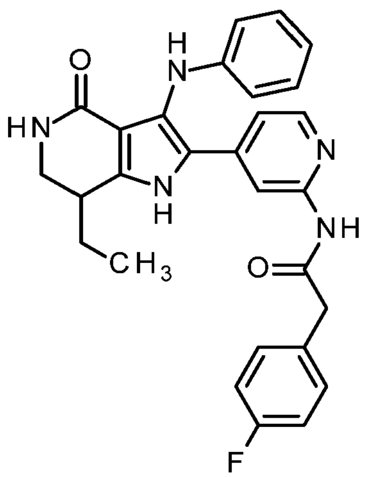

For the preparation of the racemic title compound see Example 1. Separation of enantiomers by preparative chiral HPLC (method see Example 2) gave the title compound (14 mg).

Analytical Chiral HPLC (method see Example 2): $R_t$ = 5.30 min.

$^1$H-NMR (400 MHz, DMSO-d6) δ [ppm] = 0.93 (t, 3H), 1.47 - 1.63 (m, 1H), 1.69 - 1.81 (m, 1H), 2.81 (br dd, 1H), 3.18 (dt, 1H), 3.50 (dd, 1H), 3.69 (s, 2H), 6.54 (d, 2H), 6.60 (t, 1H), 6.95 - 7.07 (m, 3H), 7.10 - 7.20 (m, 3H), 7.27 - 7.40 (m, 3H), 8.05 (d, 1H), 8.19 (s, 1H), 10.56 (s, 1H), 11.62 (s, 1H).

**Example 4**

N-{4-[3-anilino-7-ethyl-4-oxo-4,5,6,7-tetrahydro-1H-pyrrolo[3,2-c]pyridin-2-yl]pyridin-2-yl}-4,4-difluoro-2-(4-fluorophenyl)butanamide (Mixture of stereoisomers)

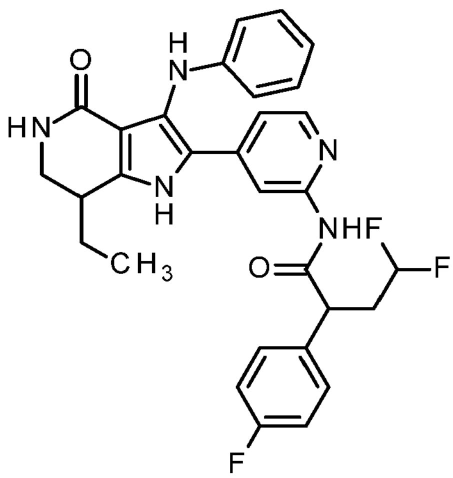

2-(2-Aminopyridin-4-yl)-3-anilino-7-ethyl-1,5,6,7-tetrahydro-4H-pyrrolo[3,2-c]pyridin-4-one (Racemate) (see

Intermediate 4, 229 mg), 4,4-difluoro-2-(4-fluorophenyl)butanoic acid (racemate) (158 mg, 725 µmol), PyBOP (1.03 g, 1.98 mmol) and N,N-diisopropylethylamine (690 µL, 4.0 mmol) were dissolved in 3.8 mL DMA and stirred at room temperature over night under nitrogen atmosphere. To the reaction mixture aqueous saturated sodium hydrogencarbonate solution,water and dichloromethane was added. The aqueous layer was extracted with dichloromethane three times. The combined organic layers were filtered through a silicone coated filter and concentrated under reduced pressure. The crude product was purified by HPLC to provide the target compound in 90% purity: 73 mg.

LC-MS (Method 1): $R_t$ = 1.16 min; MS (ESIpos): m/z = 548 $[M+H]^+$

$^1$H-NMR (400 MHz, DMSO-d6) δ [ppm] = 0.94 (td, 3H), 1.46 - 1.67 (m, 1H), 1.73 - 1.86 (m, 1H), 2.14 - 2.29 (m, 1H), 2.60 - 2.74 (m, 1H), 2.82 - 2.89 (m, 1H), 3.14 - 3.28 (m, 1H), 3.46 - 3.58 (m, 1H), 4.15 (br dd, 1H), 5.73 - 6.18 (m, 1H), 6.49 - 6.69 (m, 3H), 6.93 - 7.25 (m, 6H), 7.35 (d, 1H), 7.41 - 7.49 (m, 2H), 8.03 (d, 1H), 8.17 (d, 1H), 10.63 (d, 1H), 11.63 (s, 1H).

## Example 5

N-[4-(3-anilino-7-ethyl-4-oxo-4,5-dihydro-1H-pyrrolo[3,2-c]pyridin-2-yl)pyridin-2-yl]-4,4-difluoro-2-(4-fluorophenyl)butanamide (Racemate)

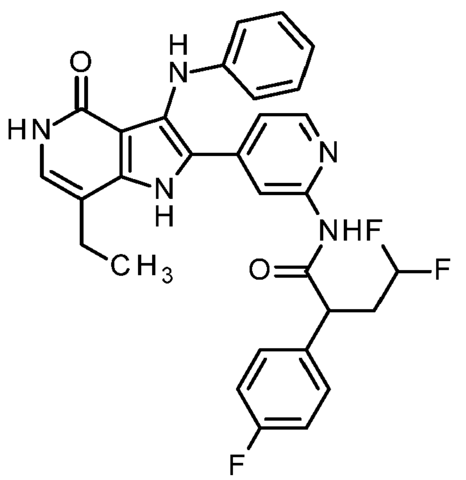

N-{4-[3-Anilino-7-ethyl-4-oxo-4,5,6,7-tetrahydro-1H-pyrrolo[3,2-c]pyridin-2-yl]pyridin-2-yl}-4,4-difluoro-2-(4-fluorophenyl)butanamide (Racemate) (see Example 4, 78.0 mg) was dissolved in 6.1 mL dioxane and 2,3-dichloro-5,6-dicyano-1,4benzochinone (35.6 mg, 157 µmol) was added. The reaction mixture was stirred at 80 °C for 1 hour. The reaction mixture was diluted with ethyl acetate and treated with 1-molar aqueous sodiumhydroxyd-solution. The layers were sperated and the aqueous layer was extracted with ethyl acetate twice. The combined organic layers were dried using a waterresistant filter and concentrated under reduced pressure. The crude product was purified by chromatography (5g spheric silica column, gradient dichloromethane/ethanol 1-10%) to provide the target compound in 98% purity: 28 mg.

LC-MS (Method 1): $R_t$ = 1.18 min; MS (ESIpos): m/z = 546 $[M+H]^+$
$^1$H-NMR (400 MHz, DMSO-d6) δ [ppm] = 1.21 (t, 3H), 2.11 - 2.29 (m, 1H), 2.60 - 2.79 (m, 3H), 4.15 (br dd, 1H), 5.74 - 6.13 (m, 1H), 6.50 - 6.63 (m, 3H), 6.78 (d, 1H), 6.95 - 7.03 (m, 2H), 7.14 - 7.24 (m, 2H), 7.36 (dd, 1H), 7.42 - 7.51 (m, 3H), 8.13 (d, 1H), 8.30 (s, 1H), 10.68 (t, 2H), 11.47 (s, 1H).

## Example 6

N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide (Racemate)

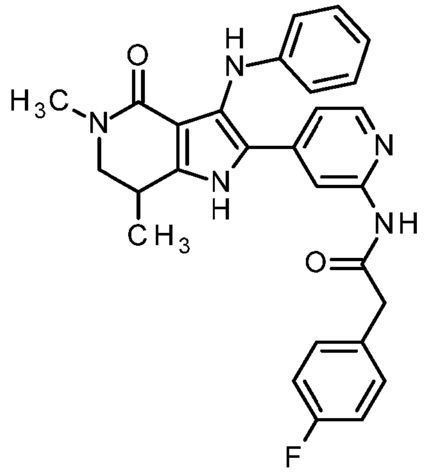

2-(2-Aminopyridin-4-yl)-3-anilino-5,7-dimethyl-1,5,6,7-tetrahydro-4H-pyrrolo[3,2-c]pyridin-4-one (Racemate) (see Intermediate 7, 98.0 mg), fluorophenyl)acetic acid (65.2 mg, 423 μmol), N,N-diisopropylethylamine (290 μL, 1.7 mmol) and PyBOP (440 mg, 846 μmol) were dissolved in 1.6 mL DMA and stirred at room temperature under nitrogen atmosphere for 2 days. The reaction mixture was diluted with water and dichloromethane. The aqueous layer was extracted with dichloromethane three times. The combined organic layers were washed with water and brine, filtered through a silicone coated filter and concentrated under reduced pressure. The crude product was purified by flash chromatography (11g column, aminophase; dichloromethane / ethanol 0%-5% ethanol) and by HPLC to provide the target compound in 98% purity: 49 mg.

LC-MS (Method 2): $R_t$ = 1.15 min; MS (ESIpos): m/z = 484 $[M+H]^+$

$^1$H-NMR (400 MHz, DMSO-d6) δ [ppm] = 1.28 (d, 3H), 2.86 (s, 3H), 3.09 - 3.17 (m, 1H), 3.18 - 3.24 (m, 1H), 3.61 (dd, 1H), 3.69 (s, 2H), 6.50 - 6.57 (m, 2H), 6.61 (t, 1H), 7.00 (dd, 2H), 7.12 - 7.23 (m, 3H), 7.30 - 7.41 (m, 3H), 8.05 (d, 1H), 8.20 (s, 1H), 10.56 (s, 1H), 11.59 (s, 1H).

## Example 7

(-)-N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide (Enantiomer 1)

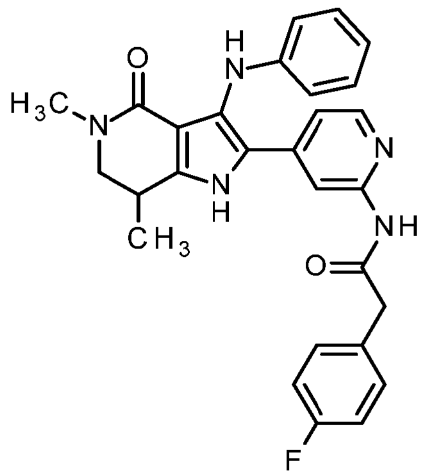

The racemic compound (see Example 6, 49 mg) was separated into enantiomers by preparative chiral HPLC to give enantiomer 1 (19 mg, x 100 ee, see Example 7) and enantiomer 2 (36 mg, 99.6 % ee, see Example 8).

Preparative chiral HPLC method:

Instrument: PrepCon Labomatic HPLC; Column: YMC Cellulose SB 5 μ, 250x30; eluent A: hexane; eluent B: ethanol + 0.1 vol % diethylamine; isocratic: 90%A+10%B; flow: 50 mL/min; temperature: 25°C; UV: 254 nm.

Analytical chiral HPLC method:

Instrument: Waters Alliance 2695; Column: YMC Cellulose SB 3 μ, 100x4.6; eluent A: hexane; eluent B: ethanol + 0.1 vol % diethylamine; isocratic: 90%A+10%B; flow: 1.4 mL/min; temperature: 25°C; UV: 254 nm.

Analytical Chiral HPLC (method Example 7): $R_t$ = 4.90 min.

$[a]_D$ = -12.45° (from solution in DMSO, c = 3.5 mg/mL)

$^1$H-NMR (400 MHz, DMSO-d6) δ [ppm] = 1.28 (d, 3H), 2.86 (s, 3H), 3.08 - 3.27 (m, 2H), 3.61 (dd, 1H), 3.68 (s, 2H), 6.49 - 6.56 (m, 2H), 6.58 - 6.68 (m, 1H), 7.00 (dd, 2H), 7.11 - 7.26 (m, 3H), 7.28 - 7.44 (m, 3H), 8.05 (d, 1H), 8.19 (s, 1H), 10.56 (s, 1H), 11.58 (s, 1H).

**Example 8**

(+)-N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide (Enantiomer 2)

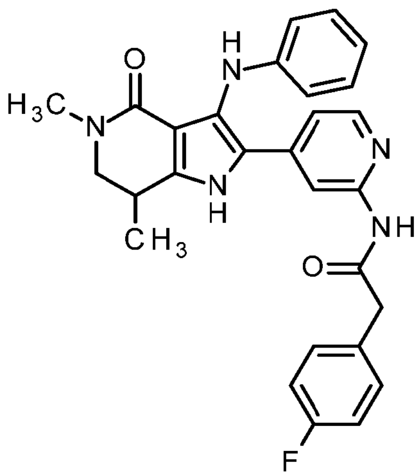

For the preparation of the racemic title compound see Example 6. Separation of enantiomers by preparative chiral HPLC (method see Example 7 gave the title compound (36 mg).

Analytical Chiral HPLC (method see Example 7: $R_t$ = 6.16 min.

$[a]_D$ = +11.25° (from solution in DMSO, c = 5.0 mg/mL)

$^1$H-NMR (400 MHz, DMSO-d6) δ [ppm] = 1.28 (d, 3H), 2.86 (s, 3H), 3.07 - 3.27 (m, 2H), 3.61 (dd, 1H), 3.69 (s, 2H), 6.49 - 6.55 (m, 2H), 6.57 - 6.69 (m, 1H), 7.00 (dd, 2H), 7.11 - 7.23 (m, 3H), 7.28 - 7.48 (m, 3H), 8.05 (d, 1H), 8.19 (s, 1H), 10.56 (s, 1H), 11.58 (s, 1H).

**Example 9**

N-{4-[(7RS)-3-anilino-5,7-dimethyl-4-oxo-4,5,6,7-tetrahydro-1H-pyrrolo[3,2-c]pyridin-2-yl]pyridin-2-yl}-2-(4-fluorophenyl)propanamide (Mixture of stereoisomers)

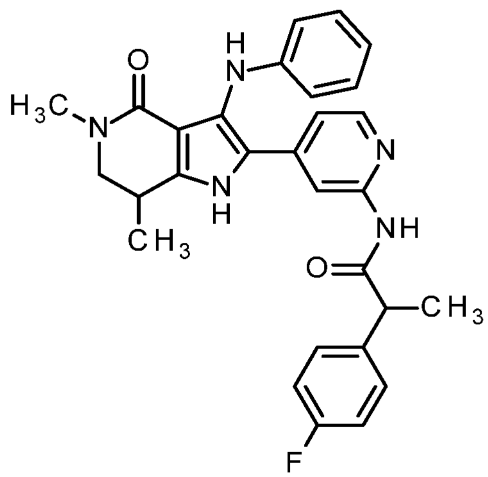

2-(2-Aminopyridin-4-yl)-3-anilino-5,7-dimethyl-1,5,6,7-tetrahydro-4H-pyrrolo[3,2-c]pyridin-4-one (Racemate) (see Intermediate 7, 300 mg), 2-(4-fluorophenyl)propanoic acid (Racemate) (218 mg, 1.30 mmol), N,N-diisopropylethylamine (900 μL, 5.2 mmol) and PyBOP (1.35 g, 2.59 mmol) were dissolved in 5.0 mL DMA and stirred at room temperature under argon atmosphere for 2 days. The reaction mixture was diluted with dichloromethane and water and aqueous saturated sodium hydorgencarbonate solution was added. It was stirred for 10 minutes and the organic layer was filtered through a silicone coated filter and concentrated under reduced pressure. The crude product was purified by flash chromatography (10g column, silica ULTRA; hexane / ethyl acetate 25%-100%) and HPLC under basic conditions to provide the target compound in 95% purity: 211 mg.

LC-MS (Method 1): $R_t$ = 1.12 min; MS (ESIpos): m/z = 499 $[M+H]^+$

$^1$H-NMR (400 MHz, DMSO-d6) δ [ppm]: 1.035 (4.98), 1.053 (9.20), 1.070 (5.17), 1.284 (3.27), 1.291 (3.64), 1.300 (3.85), 1.308 (3.63), 1.377 (6.22), 1.394 (6.54), 2.522 (5.21), 2.539 (2.26), 2.871 (16.00), 3.132 (0.46), 3.148 (0.89), 3.163 (0.91), 3.179 (0.67), 3.196 (1.47), 3.212 (0.56), 3.226 (1.30), 3.243 (0.80), 3.354 (1.15), 3.405 (0.75), 3.417 (0.78), 3.423 (2.11), 3.435 (2.20), 3.440 (2.14), 3.452 (2.12), 3.457 (0.85), 3.470 (0.77), 3.596 (0.55), 3.609 (0.94), 3.619 (0.77), 3.626 (0.74), 3.636 (0.83), 3.648 (0.56), 3.998 (1.31), 4.016 (1.33), 4.033 (0.44), 4.344 (1.44), 4.357 (2.86), 4.370 (1.44), 6.524 (3.24), 6.544 (3.47), 6.588 (0.98), 6.606 (1.95), 6.625 (1.10), 6.980 (2.35), 7.001 (3.24), 7.019 (2.07), 7.125 (2.08), 7.130 (2.28), 7.133 (2.61), 7.139 (2.64), 7.143 (2.39), 7.150 (1.32), 7.155 (4.74), 7.172 (0.96), 7.177 (2.67), 7.391 (4.29), 7.396 (3.78), 7.405 (3.07), 7.413 (2.76), 7.421 (1.18), 7.427 (2.30), 8.018 (2.77), 8.032 (2.71), 8.213 (2.65), 10.503 (3.03), 11.600 (2.24).

**Example 10**

N-{4-[3-anilino-5,7-dimethyl-4-oxo-4,5,6,7-tetrahydro-1H-pyrrolo[3,2-c]pyridin-2-yl]pyridin-2-yl}-4,4-difluoro-2-(4-fluorophenyl)butanamide (Mixture of stereoisomers)

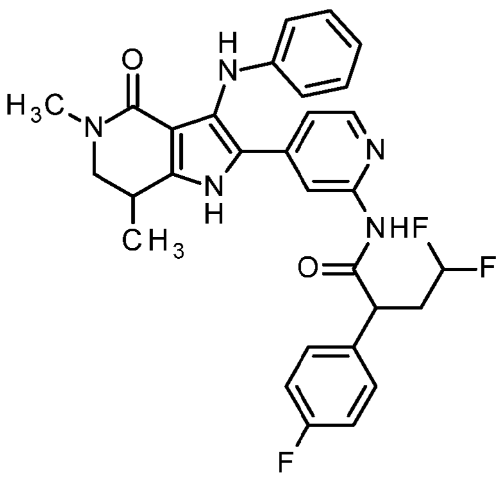

2-(2-Aminopyridin-4-yl)-3-anilino-5,7-dimethyl-1,5,6,7-tetrahydro-4H-pyrrolo[3,2-c]pyridin-4-one (Racemate) (see Intermediate 7, 300 mg), 4,4-difluoro-2-(4-fluorophenyl)butanoic acid (Racemate) (283 mg, 1.30 mmol), N,N-diisopropylethylamine (900 µL, 5.2 mmol) and PyBOP (1.35 g, 2.59 mmol) were dissolved in 5.0 mL DMA and were stirred at room temperature under Ar atmosphere for 2 days. The reaction mixture was diluted with dichloromethane and water and aqueous saturated sodium hydorgencarbonate solution was added. It was stirred for 10 minutes and the organic layer was filtered through a silicone coated filter and concentrated under reduced pressure. The crude product was purified by flash chromatography (10g column, silica ULTRA; hexane / ethyl acetate 25%-100%) and by HPLC under basic conditions to provide the target compound in 96% purity: 651 mg.

LC-MS (Method 1): $R_t$ = 1.18 min; MS (ESIpos): m/z = 549 $[M+H]^+$

$^1$H-NMR (400 MHz, DMSO-d6) δ [ppm]: 1.035 (5.51), 1.052 (9.51), 1.070 (5.85), 1.284 (3.10), 1.298 (4.07), 1.301 (4.35), 1.314 (3.43), 2.214 (0.42), 2.226 (0.44), 2.238 (0.47), 2.322 (0.55), 2.327 (0.82), 2.331 (0.62), 2.442 (0.51), 2.518 (10.22), 2.522 (7.30), 2.659 (0.57), 2.665 (0.91), 2.669 (1.14), 2.673 (0.94), 2.699 (0.40), 2.873 (16.00), 3.139 (0.49), 3.156 (0.93), 3.169 (0.92), 3.186 (0.74), 3.200 (1.52), 3.217 (0.52), 3.230 (1.26), 3.247 (0.77), 3.356 (1.71), 3.405 (0.44), 3.417 (0.50), 3.423 (1.02), 3.435 (1.12), 3.440 (1.10), 3.452 (1.05), 3.469 (0.41), 3.597 (0.53), 3.610 (0.96), 3.625 (1.12), 3.641 (0.93), 3.654 (0.52), 4.126 (0.63), 4.141 (0.79), 4.149 (0.81), 4.163 (0.69), 4.344 (0.44), 4.357 (0.81), 4.369 (0.48), 5.939 (0.73), 5.946 (0.75), 6.518 (3.00), 6.539 (3.39), 6.584 (0.85), 6.587 (0.89), 6.602 (1.75), 6.604 (1.75), 6.621 (1.01), 6.623 (1.00), 6.978 (2.05), 6.997 (3.30), 7.016 (1.79), 7.155 (2.26), 7.159 (2.84), 7.168 (2.61), 7.172 (2.49), 7.183 (4.43), 7.205 (2.47), 7.411 (2.97), 7.422 (2.42), 7.427 (1.31), 7.436 (2.55), 7.444 (2.48), 7.453 (1.08), 7.458 (2.14), 8.030 (2.71), 8.044 (2.67), 8.172 (2.32), 10.633 (2.35), 11.596 (2.06).

## Example 11

N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide (Racemate)

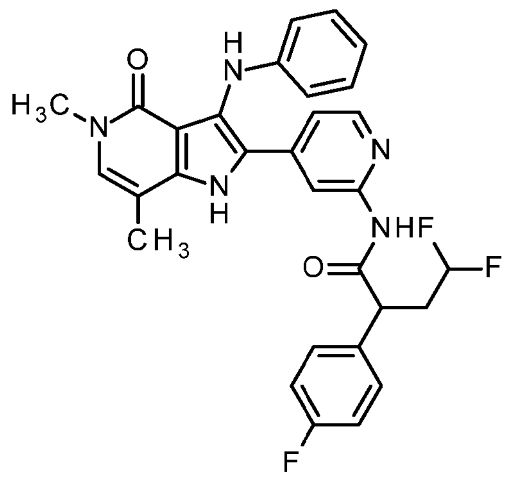

N-{4-[3-Anilino-5,7-dimethyl-4-oxo-4,5,6,7-tetrahydro-1H-pyrrolo[3,2-c]pyridin-2-yl]pyridin-2-yl}-4,4-difluoro-2-(4-fluorophenyl)butanamide (Racemate) (see Example 10, 100 mg) was dissolved in 7.8 mL dioxane and treated with 2,3-dichloro-5,6-dicyano-1,4-benzochinone (62.2 mg, 274 μmol). It was stirred at 80°C under argon atmosphere. The reaction mixture was diluted with ethyl acetate and aqueous sodiumhydroxide solution (1M) were added. The layers were separated, the aqueous layer was extracted with ethyl acetate three times. The combined organic layers were washed with water and brine, filtered through a silicone coated filter and concentrated under reduced pressure. The crude product was purified by flash chromatography (10g column, silica ULTRA; ethyl acetate / ethanol 0%-10%) and HPLC under basic conditions to provide the target compound in 92% purity: 56 mg.

LC-MS (Method 1): $R_t$ = 1.21 min; MS (ESIpos): m/z = 547 $[M+H]^+$

$^1$H-NMR (400 MHz, DMSO-d6) δ [ppm] = 2.13 - 2.29 (m, 4H), 2.67 (dt, 1H), 3.36 (s, 3H), 4.16 (dd, 1H), 5.75 - 6.12 (m, 1H), 6.53 (dd, 2H), 6.56 - 6.63 (m, 1H), 6.99 (dd, 2H), 7.13 - 7.25 (m, 3H), 7.37 (dd, 1H), 7.41 - 7.48 (m, 2H), 7.49 (s, 1H), 8.14 (d, 1H), 8.32 (s, 1H), 10.69 (s, 1H), 11.54 (s, 1H). - contains ethanol.

## Example 12

N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide (Enantiomer 1)

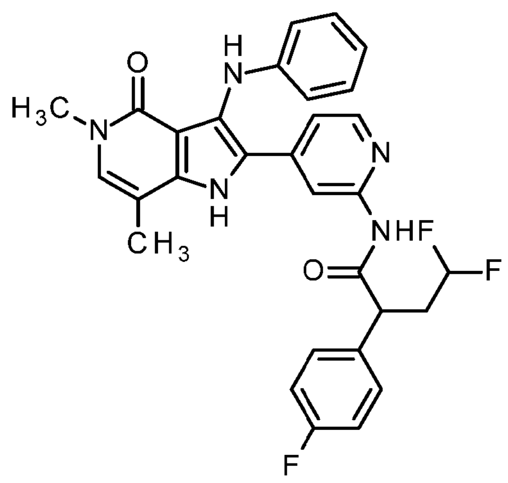

The racemic compound (see Example 11, 37 mg) was separated into enantiomers by preparative chiral HPLC to give enantiomer 1 (16 mg, 100 %ee, see Example 12) and enantiomer 2 (15 mg, 99.4 % ee, see Example 13).

Preparative chiral HPLC method:

Instrument: PrepCon Labomatic HPLC-3; Column: YMC Cellulose SB 5 µ, 250x30; eluent A: methyl tert-butyl ether + 0.1 vol % diethylamine; eluent B: acetonitril; isocratic: 90%A+10%B; flow: 40 mL/min; temperature: 25 °C; UV: 254 nm.

Analytical chiral HPLC method:

Instrument: Thermo Fisher UltiMate 3000; Column: YMC Cellulose SB 3 µ, 100x4.6; eluent A: methyl tert-butyl ether + 0.1 vol % diethylamine; eluent B: acetonitril; isocratic: 90%A+10%B; flow: 1.4 mL/min; temperature: 25 °C; UV: 254 nm.

Analytical Chiral HPLC (method Example 12): $R_t$ = 2.68 min.
$^1$H-NMR (400 MHz, DMSO-d6) δ [ppm] = 2.10 - 2.20 (m, 1H), 2.24 (d, 3H), 2.54 (s, 3H), 2.62 - 2.76 (m, 1H), 4.15 (br dd, 1H), 5.77 - 6.12 (m, 1H), 6.53 (d, 2H), 6.59 (t, 1H), 6.95 - 7.04 (m, 2H), 7.12 - 7.23 (m, 3H), 7.37 (dd, 1H), 7.40 - 7.52 (m, 3H), 8.14 (d, 1H), 8.32 (s, 1H), 10.69 (s, 1H), 11.54 (s, 1H). - impurities in the aliphatic range.

**Example 13**

N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide (Enantiomer 2)

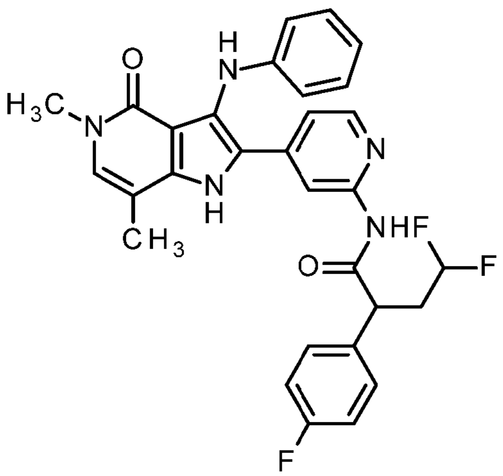

For the preparation of the racemic title compound see Example 11. Separation of enantiomers by preparative chiral HPLC (method see Example 12 gave the title compound (15 mg).

Analytical Chiral HPLC (method see Example 12): $R_t$ = 5.30 min.

$^1$H-NMR (400 MHz, DMSO-d6) δ [ppm] = 2.09 - 2.20 (m, 1H), 2.24 (d, 3H), 2.52 (s, 3H), 2.61 - 2.75 (m, 1H), 4.15 (br dd, 1H), 5.78 - 6.13 (m, 1H), 6.53 (d, 2H), 6.56 - 6.63 (m, 1H), 6.99 (dd, 2H), 7.13 - 7.23 (m, 3H), 7.37 (dd, 1H), 7.42 - 7.52 (m, 3H), 8.14 (d, 1H), 8.32 (s, 1H), 10.69 (s, 1H), 11.54 (s, 1H). - impurities in the aliphatic range.

**Example 14**

N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide (Racemate)

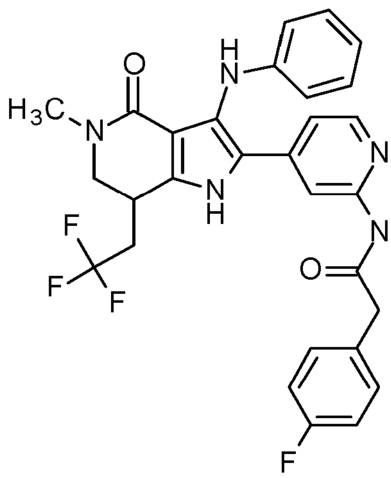

To a mixture of (4-fluorophenyl)acetic acid (66.8 mg, 433 µmol) , N,N-diisopropylethylamine (190 µl, 1.1 mmol) in N,N-dimethylformamide (10 mL) was added O-(7-azabenzotriazol-1-yl)-N,N,N,N-tetramethyluroniumhexafluorophosphate (206 mg, 542 µmol) and 2-(2-aminopyridin-4-yl)-3-anilino-5-methyl-7-(2,2,2-trifluoroethyl)-1,5,6,7-tetrahydro-4H-pyrrolo[3,2-c]pyridin-4-one (see Intermediate 17, 150 mg) at 0 °C. The mixture was stirred at room temperature for 16 hours. The mixture was poured into water and extracted with ethyl acetate. The combined organic phase was washed with brine, dried (sodium sulfate), filtered and concentrated in vacuum. The residue was purified by preparative TLC (petroleum ether: ethyl acetate = 1: 1) to give N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide (40 mg, crude) and N-{4-[3-anilino-5-methyl-4-oxo-7-(2,2,2-trifluoroethyl)-4,5-dihydro-1H-pyrrolo[3,2-c]pyridin-2-yl]pyridin-2-yl}-2-(4-fluorophenyl)acetamide (20 mg, crude). The crude product N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide (40 mg, crude) was further purified by preparative HPLC [Instrument:Gilson-281; Column: Xtimate C18 150*40mm*10um; eluent A: water (0.05% ammonia hydroxide), eluent B: acetonitrile; gradient: 0-10 min 43-73% B; flow 25 mL/min; temperature: room temperature; Detector: UV 220/254 nm.] to give N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide (20.10 mg, 98 % purity) as a white solid.

LC-MS (Method 3): $R_t$ = 0.877 min; MS (ESIpos): m/z = 552.0 $[M+H]^+$.

$^1$H-NMR (400 MHz, DMSO-$d_6$): δ [ppm] = 11.64 (s, 1H), 10.59 (s, 1H), 8.22 (s, 1H), 8.07 (d, 1H), 7.37-7.34 (m, 3H), 7.17-7.13 (m, 3H), 7.01 (t, 2H), 6.61 (t, 1H), 6.54 (d, 2H), 3.74-3.69 (m, 3H), 3.43-3.39 (m, 2H), 2.88-2.82 (m, 4H), 2.67-2.60 (m, 1H).

$^1$H-NMR (400 MHz, DMSO-$d_6$+$D_2O$): δ [ppm] =, 8.16 (s, 1H), 8.05 (d, 1H), 7.32 (dd, 2H), 7.17-7.10 (m, 3H), 6.99 (t, 2H), 6.61 (t, 1H), 6.51 (d, 2H), 3.76 (s, 2H), 3.66 (s, 1H), 3.40 (d, 2H), 2.86 (s, 3H), 2.80-2.74 (m, 1H), 2.62-2.59 (m, 1H).

## Example 15

N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide (Enantiomer 1)

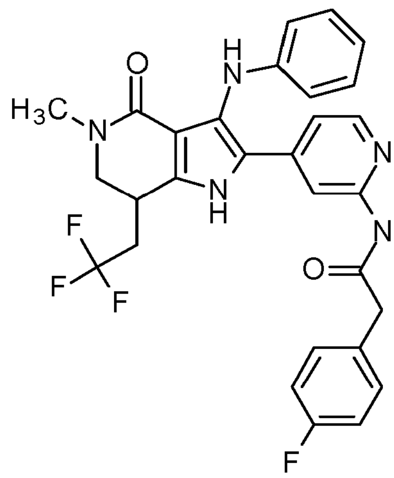

The racemic compound (see Example 14, 15 mg) was separated into enantiomers by preparative chiral HPLC to give enantiomer 1 (6.3 mg, 93.8 %ee, see Example 15) and enantiomer 2 (5.2 mg, 96.3 % ee, see Example 16).

Preparative chiral HPLC method:

Instrument: Waters 80Q SFC; Column: DAICEL CHIRALCEL OD(250mm*30mm,10um); Mobile Phase: Phase A for Supercritical carbon dioxide, Phase B for methyl alcohol (0.1% ammonia water); Isocratic elution: 40% Phase B (60% Phase A); Flow Rate:70 g/min Cycle Time:4.0 min,total time:25min Single injetion volume:4.0ml Back Pressure:100 bar to keep the carbon dioxide in Supercritical flow; temperature: room temperature; Detector: UV 220 nm).

Analytical chiral HPLC method:

SFC (Method:Column: Chiralcel OJ-3 50×4.6mm I.D., 3um; Mobile phase: Phase A for carbon dioxide, and Phase B for methyl alcohol (0.05% diethylamine); Gradient elution: methyl alcohol (0.05% diethylamine) in carbon dioxide from 5% to 40%; Flow rate: 3mL/min; Detector: PDA; Column Temp: 35C;Back Pressure: 100Bar).

Analytical Chiral HPLC (method Example 15): $R_t$ = 1.75 min.

LC-MS (Method 3): $R_t$ = 0.868 min; MS (ESIpos): m/z = 552.3 $[M+H]^+$

$^1H$ NMR (400 MHz, DMSO-$d_6$): δ [ppm] = 11.67 (s, 1H), 10.58 (s, 1H), 8.22 (s, 1H), 8.07 (d, 1H), 7.38-7.35 (m, 3H), 7.18-7.13 (m, 3H), 7.03-6.99 (m, 2H), 6.64-6.60 (m, 1H), 6.55 (d, 2H), 3.75-3.74 (m, 1H), 3.70 (s, 2H), 3.45-3.40 (m, 2H), 2.89 (s, 3H), 2.86-2.79 (m, 1H).

**Example 16**

N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide (Enantiomer 2)

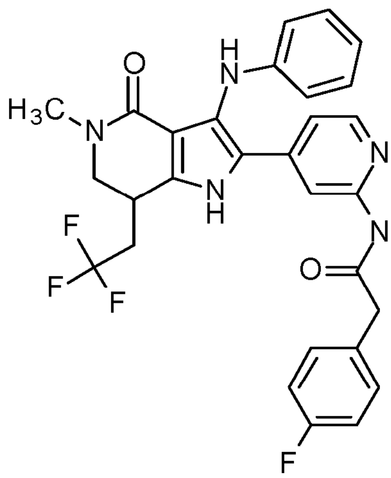

For the preparation of the racemic title compound see Example 14. Separation of enantiomers by preparative chiral HPLC (method see Example 15) gave the title compound (5.2 mg).

Analytical Chiral HPLC (method see Example 15): $R_t$ = 1.57 min.

LC-MS (Method 3): $R_t$ = 0.868 min; MS (ESIpos): m/z = 552.3 $[M+H]^+$

$^1H$ NMR (400 MHz, DMSO-$d_6$): δ [ppm] = 11.68 (s, 1H), 10.58 (s, 1H), 8.22 (s, 1H), 8.07 (d, 1H), 7.38-7.35 (m, 3H), 7.18-7.13 (m, 3H), 7.03-6.99 (m, 2H), 6.64-6.60 (m, 1H), 6.55 (d, 2H), 3.75-3.74 (m, 1H), 3.70 (s, 2H), 3.45-3.40 (m, 2H), 2.89 (s, 3H), 2.86-2.79 (m, 1H).

## Example 17

N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-4,4-difluoro-2-(4-fluorophenyl)butanamide (Mixture of 4 Stereoisomers)

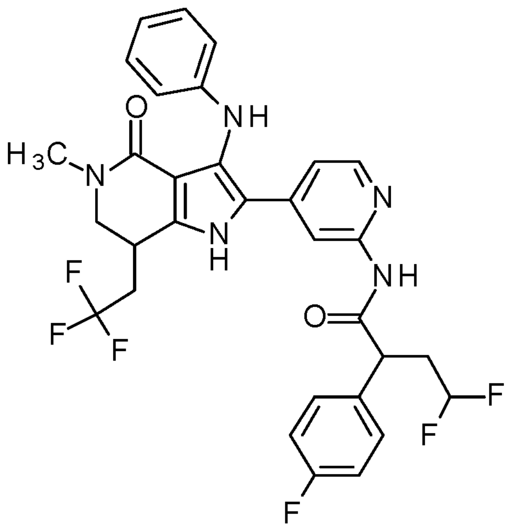

2-(2-aminopyridin-4-yl)-3-anilino-5-methyl-7-(2,2,2-trifluoroethyl)-1,5,6,7-tetrahydro-4H-pyrrolo-[3,2-c]pyridin-4-one (Racemate) (see Intermediate 17, 320 mg), 4,4-difluoro-2-(4-fluorophenyl)butanoic acid (Racemate) (CAS-RN:[1538957-14-0], 420 mg), N,N-diisopropylethylamine (810 μL, 4.6 mmol) and PYBOP (1.20 g, 2.31 mmol) were dissolved in 1.7 mL DMA and stirred at room temperature under argon atmosphere over night. To the reaction mixture aqueous saturated sodium hydrogencarbonate solution, water and ethyl acetate was added. The aqueous layer was

extracted with ethyl acetate three times. The combined organic layers were washed with brine, filtered through a waerresistant filter and concentrated under reduced pressure. The crude product was purified by flash chromatography (25 g column, aminophase coupled with 25 g silica ULTRA column; dichloromethane / ethanol 0%-4% ethanol). Afterwards it was stirred in dichloromethane and filtered to provide the target compound: 280 mg.

LC-MS (Method 2): $R_t$ = 1.35 min; MS (ESIpos): m/z = 616 $[M+H]^+$

$^1$H-NMR (400 MHz, DMSO-d6): δ [ppm] = 11.71 (s, 1H), 10.69 (s, 1H), 8.20 (d, 1H), 8.06 (d, 1H), 7.51-7.38 (m, 3H), 7.24-7.14 (m, 3H), 7.00 (t, 2H), 6.61 (td, 1H), 6.56-6.49 (m, 2H), 6.12-5.79 (m, 1H), 4.16 (dd, 1H), 3.80-3.70 (m, 1H), 3.51-3.38 (m, 2H), 2.96-2.81 (m, 4H), 2.77-2.59 (m, 2H), 2.30-2.12 (m, 1H)

**Example 18**

N-{4-[3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide (Racemate)

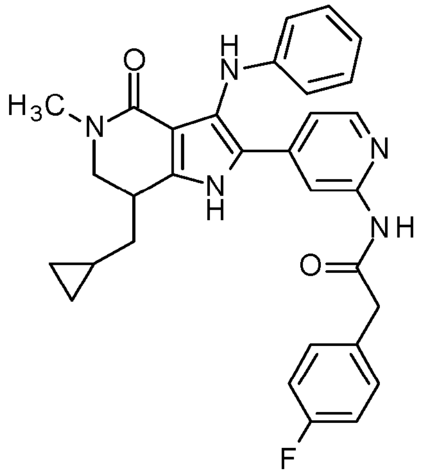

To a mixture of (4-fluorophenyl)acetic acid (119 mg, 774 μmol) and 2-(2-aminopyridin-4-yl)-3-anilino-7-(cyclopropylmethyl)-5-methyl-1,5,6,7-tetrahydro-4H-pyrrolo[3,2-c]pyridin-4-one (Racemate) (see Intermediate 27, 200 mg, 516 μmol) in N,N-dimethyl formamide (3 mL) was added benzotriazol-1-yl-oxytripyrrolidinophosphoniumhexafluorophosphate (PyBOP) (537 mg, 1.03 mmol) and N,N-Diisopropylethylamine (540 μL, 3.1 mmol) at room temperature. The mixture was stirred at room temperature for 16 hours. Then N,N-Diisopropylethylamine (540 μL, 3.1 mmol) and 2-(7-Azabenzotriazol-1-yl)-N,N,N',N'-tetramethyluronium hexafluorophosphate (393 mg, 1.03 mmol]) was added to the mixture. The mixture was stirred at room temperature for 16 hours. The mixture was diluted in dichloromethane, then washed with saturated sodium bicarbonate, and followed by 1M sodium hydroxide. The organic phase was washed with brine, dried (sodium sulfate), filtered and concentrated to give a residue. The residue was purified by HPLC [ACSWH-GX-K; Column: Phenomenex Synergi C18 150*30mm*4um; eluent A: water(0.1%TFA), eluent B: acetonitrile; gradient: 0-7min 2% B; flow 25 mL/min; temperature: room temperature; Detector: UV 220/254 nm.] and lyophilization to give 30.0 mg N-{4-[3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide (Racemate) in 98 % purity as yellow solid.

LC-MS (Method 3): $R_t$ = 0.901 min; MS (ESIpos): m/z = 524.3 $[M+H]^+$.

$^1$H-NMR (400 MHz, MeOD): δ [ppm] = 0.81 (d, 1H), 7.55-7.50 (m, 1H), 7.42 (d, 1H), 7.33-7.29 (m, 2H), 7.11-7.03 (m, 4H), 6.76 (t, 1H), 6.68 (d, 2H), 3.89 (dd, 5.2 Hz, 1H), 3.73 (s, 2H), 3.59 (dd, 1H),, 3.19-3.13 (m, 1H), 3.03 (s, 3H), 1.70-1.63 (m, 2H), 0.84-0.80 (m, 1H), 0.57-0.46 (m, 2H), 0.12-0.01 (m, 2H).

**Example 19**

N-{4-[3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide (Enantiomer 1)

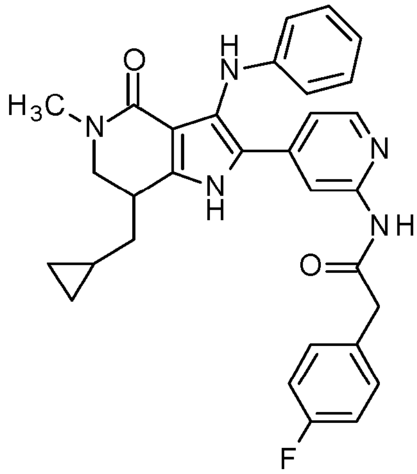

The racemic compound (see Example 18, 20 mg) was separated into enantiomers by preparative-SFC to give enantiomer 1 (4.1 mg, 97.7 %ee, see Example 19) and enantiomer 2 (5.0 mg, 96.5% ee, see Example 20).

Preparative SFC method:

Instrument: SFC, Column: DAICEL CHIRALPAK AD(250mm*30mm,10um)); Mobile Phase: 45% isopropyl alcohol (0.1% ammonia water) in Supercritical carbon dioxide; Flow Rate:70 g/min Cycle Time:3.5 min, total time:40 min Single injection volume:3.5 mL Back Pressure:100 bar to keep the carbon dioxide in Supercritical flow; temperature: room temperature; Detector: UV 220 nm)

Analytical SFC method:

Instrument: SFC, Column: Chiralcel OJ-3 50×4.6mm I.D., 3um; Mobile phase: Phase A for carbon dioxide, and Phase B for isopropyl alcohol (0.05% diethylamine); Gradient elution:40% isopropyl alcohol (0.05% diethylamine) in carbon dioxide from 5% to 40%; Flow rate: 3mUmin;Detector: PDA; Column Temp: 35C;Back Pressure: 100Bar.

Analytical SFC (method Example 19): $R_t$ = 0.932 min.

LC-MS (Method 3): $R_t$ = 0.897 min; MS (ESIpos): m/z = 524.3 $[M+H]^+$.

$^1$H NMR (400 MHz, MeOD): δ [ppm] = 0.81 (s, 1H), 7.79 (d, 1H), 7.36-7.32 (m, 2H), 7.21 (dd, 1H), 7.08-6.99 (m, 4H), 6.66 (t, 1H), 6.60 (d, 2H), 3.86 (dd, 5.2 Hz, 1H), 3.70 (s, 2H), 3.54 (dd, 1H), 3.15-3.12 (m, 1H), 3.01 (s, 3H), 1.67-1.60 (m, 2H), 0.82-0.78 (m, 1H), 0.55-0.45 (m, 2H), 0.10-0.00 (m, 2H).

**Example 20**

N-{4-[3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide (Enantiomer 2)

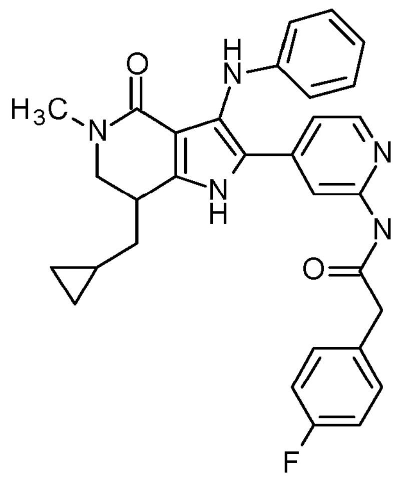

For the preparation of the racemic title compound see Example 18. Separation of enantiomers by preparative -SFC (method see Example 19) gave the title compound (5.0 mg).

Analytical SFC (method see Example 19): $R_t$ = 1.391 min.

LC-MS (Method 3): $R_t$ = 0.903 min; MS (ESIpos): m/z = 524.3 $[M+H]^+$.

$^1H$ NMR (400 MHz, MeOD): δ [ppm] = 8.00 (d, 1H), 7.76-7.61 (m, 1H), 7.36-7.30 (m, 3H), 7.08-7.03 (m, 4H), 6.73 (t, 1H), 6.65 (d, 2H), 3.88 (dd, 1H), 3.72 (s, 2H), 3.57 (dd, 1H), 3.19-3.12 (m, 1H), 3.02 (s, 3H), 1.67-1.62 (m, 2H), 0.90-0.86 (m, 1H), 0.56-0.45 (m, 2H), 0.12-0.00 (m, 2H).

## Example 21

(2RS)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide (Mixture of 4 Stereoisomers)

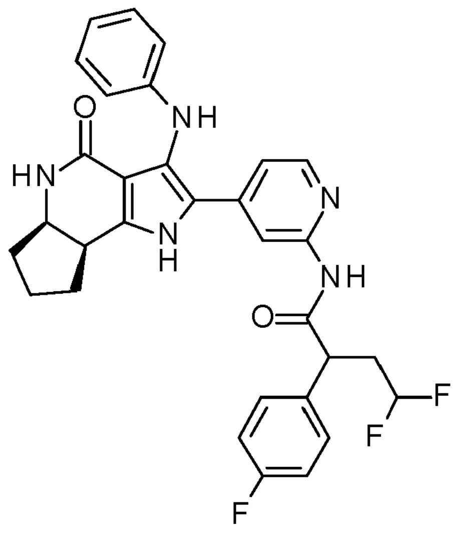

To (2RS)-N-(4-{(cis)-3-anilino-5-[(2,4-dimethoxyphenyl)methyl]-4-oxo-1,4,5,5a,6,7,8,8a-octahydrocyclopenta[b]pyrrolo[2,3-d]pyridin-2-yl}pyridin-2-yl)-4,4-difluoro-2-(4-fluorophenyl)butanamide (Mixture of 4 Stereoisomers) (see Intermediate 37, 18.0 mg) in ethanol (470μL) / tetrahydrofuran (240 μL) (2:1) hydrochloric acid in water (240 μL, 4.0 M, 940 μmol) was added and it was stirred at 80 °C for 2 h. Aqueous saturated sodium hydrogencarbonate solution was added to the reaction mixture. The aqueous layer was extracted with ethyl acetate two times. The combined organic

layers were washed with brine once, dried using a water resistant filter and concentrated under reduced pressure. The crude product was purified by flash chromatography (10 g column, silica ULTRA; gradient dichloromethane/ethanol 0-10%) to provide the target compound: 2.5 mg.

LC-MS (Method 2): $R_t$ = 1.19 min; MS (ESIpos): m/z = 560 $[M+H]^+$

$^1$H-NMR (400 MHz, DMSO-$d_6$): $\delta$ [ppm] = 11.60 (s, 1H), 10.61 (s, 1H), 8.16 (s, 1H), 8.03 (d, 1H), 7.56 (d, 1H), 7.49-7.30 (m, 4H), 7.23-7.12 (m, 5H), 7.08-6.96 (m, 3H), 6.61 (t, 1H), 6.58-6.52 (m, 3H), 6.46 (dd, 1H), 6.12-5.79 (m, 1H), 4.87 (d, 1H), 4.12 (br d, 2H), 3.88-3.81 (m, 1H), 3.78 (s, 3H), 3.73 (s, 3H), 3.49 (dt, 1H), 3.19-3.10 (m, 1H), 2.17 (br s, 1H), 2.12-1.95 (m, 3H), 1.60-1.44 (m, 3H).

**Example 22**

(+)-(2R or S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide (Enantiomer 1)

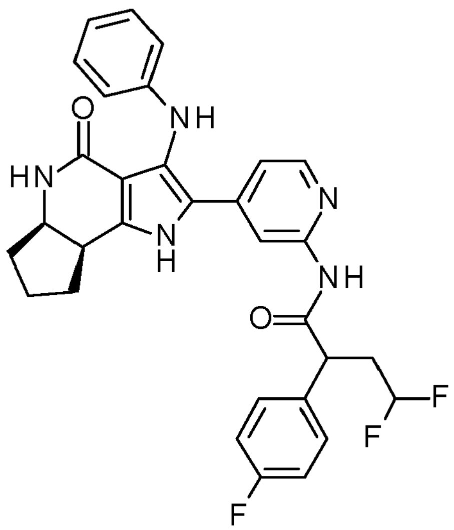

The mixture of the four stereoisomeres (see Example 21, 208 mg) was separated by preparative HPLC to give stereoisomer 1 (38 mg, 100 %ee, see Example 22), stereoisomer 2 (30 mg, 97.3% ee, see Example 23), stereoisomer 3 (35 mg, 100% ee, see Example 24) and stereoisomer 4 (38 mg, 100% ee, see Example 25).

Preparative HPLC method MTBE:

Instrument: PrepCon Labomatic HPLC-3; Column: YMC Amylose SA 10μ, 250x50; eluent A: methyl tert-butyl ether + 0.1 vol % diethylamine; eluent B: methanol; isocratic: 90%A+10%B; flow: 100 mL/min; temperature: 25°C; UV: 254 nm

Analytical HPLC method MTBE:

Instrument: Thermo Fisher UltiMate 3000; Column: YMC Amylose SA 3μ, 100x4.6; eluent A: methyl tert-butyl ether + 0.1 vol % diethylamine; eluent B: methanol; isocratic: 90%B+10%B; flow: 1.4 mL/min; temperature: 25°C; UV: 254 nm

Analytical HPLC MTBE (method Example 22): $R_t$ = 1.57 min.

$[\alpha]_D$ =: +271,9° (from solution in DMSO, c = 1,63 mg/mL)

LC-MS (Method 2): $R_t$ = 1.23 min; MS (ESIpos): m/z = 560 $[M+H]^+$

$^1$H-NMR (400 MHz, DMSO-$d_6$): δ [ppm] = 11.67 (s, 1H), 10.63 (s, 1H), 8.15 (s, 1H), 8.01 (d, 1H), 7.49-7.41 (m, 2H), 7.40 (s, 1H), 7.22-7.15 (m, 2H), 7.12 (dd, 1H), 7.05-6.96 (m, 3H), 6.64-6.58 (m, 1H), 6.55 (dd, 2H), 6.11-5.78 (m, 1H), 4.15 (dd, 1H), 4.05 (br t, 1H), 3.26-3.16 (m, 1H), 2.79-2.60 (m, 1H), 2.29-2.04 (m, 2H), 1.94-1.82 (m, 1H), 1.81-1.60 (m, 4H)

**Example 23**

(-)-(2R or S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide (Enantiomer 2)

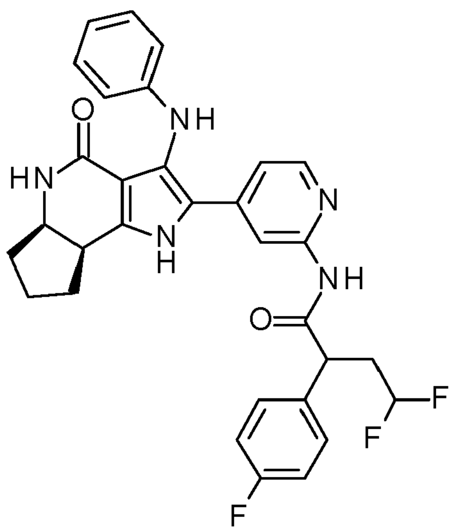

For the preparation of the compound (mixture of the four stereoisomeres) see Example 21. Separation of stereoisomeres by preparative HPLC (method see Example 22) gave the title compound (30 mg).

Analytical HPLC MTBE (method Example 22): $R_t$ = 1.77 min.

$[\alpha]_D$ = -85,7° (from solution in DMSO, c = 1,90 mg/mL)

LC-MS (Method 2): Rt = 1.23 min; MS (ESIpos): m/z = 560 $[M+H]^+$

$^1$H-NMR (400 MHz, DMSO-$d_6$): δ [ppm] = 11.67 (s, 1H), 10.63 (s, 1H), 8.15 (s, 1H), 8.01 (d, 1H), 7.47-7.41 (m, 2H), 7.40 (s, 1H), 7.22-7.15 (m, 2H), 7.12 (dd, 1H), 7.05-6.97 (m, 3H), 6.61 (t, 1H), 6.58-6.52 (m, 2H), 6.12-5.79 (m, 1H), 4.15 (dd, 1H), 4.06 (br t, 1H), 3.27-3.16 (m, 1H), 2.78-2.59 (m, 1H), 2.29-2.04 (m, 2H), 1.94-1.81 (m, 1H), 1.80-1.60 (m, 4H)

**Example 24**

(-)-(2R or S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide (Enantiomer 3)

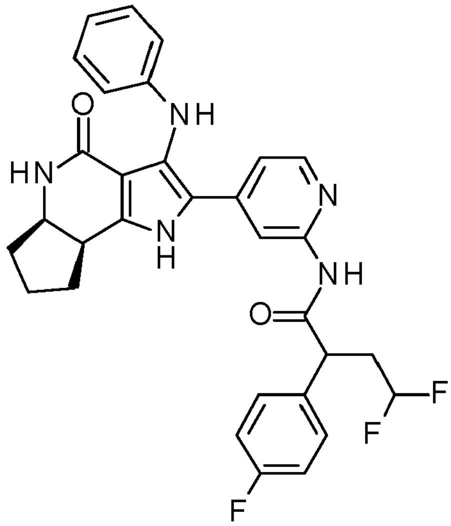

For the preparation of the compound (mixture of the four stereoisomeres) see Example 21. Separation of stereoisomeres by preparative HPLC (method see Example 22) gave the title compound (35 mg).

Analytical HPLC MTBE (method Example 22): $R_t$ = 3.42 min.

$[\alpha]_D$ = -282,5 ° (from solution in DMSO, c = 1,71 mg/mL)

LC-MS (Method 2): $R_t$ = 1.23 min; MS (ESIpos): m/z = 560 $[M+H]^+$

$^1$H-NMR (400 MHz, DMSO-$d_6$): δ [ppm] = 11.68 (s, 1H), 10.63 (s, 1H), 8.16 (s, 1H), 8.01 (d, 1H), 7.48-7.42 (m, 2H), 7.40 (s, 1H), 7.22-7.15 (m, 2H), 7.12 (dd, 1H), 7.06-6.97 (m, 3H), 6.61 (t, 1H), 6.54 (d, 2H), 6.12-5.78 (m, 1H), 4.15 (dd, 1H), 4.09-4.01 (m, 1H), 3.26-3.17 (m, 1H), 2.77-2.62 (m, 1H), 2.27-2.06 (m, 2H), 1.94-1.82 (m, 1H), 1.80-1.62 (m, 4H)

## Example 25

(+)-(2R or S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide (Enantiomer 4)

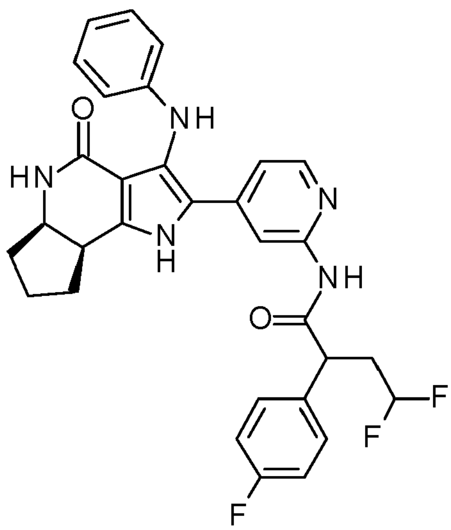

For the preparation of the compound (mixture of the four stereoisomeres) see Example 21. Separation of

stereoisomeres by preparative HPLC (method see Example 22) gave the title compound (38 mg).

Analytical HPLC MTBE (method Example 22): $R_t$ = 6.75 min.

$[\alpha]_D$ = +102,3 ° (from solution in DMSO, c = 1,82 mg/mL)

LC-MS (Method 2): Rt = 1.23 min; MS (ESIpos): m/z = 560 $[M+H]^+$

$^1$H-NMR (400 MHz, DMSO-$d_6$): $\delta$ [ppm] = 11.67 (s, 1H), 10.63 (s, 1H), 8.15 (s, 1H), 8.02 (d, 1H), 7.47-7.41 (m, 2H), 7.40 (s, 1H), 7.18 (t, 2H), 7.12 (dd, 1H), 7.05-6.97 (m, 3H), 6.61 (t, 1H), 6.55 (d, 2H), 6.13-5.79 (m, 1H), 4.15 (dd, 1H), 4.09-4.02 (m, 1H), 3.26-3.17 (m, 1H), 2.78-2.63 (m, 1H), 2.26-2.04 (m, 2H), 1.93-1.82 (m, 1H), 1.80-1.61 (m, 4H)

**Example 26**

(2S)-N-[4-(3-anilino-5-methyl-4-oxo-4,5-dihydro-1H-pyrrolo[3,2-c]pyridin-2-yl)pyridin-2-yl]-2-(4-fluorophenyl)propanamide (Enantiomer 1)

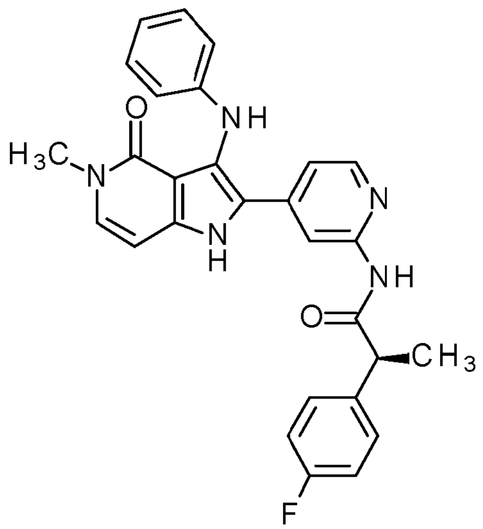

To (2S)-N-[4-(3-anilino-5-methyl-4-oxo-4,5,6,7-tetrahydro-1H-pyrrolo[3,2-c]pyridin-2-yl)pyridin-2-yl]-2-(4-fluorophenyl)propanamide (Enantiomer 1) (see Intermediate 42, 500 mg) in 1,4-dioxane (33 mL) distributed to two 20-mL-microwave tubes 4,5-dichloro-3,6-dioxocyclohexa-1,4-diene-1,2-dicarbonitrile (305 mg, 1.34 mmol) was added. The reaction mixture was stirred at 80 °C for 1 hour. The reaction mixture was diluted with aqueous saturated sodium hydrogencarbonate solution, water and treated with ethyl acetate. The layers were separated and the aqueous layer was extracted with ethyl acetate twice. The combined organic layers were dried using a water resistant filter and concentrated under reduced pressure. The crude product was purified by flash chromatography (55 g column, aminophase; dichloromethane / ethanol 0%-5% ethanol). Afterwards it was stirred in dichloromethane and filtered to provide the target compound: 275 mg.

LC-MS (Method 1): $R_t$ = 1.09 min; MS (ESIpos): m/z = 482 $[M+H]^+$

$^1$H NMR (400 MHz, DMSO-$d_6$) $\delta$ 11.84 (s, 1H), 10.60 (s, 1H), 8.33 (d, 1H), 8.11 (d, 1H), 7.46 (s, 1H), 7.39-7.45 (m, 2H), 7.34 (d, 1H), 7.32 (dd, 1H), 7.12-7.20 (m, 2H), 7.01 (dd, 2H), 6.60 (t, 1H), 6.53-6.57 (m, 2H), 6.40 (d, 1H), 4.03 (q, 1H), 3.39 (s, 3H), 1.40 (d, 3H).

**Example 27**

(2R)-N-[4-(3-anilino-5-methyl-4-oxo-4,5-dihydro-1H-pyrrolo[3,2-c]pyridin-2-yl)pyridin-2-yl]-2-(4-fluorophenyl)propanamide (Enantiomer 2)

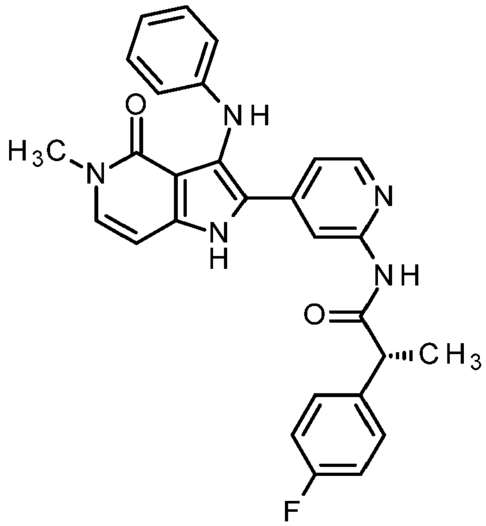

To (2R)-N-[4-(3-anilino-5-methyl-4-oxo-4,5,6,7-tetrahydro-1H-pyrrolo[3,2-c]pyridin-2-yl)pyridin-2-yl]-2-(4-fluorophenyl)propanamide (Enantiomer 2) (see Intermediate 43, 500 mg) in 1,4-dioxane (33 mL) distributed to two 20-mL-microwave tubes 4,5-dichloro-3,6-dioxocyclohexa-1,4-diene-1,2-dicarbonitrile (305 mg, 1.34 mmol) was added. The reaction mixture was stirred at 80 °C for 1 hour. The reaction mixture was diluted with aqueous saturated sodium hydrogencarbonate solution, water and treated with ethyl acetate. The layers were separated and the aqueous layer was extracted with ethyl acetate twice. The combined organic layers were dried using a water resistant filter and concentrated under reduced pressure. The crude product was purified by flash chromatography (55 g column, aminophase; dichloromethane / ethanol 0%-5% ethanol). Afterwards it was stirred in dichloromethane and filtered to provide the target compound: 262 mg.

LC-MS (Method 1): Rt = 1.12 min; MS (ESIneg): m/z = 480 $[M-H]^-$

$^1$H-NMR (400 MHz, DMSO-d6): δ [ppm] = 11.85 (s, 1H), 10.60 (s, 1H), 8.33 (d, 1H), 8.11 (d, 1H), 7.49-7.39 (m, 3H), 7.37-7.29 (m, 2H), 7.20-7.12 (m, 2H), 7.01 (dd, 2H), 6.64-6.51 (m, 3H), 6.40 (d, 1H), 4.03 (q, 1H), 3.38 (s, 3H), 1.40 (d, 3H)

**Example 28**

N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide (Racemate)

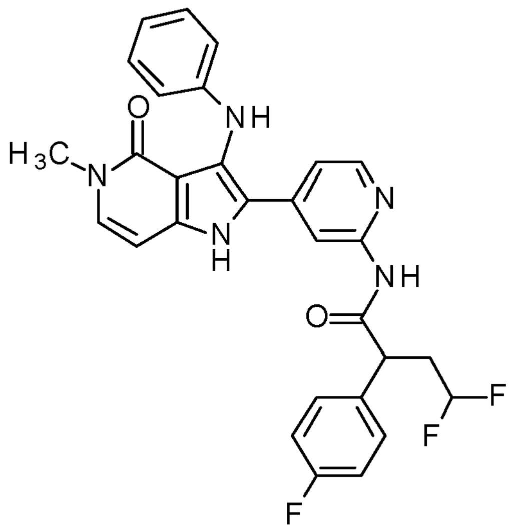

To N-[4-(3-anilino-5-methyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate) (see Intermediate 44, 1.08 g) in 1,4-dioxane (65 mL) 4,5-dichloro-3,6-dioxocyclohexa-1,4-diene-1,2-dicarbonitrile (598 mg, 2.63 mmol) was added. The reaction mixture was stirred at 80°C for 1 hour. The reaction mixture (Combined with another batch starting from 1.0 g) was diluted with aqueous saturated sodium hydrogencarbonate solution, water and treated with ethyl acetate. The layers were separated and the aqueous layer was extracted with ethyl acetate twice. The combined organic layers were dried using a water resistant filter and concentrated under reduced pressure. The crude product was purified by flash chromatography (100 g column, aminophase; dichloromethane / ethanol 0%-5% ethanol). Afterwards it was stirred in dichloromethane and filtered to provide the target compound: 1.10 g.

LC-MS (Method 1): $R_t$ = 1.17 min; MS (ESIpos): m/z = 532 $[M+H]^+$

$^1$H-NMR (400 MHz, DMSO-d6) δ [ppm]: 2.322 (0.50), 2.326 (0.70), 2.332 (0.52), 2.518 (2.28), 2.522 (1.44), 2.664 (0.75), 2.669 (0.89), 2.673 (0.77), 3.387 (16.00), 4.151 (0.64), 4.165 (0.72), 4.174 (0.72), 4.188 (0.58), 5.835 (0.56), 5.964 (0.51), 5.976 (1.14), 5.987 (0.51), 6.116 (0.51), 6.404 (4.11), 6.422 (4.07), 6.539 (2.51), 6.541 (3.16), 6.560 (3.31), 6.563 (2.72), 6.579 (0.64), 6.582 (1.00), 6.599 (1.94), 6.618 (1.10), 6.989 (2.45), 7.007 (3.09), 7.010 (2.95), 7.029 (2.00), 7.167 (2.16), 7.173 (0.71), 7.185 (0.93), 7.190 (4.46), 7.195 (0.85), 7.207 (0.78), 7.212 (2.39), 7.321 (2.02), 7.325 (2.08), 7.335 (2.35), 7.338 (4.78), 7.356 (3.16), 7.438 (2.14), 7.444 (1.00), 7.452 (2.39), 7.461 (2.20), 7.473 (5.34), 8.111 (2.60), 8.112 (2.52), 8.126 (2.40), 8.297 (2.24), 10.732 (2.66), 11.850 (1.79).

**Example 29**

N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide (Enantiomer 1)

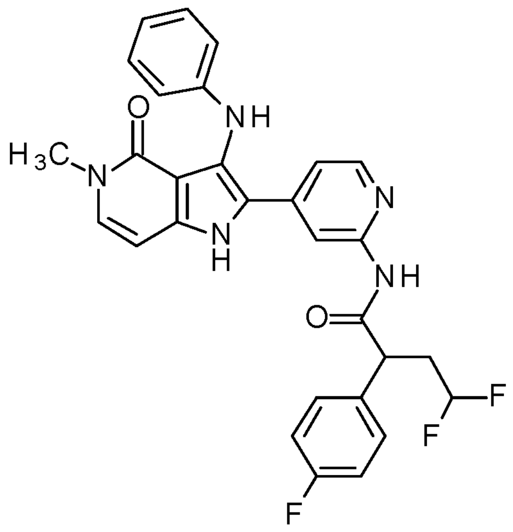

The racemic compound (see Example 28, 1090 mg) was separated into enantiomers by preparative-SFC to give enantiomer 1 (379 mg, 90.4 %ee, see Example 29) and enantiomer 2 (449 mg, 97.0% ee, see Example 30).

Preparative SFC method:

Instrument: Sepiatec: Prep SFC100; Column: Chiralpak IA 5μ 250x30mm; eluent A: CO2; eluent B: methanol + 0.2 vol % aqueous ammonia (32%); isocratic: 35%B; flow: 100 mL/min; temperature: 40°C; BPR: 150bar; UV: 254 nm

Analytical SFC method:

Instrument: Agilent: 1260, Aurora SFC-Modul; Column: Chiralpak IA 5μ 100x4.6mm; eluent A: CO2; eluent B: methanol + 0.2 vol % aqueous ammonia (32%); isocratic: 35%B; flow: 4 mL/min; temperature: 37.5°C; BPR: 100bar; UV: 254 nm

Analytical SFC (method Example 29): $R_t$ = 6.17 min.

$[\alpha]_D$ = - 188,42 ° (from solution in DMSO, c = 2,25 mg/mL)

LC-MS (Method 2): Rt = 1.18 min; MS (ESIpos): m/z = 532 $[M+H]^+$

$^1$H-NMR (400 MHz, DMSO-d6) δ [ppm]: 2.230 (0.40), 2.326 (0.50), 2.518 (3.09), 2.522 (2.09), 2.539 (16.00), 2.664 (0.62), 2.668 (0.66), 2.673 (0.60), 2.710 (0.42), 3.388 (14.84), 4.152 (0.66), 4.166 (0.77), 4.175 (0.76), 4.188 (0.64), 5.836 (0.57), 5.965 (0.51), 5.976 (1.16), 5.988 (0.53), 6.117 (0.50), 6.403 (4.01), 6.421 (3.84), 6.541 (3.13), 6.561 (3.33), 6.563 (2.78), 6.582 (1.03), 6.600 (2.02), 6.618 (1.12), 6.989 (2.47), 7.008 (3.18), 7.010 (3.06), 7.029 (1.96), 7.168 (2.06), 7.174 (0.74), 7.190 (4.32), 7.213 (2.39), 7.320 (1.90), 7.325 (1.96), 7.334 (2.23), 7.339 (5.11), 7.357 (3.36), 7.439 (2.15), 7.444 (1.04), 7.453 (2.42), 7.461 (2.28), 7.475 (5.95), 8.111 (2.57), 8.126 (2.46), 8.297 (2.38), 10.734 (2.84), 11.845 (2.49).

**Example 30**

N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide (Enantiomer 2)

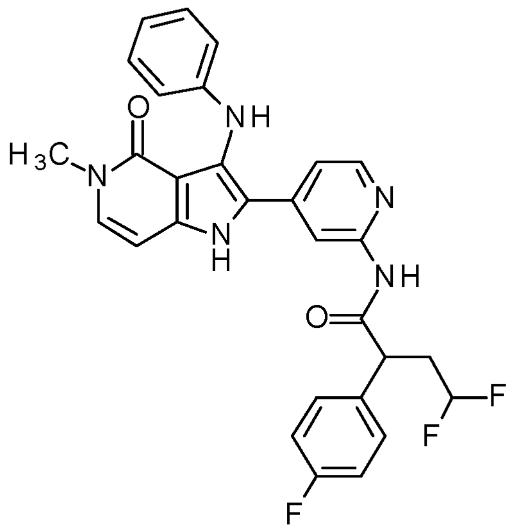

For the preparation of the racemic title compound see Example 28. Separation of enantiomers by preparative -SFC (method see Example 29) gave the title compound (449 mg).

Analytical SFC (method see Example 29): $R_t$ = 3.74 min.

$[\alpha]_D$ = +220,73° (from solution in DMSO, c = 1,58 mg/mL)

LC-MS (Method 2): Rt = 1.21 min; MS (ESIpos): m/z = 532 $[M+H]^+$

$^1$H-NMR (400 MHz, DMSO-d6) $\delta$ [ppm]: 2.327 (0.52), 2.518 (1.73), 2.523 (1.16), 2.540 (15.64), 2.665 (0.62), 2.669 (0.69), 2.674 (0.60), 2.865 (0.44), 3.388 (16.00), 4.153 (0.69), 4.167 (0.77), 4.175 (0.77), 4.189 (0.62), 5.836 (0.59), 5.966 (0.55), 5.977 (1.20), 5.988 (0.56), 6.117 (0.53), 6.404 (4.53), 6.422 (4.13), 6.540 (2.68), 6.542 (3.31), 6.561 (3.51), 6.564 (2.91), 6.580 (0.71), 6.582 (1.09), 6.601 (2.09), 6.618 (1.13), 6.990 (2.64), 7.008 (3.30), 7.011 (3.19), 7.029 (2.11), 7.169 (2.21), 7.174 (0.77), 7.185 (1.00), 7.191 (4.75), 7.196 (0.94), 7.208 (0.82), 7.213 (2.58), 7.321 (2.17), 7.325 (2.02), 7.335 (2.22), 7.339 (5.82), 7.357 (3.66), 7.440 (2.27), 7.445 (1.08), 7.453 (2.54), 7.461 (2.35), 7.475 (6.12), 8.112 (2.81), 8.126 (2.54), 8.298 (2.41), 10.733 (2.94), 11.844 (2.44).

## Example 31

4,4-difluoro-N-{4-[3-(2-fluoroanilino)-5,6-dimethyl-4-oxo-4,5-dihydro-1H-pyrrolo[3,2-c]pyridin-2-yl]pyridin-2-yl}-2-(4-fluorophenyl)butanamide (Racemate)

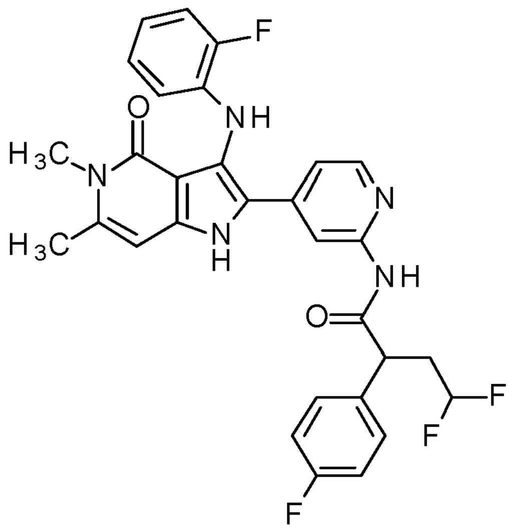

To 4,4-difluoro-N-{4-[(6R*)-3-(2-fluoroanilino)-5,6-dimethyl-4-oxo-4,5,6,7-tetrahydro-1H-pyrrolo-[3,2-c]pyridin-2-yl]pyridin-2-yl}-2-(4-fluorophenyl)butanamide (Racemate) (see Intermediate 53, 44.0 mg) in 1,4-dioxane (2.8 mL) 4,5-dichloro-3,6-dioxocyclohexa-1,4-diene-1,2-dicarbonitrile (19.4 mg, 85.6 μmol) was added. The reaction mixture was stirred at room temperature over night. Then 4,5-dichloro-3,6-dioxocyclohexa-1,4-diene-1,2-dicarbonitrile (7.1 mg) was added again and it was stirred at room temperature over night. The reaction mixture was filtered and the filtrate was concentrated under reduced pressure. The crude product was purified by HPLC (gradient 0,1% formic acid in water, 30-70% acetonitrile) to provide the target compound: 8.4 mg.

LC-MS (Method 1): $R_t$ = 1.21 min; MS (ESIpos): m/z = 564 $[M+H]^+$

$^1$H NMR (400 MHz, DMSO-$d_6$) δ [ppm]: 2.10 - 2.29 (m, 1 H), 2.40 (s, 3 H), 2.59 - 2.78 (m, 1 H), 3.39 (s, 3 H), 4.15 (br dd, 1 H), 5.80 - 6.12 (m, 1 H), 6.19 - 6.26 (m, 1 H), 6.34 (d, 1 H), 6.59 - 6.67 (m, 1 H), 6.69 - 6.76 (m, 1 H), 7.08 - 7.23 (m, 3 H), 7.30 (dd, 1 H), 7.36 - 7.47 (m, 3 H), 8.15 (d, 1 H), 8.27 (s, 1 H), 10.72 (s, 1 H), 11.75 (s, 1 H)

## Example 32

N-{4-[3-anilino-7-(cyclopropylmethyl)-5-methyl-4-oxo-4,5-dihydro-1H-pyrrolo[3,2-c]pyridin-2-yl]pyridin-2-yl}-2-(4-fluorophenyl)acetamide

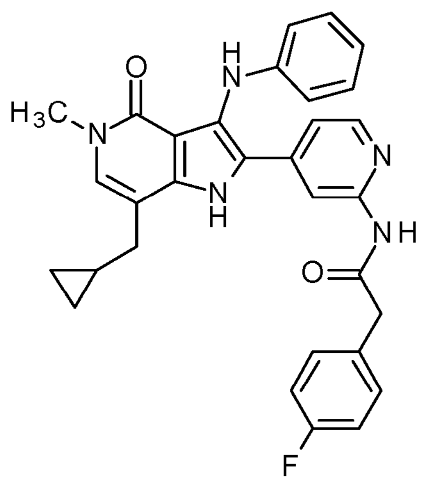

A solution of N-{4-[3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide (Racemate) (see Example 18, 220 mg) and 2,3-dichloro-5,6-dicyano-1,4-benzoquinone (105 mg, 462 μmol) in 1,4-dioxane (20 mL) was stirred at 100 °C for 4 hours. The mixture was extrated with ethyl acetate. The organic layer was concentrated to give a residue. The residue was purified by preparative HPLC [Instrument:Gilson-281; Column: Phenomenex Synergi C18 150*30mm*4um; eluent A: water (0.225% formic acid in water), eluent B: acetonitrile; gradient: 0-10.5 min 5-35% B; flow 25 mL/min; temperature: room temperature; Detector: UV 220/254 nm.] to give 1.80 mg of the title compound as formic acid salt as a yellow solid.

LC-MS (Method 3): $R_t$ = 0.910 min; MS (ESIpos): m/z = 522 $[M+H]^+$

$^1H$ NMR (400 MHz, DMSO-$d_6$): δ [ppm] = 11.48 (s, 1H), 10.61 (s, 1H), 8.44 (s, 1H), 8.34 (s, 1H), 8.14 (d, 1H), 7.46 (s, 1H), 7.38-7.33 (m, 3H), 7.19-7.12 (m, 3H), 7.01-6.97 (m, 2H), 6.59 (t, 1H), 6.52 (d, 2H), 3.69 (s, 2H), 3.38 (s, 3H), 2.56 (d, 2H), 1.12-1.05 (m, 1H), 0.53-0.48 (m, 2H), 0.25-0.21 (m, 2H).

**Example 33**

2-(4-fluorophenyl)-N-{4-[5-methyl-3-(2-methylanilino)-4-oxo-7-(2,2,2-trifluoroethyl)-4,5-dihydro-1H-pyrrolo[3,2-c]pyridin-2-yl]pyridin-2-yl}acetamide

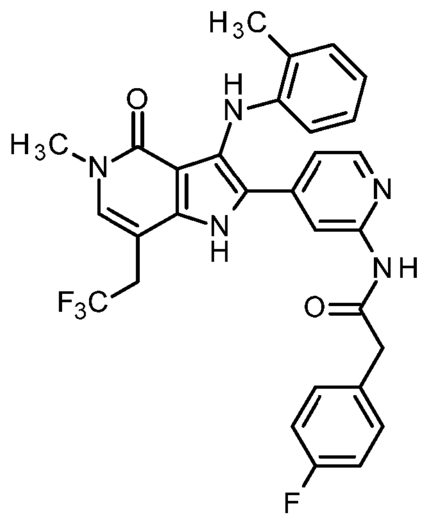

A solution of 2-(4-fluorophenyl)-N-{4-[5-methyl-3-(2-methylanilino)-4-oxo-7-(2,2,2-trifluoroethyl)-4,5,6,7-tetrahydro-1H-pyrrolo[3,2-c]pyridin-2-yl]pyridin-2-yl}acetamide (Racemate) (see Intermediate 57, 300 mg) and 2,3-dichloro-5,6-dicyano-1,4-benzoquinone (DDQ) (132 mg, 583 μmol) in 1,4-dioxane (5.0 mL) was stirred at 100 °C for 4 hours. Saturated sodium hydrogencarbonate solution was added until moist potassium iodide test strip tured white. The mixture was extrated with ethyl acetate. The organic layer was concentrated to give a residue. The residue was purified by flash silca column chromatography (petroleum ether: ethyl acetate = 10: 1 then 1: 1) to give 27.7 mg 2-(4-fluorophenyl)-N-{4-[5-methyl-3-(2-methylanilino)-4-oxo-7-(2,2,2-trifluoroethyl)-4,5-dihydro-1H-pyrrolo[3,2-c]pyridin-2-yl]pyridin-2-yl}acetamide as yellow solid.

LC-MS (Method 3): $R_t$ = 0.837 min; MS (ESIpos): m/z = 564.2 $[M+H]^+$.

$^1H$ NMR (400 MHz, DMSO-$d_6$): δ [ppm] = 11.60 (s, 1 H), 10.61 (s, 1 H), 8.32 (s, 1 H), 8.16 (d, 2H), 7.41 (s, 1H), 7.36-7.32 (m, 2H), 7.25 (d, 1H), 7.16 (t, 2H), 7.07-7.04 (m, 2H), 6.72 (t, 1H), 6.59 (t, 1H), 6.16 (d, 1H), 3.80-3.77 (m, 2H), 3.68 (s, 2H), 3.41 (s, 3H), 3.10 (s, 3H).

**Example 34**

N-{4-[3-anilino-5-methyl-4-oxo-7-(2,2,2-trifluoroethyl)-4,5-dihydro-1H-pyrrolo[3,2-c]pyridin-2-yl]pyridin-2-yl}-2-(4-fluorophenyl)acetamide

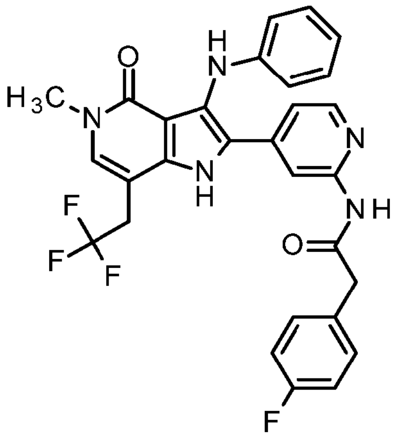

To a mixture of N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide (Racemate) (see Example 14, 70.0 mg) and 2,3-Dichloro-5,6-dicyano-1,4-benzoquinone (28.8 mg, 0.127 mmol) in 1,4-dioxane (7 mL) was stirred at 100 °C for 3 hours. The mixture (combined with another batch starting from 50.0 mg) was cooled to room temperature and poured into Saturated sodium thiosulfate solution: Saturated sodium bicarbonate solution = 1: 1 and the mixture was extracted with ethyl acetate The combined organic phase was washed with brine, dried over anhydrous sodium sulfate, filtered and concentrated in vacuum. The residue was purified by by preparative TLC (petroleum ether: ethyl acetate = 1: 1) to give 28.6 mg N-{4-[3-anilino-5-methyl-4-oxo-7-(2,2,2-trifluoroethyl)-4,5-dihydro-1H-pyrrolo[3,2-c]pyridin-2-yl]pyridin-2-yl}-2-(4-fluorophenyl)acetamide as a yellow solid.

LC-MS (Method 3): $R_t$ = 0.891 min; MS (ESIpos): m/z = 550.3 $[M+H]^+$.

$^1$H NMR (400 MHz, DMSO-$d_6$): δ [ppm] = 11.62 (s, 1H), 10.66 (s, 1H), 8.38 (s, 1H), 8.33 (s, 1H), 8.18 (d, 1H), 7.42 (d, 2H), 7.36-7.34 (m, 3H), 7.17-7.15 (m, 2H), 7.00-6.98 (m, 2H), 6.61-6.58 (m, 1H), 6.53 (d, 2H), 3.82-3.74 (m, 2H), 3.7 (s, 2H), 3.40 (s, 3H).

## Example 35

N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide (Racemate)

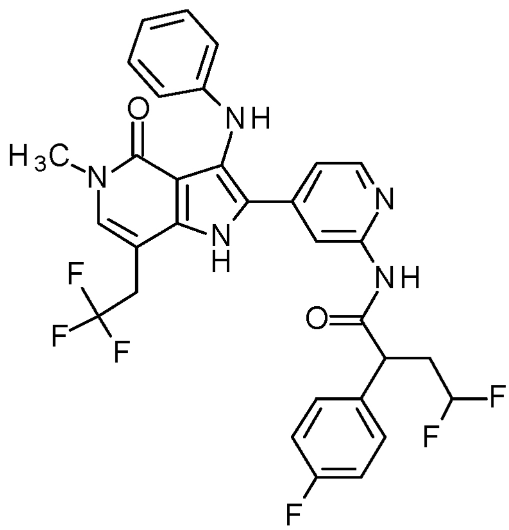

To N-{4-[(7RS)-3-anilino-5-methyl-4-oxo-7-(2,2,2-trifluoroethyl)-4,5,6,7-tetrahydro-1H-pyrrolo-[3,2-c]pyridin-2-yl]pyridin-2-yl}-4,4-difluoro-2-(4-fluorophenyl)butanamide (Mixture of 4 Stereoisomers) (see Example 17, 242 mg) in 1,4-dioxane (15 mL) 2,3-dichloro-5,6-dicyano-1,4-benzochinone (CAS-RN:[84-58-2], 116 mg, 511 μmol) was added. The reaction mixture was stirred at 80 °C for 1 hour. The reaction mixture was directly purified by flash chromatography (25 g silica ULTRA column; gradient: dichloromethane / ethanol 0%-50% ethanol). Afterwards it was stirred in dichloromethane and filtered to provide the target compound: 30 mg.

LC-MS (Method 1): $R_t$ = 1.26 min; MS (ESIneg): m/z = 612 $[M-H]^-$

$^1$H-NMR (400 MHz, DMSO-$d_6$) δ [ppm]: 2.194 (0.40), 2.231 (0.45), 2.244 (0.43), 2.518 (0.90), 2.522 (0.58), 2.665 (0.54), 2.669 (0.51), 2.673 (0.48), 2.687 (0.42), 2.699 (0.46), 2.891 (2.03), 3.404 (16.00), 3.756 (0.50), 3.784 (1.23), 3.812 (1.20), 3.840 (0.41), 4.151 (0.72), 4.165 (0.91), 4.173 (0.86), 4.187 (0.66), 5.808 (0.63), 5.938 (0.56), 5.950 (1.25), 5.960 (0.65), 6.090 (0.57), 6.518 (3.42), 6.538 (3.66), 6.569 (1.01), 6.587 (2.19), 6.605 (1.36), 6.975 (2.60), 6.994 (3.45), 6.996 (3.39), 7.015 (2.16), 7.164 (2.48), 7.186 (5.32), 7.203 (0.96), 7.208 (2.79), 7.345 (2.08), 7.349 (2.01), 7.358 (1.99), 7.362 (2.00), 7.404 (0.44), 7.419 (4.44), 7.435 (2.53), 7.441 (1.21), 7.449 (2.83), 7.457 (2.55), 7.466 (1.19), 7.471 (2.41), 7.479 (4.82), 8.162 (2.91), 8.176 (2.72), 8.316 (2.60), 10.740 (3.12), 11.643 (2.90).

**Example 36**

N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide (Enantiomer 1)

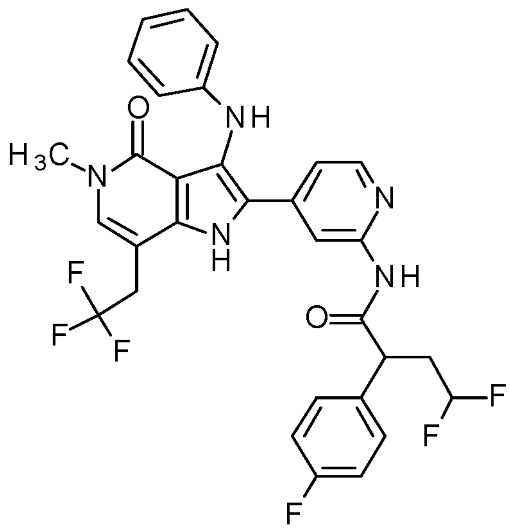

The racemic compound (see Example 35, 60 mg) was separated into enantiomers by preparative chiral HPLC to give enantiomer 1 (11 mg, 65.4 % ee, see Example 36) and enantiomer 2 (10.0 mg, 99.5 % ee, see Example 37).

Preparative chiral HPLC method:

Instrument: PrepCon Labomatic HPLC; Column: YMC Cellulose SB 5μ, 250x30; eluent A: hexane + 0.1 vol % diethylamine; eluent B: ethanol + 0.1 vol % diethylamine; isocratic: 70%A+30%B; flow: 50 mL/min; temperature: 25°C; UV: 254 nm

Analytical chiral HPLC method:

Instrument: Waters Alliance 2695; Column: YMC Cellulose SB 3μ, 100x4.6; eluent A: hexane + 0.1 vol % diethylamine; eluent B: ethanol; isocratic: 70%A+30%B; flow: 1.4 mL/min; temperature: 25°C; UV: 254 nm

Analytical Chiral HPLC (method Example 36): $R_t$ = 1.88 min.

LC-MS (Method 1): $R_t$ = 1.27 min; MS (ESIpos): m/z = 614 $[M+H]^+$

$^1$H-NMR (400 MHz, DMSO-$d_6$) δ [ppm]: 0.814 (0.72), 0.831 (1.50), 0.836 (0.63), 0.850 (0.64), 0.853 (0.67), 0.858 (0.51), 0.936 (0.44), 0.953 (0.48), 1.238 (0.56), 2.230 (0.43), 2.243 (0.41), 2.323 (0.61), 2.327 (0.85), 2.332 (0.61), 2.518 (3.48), 2.523 (2.30), 2.665 (0.91), 2.669 (1.08), 2.674 (0.93), 3.403 (16.00), 3.755 (0.44), 3.783 (1.22), 3.812 (1.19), 3.840 (0.41), 3.879 (0.86), 4.149 (0.68), 4.163 (0.82), 4.171 (0.82), 4.185 (0.65), 5.807 (0.61), 5.937 (0.54), 5.948 (1.20), 5.959 (0.58), 6.088 (0.53), 6.517 (3.34), 6.536 (3.54), 6.568 (1.02), 6.587 (2.06), 6.604 (1.18), 6.974 (2.56), 6.993 (3.30), 6.995 (3.27), 7.014 (2.06), 7.163 (2.23), 7.168 (0.90), 7.185 (4.61), 7.208 (2.50), 7.343 (2.03), 7.347 (2.07), 7.357 (1.98), 7.361 (2.03), 7.419 (4.31), 7.433 (2.35), 7.439 (1.15), 7.447 (2.63), 7.456 (2.42), 7.469 (2.41), 7.477 (4.68), 8.162 (2.73), 8.176 (2.57), 8.313 (2.54), 10.737 (2.99), 11.640 (2.59).

**Example 37**

N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide (Enantiomer 2)

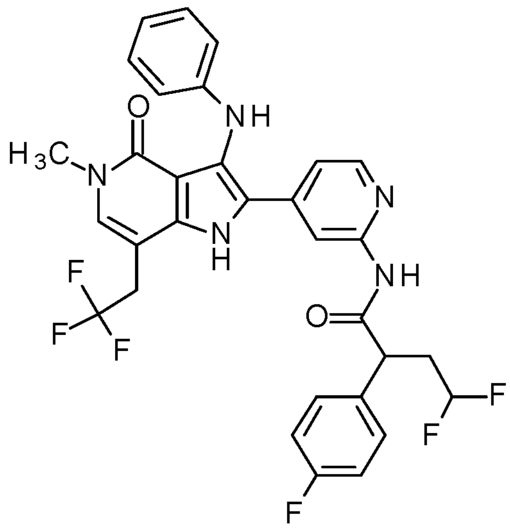

For the preparation of the racemic title compound see Example 35. Separation of enantiomers by preparative chiral HPLC (method see Example 36) gave the title compound (10.0 mg).

Analytical Chiral HPLC (method see Example 36): Rt = 2.57 min.

LC-MS (Method 1): $R_t$ = 1.28 min; MS (ESIpos): m/z = 614 [M+H]$^+$

$^1$H-NMR (400 MHz, DMSO-$d_6$) δ [ppm]: 0.815 (0.96), 0.831 (1.94), 0.837 (0.88), 0.851 (0.82), 0.854 (0.85), 0.858 (0.64), 0.936 (0.65), 0.953 (0.66), 1.239 (0.86), 1.275 (0.56), 1.338 (0.73), 1.380 (0.56), 1.395 (0.47), 1.905 (0.63), 2.192 (0.42), 2.229 (0.46), 2.243 (0.45), 2.323 (0.92), 2.327 (1.27), 2.332 (0.90), 2.518 (4.87), 2.523 (3.17), 2.665 (1.17), 2.669 (1.48), 2.674 (1.20), 3.403 (16.00), 3.754 (0.44), 3.783 (1.22), 3.812 (1.20), 3.840 (0.41), 4.149 (0.68), 4.163 (0.82), 4.171 (0.81), 4.185 (0.63), 5.807 (0.61), 5.937 (0.54), 5.948 (1.21), 5.959 (0.56), 6.088 (0.54), 6.516 (3.37), 6.535 (3.53), 6.568 (1.02), 6.586 (2.06), 6.604 (1.16), 6.974 (2.56), 6.993 (3.28), 6.995 (3.24), 7.014 (2.04), 7.163 (2.23), 7.168 (0.87), 7.185 (4.64), 7.208 (2.49), 7.343 (2.03), 7.347 (1.98), 7.356 (1.93), 7.361 (2.02), 7.419 (4.34), 7.433 (2.34), 7.439 (1.14), 7.447 (2.55), 7.456 (2.38), 7.469 (2.35), 7.476 (4.68), 8.162 (2.72), 8.174 (2.56), 8.313 (2.52), 10.736 (3.02), 11.640 (2.36).

**Example 38**

N-[4-(3-anilino-5,7-dimethyl-4-oxo-4,5-dihydro-1H-pyrrolo[3,2-c]pyridin-2-yl)pyridin-2-yl]-2-(4-fluorophenyl)acetamide

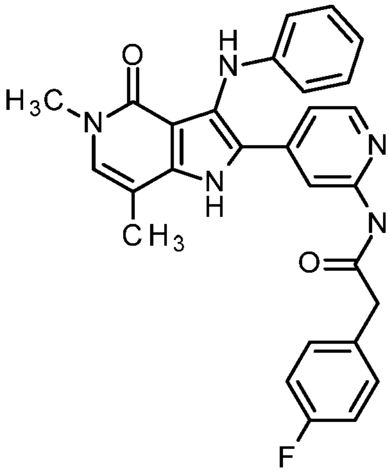

N-{4-[(7S)-3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide (see Example 6, 95.0 mg) was dissolved in 1,4-dioxane (8.4 mL) and 2,3-dichloro-5,6-dicyano-1,4-benzochinone (CAS-RN:[84-58-2], 51.3 mg, 226 μmol) was added. The reaction mixture was stirred at 80 °C for 1 hour under argon atmosphere. The reaction mixture was diluted with ethyl acetate and treated with 1-molar aqueous sodiumhydroxyd-solution. The layers were sperated and the aqueous layer was extracted with ethyl acetate three times. The combined organic layers were washed with water and brine, dried using a waterresistant filter and concentrated under reduced pressure. The crude product was purified by chromatography (10 g column, silica ULTRA, gradient: dichloromethane/ethanol 0-10% ethanol) to provide the target compound: 39 mg.

LC-MS (Method 1): $R_t$ = 1.10 min; MS (ESIpos): m/z = 483 $[M+H]^+$

$^1$H-NMR (400MHz, DMSO-$d_6$): δ [ppm]= 2.22 (d, 3H), 3.69 (s, 2H), 6.53 (dd, 2H), 6.57 - 6.64 (m, 1H), 6.99 (dd, 2H), 7.11 - 7.21 (m, 3H), 7.31 - 7.41 (m, 3H), 7.46 (s, 1H), 8.15 (d, 1H), 8.35 (s, 1H), 10.63 (s, 1H), 11.53 (s, 1H).

## Example 39

N-[4-(3-anilino-5,7-dimethyl-4-oxo-4,5-dihydro-1H-pyrrolo[3,2-c]pyridin-2-yl)pyridin-2-yl]-2-(4-fluorophenyl)propanamide (Racemate)

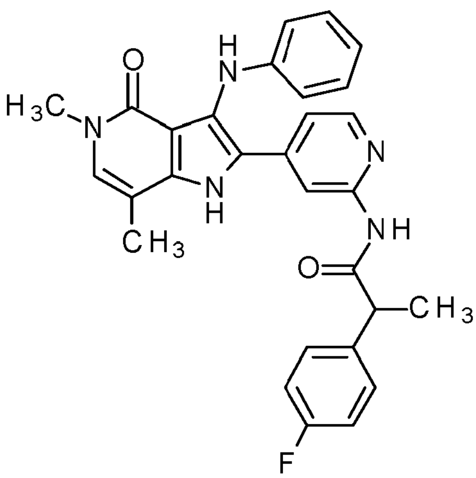

N-{4-[(7RS)-3-anilino-5,7-dimethyl-4-oxo-4,5,6,7-tetrahydro-1H-pyrrolo[3,2-c]pyridin-2-yl]pyridin-2-yl}-2-(4-fluorophenyl)propanamide (Racemate.) (see Example 9, 100 mg) was dissolved in 1,4-dioxane (8.6 mL) and 2,3-dichloro-5,6-dicyano-1,4-benzochinone (CAS RN:[84-58-2], 52.5 mg, 231 μmol) was added. The reaction mixture was stirred at

80 °C for 1 hour under argon atmosphere. The reaction mixture was diluted with ethyl acetate and treated with 1-molar aqueous sodiumhydroxyd-solution. The layers were sperated and the aqueous layer was extracted with ethyl acetate three times. The combined organic layers were washed with water and brine, dried using a waterresistant filter and concentrated under reduced pressure. The crude product was purified by HPLC under acidic conditions to provide the target compound: 36 mg.

LC-MS (Method 1): $R_t$ = 1.14 min; MS (ESIpos): m/z = 496 $[M+H]^+$

$^1$H-NMR (400MHz, DMSO-$d_6$): δ [ppm]= 1.39 (d, 3H), 2.24 (d, 3H), 3.35 (s, 3H), 4.02 (q, 1H), 6.48 - 6.56 (m, 2H), 6.56 - 6.66 (m, 1H), 6.99 (dd, 2H), 7.11 - 7.20 (m, 3H), 7.34 (dd, 1H), 7.38 - 7.45 (m, 2H), 7.48 (s, 1H), 8.12 (d, 1H), 8.36 (s, 1H), 10.56 (s, 1H), 11.55 (s, 1H).

**Example 40**

N-[4-(3-anilino-5,7-dimethyl-4-oxo-4,5-dihydro-1H-pyrrolo[3,2-c]pyridin-2-yl)pyridin-2-yl]-2-(4-fluorophenyl)propanamide (Enantiomer 1)

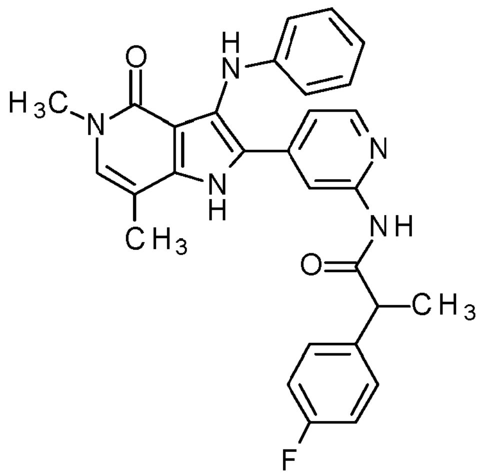

The racemic compound (see Example 39, 34 mg) was separated into enantiomers by preparative chiral HPLC to give enantiomer 1 (11 mg, 99.4 %ee, see Example 40) and enantiomer 2 (12 mg, 98.4 % ee, see Example 41).

Preparative chiral HPLC method:

Instrument: PrepCon Labomatic HPLC; Column: YMC Amylose SA 5μ, 250x30; eluent A: hexane + 0.1 vol % diethylamine; eluent B: ethanol; isocratic: 50%B+50%B; flow: 40 mL/min; temperature: 25°C; UV: 254 nm

Analytical chiral HPLC method:

Instrument: Thermo Fisher UltiMate 3000; Column: YMC Amylose SA 3μ, 100x4.6; eluent A: hexane + 0.1 vol % diethylamine; eluent B: ethanol; isocratic: 50%B+50%B; flow: 1.4 mL/min; temperature: 25°C; UV: 254 nm

Analytical Chiral HPLC (method Example 40): $R_t$ = 2.10 min.

$^1$H-NMR (400MHz, DMSO-$d_6$): δ [ppm]= 1.39 (d, 3H), 2.24 (d, 3H), 3.35 (s, 3H), 4.02 (q, 1H), 6.43 - 6.66 (m, 3H), 6.99 (dd, 2H), 7.11 - 7.22 (m, 3H), 7.34 (dd, 1H), 7.38 - 7.46 (m, 2H), 7.48 (s, 1H), 8.12 (d, 1H), 8.36 (s, 1H), 10.57 (s, 1H), 11.55 (s, 1H).

**Example 41**

N-[4-(3-anilino-5,7-dimethyl-4-oxo-4,5-dihydro-1H-pyrrolo[3,2-c]pyridin-2-yl)pyridin-2-yl]-2-(4-fluorophenyl)propanamide (Enantiomer 2)

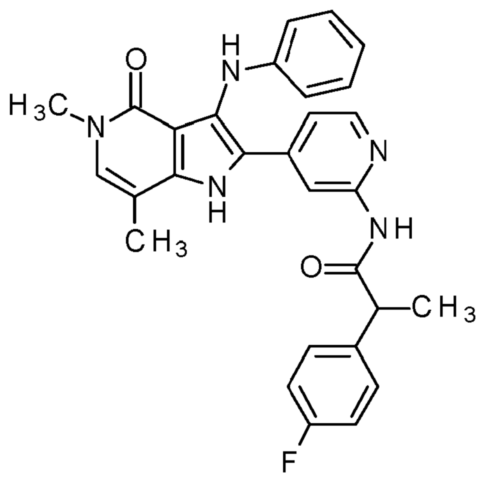

For the preparation of the racemic title compound see Example 39. Separation of enantiomers by preparative chiral HPLC (method see Example 40) gave the title compound (12 mg).

Analytical Chiral HPLC (method see Example 40): $R_t$ = 3.13 min.

$^1$H-NMR (400MHz, DMSO-$d_6$): δ [ppm]= 1.39 (d, 3H), 2.24 (d, 3H), 3.31 - 3.35 (m, 3H), 4.02 (q, 1H), 6.49 - 6.64 (m, 3H), 6.99 (dd, 2H), 7.11 - 7.21 (m, 3H), 7.34 (dd, 1H), 7.39 - 7.45 (m, 2H), 7.48 (s, 1H), 8.12 (d, 1H), 8.36 (s, 1H), 10.57 (s, 1H), 11.55 (s, 1H).

**Example 42**

N-{4-[3-anilino-7-(2,2-difluoroethyl)-5-methyl-4-oxo-4,5,6,7-tetrahydro-1 H-pyrrolo[3,2-c]pyridin-2-yl]pyridin-2-yl}-2-(4-fluorophenyl)acetamide (Racemate)

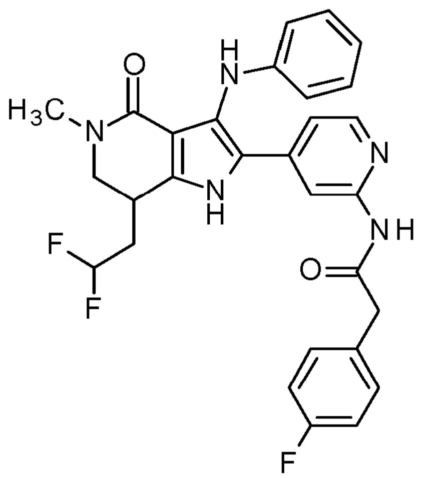

To a mixture of (4-fluorophenyl)acetic acid (493 mg, 3.20 mmol), N,N-diisopropylethylamine (930 µL, 5.3 mmol)

in N,N-dimethylformamide (5 mL) was added (dimethylamino)-N,N-dimethyl(3H-[1,2,3]triazolo[4,5-b]pyridin-3-yloxy)methaniminium hexafluorophosphate(1-) (1.22 g, 3.20 mmol) and 2-(2-aminopyridin-4-yl)-3-anilino-7-(2,2-difluoroethyl)-5-methyl-1,5,6,7-tetrahydro-4H-pyrrolo[3,2-c]pyridin-4-one (Racemate) (see Intermediate 67, 424 mg) at room temperature. The mixture was stirred at 40 °C for 16 hours. Water and ethyl acetate were added to the mixture (combined with another batch starting from 50.0 mg) and the mixture was extracted with ethyl acetate. The organic phase was washed with brine, dried over anhydrous sodium sulfate, filtered and concentrated to give a residue. The residue was purified by purified by twice flash chromatography ((ethyl acetate: methanol = 10: 1) in petroleum ether= 0% to 50%) to give 479.1 mg of the target compound as a yellow solid and as byproduct 8.9 mg Example 45 as a yellow solid.

Analytics of Example 42:

LC-MS (Method 3): $R_t$ = 0.864 min; MS (ESIpos): m/z = 534.3 $[M+H]^+$

$^1H$ NMR (400 MHz, DMSO-$d_6$ + $D_2O$): $\delta$ [ppm] = 8.13 (s, 1H), 8.04 (d, 1H), 7.33-7.31 (m, 2H), 7.17 (dd, 1.6 Hz, 1H), 7.14-7.10 (m, 2H), 7.01-6.97 (m, 2H), 6.64-6.61 (m, 1H), 6.51 (d, 2H), 6.31-6.03 (m, 1H), 3.74-3.70 (m, 1H), 3.64 (s, 2H), 3.41-3.36 (m, 1H), 3.29-3.26 (m, 1H), 2.87 (s, 3H), 2.28-2.08 (m, 2H).

## Example 43

N-{4-[3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide (Enantiomer 1)

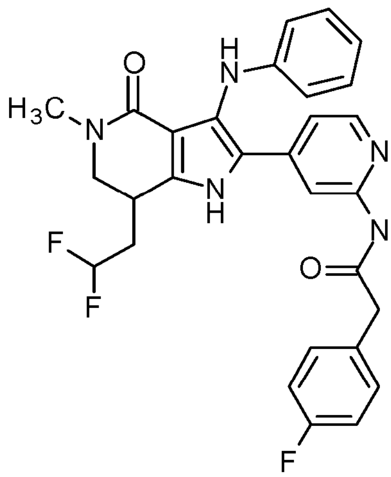

The racemic compound (see Example 42, 120 mg) was separated into enantiomers by preparative-SFC to give enantiomer 1 (48.8 mg, 99 %ee, see Example 43) and enantiomer 2 (41.0 mg, 98 % ee, see Example 44).

Preparative SFC method:

Instrument: CAS-WH-SFC-D; Column: dicel chiralcel od (250mm*30mm,10 μm); Mobile phase: phase A for supercritical carbon dioxide, phase B for ethanol: acetonitrile=3:1 (0.1% ammonia water); Isocratic elution: 40% phase B (60% phase A); Flow rate:70 g/min; Cycle time:4.8 min, total time: 85min; Single injetion volume: 1.5 mL; Back pressure: 100 bar to keep the carbon dioxide in supercritical flow; temperature: room temperature; Detector: UV 220 nm

Analytical SFC method:

Instrument: SFC, column: Chiralcel AD-3 50×4.6mm I.D., 3um; Mobile phase: phase A for carbon dioxide, and phase B for ethanol (0.05% diethylamine); Gradient elution: 40% ethanol (0.05% diethylamine) in carbon dioxide; Flow rate: 3mL/min; Detector: PDA; Column temperature: 35 °C; Back pressure: 100 bar.

Analytical SFC (method Example 43): $R_t$ = 1.079 min.

LC-MS (Method 3): $R_t$ = 0.859 min; MS (ESIpos): m/z = 534.2 $[M+H]^+$

$^1H$ NMR (400 MHz, DMSO-$d_6$ + $D_2O$): δ [ppm] = 8.10 (s, 1H), 8.03 (d, 1H), 7.31-7.27 (m, 2H), 7.17-7.15 (m, 1H), 7.12-7.08 (m, 2H), 7.00-6.95 (m, 2H), 6.63-6.59 (m, 1H), 6.49 (d, 2H), 6.28-6.00 (m, 1H), 3.72-3.68 (m, 1H), 3.62 (s, 2H), 3.39-3.35 (m, 1H), 3.26-3.24 (m, 1H), 2.85 (s, 3H), 2.30-2.11 (m, 2H).

**Example 44**

N-{4-[3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide (Enantiomer 2)

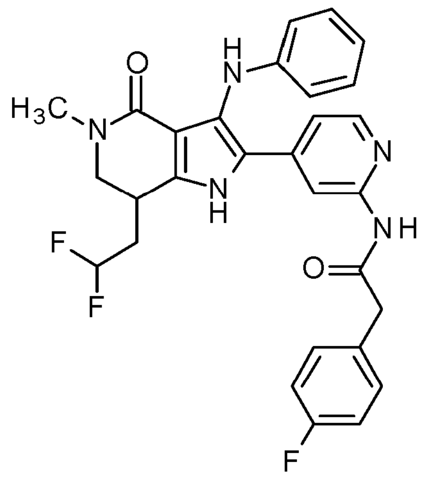

For the preparation of the racemic title compound see Example 42. Separation of enantiomers by preparative -SFC (method see Example 43) gave the title compound (41 mg).

Analytical SFC (method see Example 43): $R_t$ = 1.923 min.

LC-MS (Method 3): $R_t$ = 0.862 min; MS (ESIpos): m/z = 534.2 $[M+H]^+$

$^1H$ NMR (400 MHz, DMSO-$d_6$ + $D_2O$): δ [ppm] = 11.66 (s, 1H), 10.57 (s, 1H), 8.20 (s, 1H), 8.06 (d, 1H), 7.37-7.34 (m, 3H), 7.17-7.13 (m, 3H), 7.02-6.98 (m, 2H), 6.63-6.59 (m, 1H), 6.54 (d, 2H), 6.41-6.13 (m, 1H), 3.74-3.71 (m, 1H), 3.69 (s, 2H), 3.43-3.37 (m, 2H), 2.88 (s, 3H), 2.44-2.36 (m, 1H), 2.19-2.11 (m, 1H).

**Example 45**

N-{4-[3-anilino-7-(2,2-difluoroethyl)-5-methyl-4-oxo-4,5-dihydro-1H-pyrrolo[3,2-c]pyridin-2-yl]pyridin-2-yl}-2-(4-fluorophenyl)acetamide

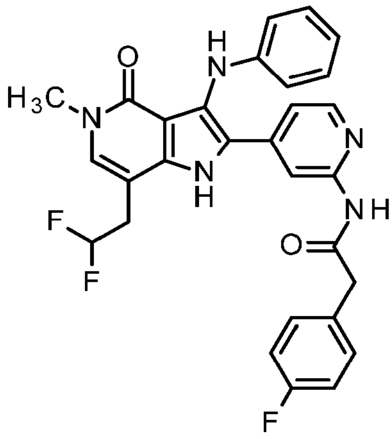

8.9 mg target compound was obtained as a yellow solid as a byproduct of Example 42.

LC-MS (Method 3): $R_t$ = 0.855 min; MS (ESIpos): m/z = 532.3 $[M+H]^+$.

$^1$H NMR (400 MHz, DMSO-$d_6$ + $D_2O$): δ [ppm] = 8.30 (s, 1H), 8.14 (d, 1H), 7.36 (dd, 1H), 7.33-7.29 (m, 2H), 7.26 (s, 1H), 7.14-7.10 (m, 2H), 7.00-6.95 (m, 2H), 6.62-6.58 (m, 1H), 6.51-6.48 (m, 2H), 6.33-6.04 (m, 1H), 3.65 (s, 2H), 3.37 (s, 3H), 3.31-3.21 (m, 2H).

## Example 46

2-(4-fluorophenyl)-N-{4-[(7RS)-5-methyl-4-oxo-3-(1, 3-thiazol-4-ylamino)-7-(2,2,2-trifluoroethyl)-4,5,6,7-tetrahydro-1H-pyrrolo[3,2-c]pyridin-2-yl]pyridin-2-yl}acetamide (Racemate)

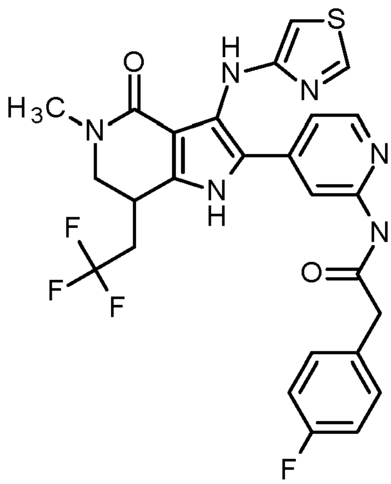

To a mixture of (4-fluorophenyl)acetic acid (140 mg, 909 μmol) and N,N-diisopropylethylamine (400 μl, 2.3 mmol) in N,N-dimethylformamide (8.0 ml) was added 2-(7-azabenzotriazol-1-yl)-N,N,N',N'-tetramethyluronium hexafluorophosphate (HATU) (576 mg, 1.52 mmol) and (7RS)-2-(2-aminopyridin-4-yl)-5-methyl-3-(1,3-thiazol-4-ylamino)-7-(2,2,2-trifluoroethyl)-1,5,6,7-tetrahydro-4H-pyrrolo[3,2-c]pyridin-4-one (320 mg, 758 μmol, see Intermediate 72) at room temperature. The mixture was stirred at 40 °C for 16 hours. Water and ethyl acetate were added and the mixture was extracted with ethyl acetate. The organic phases were washed with brine, dried over anhydrous sodium sulfate, filtered and concentrated in vacuum to give a residue. The residue was purified by preparative TLC (petroleum ether: ethyl acetate = 1: 2) to give the target compound as a crude product (50 mg, crude). The crude target compound was purified by preparative HPLC [Instrument:Gilson-281; Column: Xtimate C18 150*40mm*10μm; eluent A: water (0.05% ammonia hydroxide), eluent B: acetonitrile; gradient: 0-10 minutes 43-73% B; flow 25 ml/minute; temperature: rt; Detector: UV 220/254 nm] followed by preparative TLC (ethyl acetate) to give the target compound (2.10 mg, 95 % purity) as a

yellow solid.

LC-MS (Method 3): $R_t$ = 0.835 min; MS (ESIpos): m/z = 559.4 $[M+H]^+$.

$^1$H-NMR (400 MHz, DMSO-$d_6$): δ [ppm] = 10.61 (s, 1H), 8.70 (d, *J*= 2.0 Hz, 1H), 8.15 (brs, 1H), 8.12 (d, *J*= 5.2 Hz, 1H), 8.01 (s, 1H), 7.38-7.34 (m, 3H), 7.18-7.14 (m, 2H), 7.11-7.09 (m, 1H), 5.47 (d, *J*= 2.0 Hz, 1H), 3.77-7.71 (m, 1H), 3.70 (s, 2H), 3.48-3.40 (m, 3H), 2.92 (s, 3H), 2.89-2.85 (m, 1H).

**Example 47**

2-(4-fluorophenyl)-N-{4-[(7R or S)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide (Enantiomer 1)

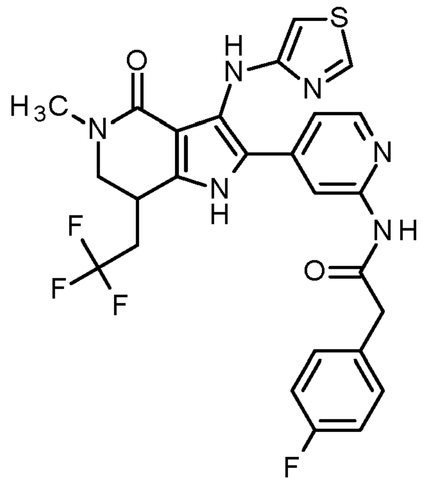

2-(4-Fluorophenyl)-N-{4-[(7RS)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide (50 mg, 89.516 μmol, see Example 46) was separated into the enantiomers by preparative chiral SFC to give enantiomer 1 (first eluting isomer, 7.7 mg, 96.5% purity, see Example 47) as a yellow solid and enantiomer 2 (second eluting isomer, 7.5 mg, 96% purity, see Example 48) as a yellow solid.

Preparative chiral HPLC method:

Preparative SFC; Instrument: ACSWH-PREP-SFC-B; Column: daicel chiralcel od (250mm*30mm,10μm); mobile phase: 30% methanol (0.1% ammonium hydroxide) in supercritical carbon dioxide; flow rate: 65 g/minute; Cycle time: 7.5 minutes, total time 95 minutes; Single injection volume: 4.0 ml; Back pressure: 100 bar to keep the carbon dioxide in supercritical flow.

Analytical chiral HPLC method:

SFC (Method:Cellucoat-Isopropanol (diethylamine)-5-40-3 ml-35T); Instrument: CAS-WH-ANA-SFC-C (SHIMADZU LC-30ADsf); Column: Cellucoat 50×4.6mm I.D., 3μm; Mobile phase: methyl alcohol (0.05% diethylamine) in carbon dioxide from 5% to 40%; Flow rate: 3ml/minute; temperature: 35 °C; Detector: 220 nm.

Analytical Chiral SFC (Method see Example 47): $R_t$ = 1.807 min.

LC-MS (Method 3): $R_t$ = 0.845 min; MS (ESIpos): m/z = 559.1 $[M+H]^+$.

$^1$H-NMR (400 MHz, DMSO-$d_6$): δ [ppm] = 11.66 (s, 1H), 10.60 (s, 1H), 8.76 (d, *J*= 2.0 Hz, 1H), 8.15 (s, 1H), 8.12 (d, *J* = 5.6 Hz, 1H), 8.00 (s, 1H), 7.37-7.34 (m, 2H), 7.17-7.13 (m, 2H), 7.08 (dd, *J* = 5.2 Hz, 1.6 Hz, 1H), 5.47 (d, *J*= 2.0 Hz, 1H), 3.75-3.73 (m, 1H), 3.68 (s, 2H), 3.44-3.40 (m, 2H), 2.90 (s, 3H), 2.86-2.82 (m, 1H), 2.67-2.63 (m, 1H).

**Example 48**

2-(4-fluorophenyl)-N-{4-[(7R or S)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide (Enantiomer 2)

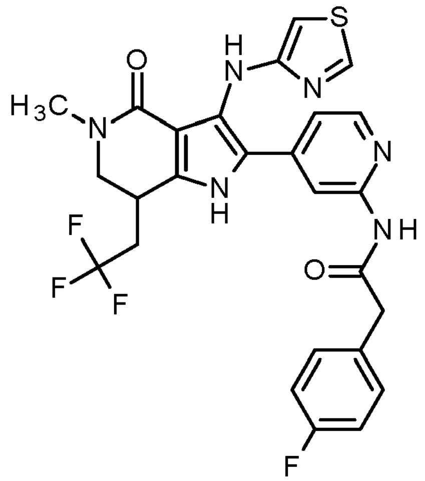

For the preparation of the racemic title compound see Example 46. Separation of the enantiomers by preparative chiral SFC (method see Example 47) gave the title compound, enantiomer 2 (second eluting isomer, 7.5 mg, 96% purity, see Example 48) as a yellow solid.

Analytical Chiral SFC (Method see Example 47): $R_t$ = 1.911 min.

LC-MS (Method 3): $R_t$ = 0.852 min; MS (ESIpos): m/z = 559.2 $[M+H]^+$.

$^1$H-NMR (400 MHz, DMSO-$d_6$): δ [ppm] = 11.66 (s, 1H), 10.61 (s, 1H), 8.76 (d, *J*= 2.0 Hz, 1H), 8.15 (s, 1H), 8.11 (d, *J*= 5.2 Hz, 1H), 8.00 (s, 1H), 7.38-7.34 (m, 2H), 7.18-7.13 (m, 2H), 7.09 (dd, *J* = 5.6 Hz, 1.6 Hz, 1H), 5.47 (d, *J*= 2.0 Hz, 1H), 3.75-3.72 (m, 1H), 3.69 (s, 2H), 3.46-3.43 (m, 2H), 2.90 (s, 3H), 2.87-2.84 (m, 1H), 2.67-2.66 (m, 1H).

**Example 49**

N-{4-[(7RS)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide (Racemate)

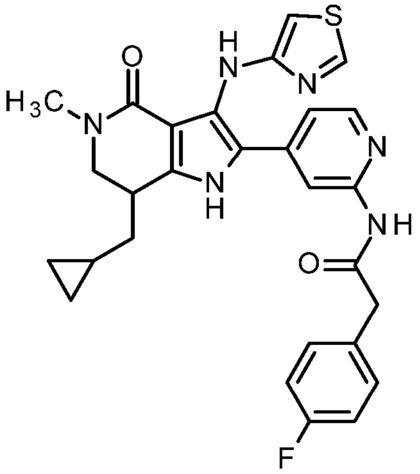

To a solution of (4-fluorophenyl)acetic acid (234 mg, 1.52 mmol) in N,N-dimethylacetamide (10 ml) was added 2-(7-azabenzotriazol-1-yl)-N,N,N',N'-tetramethyluronium hexafluorophosphate (HATU) (578.3 mg, 1.52 mmol) and N,N-diisopropylethylamine (0.53 ml, 3.04 mmol). Then (7RS)-2-(2-aminopyridin-4-yl)-7-(cyclopropylmethyl)-5-methyl-3-(1,3-thiazol-4-ylamino)-1,5,6,7-tetrahydro-4H-pyrrolo[3,2-c]pyridin-4-one (200 mg, 507 μmol, see Intermediate 75) was added and the mixture was stirred at room temperature for 16 hours. The mixture was diluted with dichloromethane, then washed with saturated sodium bicarbonate solution followed by 1 M sodium hydroxide solution. The combined organic phases were washed with brine, dried over anhydrous sodium sulfate, filtered and concentrated in vacuum to give a residue. The residue was purified twice by preparative TLC (petroleum ether: ethyl acetate = 0: 1) to give the title compound as a crude product. The crude product was triturated with ethyl acetate at room temperature for 1 h, then filtered to give the title compound (2.20 mg, 97 % purity) as a yellow solid.

LC-MS (Method 3): $R_t$ = 0.886 min; MS (ESIpos): m/z = 531.2 $[M+H]^+$.

$^1$H-NMR (400 MHz, DMSO-$d_6$): δ [ppm] = 11.52 (s, 1 H), 10.56 (s, 1 H), 8.75 (d, *J*= 2.0 Hz, 1H), 8.11 (s, 1H), 8.09 (d, *J* = 5.2 Hz, 1H), 8.01 (s, 1H), 7.36-7.33 (m, 2H), 7.17-7.13 (m, 2H), 7.06 (dd, *J* = 5.2 Hz, 1.2 Hz, 1H), 5.44 (d, *J*= 2.0 Hz, 1H), 3.76-3.72 (m, 1H), 3.68 (s, 2H), 3.49-3.45 (m, 1H), 3.09-3.06 (m, 1H), 2.91 (s, 3H), 1.57-1.54 (m, 2H), 0.78-0.78 (m, 1H), 0.49-0.40 (m, 2H), 0.06-0.02 (m, 2H).

## Example 50

N-{4-[(7R or S)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide (Enantiomer 1)

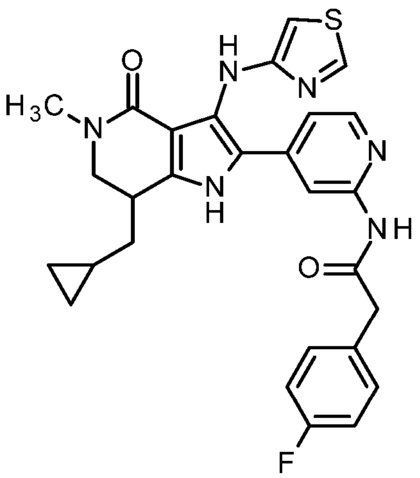

N-{4-[(7RS)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide (30.0 mg, 97 % purity, 54.6 μmol, see example 49) was separated into the enantiomers by preparative chiral SFC to give enantiomer 1 (first eluting isomer, 7.7 mg, 96.9% purity, see Example 50) as a yellow solid and enantiomer 2 (second eluting isomer, 12.6 mg, 94.8% purity, see Example 51) as a yellow solid.

Preparative chiral HPLC method:

Preparative-SFC: Instrument: ACSWH-PREP-SFC-B; Column: daicel chiralpak ad (250mm*30mm,10μm); mobile phase: 60% isopropanol + acetonitrile (0.1% ammonium hydroxide in supercritical carbon dioxide); flow rate: 80 g/minute; cycle time:7.5 minutes, total time: 75 minutes; single injetion volume:4.0 ml; Back pressure:100 bar to keep the carbon dioxide in supercritical flow.

Analytical chiral HPLC method:

SFC (Method: Column: Chiralpak AD-3 50×4.6mm I.D., 3μm; Mobile phase: Phase A for carbon dioxide, and phase B for isopropanol + acetonitrile (0.05% diethanol amine); Gradient elution: 50% isopropanol + acetonitrile (0.05% diethanol amine) in carbon dioxide. Flow rate: 3ml/minute; Detector: PDA Column temperature: 35 °C: 35C; Back pressure: 100 Bar)

Analytical Chiral SFC (Method see Example 50): $R_t$ = 0.564 min.

LC-MS (Method 3): $R_t$ = 0.861 min; MS (ESIpos): m/z = 531.3 $[M+H]^+$.

$^1$H-NMR (400 MHz, DMSO-$d_6$): δ [ppm] = 11.53 (s, 1H), 10.58 (s, 1H), 8.76 (d, *J* = 2.0 Hz, 1H), 8.10-8.08 (m, 2H), 8.02 (s, 1H), 7.36-7.33 (m, 2H), 7.17-7.13 (m, 2H), 7.07 (dd, *J* = 5.6 Hz, 1.2 Hz, 1H), 5.44 (d, *J*= 2.0 Hz, 1H), 3.76-3.72 (m, 1H), 3.68 (s, 2H), 3.49-3.44 (m, 1H), 3.09-3.06 (m, 1H), 2.91 (s, 3H), 1.55 (t, *J* = 6.8 Hz, 2H), 0.80-0.76 (m, 1H), 0.50-0.38 (m, 2H), 0.07-0.01 (m, 2H).

## Example 51

N-{4-[(7R or S)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide (Enantiomer 2)

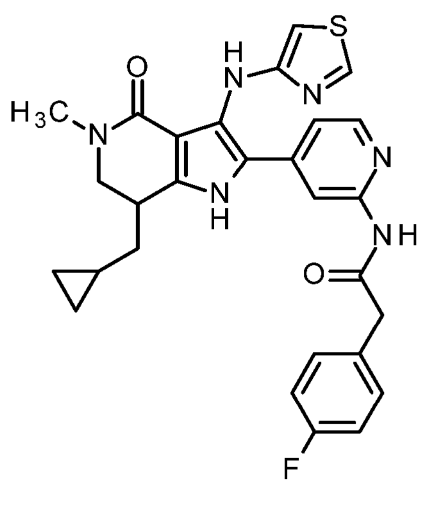

For the preparation of the racemic title compound see Example 49. Separation of the enantiomers by preparative chiral SFC (method see Example 50) gave the title compound, enantiomer 2 (second eluting isomer, 12.6 mg, 94.8% purity, see Example 51) as a yellow solid.

Analytical Chiral SFC (Method see Example 50): $R_t$ = 1.714 min.

LC-MS (Method 3): $R_t$ = 0.854 min; MS (ESIpos): m/z = 531.3 $[M+H]^+$.

$^1$H-NMR (400 MHz, DMSO-$d_6$): δ [ppm] = 11.60 (s, 1 H), 10.74 (s, 1 H), 8.78 (d, *J* = 2.0 Hz, 1H), 8.11 (s, 1H), 8.09 (s, 1H), 8.00 (s, 1H), 7.37-7.33 (m, 2H), 7.18-7.14 (m, 3H), 5.45 (s, 1H), 3.77-3.73 (m, 1H), 3.69 (s, 2H), 3.50-3.46 (m, 1H), 3.11-3.08 (m, 1H), 2.97 (s, 3H), 1.57 (t, *J* = 6.8 Hz, 2H), 0.81-0.77 (m, 1H), 0.50-0.39 (m, 2H), 0.08-0.01 (m, 2H).

**Example 52**

(2RS)-4,4-difluoro-2-(4-fluorophenyl)-N-{4-[(7RS)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide (Mixture of 4 Stereoisomers)

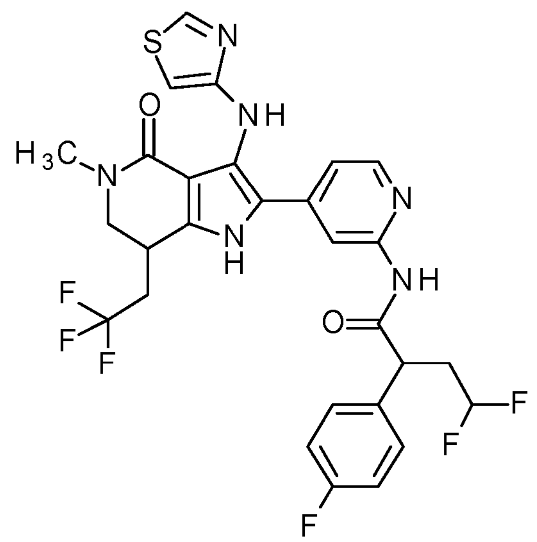

To a stirred solution of (7RS)-2-(2-aminopyridin-4-yl)-5-methyl-3-[(1,3-thiazol-4-yl)amino]-7-(2,2,2-trifluoroethyl)-1,5,6,7-tetrahydro-4H-pyrrolo[3,2-c]pyridin-4-one (144 mg, 342 μmol, see Intermediate 72) in DMA (570 μL) was added N,N-diisopropylethylamine (360 μl, 2.0 mmol; CAS-RN:[7087-68-5]), (2RS)-4,4-difluoro-2-(4-fluorophenyl)butanoic acid (96.9 mg, 444 μmol) and PyBOP (533 mg, 1.02 mmol; CAS-RN:[128625-52-5]). The mixture was stirred at r.t. for 16 h. An aqueous half-saturated solution of sodium bicarbonate was added, the mixture was stirred for 10 minutes and the mixture was extracted with ethyl acetate. The combined organic phases were washed with half-saturated sodium chloride solution (three times), dried (sodium sulfate), filtered and the solvent was removed in vacuum. The crude product was combined with the crude product of a further analogous reaction starting with 100 mg (237 μM) of (7RS)-2-(2-aminopyridin-4-yl)-5-methyl-3-[(1,3-thiazol-4-yl)amino]-7-(2,2,2-trifluoroethyl)-1,5,6,7-tetrahydro-4H-pyrrolo[3,2-c]pyridin-4-one. Silicagel chromatography (Gradient: dichloromethane / ethanol 0-15%) of the combined crude products gave 210 mg of the title compound as a solid.

LC-MS (Method 2): $R_t$ = 1.25 min; MS (ESIpos): m/z = 623 $[M+H]^+$

$^1$H-NMR (400 MHz, DMSO-d6) δ [ppm]: 0.814 (1.06), 0.821 (0.63), 0.830 (1.71), 0.836 (1.00), 0.840 (0.58), 0.852 (1.20), 0.858 (0.98), 0.935 (0.83), 0.952 (0.87), 1.237 (0.82), 1.255 (0.52), 1.394 (1.38), 1.955 (5.72), 2.221 (0.41), 2.322 (0.48), 2.326 (0.66), 2.331 (0.49), 2.518 (3.26), 2.522 (2.24), 2.664 (1.03), 2.669 (1.20), 2.673 (1.16), 2.702 (0.56), 2.781 (5.40), 2.863 (0.41), 2.912 (16.00), 2.924 (1.31), 2.941 (8.10), 3.423 (0.84), 3.431 (1.11), 3.451 (1.61), 3.730 (0.66), 3.746 (0.69), 3.763 (0.54), 4.145 (0.70), 4.160 (0.82), 4.167 (0.84), 4.181 (0.66), 5.440 (2.18), 5.444 (2.68), 5.448 (2.02), 5.827 (0.60), 5.956 (0.56), 5.967 (1.21), 5.979 (0.58), 6.108 (0.54), 7.081 (1.16), 7.085 (1.96), 7.089 (1.20), 7.094 (1.22), 7.098 (1.98), 7.102 (1.12), 7.174 (2.04), 7.196 (4.23), 7.218 (2.30), 7.426 (1.32), 7.432 (1.76), 7.440 (1.65), 7.446 (2.14), 7.454 (1.47), 7.462 (1.45), 7.467 (1.13), 8.036 (2.59), 8.040 (2.57), 8.087 (2.46), 8.101 (2.35), 8.128 (1.36), 8.139 (1.40), 8.762 (2.43), 8.767 (3.58), 8.772 (2.44), 10.697 (1.83), 10.703 (1.79), 11.676 (2.04).

**Example 53**

(2R or S)-4,4-difluoro-2-(4-fluorophenyl)-N-{4-[(7R or S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide (Stereoisomer 1)

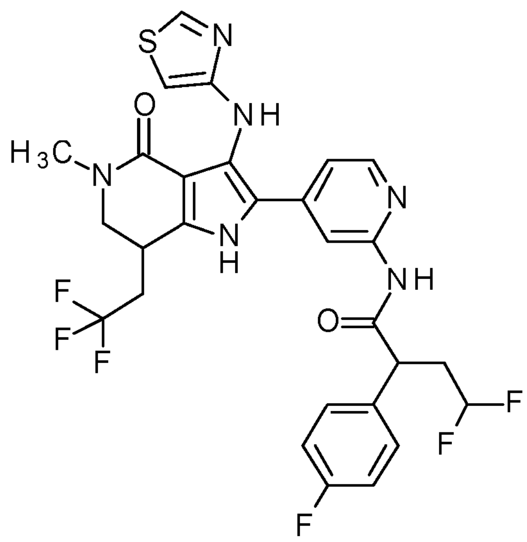

(2RS)-4,4-difluoro-2-(4-fluorophenyl)-N-{4-[(7RS)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide (mixture of 4 stereoisomers, 204 mg, 328 µmol, see Example 52) was separated into the single isomers by preparative chiral HPLC (Method 53-1) to give 43 mg of crude isomer 1 (first eluting isomer), that was triturated with a mixture of dichloromethane and hexane to give the title compound, enantiomer 1 (first eluting, 28 mg, 98.09% purity, see Example 53) as a solid and a mixture of isomer 2 and isomer 3 (second eluting, 84 mg, see Example 54 and Example 55) and 48 mg of crude isomer 4 (last eluting isomer) as a solid that was triturated with a mixture of dichloromethane and hexane to give isomer 4 (last eluting, 27 mg, 94.36% purity, see Example 56) as a solid. The mixture of isomer 2 and isomer 3 (84 mg) was separated into the single isomers by preparative chiral HPLC (Method 53-2) to give 32 mg of crude isomer 2 (first eluting isomer) that was triturated with a mixture of dichloromethane and hexane to give isomer 2 (first eluting, 11 mg, 99.34% purity, see Example 54) as a solid and 41 mg of crude isomer 3 (second eluting isomer) as a solid that was triturated with a mixture of dichloromethane and hexane to give isomer 3 (second eluting, 11 mg, 99.82% purity, see Example 55) as a solid.

Preparative chiral HPLC method 53-1:

Instrument: PrepCon Labomatic HPLC-3; Column: YMC Cellulose SB 10µ, 250x50; eluent A: methyl tert-butyl ether + 0.1 vol % diethylamine; eluent B: acetonitrile; isocratic: 95%A+5%B; flow: 80 ml/min; temperature: 25°C; UV: 254 nm

Preparative chiral HPLC method 53-2:

Instrument: PrepCon Labomatic HPLC-3; Column: YMC Amylose SA 10µ, 250x50; eluent A: methyl tert-butyl ether + 0.1 vol % diethylamine; eluent B: acetonitrile + 0.1 vol % diethylamine; isocratic: 50%A+50%B; flow: 100 mL/min; temperature: 25°C; UV: 254 nm

Analytical chiral HPLC, analytical method 53-1:

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 95%A+5%B; flow: 1.4 ml/min; temperature: 25°C; UV: 254 nm

Analytical chiral HPLC, analytical method 53-2:

Instrument: Waters Alliance 2695; Column: YMC Amylose SA 3μ, 100x4.6; eluent A: methyl tert-butyl ether + 0.1 vol % diethylamine; eluent B: acetonitrile; isocratic: 50%A+50%B; flow: 1.4 ml/min; temperature: 25°C; UV: 254 nm

Analytical Chiral HPLC (Analytical Method 53-1): $R_t$ = 2.58 min.

$[\alpha]_D$ = -178.1° (from solution in DMSO, c = 1.5 mg/mL)

LC-MS (Method 2): $R_t$ = 1.24 min; MS (ESIneg): m/z = 621 $[M-H]^-$

$^1$H-NMR (400 MHz, DMSO-$d_6$): δ [ppm] = 11.67 (s, 1H), 10.70 (s, 1H), 8.77 (d, 1H), 8.14 (s, 1H), 8.09 (d, 1H), 8.04 (s, 1H), 7.48-7.42 (m, 2H), 7.23-7.16 (m, 2H), 7.09 (dd, 1H), 6.13-5.81 (m, 1H), 5.44 (d, 1H), 4.16 (dd, 1H), 3.79-3.69 (m, 1H), 3.49-3.40 (m, 2H), 2.96-2.82 (m, 4H), 2.77-2.59 (m, 2H), 2.30-2.13 (m, 1H).

**Example 54**

(2R or S)-4,4-difluoro-2-(4-fluorophenyl)-N-{4-[(7R or S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide (Stereoisomer 2)

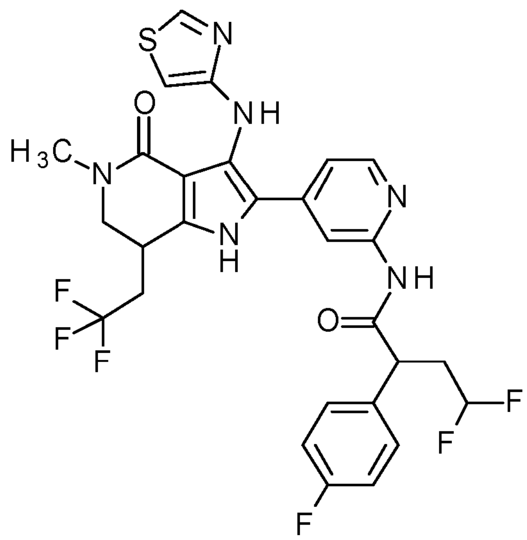

For the preparation of the racemic title compound see Example 52. Separation of the enantiomers by preparative chiral HPLC using preparative chiral HPLC method 53-1 followed by preparative chiral HPLC method 53-2 (see Example 53) gave 32 mg of crude isomer 2 that was triturated with a mixture of dichloromethane and hexane to give the title compound, isomer 2 (11 mg, 99.34% purity, see Example 54) as a solid.

Analytical Chiral HPLC (Analytical Method 53-1): $R_t$ = 3.12 min.

Analytical Chiral HPLC (Analytical Method 53-2): $R_t$ = 1.04 min.

$[\alpha]_D$ = +110.3° (from solution in DMSO, c = 1.5 mg/mL)

LC-MS (Method 2): $R_t$ = 1.23 min; MS (ESIpos): m/z = 623 $[M+H]^+$

$^1$H-NMR (400 MHz, DMSO-$d_6$): δ [ppm] = 11.67 (s, 1H), 10.70 (s, 1H), 8.77 (d, 1H), 8.13 (s, 1H), 8.10 (d, 1H), 8.04 (s, 1H), 7.48-7.40 (m, 2H), 7.23-7.16 (m, 2H), 7.09 (dd, 1H), 6.14-5.80 (m, 1H), 5.45 (d, 1H), 4.16 (br dd, 1H), 3.81-3.71 (m, 1H), 3.49-3.39 (m, 2H), 2.91 (s, 3H), 2.90-2.82 (m, 1H), 2.78-2.60 (m, 2H), 2.29-2.12 (m, 1H).

**Example 55**

(2R or S)-4,4-difluoro-2-(4-fluorophenyl)-N-{4-[(7R or S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide (Stereoisomer 3)

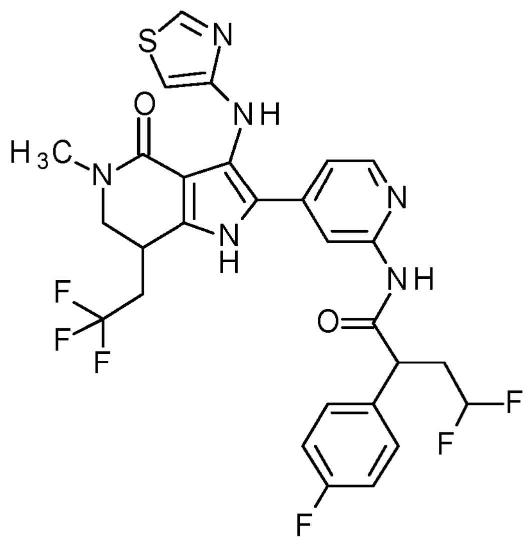

For the preparation of the racemic title compound see Example 52. Separation of the enantiomers by preparative chiral HPLC using preparative chiral HPLC method 53-1 followed by preparative chiral HPLC method 53-2 (see Example 53) gave 41 mg of crude isomer 3 that was triturated with a mixture of dichloromethane and hexane to give the title compound, isomer 3 (11 mg, 99.82% purity, see Example 55) as a solid.

Analytical Chiral HPLC (Analytical Method 53-1): $R_t$ = 3.36 min.

Analytical Chiral HPLC (Analytical Method 53-2): $R_t$ = 1.54 min.

$[\alpha]_D$ = -37.6° (from solution in DMSO, c = 6.3 mg/mL)

LC-MS (Method 2): $R_t$ = 1.23 min; MS (ESIpos): m/z = 623 $[M+H]^+$

$^1$H-NMR (400 MHz, DMSO-$d_6$): $\delta$ [ppm] = 11.67 (s, 1H), 10.70 (s, 1H), 8.77 (d, 1H), 8.13 (s, 1H), 8.10 (d, 1H), 8.04 (s, 1H), 7.48-7.41 (m, 2H), 7.23-7.16 (m, 2H), 7.09 (dd, 1H), 6.13-5.80 (m, 1H), 5.45 (d, 1H), 4.16 (br dd, 1H), 3.81-3.71 (m, 1H), 3.51-3.40 (m, 2H), 2.91 (s, 3H), 2.90-2.81 (m, 1H), 2.77-2.58 (m, 2H), 2.29-2.12 (m, 1H).

**Example 56**

(2R or S)-4,4-difluoro-2-(4-fluorophenyl)-N-{4-[(7R or S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide (Stereoisomer 4)

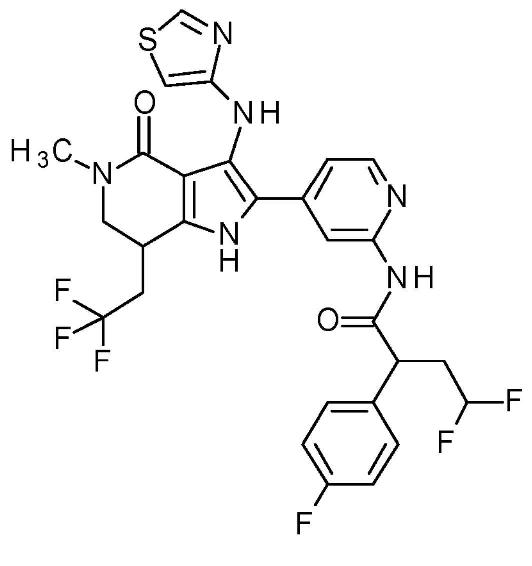

For the preparation of the racemic title compound see Example 52. Separation of the enantiomers by preparative chiral HPLC using preparative chiral HPLC method 53-1 (see Example 53) gave 48 mg of crude isomer 4 (last eluting isomer) as a solid that was triturated with a mixture of dichloromethane and hexane to give the title compound isomer 4 (last eluting, 27 mg, 94.36% purity, see Example 56) as a solid.

Analytical Chiral HPLC (Analytical Method 53-1): $R_t$ = 5.04 min.

$[\alpha]_D$ = +152.7° (from solution in DMSO, c = 2.6 mg/mL)

LC-MS (Method 2): $R_t$ = 1.23 min; MS (ESIpos): m/z = 623 $[M+H]^+$

$^1$H-NMR (400 MHz, DMSO-$d_6$): $\delta$ [ppm] = 11.67 (s, 1H), 10.70 (s, 1H), 8.77 (d, 1H), 8.14 (s, 1H), 8.09 (d, 1H), 8.04 (s, 1H), 7.48-7.42 (m, 2H), 7.23-7.16 (m, 2H), 7.09 (dd, 1H), 6.14-5.80 (m, 1H), 5.44 (d, 1H), 4.16 (dd, 1H), 3.80-3.70 (m, 1H), 3.51-3.41 (m, 2H), 2.97-2.80 (m, 4H), 2.76-2.60 (m, 2H), 2.29-2.12 (m, 1H).

## Co-crystallization and structure determination of Casein kinase inhibitors with Casein Kinase 1D

### Crystallization

Crystals of Casein Kinase 1D (residues 1-294 with mutation Arg13Asn) in complex with Intermediate 42 were obtained using hanging-drop vapor diffusion set-ups. Casein Kinase 1D at a concentration of 13.77 mg/mL (50 mM Hepes, 290 mM NaCl, 1 mM EDTA, 1 mM DTT, 5 mM ß-OG, pH 7.5) was pre-incubated with 2 mM (5.0-fold molar excess) of Intermediate 42 (150 mM in DMSO) for 1 h. 0.8 µL of the protein solution was then mixed with 0.8 µL of reservoir solution (0.10 M sodium citrate pH 4.90, 20 % (w/v) PEG 3350) and equilibrated at 20°C over 0.4 mL of reservoir solution. Well-diffracting plates grew within a week and data were collected from a single crystal cryo-protected with 20% (v/v) ethylene glycol.

### Data Collection, Structure determination and Refinement

A complete 1.8 Å data set of a Casein Kinase 1D/Intermediate 42 crystal was collected at the ESRF (Grenoble, FR, beamline ID30a1) (Table 5). Molecular replacement was done using a previously determined model of Casein Kinase 1D as starting model. Several rounds of alternating manual re-building and refinement with REFMAC5 resulted in the final model (Table 5). The B-factors were modelled through a combination of an isotropic B-factor for each atom and a single TLS group for each of the four copies of Casein Kinase 1D in the asymmetric unit. Local NCS restraints as implemented in REFMAC5 were used throughout refinement.

| **Table 5:** Data collection and refinement statistics | |
|---|---|
| Space group | P1 |

(continued)

| Table 5: Data collection and refinement statistics | |
|---|---|
| Unit cell parameters, | |
| axes a, b, c [Å], | 48.9, 83.9, 89.9, |
| angles $\alpha$, $\beta$, $\gamma$ (°) | 69.3, $\beta$=74.2, $\gamma$=87.9 |
| Resolution [Å] | 80.75-1.80 (1.83-1.80) |
| Number of unique reflections | 115984 (5666) |
| Mean I/$\sigma$ | 9.9 (3.1) |
| Completeness | 97.6 (96.3) |
| Multiplicity | 2.8 (2.9) |
| Rmeas | 0.065 (0.320) |
| Resolution [Å] | 80.75-1.80 (1.85-1.80) |
| $R_{work}$ | 0.186 (0.241) |
| $R_{free}$ | 0.232 (0.278) |
| Completeness | 97.5 (96.4) |
| r.m.s.d. bonds [Å] | 0.019 |
| r.m.s.d. angles | 1.932 |

Values in brackets refer to the highest resolution

shell

**Absolute configuration of Intermediate 42 (bound to Casein Kinase 1D)**

The complex of Casein Kinase 1D and Intermediate 42 crystallized with four molecules of Casein Kinase 1D in the asymmetric unit. A single molecule of the Intermediate 42 is present in the active site of all four molecules of Casein Kinase 1D. For co-crystallization, an enantiomer-pure

batch of Intermediate 42 was used for which the exact stereo-configuration was not known. The electron density maps allowed the deduction of the configuration at C6 of the stereoisomer bound in the crystal. The stereochemistry at the central carbon atom C6 of Intermediate 42 (Figure 1) is unambiguously defined by the knowledge of the stereochemistry of the protein Casein Kinase 1D. Intermediate 42 unambiguously features the (S)-configuration on carbon atom C6 (Figure 1).

**EXPERIMENTAL SECTION - BIOLOGICAL ASSAYS**

The following assays can be used to illustrate the commercial utility of the compounds according to the present invention.

Examples were tested in selected biological assays one or more times. When tested more than once, data are reported as either average values or as median values or single individual measurements, wherein

- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.
- Individual measurements are shown when median or average values cannot be computed. Examples were synthesized one or more times. When synthesized more than once, data from biological assays represent average values calculated utilizing data sets obtained from testing of one or more synthetic batch.

The ***in vitro* activity** of the compounds of the present invention can be demonstrated in the following assays:

**CSNK1A1 assay 1**

CSNK1A1-inhibitory activity of compounds of the present invention in presence of 1 $\mu$M adenosine-tri-phosphate

(ATP) was quantified employing the CSNK1A1 assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent"). Recombinant fusion protein of N-terminal Glutathion-S-Transferase (GST) and full-length human CSNK1A1, expressed by baculovirus infected insect cells and purified via Glutathion affinity chromatography, was purchased from Life Technologies (product no. PV4174) and used as enzyme. As substrate for the kinase reaction the biotinylated peptide Btn-Ahx-SGSEGDSESGEEEG (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1A1 in aqueous assay buffer [50 mM HEPES pH 7.5, 10% (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1A1 was adjusted depending of the activity of the enzyme lot and was chosen appropriate to have the assay in the linear range, a typical concentration is 0.15 ng/μL. The reaction was stopped by the addition of 2.5 μL of "ADP-Glo-reagent" (1:1.5 fold diluted with water) and the resulting mixture was incubated at 22°C for 1 h to convert the ATP not consumed in the kinase reaction completely to cAMP. Subsequently 2.5 μL of the "kinase detection reagent" (1.2 fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1A1.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1A1 assay 2**

CSNK1A1-inhibitory activity of compounds of the present invention in presence of 1 μM adenosine-tri-phosphate (ATP) was quantified employing the CSNK1A1 assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows: In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent").

Recombinant fusion protein of N-terminal Glutathion-S-Transferase (GST) and full-length human CSNK1A1, expressed by baculovirus infected insect cells and purified via Glutathion affinity chromatography, was purchased from Life Technologies (product no. PV4174) and used as enzyme. As substrate for the kinase reaction the biotinylated peptide biotin-Ahx-KRRRAL-pS-VASLPGL (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1A1 in aqueous assay buffer [50 mM HEPES pH 7.5, 10% (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1A1 was adjusted depending of the activity of the enzyme lot and was chosen appropriate to have the assay in the linear range, a typical concentrations are about 0.0375 ng/μL. The reaction was stopped by the addition of 2.5 μL of "ADP-Glo-reagent" (1:1.5 fold diluted with water)

and the resulting mixture was incubated at 22°C for 1 h to convert the ATP not consumed in the kinase reaction completely to cAMP. Subsequently 2.5 µL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1A1.

The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1A1 high ATP assay**

CSNK1A1-inhibitory activity of compounds of the present invention in presence of 1 mM adenosine-tri-phosphate (ATP) was quantified employing the CSNK1A1-high-ATP-assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent").

Recombinant fusion protein of N-terminal Glutathion-S-Transferase (GST) and full-length human CSNK1A1, expressed by baculovirus infected insect cells and purified via Glutathion affinity chromatography, was purchased from Life Technologies (product no. PV4174) and used as enzyme. As substrate for the kinase reaction the biotinylated peptide biotin-Ahx-KRRRAL-pS-VASLPGL (C-terminus in amide form) was used which can be purchased e.g. from the company Biosyntan (Berlin-Buch, Germany).

For the assay 50 nl of a 100fold concentrated solution of the test compound in DMSO was pipetted into a white low volume 384-well microtiter plate (Greiner Bio-One, Frickenhausen, Germany), 2 µL of a solution of CSNK1A1 in aqueous assay buffer [50 mM HEPES pH 7.5, 10 % (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 µL of a solution of ATP (1.67 mM => final conc. in the 5 µL assay volume is 1 mM) and peptide substrate (167 µM => final conc. in the 5 µL assay volume is 100 µM) in assay buffer and the resulting mixture was incubated for a reaction time of 30 min at 22°C. The concentration of CSNK1A1 was adjusted depending of the activity of the enzyme lot and was chosen appropriate to have the assay in the linear range, a typical concentration is about 0.4 ng/µL. The reaction was stopped by the addition of 2.5 µL of "ADP-Glo-reagent" (1:1.5fold diluted with water) and the resulting mixture was incubated at 22°C for 1 h to convert the ATP not consumed in the kinase reaction completely to cAMP. Subsequently 2.5 µL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1A1.

The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1D assay**

CSNK1D-inhibitory activity of compounds of the present invention in presence of 1 µM adenosine-tri-phosphate (ATP) was quantified employing the CSNK1D assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection

Reagent").

Recombinant fusion protein of N-terminal Glutathion-S-Transferase (GST) and full-length human CSNK1D, expressed by baculovirus infected insect cells and purified via Glutathion affinity chromatography, was purchased from Life Technologies (product no. PV3665) and used as enzyme. As substrate for the kinase reaction the biotinylated peptide Btn-Ahx-SGSEGDSESGEEEG (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1D in aqueous assay buffer [50 mM HEPES pH 7.5, 10 % (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1D was adjusted depending of the activity of the enzyme lot and was chosen appropriate to have the assay in the linear range, a typical concentration is about 0.5 ng/μL. The reaction was stopped by the addition of 2.5 μL of "ADP-Glo-reagent" (1:1.5fold diluted with water) and the resulting mixture was incubated at 22°C for 1 h to convert the ATP not consumed in the kinase reaction completely to cAMP. Subsequently 2.5 μL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1D.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1G3 assay**

CSNK1G3-inhibitory activity of compounds of the present invention in presence of 1 μM adenosine-tri-phosphate (ATP) was quantified employing the CSNK1G3 assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent").

Recombinant fusion protein of N-terminal Glutathion-S-Transferase (GST) and full-length human CSNK1G3, expressed by baculovirus infected insect cells and purified via Glutathion affinity chromatography, was purchased from Life Technologies (product no. PV3838) and used as enzyme. As substrate for the kinase reaction the biotinylated peptide biotin-Ahx-KRRRAL-pS-VASLPGL (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1G3 in aqueous assay buffer [50 mM HEPES pH 7.5, 10 % (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1G3 was adjusted depending of the activity of the enzyme lot and was chosen appropriate to have the assay in the linear range, a typical concentration is about 0.06 ng/μL. The reaction was stopped by the addition of 2.5 μL of "ADP-Glo-reagent" (1:1.5fold diluted with water) and the resulting mixture was incubated at 22°C for 1 h to convert the ATP not consumed in the kinase reaction completely to cAMP. Subsequently 2.5 μL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1 G3.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**WT-EGFR kinase assay**

Inhibitory activity of compounds of the present invention against wild-type Epidermal Growth Factor Receptor (EGFR) was quantified employing the TR-FRET based EGFR assay as described in the following paragraphs.

Recombinant fusion protein of N-terminal Glutathion-S-Transferase (GST) and a fragment of human EGFR (amino acids R669 to A1210), expressed in Sf9 insect cells and purified via affinity chromatography using Glutathion Sepharose as described above, was used as kinase. As substrate for the kinase reaction the biotinylated peptide biotin-Ahx-AEEEEYFELVAKKK (C-terminus in amide form) was used which can be purchased e.g. form the company Biosynthan GmbH (Berlin-Buch, Germany).

For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 2 μL of a solution of EGFR in aqueous assay buffer [50 mM Hepes pH 7.0, 10 mM $MgCl_2$, 1mM dithiothreitol, 0.5 mM EGTA, 0.3 mM activated sodium ortho-vanadate, 0.005 % (w/v) bovine serum albumin, 0.005% (v/v) Tween-20] were added and the mixture was incubated for 15 min at 22°C to allow pre-binding of the test compounds to the enzyme before the start of the kinase reaction. Then the kinase reaction was started by the addition of 3 μL of a solution of adenosine-tri-phosphate (ATP, 3.33 mM => final conc. in the 5 μL assay volume is 2 mM) and substrate (1.67 μM => final conc. in the 5 μL assay volume is 1 μM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 7.6 pg/μL. The reaction was stopped by the addition of 3 μL of a solution of HTRF detection reagents (83.3 nM streptavidine-XL665 [Cisbio Bioassays, Codolet, France] and 1.67 nM PT66-Tb-Cryptate, a terbium-cryptate labelled anti-phospho-tyrosine antibody from Cisbio Bioassays [instead of the PT66-Tb-cryptate PT66-Eu-Chelate from Perkin Elmer can also be used]) in an aqueous EDTA-solution (133.3 mM EDTA, 0.2 % (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC50 values were calculated using Genedata Screener™ software.

**Bub1 kinase assay**

Bub1-inhibitory activity of compounds of the present invention was quantified employing the Bub1 TR-FRET assay as described in the following paragraphs.

N-terminally Hiss-tagged recombinant catalytic domain of human Bub1 (amino acids 704-1085), expressed in insect cells (Hi5) and purified by Ni-NTA affinity chromatography and subsequent size exclusion chromatography, was used as enzyme. As substrate for the kinase reaction the biotinylated peptide biotin-Ahx-VLLPKKSFAEPG (C-terminus in amid form) was used which can be purchased e.g. form the company Biosyntan (Berlin, Germany).

For the assay 50 nl of a 100 fold concentrated solution of the test compound in DMSO was pipetted into either a black low volume 384well microtiter plate or a black 1536well microtiter plate (both Greiner Bio-One, Frickenhausen, Germany), 2 μL of a solution of Bub1 in aqueous assay buffer [50 mM Tris/HCl pH 7.5, 10 mM magnesium chloride ($MgCl_2$), 200 mM potassium chloride (KCl), 1.0 mM dithiothreitol (DTT), 0.1 mM sodium ortho-vanadate, 1% (v/v) glycerol, 0.01 % (w/v) bovine serum albumine (BSA), 0.005% (v/v) Trition X-100 (Sigma), 1x *Complete EDTA-free* protease inhibitor mixture (Roche)] were added and the mixture was incubated for 15 min at 22°C to allow pre-binding of the test compounds to the enzyme before the start of the kinase reaction. Then the kinase reaction was started by the addition

of 3 μL of a solution of adenosine-tri-phosphate (ATP, 16.7 μM => final conc. in the 5 μL assay volume is 10 μM) and substrate (1.67 μM => final conc. in the 5 μL assay volume is 1 μM) in assay buffer and the resulting mixture was incubated for a reaction time of 60 min at 22°C. The concentration of Bub1 was adjusted depending of the activity of the enzyme lot and was chosen appropriate to have the assay in the linear range, typical concentrations were in the range of 200 ng/mL. The reaction was stopped by the addition of 5 μL of a solution of TR-FRET detection reagents (0.2 μM streptavidine-XL665 [Cisbio Bioassays, Codolet, France] and 1 nM anti-phosho-Serine antibody [Merck Millipore, cat. # 35-002] and 0.4 nM LANCE EU-W1024 labeled anti-mouse IgG antibody [Perkin-Elmer, product no. AD0077, as an alternative a Terbium-cryptate-labeled anti-mouse IgG antibody from Cisbio Bioassays can be used]) in an aqueous EDTA-solution (50 mM EDTA, 0.2 % (w/v) bovine serum albumin in 100 mM HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar or Pherastar FS (both from BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). The test compounds were tested on the same microtiterplate, usually in 11 different concentrations in the range of 20 μM to 0.1 nM (20 μM, 5.9 μM, 1.7 μM, 0.51 μM, 0.15 μM, 44 nM, 13 nM,

3.8 nM, 1.1 nM, 0.33 nM and 0.1 nM, the dilution series prepared separately before the assay on the level of the 100fold concentrated solutions in DMSO by serial 1:3.4 dilutions, the exact concentrations and the number of tested concentrations may vary depending on the liquid handling instrumentation used for test sample preparation) in duplicate values for each concentration and $IC_{50}$ values were calculated by a 4 parameter fit.

**Bub1 high ATP kinase assay**

Bub1-inhibitory activity of compounds of the present invention at a high ATP concentration was quantified employing the Bub1 TR-FRET high ATP kinase assay as described in the following paragraphs.

N-terminally Hiss-tagged recombinant catalytic domain of human Bub1 (amino acids 704-1085), expressed in insect cells (Hi5) and purified by Ni-NTA affinity chromatography and subsequent size exclusion chromatography, was used as enzyme. As substrate for the kinase reaction the biotinylated peptide biotin-Ahx-VLLPKKSFAEPG (C-terminus in amid form) was used which can be purchased e.g. form the company Biosyntan (Berlin, Germany).

For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 3 μL of a solution of adenosine-tri-phosphate (ATP, 3.33 mM => final conc. in the 5 μL assay volume is 2 mM) and substrate (1.67 μM => final conc. in the 5 μL assay volume is 1 μM) in aqueous assay buffer [50 mM Tris/HCl pH 7.5, 10 mM magnesium chloride ($MgCl_2$), 200 mM potassium chloride (KCl), 1.0 mM dithiothreitol (DTT), 0.1 mM sodium ortho-vanadate, 1% (v/v) glycerol, 0.01 % (w/v) bovine serum albumine (BSA), 0.005% (v/v) Trition X-100 (Sigma), 1x *Complete EDTA-free* protease inhibitor mixture (Roche)] were added. Then the kinase reaction was started by the addition of 2 μL of a solution of Bub1 in assay buffer and the resulting mixture was incubated for a reaction time of 60 min at 22°C. The concentration of Bub1 was adjusted depending of the activity of the enzyme lot and was chosen appropriate to have the assay in the linear range, a typical concentration is about 200 ng/mL. The reaction was stopped by the addition of 3 μL of a solution of TR-FRET detection reagents (0.167 μM streptavidine-XL665 [Cisbio Bioassays, Codolet, France] and 1.67 nM anti-phosho-Serine antibody [Merck Millipore, cat. # 35-002] and 0.67 nM LANCE EU-W1024 labeled anti-mouse IgG antibody [Perkin-Elmer, product no. AD0077, as an alternative a Terbium-cryptate-labeled anti-mouse IgG antibody from Cisbio Bioassays can be used]) in an aqueous EDTA-solution (83.3 mM EDTA, 0.2 % (w/v) bovine serum albumin in 100 mM HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar or Pherastar FS (both from BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.0.7 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC50 values were calculated by a 4 parameter fit.

**Cellular Mechanistic assays**

**P-RPS6 (Ser244/247) Ribosomal protein S6**

The kinase CSNK1A phosphorylates Ribosomal protein S6 at Ser247. In-cell Western assay simultaneously detects two targets at 700 and 800 nm using two spectrally distinct near-infrared dyes. With a specific antibody, Ser244/247-phosphorylated RPS6 (Thermo Fisher 44-923) can be quantified and the samples can be normalized with cell stains Draq5 (Cell signaling, 4084L) and Sapphire700 (LiCor, 928-40022) in parallel.

2000 HCT 116 cells were seeded in growth medium (DMEM / Ham's F12, 10% FCS) in 96well plate (Falcon 353075) over night at 37°C. Cells were treated with varying concentrations of test compounds at 37°C for4h. Cells were fixed with 4% paraformaldehyde, washed (Sigma-Aldrich, AB351787, Tween 20, 1%) and blocked with buffer (Odyssey blocking buffer, LiCor, 927-40000) before incubating with the primary antibody (Ser244/247-phosphorylated RPS6 (Thermo Fisher 44-923) overnight at 2-8°C. After washing, secondary IRDye-labeled antibody mix with cell stains was added for 1h and washed again. Plates were scanned with LiCor Odyssey Infrared Imager CLX at 800 nm for pRPS6 and at 700 nm for cell stains Draq5/Sapphire. The quotient of 800 nm and 700 nm for standard compound-treated cells was set as 0% and the quotient of 800 nm and 700 nm of DMSO treated cells was set as 100%. The results given as % reflecting the inhibition of Casein kinase activity compared to control and normalized according to cell number.

The $IC_{50}$ values were determined by means of a 4 parameter fit.

**P-ß-Catenin (Ser45)**

50,000 DLD-1 cells were seeded in 96 well plates (nunc #161093) in RPMI 1640 (Biochrom; # FG 1215, 10%FCS, 2mM L-Glutamine). After 24h, cells were treated with varying concentrations of test compounds at 37°C for 30 min. Cells were washed twice with ice-cold PBS buffer, treated with lysis buffer and all next steps were performed to the supplier's manual (ß-Catenin pS45 ELISA Kit; ab205703). The content of pS45 was measured with ELISA at 450 nm, calculated with calibration curve and normalized to protein content. The normalized quotient of control compound-treated cells treated cells was set as 0% and the normalized quotient of untreated cells was set as 100%.The results given as % reflecting the content of ß-Catenin pS45 compared to control. The $IC_{50}$ values were determined by means of a 4 parameter fit.

**Proliferation Assays**

**HCT 116**

400 HCT 116 cells/30 µL/well were plated in growth medium (DMEM / Ham's F12, 10% FCS) in a 384-well plate (CORNING #3571) at day 1. Reference plate was seeded for time zero determination. All plates were incubated overnight 37°C. Day 2: test compound was added in 7-step dilution and incubate at 37°C for 96h. Day 2: time zero plate: 30 µL/well CTG solution (Promega Cell Titer Glo solution; catalog # G755B and G756B) were added, incubated for 30 minutes, read luminescence on PheraStar. Day 6: compound treated plates: 30 µL/well CTG solution (Promega Cell Titer Glo solution; catalog # G755B and G756B) were added, incubated for 30 minutes, luminescence was read on PheraStar. Proliferation was calculated after subtracting time zero luminescence values from day 6 values and comparing to untreated wells. The $IC_{50}$ values were determined using the four parameter fit.

**A549**

400 A549 cells/30 µL/well were plated in growth medium (DMEM/Ham's F12, 10% FCS) in a 384-well plate (CORNING #3571) at day 1. Reference plate was seeded for time zero determination. All plates were incubated overnight 37°C. Day 2: test compound was added in 7-step dilution and incubated at 37°C for 96h. Day 2: time zero plate: 30 µL/well CTG solution were added (Promega Cell Titer Glo solution; catalog # G755B and G756B), incubated for 30 minutes, luminescence was read on PheraStar. Day 6: compound treated plates: 30 µL/well CTG solution (Promega Cell Titer Glo solution; catalog # G755B and G756B) were added, incubated for 30 minutes, luminescence was read on PheraStar. Proliferation was calculated after subtracting time zero luminescence values from day 6 values and comparing to untreated wells.

The $IC_{50}$ values were determined using the four parameter fit.

**TMD8**

400 TMD8 cells/30 µL/well were plated in growth medium (RPMI1640, 20% FCS) in a 384-well plate (CORNING

#3571) at day 1. Reference plate was seeded for time zero determination. All plates were incubated overnight 37°C. Day 2: test compound was added in 7-step dilution and incubated at 37°C for 96h. Day 2: time zero plate: 30 μL/well CTG solution (Promega Cell Titer Glo solution; catalog # G755B and G756B) were added, incubated 30 minutes, luminescence was read on PheraStar. Day 6: compound treated plates: 30 μL/well CTG solution (Promega Cell Titer Glo solution; catalog # G755B and G756B) were added, incubated 30 minutes, luminescence was read on PheraStar. Proliferation was calculated after subtracting time zero luminescence values from day 6 values and comparing to untreated wells.

The $IC_{50}$ values were determined using the four parameter fit.

**Results:**

Table 2 shows the results of the inhibition in the CSNK1A1 and CSNK1D biochemical assays.

**Table 2**

| Example No | CSNK1A1 Assay 2 $IC_{50}$ [mol/l] (median) | CSNK1D $IC_{50}$ [mol/l] (median) |
|---|---|---|
| 1 | 1.5 E-9 | 5.5 E-9 |
| 2 | 3.3 E-9 | 5.2 E-9 |
| 3 | 3.8 E-9 | 1.0 E-8 |
| 4 | 1.6 E-8 | 1.6 E-8 |
| 5 | 7.2 E-9 | 6.4 E-9 |
| 6 | 2.6 E-9 | 6.5 E-9 |
| 7 | 5.5 E-9 | 1.1 E-8 |
| 8 | 1.2 E-8 | 2.6 E-8 |
| 9 | 5.6 E-9 | 1.2 E-8 |
| 10 | 5.4 E-9 | 9.1 E-9 |
| 11 | 1.9 E-8 | 2.5 E-8 |
| 12 | 1.8 E-7 | 1.6 E-7 |
| 13 | 1.9 E-8 | 2.2 E-8 |
| 14 | 3.1 E-9 | 5.1 E-9 |
| 15 | 9.4 E-9 | 2.0 E-8 |
| 16 | 4.3 E-9 | 7.1 E-9 |
| 17 | 1.7 E-8 | 1.7 E-8 |
| 18 | 6.6 E-9 | 1.2 E-8 |
| 19 | 3.9 E-9 | 6.5 E-9 |
| 20 | 1.7 E-8 | 2.9 E-8 |
| 21 | 1.1 E-8 | 1.8 E-8 |
| 22 | 5.4 E-9 | 6.3 E-9 |
| 23 | 2.4 E-8 | 3.8 E-8 |
| 24 | 3.0 E-8 | 2.4 E-8 |
| 25 | 2.3 E-9 | 3.1 E-9 |
| 26 | 5.9 E-9 | 2.2 E-8 |
| 27 | 2.1 E-8 | 2.8 E-8 |
| 28 | 5.2 E-9 | 8.1 E-9 |

(continued)

| Example No | CSNK1A1 Assay 2 $IC_{50}$ [mol/l] (median) | CSNK1D $IC_{50}$ [mol/l] (median) |
|---|---|---|
| **29** | 5.0 E-8 | 5.3 E-8 |
| **30** | 3.7 E-9 | 7.1 E-9 |
| **31** | 3.1 E-8 | 3.2 E-8 |
| **32** | 2.7 E-8 | 3.6 E-8 |
| **33** | 8.6 E-9 | 6.2 E-9 |
| **34** | 4.3 E-9 | 9.1 E-9 |
| **35** | 8.0 E-9 | 5.9 E-9 |
| **36** | 6.2 E-9 | 7.6 E-9 |
| **37** | 1.2 E-8 | 1.5 E-8 |
| **38** | 4.2 E-9 | 6.6 E-9 |
| **39** | 5.1 E-8 | 5.6 E-8 |
| **40** | 1.5 E-8 | 1.9 E-8 |
| **41** | 1.0 E-7 | 8.8 E-8 |
| **42** | 1.8 E-9 | 4.1 E-9 |
| **43** | 2.8 E-9 | 4.3 E-9 |
| **44** | 3.3 E-9 | 5.9 E-9 |
| **45** | 5.7 E-9 | 9.7 E-9 |
| **46** | 2.3 E-8 | 7.2 E-8 |
| **47** | 3.4 E-9 | 8.4 E-9 |
| **48** | 7.5 E-9 | 3.2 E-8 |
| **49** | 5.9 E-9 | 1.4 E-8 |
| **50** | 5.1 E-9 | 9.9 E-9 |
| **51** | 2.0 E-8 | 5.7 E-8 |
| **52** | 7.9 E-9 | 1.2 E-8 |
| **53** | 2.1 E-8 | 3.5 E-8 |
| **54** | 3.0 E-9 | 4.0 E-9 |
| **55** | 5.9 E-8 | 1.1 E-7 |
| **56** | 7.7 E-9 | 1.3 E-8 |

Table 3 shows the results of the inhibition in the WT-EGFR assay.

**Table 3**

| Example No | EGFR Wildtyp-2mM ATP $IC_{50}$ [mol/l] (median) |
|---|---|
| **1** | 5.7 E-8 |
| **2** | 1.9 E-7 |
| **3** | 3.2 E-7 |
| **4** | 1.2 E-6 |

(continued)

| Example No | EGFR Wildtyp-2mM ATP $IC_{50}$ [mol/l] (median) |
|---|---|
| **5** | 7.3 E-6 |
| **6** | 1.4 E-6 |
| **7** | 1.3 E-6 |
| **8** | 3.0 E-6 |
| **9** | 1.3 E-5 |
| **10** | > 2.0 E-5 |
| **11** | > 2.0 E-5 |
| **12** | > 2.0 E-5 |
| **13** | > 2.0 E-5 |
| **14** | 1.1 E-6 |
| **15** | 2.9 E-6 |
| **16** | 1.8 E-6 |
| **17** | > 2.0 E-5<br>> 5.7 E-6<br>3.7 E-6<br>3.2 E-6 |
| **18** | 4.3 E-6 |
| **19** | 1.8 E-6 |
| **20** | 8.0 E-6 |
| **21** | 4.5 E-6 |
| **22** | > 2.0 E-5 |
| **23** | 1.3 E-5 |
| **24** | 3.1 E-7 |
| **25** | > 2.0 E-5 |
| **26** | > 2.0 E-5 |
| **27** | > 2.0 E-5 |
| **28** | 1.2 E-5 |
| **29** | > 2.0 E-5 |
| **30** | > 2.0 E-5<br>> 2.0 E-5<br>3.9 E-6<br>5.1 E-6<br>5.5 E-6<br>7.2 E-6<br>1.5 E-5<br>1.0 E-5 |
| **31** | > 2.0 E-5 |
| **32** | 8.6 E-7 |
| **33** | 7.0 E-9 |

(continued)

| Example No | EGFR Wildtyp-2mM ATP $IC_{50}$ [mol/l] (median) |
|---|---|
| **34** | 2.0 E-8 |
| **35** | |
| **36** | 1.1 E-6 |
| **37** | 1.2 E-7 |
| **38** | 1.4 E-6 |
| **39** | 1.8 E-5 |
| **40** | 1.7 E-5 |
| **41** | 9.1 E-6 |
| **42** | 1.1 E-6 |
| **43** | 4.1 E-7 |
| **44** | 4.9 E-7 |
| **45** | 1.5 E-7 |
| **46** | > 2.00 E-5 |
| **47** | > 2.00 E-5 |
| **48** | > 2.00 E-5 |
| **49** | > 2.00 E-5 |
| **50** | > 2.00 E-5 |
| **51** | 1.72 E-5<br>> 2.00 E-5 |
| **52** | > 2.00 E-5 |
| **53** | > 2.00 E-5 |
| **54** | > 2.00 E-5 |
| **55** | 1.08 E-5<br>9.47 E-6 |
| **56** | > 2.00 E-5 |

Compounds of the present invention have surprisingly been found to show advantageous properties, such as effectively inhibiting CSNK1A1 kinase. In particular the exemplified compounds of the present invention display an $IC_{50}$ of below 100 nM in a CSNK1A1 kinase assay in the presence of 1 μM ATP. Furthermore it has been found that said compounds of the present invention additionally show low inhibition of wild type-EGFR kinase. In particular, the exemplified compounds of the present invention are less potent than 600 nM in a wild type-EGFR kinase assay in the presence of 2 mM ATP, which may be useful to reduce potential toxicity arising from excessive inhibition of wild-type EGFR.

In contrast to the claimed compounds of this invention the compounds claimed in WO 2016/120196 do not show the advantageous combined properties described above. This can be seen in Table 4.

**Table 4**

| WO 2016/120196 **Example No.** | **CSNK1A1 Assay 1 $IC_{50}$ [mol/l] (median)** | **CSNK1A1 Assay 2 $IC_{50}$ [mol/l] (median)** | **EGFR Wildtyp-2mM ATP $IC_{50}$ [mol/l] (median)** |
|---|---|---|---|
| **14** | 2.0E-8 | | 1.0E-7 |
| **16** | 4.0E-8 | | 3.0E-7 |
| **23** | | 1.1E-8 | 1.5E-7 |

(continued)

| WO 2016/120196 **Example No.** | **CSNK1A1 Assay 1 $IC_{50}$ [mol/l] (median)** | **CSNK1A1 Assay 2 $IC_{50}$ [mol/l] (median)** | **EGFR Wildtyp-2mM ATP $IC_{50}$ [mol/l] (median)** |
|---|---|---|---|
| **24** | < 3.4E-9<br>9.3E-9<br>1.0E-8<br>5.6E-9<br>4.4E-9 | 4.51E-9 | 4.3E-8 |
| **25** | 6.3E-8 | | 3.2E-7 |
| **40** | 2.4E-8 | | 8.6E-8 |

The following paragraphs are not claims, but represent preferred aspects and embodiments of the invention.

1. A compound of formula (I)

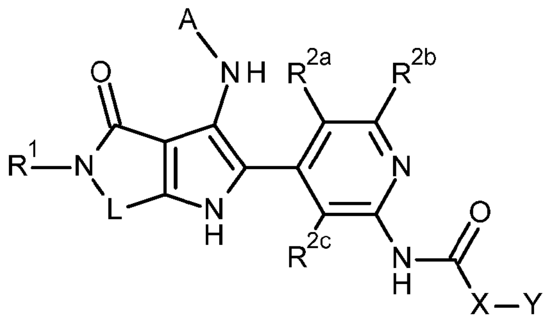

(I)

in which:

$R^1$ represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen;

$R^{2a}$ represents hydrogen, hydroxy, halogen, cyano, $-NH_2$, -NHMe, $-NMe_2$, $C_1$-$C_2$-alkoxy, $C_1$-$C_2$-haloalkyl, or $C_1$-$C_2$-haloalkoxy;

$R^{2b}$ represents hydrogen, hydroxy, halogen, cyano, $-NH_2$, $-NHR^9$, or-$NMe_2$;

$R^{2c}$ represents hydrogen, halogen, cyano, $-NMe_2$, $C_1$-$C_2$-alkoxy, or $C_1$-$C_2$-haloalkoxy;

L represents

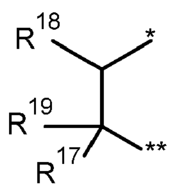 or 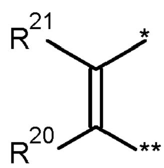 ,

wherein * represents the point of direct attachment to the adjacent N-atom and ** represents the point of direct attachment to the adjacent C-atom;

$R^{17}$ represents $C_1$-$C_3$-alkyl, $C_1$-$C_3$-alkoxy, $C_1$-$C_3$-haloalkyl or $C_3$-$C_6$-cycloalkyl-$C_1$-$C_4$-alkyl-;

$R^{18}$ represents hydrogen, $C_1$-$C_3$-alkyl, $C_1$-$C_3$-haloalkyl or $C_3$-$C_6$-cycloalkyl-$C_1$-$C_4$-alkyl-, or

$R^{17}$ and $R^{18}$, together with the carbon atoms to which they are attached, form a $C_3$-$C_6$-cycloalkyl ring;

$R^{19}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, $C_1$-$C_3$-alkyl, $C_1$-$C_3$-haloalkyl or $C_3$-$C_6$-cycloalkyl-$C_1$-$C_4$-alkyl-;

X represents methylene or ethylene, wherein said methylene and ethylene group are each independently optionally substituted, one or more times, with $R^3$;

Y represents phenyl or a heteroaryl group, wherein said phenyl and heteroaryl group are each independently optionally substituted, one or more times, with $R^4$;

$R^3$ represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, $C_3$-$C_4$-cycloalkyl, -$NHR^6$, $R^5O$-$C_1$-$C_3$-alkyl, $R^6R^7N$-$C_1$-$C_5$-alkyl, or $R^6R^7N$-X'-$C(R^{10})(R^{11})$-$C_1$-$C_2$-alkyl-;

X' represents methylene or ethylene;

$R^4$ represents hydroxy, halogen, cyano, $C_1$-$C_4$-alkyl, $C_1$-$C_4$-alkoxy, $C_1$-$C_4$-haloalkyl, $C_1$-$C_4$-haloalkoxy, -$NMe_2$, -$CO_2R^{14}$, -$CONR^{15}R^{16}$, or -$SO_2Me$;

$R^5$ represents hydrogen, $C_1$-$C_2$-alkyl, $C_1$-$C_2$-haloalkyl, or $C_3$-$C_4$-cycloalkyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or -$CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, -$NH_2$, -$NHR^9$, -$NR^9R^{9a}$, or hydroxy;

A represents a group selected from:

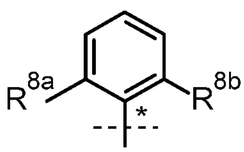, 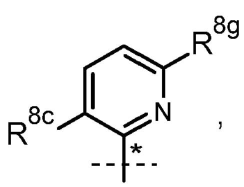, 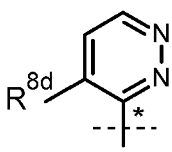, 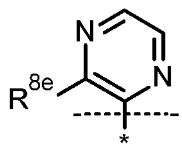,

and

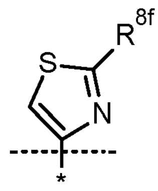

,

wherein * indicates the point of attachment of said group to the -NH- of formula (I);

$R^{8a}$ represents hydrogen, hydroxy, halogen, cyano, $C_1$-$C_2$-alkyl, or $C_1$-$C_2$-haloalkyl;

$R^{8b}$ represents hydrogen, hydroxy, halogen, cyano, $C_1$-$C_2$-alkyl, or $C_1$-$C_2$-haloalkyl;

$R^{8c}$ represents hydrogen, hydroxy, halogen, cyano, $C_1$-$C_2$-alkyl, or $C_1$-$C_2$-haloalkyl;

$R^{8d}$ represents hydrogen, hydroxy, halogen, cyano, $C_1$-$C_2$-alkyl, or $C_1$-$C_2$-haloalkyl;

$R^{8e}$ represents hydrogen, hydroxy, halogen, cyano, $C_1$-$C_2$-alkyl, or $C_1$-$C_2$-haloalkyl;

$R^{8g}$ represents hydrogen or halogen;

$R^{8f}$ represents hydrogen or $C_1$-$C_2$-alkyl;

$R^9$ represents $C_1$-$C_3$-alkyl or cyclopropyl;

$R^{9a}$ represents $C_1$-$C_3$-alkyl or cyclopropyl;

$R^{10}$ represents methyl, halogen, or hydroxy;

$R^{11}$ represents methyl, halogen, or hydroxy;

$R^{14}$ represents hydrogen, methyl or ethyl;

$R^{15}$ represents hydrogen, methyl, or ethyl;

$R^{16}$ represents hydrogen, methyl, ethyl, methoxyethyl, or dimethylaminoethyl, or

$R^{15}$ and $R^{16}$, together with the nitrogen atom to which they are attached, represent a 5- to 6-membered heterocyclic ring, wherein said heterocyclic ring optionally contains one or two further heteroatoms selected from N, O, S, or groups selected from C(=O), S(=O), and $SO_2$;

or an N-oxide, a salt, a tautomer or a stereoisomer of said compound, or a salt of said N-oxide, tautomer or stereoisomer.

2. The compound of formula (I) according to paragraph 1, wherein:

$R^1$ represents hydrogen or a group selected from $C_1$-$C_4$-alkyl and $C_3$-$C_6$-cycloalkyl, wherein said groups are each independently optionally substituted, one or more times, with halogen;

$R^{2a}$ represents hydrogen or halogen;

$R^{2b}$ represents hydrogen, halogen, $-NH_2$ or $-NHR^9$;

$R^{2c}$ represents hydrogen or halogen;

L represents

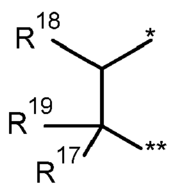 or 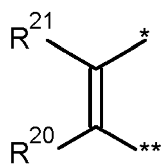 ,

wherein * represents the point of direct attachment to the adjacent N-atom and ** represents the point of direct attachment to the adjacent C-atom;

$R^{17}$ represents $C_1$-$C_3$-alkyl, $C_1$-$C_3$-haloalkyl or $C_3$-$C_6$-cycloalkyl-$C_1$-$C_4$-alkyl-;

$R^{18}$ represents hydrogen, $C_1$-$C_3$-alkyl, $C_1$-$C_3$-haloalkyl or $C_3$-$C_6$-cycloalkyl-$C_1$-$C_4$-alkyl-, or

$R^{17}$ and $R^{18}$, together with the carbon atoms to which they are attached, form a $C_3$-$C_6$-cycloalkyl ring;

$R^{19}$ represents hydrogen or $C_1$-$C_3$-alkyl;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, $C_1$-$C_3$-alkyl, or $C_1$-$C_3$-haloalkyl;

X represents methylene, wherein said methylene group is optionally substituted, one or more times, with $R^3$;

Y represents phenyl or a heteroaryl group, wherein said phenyl and heteroaryl group are each independently optionally substituted, one or more times, with $R^4$;

$R^3$ represents $C_1$-$C_3$-alkyl, $C_1$-$C_3$-haloalkyl, $R^5O$-$C_1$-$C_3$-alkyl, or $R^6R^7N$-$C_1$-$C_5$-alkyl;

$R^4$ represents halogen, $C_1$-$C_4$-alkyl, $C_1$-$C_4$-haloalkyl, -$OCF_3$, -$CO_2R^{14}$, -$CONR^{15}R^{16}$, or -SOzMe;

$R^5$ represents hydrogen, methyl, or trifluoromethyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or -$CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, and is independently optionally substituted, one or more times, with halogen, $C_1$-$C_3$-alkyl, $C_3$-cycloalkyl-$C_1$-$C_2$-alkyl-, -$NH_2$, -$NHR^9$, -$NR^9R^{9a}$, or hydroxy;

A represents a group selected from:

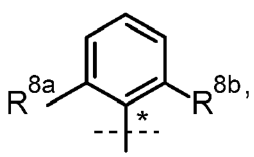 , 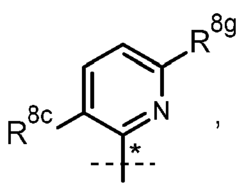 , and 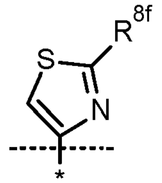 ,

wherein * indicates the point of attachment of said group to the -NH- of formula (I);

$R^{8a}$ represents hydrogen, halogen, or $C_1$-$C_2$-alkyl;

$R^{8b}$ represents hydrogen or halogen;

$R^{8c}$ represents hydrogen or halogen;

$R^{8g}$ represents hydrogen or halogen;

$R^{8f}$ represents hydrogen or methyl;

$R^9$ represents $C_1$-$C_3$-alkyl or cyclopropyl;

$R^{9a}$ represents $C_1$-$C_3$-alkyl or cyclopropyl;

$R^{14}$ represents hydrogen, methyl or ethyl;

$R^{15}$ represents hydrogen or methyl;

$R^{16}$ represents methyl, ethyl, methoxyethyl, or dimethylaminoethyl, or

$R^{15}$ and $R^{16}$, together with the nitrogen atom to which they are attached, represent a 5- to 6-membered heterocyclic ring, wherein said heterocyclic ring optionally contains one further heteroatom selected from N and O;

or an N-oxide, a salt, a tautomer or a stereoisomer of said compound, or a salt of said N-oxide, tautomer or stereoisomer.

3. The compound of formula (I) according to paragraph 1 or 2, wherein:

$R^1$ represents hydrogen or $C_1$-$C_2$-alkyl, wherein said alkyl is optionally substituted, one or more times, with halogen;

$R^{2a}$ represents hydrogen or halogen;

$R^{2b}$ represents hydrogen, $-NH_2$ or $-NHR^9$;

$R^{2c}$ represents hydrogen or halogen;

L represents

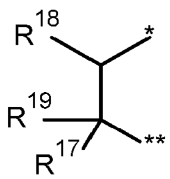

or

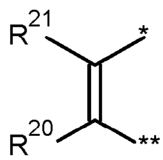

,

wherein * represents the point of direct attachment to the adjacent N-atom and ** represents the point of direct attachment to the adjacent C-atom;

$R^{17}$ represents $C_1$-$C_3$-alkyl, $C_1$-$C_3$-haloalkyl or $C_3$-$C_4$-cycloalkyl-$C_1$-$C_3$-alkyl-;

$R^{18}$ represents hydrogen, $C_1$-$C_3$-alkyl, or $C_1$-$C_3$-haloalkyl, or

$R^{17}$ and $R^{18}$, together with the carbon atoms to which they are attached, form a $C_3$-$C_6$-cycloalkyl ring;

$R^{19}$ represents hydrogen or $C_1$-$C_3$-alkyl;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, $C_1$-$C_3$-alkyl, or $C_1$-$C_3$-haloalkyl;

X represents methylene, wherein said methylene group is optionally substituted, one or more times, with $R^3$;

Y represents phenyl or a pyridine group, wherein said phenyl and pyridine group are each independently optionally substituted, one or more times, with $R^4$;

$R^3$ represents $C_1$-$C_3$-alkyl, $C_1$-$C_3$-haloalkyl, $R^5O$-$C_1$-$C_2$-alkyl, or $R^6R^7N$-$C_1$-$C_5$-alkyl;

$R^4$ represents halogen, $C_1$-$C_4$-alkyl or $C_1$-$C_4$-haloalkyl;

$R^5$ represents hydrogen;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or

$R^6$ and $R^7$, together with the nitrogen atom to which they are attached, represent a 4- to 6-membered heterocyclic ring, wherein said heterocyclic ring is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;

A represents a group:

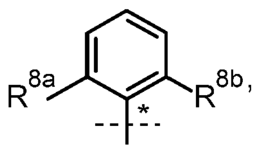 and 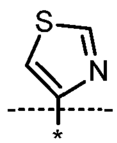 ,

wherein * indicates the point of attachment of said group to the -NH- of formula (I);

$R^{8a}$ represents hydrogen, halogen, or $C_1$-$C_2$-alkyl;

$R^{8b}$ represents hydrogen;

$R^9$ represents $C_1$-$C_3$-alkyl or cyclopropyl;

$R^{9a}$ represents $C_1$-$C_3$-alkyl or cyclopropyl;

or an N-oxide, a salt, a tautomer or a stereoisomer of said compound, or a salt of said N-oxide, tautomer or stereoisomer.

4. The compound of formula (I) according to paragraph 1, 2 or 3, wherein:

$R^1$ represents hydrogen or $C_1$-$C_2$-alkyl;

$R^{2a}$ represents hydrogen;

$R^{2b}$ represents hydrogen;

$R^{2c}$ represents hydrogen;

L represents

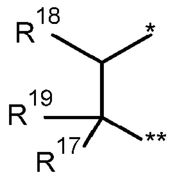 or 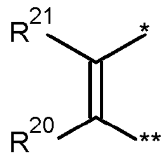 ,

wherein * represents the point of direct attachment to the adjacent N-atom and ** represents the point of direct attachment to the adjacent C-atom;

$R^{17}$ represents $C_1$-$C_3$-alkyl, $C_1$-$C_3$-haloalkyl or $C_3$-$C_4$-cycloalkyl-$C_1$-$C_3$-alkyl-;

$R^{18}$ represents hydrogen or $C_1$-$C_3$-alkyl, or

$R^{17}$ and $R^{18}$, together with the carbon atoms to which they are attached, form a $C_3$-$C_5$-cycloalkyl ring;

$R^{19}$ represents hydrogen;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, or $C_1$-$C_2$-alkyl;

X represents methylene, wherein said methylene group is optionally substituted, one or more times, with $R^3$;

Y represents phenyl, wherein said phenyl is independently optionally substituted, one or more times, with $R^4$;

$R^3$ represents $C_1$-$C_3$-alkyl, or $C_1$-$C_3$-haloalkyl;

$R^4$ represents halogen;

A represents a group:

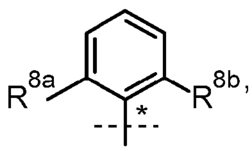 and 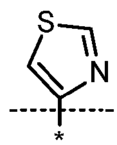 ,

wherein * indicates the point of attachment of said group to the -NH- of formula (I);

$R^{8a}$ represents hydrogen, halogen, or methyl;

$R^{8b}$ represents hydrogen;

or an N-oxide, a salt, a tautomer or a stereoisomer of said compound, or a salt of said N-oxide, tautomer or stereoisomer.

5. The compound of formula (I) according to any of paragraphs 1 to 4, which is selected from the group consisting of:

N-{4-[3-anilino-7-ethyl-4-oxo-4,5,6,7-tetrahydro-1H-pyrrolo[3,2-c]pyridin-2-yl]pyridin-2-yl}-2-(4-fluorophenyl)acetamide;

N-{4-[(7R)-3-anilino-7-ethyl-4-oxo-4,5,6,7-tetrahydro-1H-pyrrolo[3,2-c]pyridin-2-yl]pyridin-2-yl}-2-(4-fluorophenyl)acetamide;

N-{4-[(7S)-3-anilino-7-ethyl-4-oxo-4,5,6,7-tetrahydro-1H-pyrrolo[3,2-c]pyridin-2-yl]pyridin-2-yl}-2-(4-fluorophenyl)acetamide;

N-{4-[3-anilino-7-ethyl-4-oxo-4,5,6,7-tetrahydro-1H-pyrrolo[3,2-c]pyridin-2-yl]pyridin-2-yl}-4,4-difluoro-2-(4-fluorophenyl)butanamide;

N-[4-(3-anilino-7-ethyl-4-oxo-4,5-dihydro-1H-pyrrolo[3,2-c]pyridin-2-yl)pyridin-2-yl]-4,4-difluoro-2-(4-fluorophenyl)butanamide;

N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide;

(-)-N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide;

(+)-N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide;

N-{4-[(7RS)-3-anilino-5,7-dimethyl-4-oxo-4,5,6,7-tetrahydro-1H-pyrrolo[3,2-c]pyridin-2-yl]pyridin-2-yl}-2-(4-fluorophenyl)propenamide;

N-{4-[3-anilino-5,7-dimethyl-4-oxo-4,5,6,7-tetrahydro-1H-pyrrolo[3,2-c]pyridin-2-yl]pyridin-2-yl}-4,4-difluoro-2-(4-fluorophenyl)butanamide;

N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide;

(2R)-N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide;

(2S)-N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide;

N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide;

N-{4-[(7R)-3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide;

N-{4-[(7S)-3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide;

N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-4,4-difluoro-2-(4-fluorophenyl)butanamide;

N-{4-[3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide;

N-{4-[(7R)-3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide;

N-{4-[(7S)-3-anilino-7-(cyclopropylmethyl)-5-methyl-4-oxo-4,5,6,7-tetrahydro-1H-pyrrolo[3,2-c] pyridin-2-yl]pyridin-2-yl}-2-(4-fluorophenyl)acetamide;

(2RS)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;

(+)-(2R)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;

(-)-(2S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;

(-)-(2R)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;

(+)-(2S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;

(2S)-N-[4-(3-anilino-5-methyl-4-oxo-4,5-dihydro-1H-pyrrolo[3,2-c]pyridin-2-yl)pyridin-2-yl]-2-(4-fluorophenyl)propenamide;

(2R)-N-[4-(3-anilino-5-methyl-4-oxo-4,5-dihydro-1H-pyrrolo[3,2-c]pyridin-2-yl)pyridin-2-yl]-2-(4-fluorophenyl)propenamide;

N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide;

(2R)-N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide;

(2S)-N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide;

4,4-difluoro-N-{4-[3-(2-fluoroanilino)-5,6-dimethyl-4-oxo-4,5-dihydro-1H-pyrrolo[3,2-c]pyridin-2-yl]pyridin-2-yl}-2-(4-fluorophenyl)butanamide;

N-{4-[3-anilino-7-(cyclopropylmethyl)-5-methyl-4-oxo-4,5-dihydro-1H-pyrrolo[3,2-c]pyridin-2-yl]pyridin-2-yl}-2-(4-fluorophenyl)acetamide;

2-(4-fluorophenyl)-N-{4-[5-methyl-3-(2-methylanilino)-4-oxo-7-(2,2,2-trifluoroethyl)-4,5-dihydro-1H-pyrrolo[3,2-c]pyridin-2-yl]pyridin-2-yl}acetamide;

N-{4-[3-anilino-5-methyl-4-oxo-7-(2,2,2-trifluoroethyl)-4,5-dihydro-1H-pyrrolo[3,2-c]pyridin-2-yl]pyridin-2-yl}-2-(4-fluorophenyl)acetamide;

N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide;

(2R)-N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide;

(2S)-N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide;

N-[4-(3-anilino-5,7-dimethyl-4-oxo-4,5-dihydro-1H-pyrrolo[3,2-c]pyridin-2-yl)pyridin-2-yl]-2-(4-fluorophenyl)acetamide;

N-[4-(3-anilino-5,7-dimethyl-4-oxo-4,5-dihydro-1H-pyrrolo[3,2-c]pyridin-2-yl)pyridin-2-yl]-2-(4-fluorophenyl)propenamide;

(2R)-N-[4-(3-anilino-5,7-dimethyl-4-oxo-4,5-dihydro-1H-pyrrolo[3,2-c]pyridin-2-yl)pyridin-2-yl]-2-(4-fluorophenyl)propenamide;

(2S)-N-[4-(3-anilino-5,7-dimethyl-4-oxo-4,5-dihydro-1H-pyrrolo[3,2-c]pyridin-2-yl)pyridin-2-yl]-2-(4-fluorophenyl)propenamide;

N-{4-[3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide;

N-{4-[(7R)-3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide;

N-{4-[(7S)-3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide;

N-{4-[3-anilino-7-(2,2-difluoroethyl)-5-methyl-4-oxo-4,5-dihydro-1H-pyrrolo[3,2-c]pyridin-2-yl]pyridin-2-yl}-2-(4-fluorophenyl)acetamide;

2-(4-fluorophenyl)-N-{4-[(7RS)-5-methyl-4-oxo-3-(1, 3-thiazol-4-ylamino)-7-(2,2,2-trifluoroethyl)-4,5,6,7-tetrahydro-1H-pyrrolo[3,2-c]pyridin-2-yl]pyridin-2-yl}acetamide;

2-(4-fluorophenyl)-N-{4-[(7R)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide;

2-(4-fluorophenyl)-N-{4-[(7S)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide;

N-{4-[(7RS)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide;

N-{4-[(7R)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide;

N-{4-[(7S)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide;

(2RS)-4,4-difluoro-2-(4-fluorophenyl)-N-{4-[(7RS)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;

(2R)-4,4-difluoro-2-(4-fluorophenyl)-N-{4-[(7R)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;

(2R)-4,4-difluoro-2-(4-fluorophenyl)-N-{4-[(7S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;

(2S)-4,4-difluoro-2-(4-fluorophenyl)-N-{4-[(7R)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;

(2S)-4,4-difluoro-2-(4-fluorophenyl)-N-{4-[(7S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;

2-(4-fluorophenyl)-N-{4-[5-methyl-3-(2-methylanilino)-4-oxo-7-(2,2,2-trifluoroethyl)-4,5,6,7-tetrahydro-1H-pyrrolo[3,2-c]pyridin-2-yl]pyridin-2-yl}acetamide;

or an N-oxide, a salt, a tautomer or a stereoisomer of said compound, or a salt of said N-oxide, tautomer or stereoisomer.

6. A method of preparing a compound of general formula **(I)** according to any one of paragraphs 1 to 5, said method comprising reacting an intermediate compound of general formula (1-3):

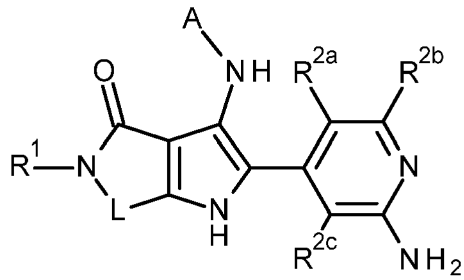

(1-3),

in which $R^1$, $R^{2a}$, $R^{2b}$, $R^{2c}$, A and L are as defined for the compound of general formula (I) according to any one of paragraphs 1 to 4,
with an acylating reagent selected from:

a) a carboxylic acid of formula

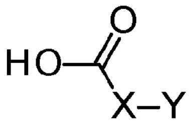

,

b) an acyl halide of formula

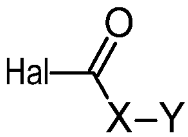

,

wherein Hal represents F, Cl or Br, or
c) an anhydride of formula

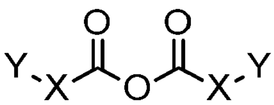

or

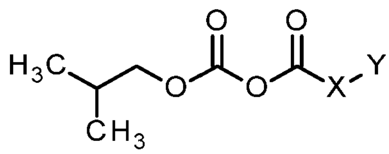

in which X and Y are as defined for the compound of general formula (I) according to any one of paragraphs 1 to 4,

to give a compound of general formula (I) :

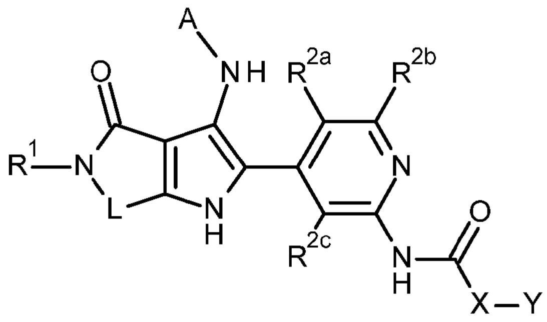

(I),

in which $R^1$, $R^{2a}$, $R^{2b}$, $R^{2c}$, A, L, X and Y are as defined for the compound of general formula (I) according to any one of paragraphs 1 to 4.

7. Use of a compound of general formula (I) according to any of paragraphs 1 to 5 for the treatment or prophylaxis of diseases.

8. Use of a compound of general formula (I) according to paragraph 7, wherein the diseases are hyperproliferative diseases and/or disorders responsive to induction of cell death.

9. Use of a compound of general formula (I) according to paragraph 8, wherein the hyperproliferative diseases and/or disorders responsive to induction of cell death are haematological tumours, solid tumours and/or metastases thereof.

10. Use of a compound of formula (I) according to paragraph 9, wherein the haematological tumour is a lymphoma and/or metastases thereof.

11. Use of a compound of formula (I) according to paragraph 10, wherein the lymphoma is diffuse large B-cell lymphoma and/or metastases thereof.

12. Use of a compound of formula (I) according to paragraph 9, wherein the solid tumour is a cervical tumour, a lung tumour, a colon tumour and/or metastases thereof.

13. Use of a compound of formula (I) according to paragraph 12, wherein the lung tumour is a lung carcinoma and/or metastases thereof.

14. Use of a compound of formula (I) according to paragraph 12, wherein the colon tumour is a colorectal carcinoma and/or metastases thereof.

15. A pharmaceutical composition comprising at least one compound of general formula (I) according to any of paragraphs 1 to 5, together with at least one pharmaceutically acceptable excipient.

16. A composition according to paragraph 15 for the treatment of haematological tumours, solid tumours and/or metastases thereof.

17. A combination comprising one or more first active ingredients selected from a compound of general formula (I) according to any of paragraphs 1 to 5, and one or more second active ingredients selected from chemotherapeutic anti-cancer agents and target-specific anti-cancer agents.

18. An intermediate compound 1-3, or a salt thereof:

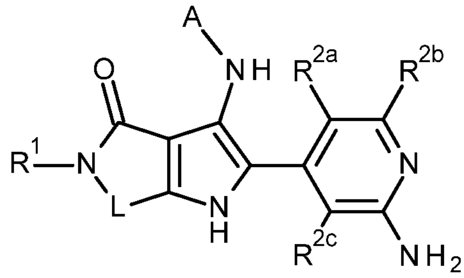

1-3,

in which $R^1$, $R^{2a}$, $R^{2b}$, $R^{2c}$, A, and L are as defined for the compound of general formula (I) according to any one of paragraphs 1 to 4.

19. Use of a compound of formula 1-3, or a salt thereof:

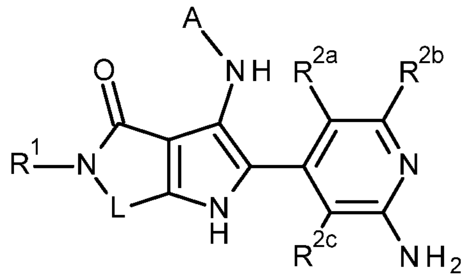

1-3,

in which $R^1$, $R^{2a}$, $R^{2b}$, $R^{2c}$, A, and L are as defined for the compound of general formula (I) according to any one of paragraphs 1 to 4, for the preparation of a compound of formula (I) as defined in any one of paragraphs 1 to 5.

20. A method of preparing a compound of general formula (Ib) according to any one of paragraphs 1 to 5, said method comprising reacting an intermediate compound of general formula (6-1):

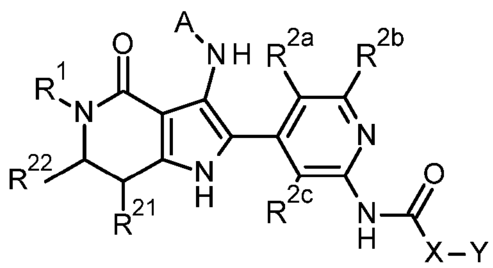

**6-1**

,

in which $R^1$, $R^{2a}$, $R^{2b}$, $R^{2c}$, $R^{21}$, $R^{22}$, A, X, and Y are as defined for the compound of general formula (I) according to any one of paragraphs 1 to 4, with an oxidizing reagent to give a compound of general formula (Ib):

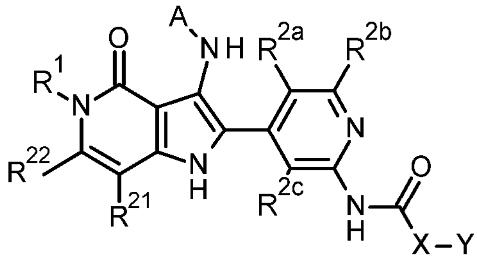

(Ib),

in which $R^1$, $R^{2a}$, $R^{2b}$, $R^{2c}$, $R^{21}$, $R^{22}$, A, X, and Y are as defined for the compound of general formula (I) according to any one of paragraphs 1 to 4.

21. Use of a compound of formula 6-1:

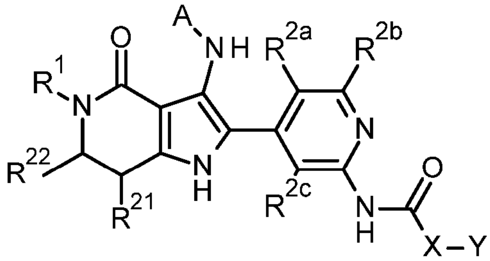

**6-1**

,

in which $R^1$, $R^{2a}$, $R^{2b}$, $R^{2c}$, $R^{21}$, $R^{22}$, A, X, and Y are as defined for the compound of general formula (I) according to any one of paragraphs 1 to 4, for the preparation of a compound of formula (I) as defined in any one of paragraphs 1 to 5.

## Claims

**1.** A compound of formula (I) for use in the treatment of a disease mediated by CSNK1A1 or CSNK1D activity in a subject:

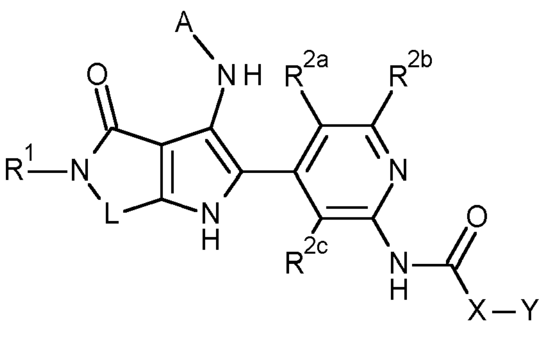

(I)

wherein:

$R^1$ represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen;

$R^{2a}$ represents hydrogen, hydroxy, halogen, cyano, $-NH_2$, -NHMe, $-NMe_2$, $C_1$-$C_2$-alkoxy, $C_1$-$C_2$-haloalkyl, or $C_1$-$C_2$-haloalkoxy;

$R^{2b}$ represents hydrogen, hydroxy, halogen, cyano, $-NH_2$, $-NHR^9$, or $-NMe_2$;

$R^{2c}$ represents hydrogen, halogen, cyano, $-NMe_2$, $C_1$-$C_2$-alkoxy, or $C_1$-$C_2$-haloalkoxy;

L represents

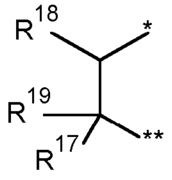 or 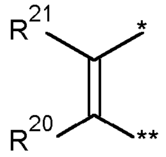 ,

wherein * represents the point of direct attachment to the adjacent N-atom and ** represents the point of direct attachment to the adjacent C-atom;

$R^{17}$ represents $C_1$-$C_3$-alkyl, $C_1$-$C_3$-alkoxy, $C_1$-$C_3$-haloalkyl or $C_3$-$C_6$-cycloalkyl-$C_1$-$C_4$-alkyl-;

$R^{18}$ represents hydrogen, $C_1$-$C_3$-alkyl, $C_1$-$C_3$-haloalkyl or $C_3$-$C_6$-cycloalkyl-$C_1$-$C_4$-alkyl-, or

$R^{17}$ and $R^{18}$, together with the carbon atoms to which they are attached, form a $C_3$-$C_6$-cycloalkyl ring;

$R^{19}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;

$R^{21}$ represents hydrogen, $C_1$-$C_3$-alkyl, $C_1$-$C_3$-haloalkyl or $C_3$-$C_6$-cycloalkyl-$C_1$-$C_4$-alkyl-;

X represents methylene or ethylene, wherein said methylene and ethylene group are each independently optionally substituted, one or more times, with $R^3$;

Y represents phenyl or a heteroaryl group, wherein said phenyl and heteroaryl group are each independently optionally substituted, one or more times, with $R^4$;

$R^3$ represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, $C_3$-$C_4$-cycloalkyl, $-NHR^6$, $R^5O$-$C_1$-$C_3$-alkyl, $R^6R^7N$-$C_1$-$C_5$-alkyl, or $R^6R^7N$-X'-C($R^{10}$)($R^{11}$)-$C_1$-$C_2$-alkyl-;

X' represents methylene or ethylene;

$R^4$ represents hydroxy, halogen, cyano, $C_1$-$C_4$-alkyl, $C_1$-$C_4$-alkoxy, $C_1$-$C_4$-haloalkyl, $C_1$-$C_4$-haloalkoxy, $-NMe_2$, $-CO_2R^{14}$, $-CONR^{15}R^{16}$, or $-SO_2Me$;

$R^5$ represents hydrogen, $C_1$-$C_2$-alkyl, $C_1$-$C_2$-haloalkyl, or $C_3$-$C_4$-cycloalkyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;

A represents a group selected from:

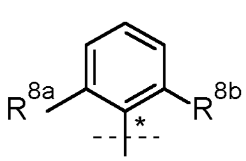 , 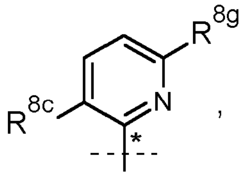 , 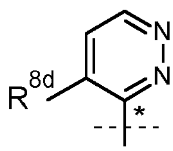 , 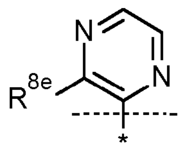 ,

and

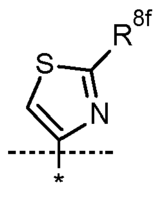 ,

wherein * indicates the point of attachment of said group to the -NH- of formula (I);
$R^{8a}$ represents hydrogen, hydroxy, halogen, cyano, $C_1$-$C_2$-alkyl, or $C_1$-$C_2$-haloalkyl;
$R^{8b}$ represents hydrogen, hydroxy, halogen, cyano, $C_1$-$C_2$-alkyl, or $C_1$-$C_2$-haloalkyl;
$R^{8c}$ represents hydrogen, hydroxy, halogen, cyano, $C_1$-$C_2$-alkyl, or $C_1$-$C_2$-haloalkyl;
$R^{8d}$ represents hydrogen, hydroxy, halogen, cyano, $C_1$-$C_2$-alkyl, or $C_1$-$C_2$-haloalkyl;
$R^{8e}$ represents hydrogen, hydroxy, halogen, cyano, $C_1$-$C_2$-alkyl, or $C_1$-$C_2$-haloalkyl;
$R^{8g}$ represents hydrogen or halogen;
$R^{8f}$ represents hydrogen or $C_1$-$C_2$-alkyl;
$R^9$ represents $C_1$-$C_3$-alkyl or cyclopropyl;
$R^{9a}$ represents $C_1$-$C_3$-alkyl or cyclopropyl;
$R^{10}$ represents methyl, halogen, or hydroxy;
$R^{11}$ represents methyl, halogen, or hydroxy;
$R^{14}$ represents hydrogen, methyl or ethyl;
$R^{15}$ represents hydrogen, methyl, or ethyl;
$R^{16}$ represents hydrogen, methyl, ethyl, methoxyethyl, or dimethylaminoethyl, or
$R^{15}$ and $R^{16}$, together with the nitrogen atom to which they are attached, represent a 5- to 6-membered heterocyclic ring, wherein said heterocyclic ring optionally contains one or two further heteroatoms selected from N, O, S, or groups selected from C(=O), S(=O), and $SO_2$;
or an N-oxide, a salt, a tautomer or a stereoisomer of said compound, or a salt of said N-oxide, tautomer or stereoisomer.

2. The compound for use according to claim 1, wherein:

$R^1$ represents hydrogen or a group selected from $C_1$-$C_4$-alkyl and $C_3$-$C_6$-cycloalkyl, wherein said groups are each independently optionally substituted, one or more times, with halogen;
$R^{2a}$ represents hydrogen or halogen;
$R^{2b}$ represents hydrogen, halogen, $-NH_2$ or $-NHR^9$;
$R^{2c}$ represents hydrogen or halogen;
L represents

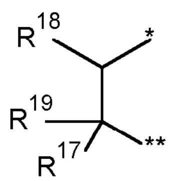

or

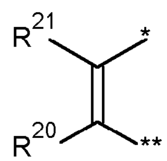

,

wherein * represents the point of direct attachment to the adjacent N-atom and ** represents the point of direct attachment to the adjacent C-atom;
$R^{17}$ represents $C_1$-$C_3$-alkyl, $C_1$-$C_3$-haloalkyl or $C_3$-$C_6$-cycloalkyl-$C_1$-$C_4$-alkyl-;
$R^{18}$ represents hydrogen, $C_1$-$C_3$-alkyl, $C_1$-$C_3$-haloalkyl or $C_3$-$C_6$-cycloalkyl-$C_1$-$C_4$-alkyl-, or
$R^{17}$ and $R^{18}$, together with the carbon atoms to which they are attached, form a $C_3$-$C_6$-cycloalkyl ring;
$R^{19}$ represents hydrogen or $C_1$-$C_3$-alkyl;
$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;
$R^{21}$ represents hydrogen, $C_1$-$C_3$-alkyl, or $C_1$-$C_3$-haloalkyl;
X represents methylene, wherein said methylene group is optionally substituted, one or more times, with $R^3$;
Y represents phenyl or a heteroaryl group, wherein said phenyl and heteroaryl group are each independently optionally substituted, one or more times, with $R^4$;
$R^3$ represents $C_1$-$C_3$-alkyl, $C_1$-$C_3$-haloalkyl, $R^5O$-$C_1$-$C_3$-alkyl, or $R^6R^7N$-$C_1$-$C_5$-alkyl;
$R^4$ represents halogen, $C_1$-$C_4$-alkyl, $C_1$-$C_4$-haloalkyl, $-OCF_3$, $-CO_2R^{14}$, $-CONR^{15}R^{16}$, or $-SO_2Me$;
$R^5$ represents hydrogen, methyl, or trifluoromethyl;
$R^6$ represents hydrogen, $C_1$-$C_2$-alkyl, $C_1$-$C_2$-haloalkyl, or $C_3$-$C_4$-cycloalkyl;
$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or
$R^6$ and $R^7$, together with the nitrogen atom to which they are attached, represent a 4- to 7-membered heterocyclic ring, wherein said heterocyclic ring optionally contains one or more further heteroatoms selected from N, O, S, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;
A represents a group selected from:

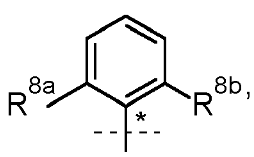, 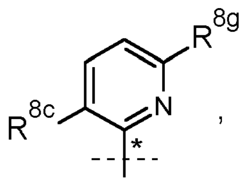 , and 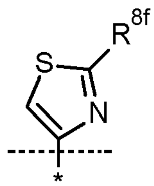 ,

wherein * indicates the point of attachment of said group to the -NH- of formula (I);
$R^{8a}$ represents hydrogen, halogen, or $C_1$-$C_2$-alkyl;
$R^{8b}$ represents hydrogen or halogen;
$R^{8c}$ represents hydrogen or halogen;
$R^{8g}$ represents hydrogen or halogen;
$R^{8f}$ represents hydrogen or methyl;
$R^9$ represents $C_1$-$C_3$-alkyl or cyclopropyl;
$R^{9a}$ represents $C_1$-$C_3$-alkyl or cyclopropyl;
$R^{14}$ represents hydrogen, methyl or ethyl;
$R^{15}$ represents hydrogen or methyl;
$R^{16}$ represents methyl, ethyl, methoxyethyl, or dimethylaminoethyl, or
$R^{15}$ and $R^{16}$, together with the nitrogen atom to which they are attached, represent a 5- to 6-membered heterocyclic ring, wherein said heterocyclic ring optionally contains one further heteroatom selected from N and O;
or an N-oxide, a salt, a tautomer or a stereoisomer of said compound, or a salt of said N-oxide, tautomer or stereoisomer.

**3.** The compound for use of claim 1 or 2, wherein:

$R^1$ represents hydrogen or $C_1$-$C_2$-alkyl, wherein said alkyl is optionally substituted, one or more times, with halogen;
$R^{2a}$ represents hydrogen or halogen;
$R^{2b}$ represents hydrogen, -$NH_2$ or -$NHR^9$;
$R^{2c}$ represents hydrogen or halogen;
L represents

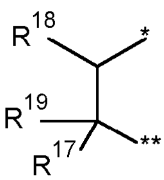 or 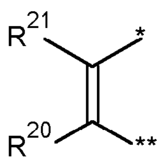 ,

wherein * represents the point of direct attachment to the adjacent N-atom and ** represents the point of direct attachment to the adjacent C-atom;
$R^{17}$ represents $C_1$-$C_3$-alkyl, $C_1$-$C_3$-haloalkyl or $C_3$-$C_4$-cycloalkyl-$C_1$-$C_3$-alkyl-;
$R^{18}$ represents hydrogen, $C_1$-$C_3$-alkyl, or $C_1$-$C_3$-haloalkyl, or
$R^{17}$ and $R^{18}$, together with the carbon atoms to which they are attached, form a $C_3$-$C_6$-cycloalkyl ring;
$R^{19}$ represents hydrogen or $C_1$-$C_3$-alkyl;
$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;
$R^{21}$ represents hydrogen, $C_1$-$C_3$-alkyl, or $C_1$-$C_3$-haloalkyl;
$R^{22}$ represents hydrogen, $C_1$-$C_3$-alkyl, $C_1$-$C_3$-haloalkyl or $C_3$-$C_6$-cycloalkyl-$C_1$-$C_4$-alkyl-;
X represents methylene, wherein said methylene group is optionally substituted, one or more times, with $R^3$;
Y represents phenyl or a pyridine group, wherein said phenyl and pyridine group are each independently optionally substituted, one or more times, with $R^4$;
$R^3$ represents $C_1$-$C_3$-alkyl, $C_1$-$C_3$-haloalkyl, $R^SO$-$C_1$-$C_2$-alkyl, or $R^6R^7N$-$C_1$-$C_5$-alkyl;
$R^4$ represents halogen, $C_1$-$C_4$-alkyl or $C_1$-$C_4$-haloalkyl;
$R^5$ represents hydrogen;
$R^6$ represents hydrogen, $C_1$-$C_2$-alkyl, $C_1$-$C_2$-haloalkyl, or $C_3$-$C_4$-cycloalkyl;
$R^7$ represents hydrogen, $C_1$-$C_2$-alkyl, $C_1$-$C_2$-haloalkyl, $C_3$-$C_4$-cycloalkyl, or -$CO_2$-$C_1$-$C_4$-alkyl, or

$R^6$ and $R^7$, together with the nitrogen atom to which they are attached, represent a 4- to 6-membered heterocyclic ring, wherein said heterocyclic ring is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;
A represents a group:

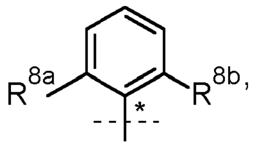
and
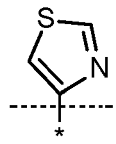
,

wherein * indicates the point of attachment of said group to the -NH- of formula (I);
$R^{8a}$ represents hydrogen, halogen, or $C_1$-$C_2$-alkyl;
$R^{8b}$ represents hydrogen;
$R^9$ represents $C_1$-$C_3$-alkyl or cyclopropyl;
$R^{9a}$ represents $C_1$-$C_3$-alkyl or cyclopropyl;
or an N-oxide, a salt, a tautomer or a stereoisomer of said compound, or a salt of said N-oxide, tautomer or stereoisomer.

**4.** The compound for use of claims 1, 2, or 3, wherein:

$R^1$ represents hydrogen or $C_1$-$C_2$-alkyl;
$R^{2a}$ represents hydrogen;
$R^{2b}$ represents hydrogen;
$R^{2c}$ represents hydrogen;
L represents

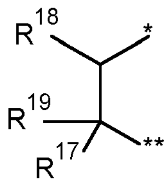
or
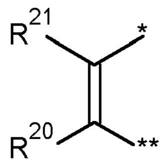
,

wherein * represents the point of direct attachment to the adjacent N-atom and ** represents the point of direct attachment to the adjacent C-atom;
$R^{17}$ represents $C_1$-$C_3$-alkyl, $C_1$-$C_3$-haloalkyl or $C_3$-$C_4$-cycloalkyl-$C_1$-$C_3$-alkyl-;
$R^{18}$ represents hydrogen or $C_1$-$C_3$-alkyl, or
$R^{17}$ and $R^{18}$, together with the carbon atoms to which they are attached, form a $C_3$-$C_5$-cycloalkyl ring;
$R^{19}$ represents hydrogen;
$R^{20}$ represents hydrogen, $C_1$-$C_3$-alkyl, $C_1$-$C_3$-haloalkyl or $C_3$-$C_6$-cycloalkyl-$C_1$-$C_4$-alkyl-;
$R^{21}$ represents hydrogen, or $C_1$-$C_2$-alkyl;
$R^{22}$ represents hydrogen, $C_1$-$C_3$-alkyl, or $C_1$-$C_3$-haloalkyl;
X represents methylene, wherein said methylene group is optionally substituted, one or more times, with $R^3$;
Y represents phenyl, wherein said phenyl is independently optionally substituted, one or more times, with $R^4$;
$R^3$ represents $C_1$-$C_3$-alkyl, or $C_1$-$C_3$-haloalkyl;
$R^4$ represents halogen;
A represents a group:

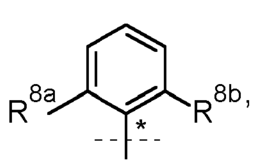
and
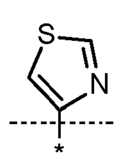
,

wherein * indicates the point of attachment of said group to the -NH- of formula (I);
$R^{8a}$ represents hydrogen, halogen, or methyl;
$R^{8b}$ represents hydrogen;
or an N-oxide, a salt, a tautomer or a stereoisomer of said compound, or a salt of said N-oxide, tautomer or stereoisomer.

**5.** The compound for use according of any one of claims 1 to 4, selected from:

N-{4-[3-anilino-7-ethyl-4-oxo-4,5,6,7-tetrahydro-1H-pyrrolo[3,2-c]pyridin-2-yl]pyridin-2-yl}-2-(4-fluorophenyl)acetamide;
N-{4-[(7R)-3-anilino-7-ethyl-4-oxo-4,5,6,7-tetrahydro-1H-pyrrolo[3,2-c]pyridin-2-yl]pyridin-2-yl}-2-(4-fluorophenyl)acetamide;
N-{4-[(7S)-3-anilino-7-ethyl-4-oxo-4,5,6,7-tetrahydro-1H-pyrrolo[3,2-c]pyridin-2-yl]pyridin-2-yl}-2-(4-fluorophenyl)acetamide;
N-{4-[3-anilino-7-ethyl-4-oxo-4,5,6,7-tetrahydro-1H-pyrrolo[3,2-c]pyridin-2-yl]pyridin-2-yl}-4,4-difluoro-2-(4-fluorophenyl)butanamide;
N-[4-(3-anilino-7-ethyl-4-oxo-4,5-dihydro-1H-pyrrolo[3,2-c]pyridin-2-yl)pyridin-2-yl]-4,4-difluoro-2-(4-fluorophenyl)butanamide;
N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide;
(-)-N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide;
(+)-N-{4-[3-anilino-5,7-dimethyl-4-oxo-4,5,6,7-tetrahydro-1H-pyrrolo[3,2-c]pyridin-2-yl]pyridin-2-yl}-2-(4-fluorophenyl)acetamide;
N-{4-[(7RS)-3-anilino-5,7-dimethyl-4-oxo-4,5,6,7-tetrahydro-1H-pyrrolo[3,2-c]pyridin-2-yl]pyridin-2-yl}-2-(4-fluorophenyl)propenamide;
N-{4-[3-anilino-5,7-dimethyl-4-oxo-4,5,6,7-tetrahydro-1H-pyrrolo[3,2-c]pyridin-2-yl]pyridin-2-yl}-4,4-difluoro-2-(4-fluorophenyl)butanamide;
N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide;
(2R)-N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide;
(2S)-N-[4-(3-anilino-5,7-dimethyl-4-oxo-4,5-dihydro-1H-pyrrolo[3,2-c]pyridin-2-yl)pyridin-2-yl]-4,4-difluoro-2-(4-fluorophenyl)butanamide;
N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide;
N-{4-[(7R)-3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide;
N-{4-[(7S)-3-anilino-5-methyl-4-oxo-7-(2,2,2-trifluoroethyl)-4,5,6,7-tetrahydro-1H-pyrrolo[3,2-c]pyridin-2-yl]pyridin-2-yl}-2-(4-fluorophenyl)acetamide;
N-{4-[3-anilino-5-methyl-4-oxo-7-(2,2,2-trifluoroethyl)-4,5,6,7-tetrahydro-1H-pyrrolo[3,2-c]pyridin-2-yl]pyridin-2-yl}-4,4-difluoro-2-(4-fluorophenyl)butanamide;
N-{4-[3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide;
N-{4-[(7R)-3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide;
N-{4-[(7S)-3-anilino-7-(cyclopropylmethyl)-5-methyl-4-oxo-4,5,6,7-tetrahydro-1H-pyrrolo[3,2-c]pyridin-2-yl]pyridin-2-yl}-2-(4-fluorophenyl)acetamide;
(2RS)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;
(+)-(2R)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;
(-)-(2S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;
(-)-(2R)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;
(+)-(2S)-N-{4-[cis-3-anilino-4-oxo-1,4,5,5a,6,7,8,8a-octahydrocyclopenta[b]pyrrolo[2,3-d]pyridin-2-yl]pyridin-2-yl}-4,4-difluoro-2-(4-fluorophenyl)butanamide;

(2S)-N-[4-(3-anilino-5-methyl-4-oxo-4,5-dihydro-1H-pyrrolo[3,2-c]pyridin-2-yl)pyridin-2-yl]-2-(4-fluorophenyl)propenamide;
(2R)-N-[4-(3-anilino-5-methyl-4-oxo-4,5-dihydro-1H-pyrrolo[3,2-c]pyridin-2-yl)pyridin-2-yl]-2-(4-fluorophenyl)propenamide;
N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide;
(2R)-N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide;
(2S)-N-[4-(3-anilino-5-methyl-4-oxo-4,5-dihydro-1H-pyrrolo[3,2-c]pyridin-2-yl)pyridin-2-yl]-4,4-difluoro-2-(4-fluorophenyl)butanamide;
4,4-difluoro-N-{4-[3-(2-fluoroanilino)-5,6-dimethyl-4-oxo-4,5-dihydro-1H-pyrrolo[3,2-c]pyridin-2-yl]pyridin-2-yl}-2-(4-fluorophenyl)butanamide;
N-{4-[3-anilino-7-(cyclopropylmethyl)-5-methyl-4-oxo-4,5-dihydro-1H-pyrrolo[3,2-c]pyridin-2-yl]pyridin-2-yl}-2-(4-fluorophenyl)acetamide;
2-(4-fluorophenyl)-N-{4-[5-methyl-3-(2-methylanilino)-4-oxo-7-(2,2,2-trifluoroethyl)-4,5-dihydro-1H-pyrrolo[3,2-c]pyridin-2-yl]pyridin-2-yl}acetamide;
N-{4-[3-anilino-5-methyl-4-oxo-7-(2,2,2-trifluoroethyl)-4,5-dihydro-1H-pyrrolo[3,2-c]pyridin-2-yl]pyridin-2-yl}-2-(4-fluorophenyl)acetamide;
N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide;
(2R)-N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide;
(2S)-N-{4-[3-anilino-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide;
N-[4-(3-anilino-5,7-dimethyl-4-oxo-4,5-dihydro-1H-pyrrolo[3,2-c]pyridin-2-yl)pyridin-2-yl]-2-(4-fluorophenyl)acetamide;
N-[4-(3-anilino-5,7-dimethyl-4-oxo-4,5-dihydro-1H-pyrrolo[3,2-c]pyridin-2-yl)pyridin-2-yl]-2-(4-fluorophenyl)propenamide;
(2R)-N-[4-(3-anilino-5,7-dimethyl-4-oxo-4,5-dihydro-1H-pyrrolo[3,2-c]pyridin-2-yl)pyridin-2-yl]-2-(4-fluorophenyl)propenamide;
(2S)-N-[4-(3-anilino-5,7-dimethyl-4-oxo-4,5-dihydro-1H-pyrrolo[3,2-c]pyridin-2-yl)pyridin-2-yl]-2-(4-fluorophenyl)propenamide;
N-{4-[3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide;
N-{4-[(7R)-3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide;
N-{4-[(7S)-3-anilino-7-(2,2-difluoroethyl)-5-methyl-4-oxo-4,5,6,7-tetrahydro-1H-pyrrolo[3,2-c]pyridin-2-yl]pyridin-2-yl}-2-(4-fluorophenyl)acetamide;
N-{4-[3-anilino-7-(2,2-difluoroethyl)-5-methyl-4-oxo-4,5-dihydro-1H-pyrrolo[3,2-c]pyridin-2-yl]pyridin-2-yl}-2-(4-fluorophenyl)acetamide;
2-(4-fluorophenyl)-N-{4-[(7RS)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide;
2-(4-fluorophenyl)-N-{4-[(7R)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide;
2-(4-fluorophenyl)-N-{4-[(7S)-5-methyl-4-oxo-3-(1,3-thiazol-4-ylamino)-7-(2,2,2-trifluoroethyl)-4,5,6,7-tetrahydro-1H-pyrrolo[3,2-c]pyridin-2-yl]pyridin-2-yl}acetamide;
N-{4-[(7RS)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide;
N-{4-[(7R)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide;
N-{4-[(7S)-7-(cyclopropylmethyl)-5-methyl-4-oxo-3-(1,3-thiazol-4-ylamino)-4,5,6,7-tetrahydro-1H-pyrrolo[3,2-c]pyridin-2-yl]pyridin-2-yl}-2-(4-fluorophenyl)acetamide;
(2RS)-4,4-difluoro-2-(4-fluorophenyl)-N-{4-[(7RS)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;
(2R)-4,4-difluoro-2-(4-fluorophenyl)-N-{4-[(7R)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;
(2R)-4,4-difluoro-2-(4-fluorophenyl)-N-{4-[(7S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;

(2S)-4,4-difluoro-2-(4-fluorophenyl)-N-{4-[(7R)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;
(2S)-4,4-difluoro-2-(4-fluorophenyl)-N-{4-[(7S)-5-methyl-4-oxo-3-[(1,3-thiazol-4-yl)amino]-7-(2,2,2-trifluoroethyl)-4,5,6,7-tetrahydro-1H-pyrrolo[3,2-c]pyridin-2-yl]pyridin-2-yl}butanamide;
2-(4-fluorophenyl)-N-{4-[5-methyl-3-(2-methylanilino)-4-oxo-7-(2,2,2-trifluoroethyl)-4,5,6,7-tetrahydro-1H-pyrrolo[3,2-c]pyridin-2-yl]pyridin-2-yl}acetamide;
or an N-oxide, a salt, a tautomer or a stereoisomer of said compound, or a salt of said N-oxide, tautomer or stereoisomer.

**6.** The compound for use of any one of claims 1 to 5, wherein the subject has a hyperproliferative disease and/or disorder responsive to induction of cell death.

**7.** The compound for use of claim 6, wherein the hyperproliferative disease and/or disorder responsive to induction of cell death is a haematological tumour, solid tumour and/or metastases thereof.

**8.** The compound for use of claim 7, wherein the haematological tumour is a lymphoma and/or metastases thereof; and the solid tumour is a cervical tumour, a lung tumour, a colon tumour and/or metastases thereof.

**9.** The compound for use of claim 8, wherein the lymphoma is diffuse large B-cell lymphoma and/or metastases thereof; the lung tumour is a lung carcinoma and/or metastases thereof; and the colon tumour is a colorectal carcinoma and/or metastases thereof.

**10.** The compound for use of any one of claims 1 to 5, wherein the subject has an angiogenesis-related disease.

**11.** The compound for use of claim 10, wherein the angiogenesis-related disease is angiogenesis of a tumor.

**12.** The compound for use of claim 10 or 11, wherein the angiogenesis related disease is diabetic neuropathy, a pathological condition, ischemic retinal-vein occlusion, retinopathy of prematurity, age-related macular degeneration (AMD), neovascular glaucoma, psoriasis, retrolental fibroplasias, angiofibroma, inflammation, rheumatoid arthritis (RA), restenosis, in-stent restenosis, or vascular graft restenosis.

**13.** The compound for use of any one of claims 1 to 12, wherein the compound is for use in inhibiting CSNK1A1.

**14.** The compound for use of any one of claims 1 to 13, wherein the compound has an $IC_{50}$ of below 100 nM for CSNK1A1.

**15.** The compound for use of any one of claims 1 to 14, wherein the compound has an $IC_{50}$ of greater than 600 nM for wild type-EGFR kinase.

**Figure 1:**

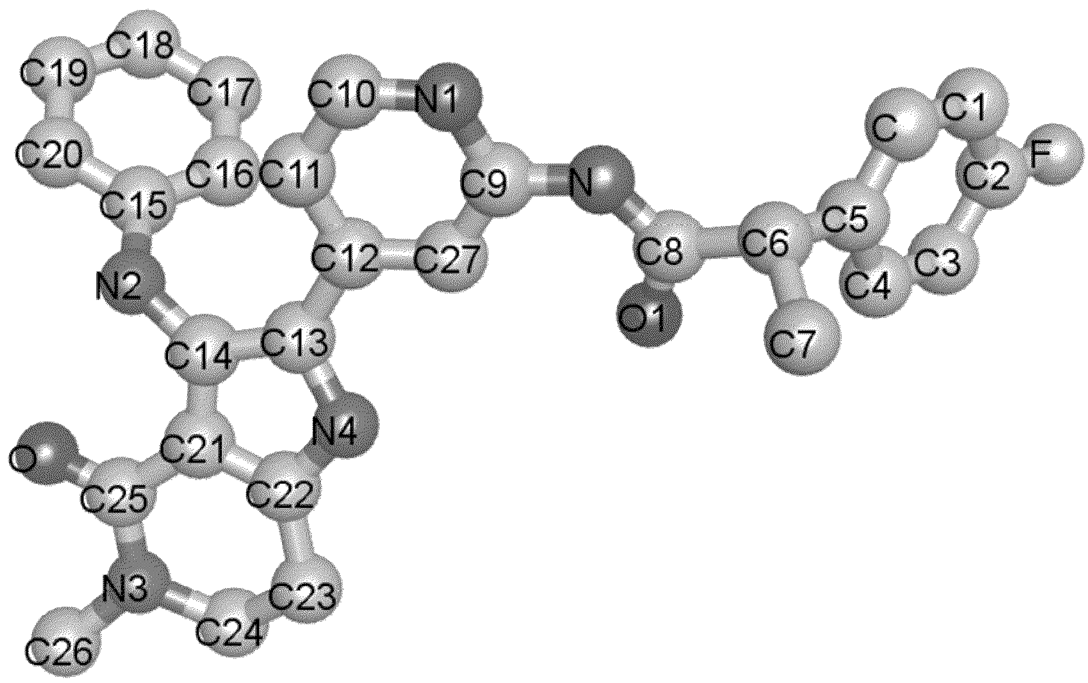

**Figure 1: Structure of Intermediate 42**

For clarity only the ligand atoms are shown. Carbon atom C6 unambiguously features the (*S*)-configuration.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016120196 A
- US 3966781 A, J. G. Atkinson
- WO 2012112363 A, K. Kassahun
- WO 201472220 A1, Menichincheri
- WO 201422752 A1, Cee
- WO 200940399 A1, Marchionni
- WO 201362344 A1, Kim
- WO 201522073 A1, Voss
- WO 200739740 A2
- WO 2016100166 A
- US 5011472 A


### Non-patent literature cited in the description


- **KNIPPSCHILD.** *Onkologie,* 2005, vol. 28, 508-514
- **KNIPPSCHILD et al.** *Front Oncol.,* 19 May 2014, vol. 4, 96
- **SCHITTEK ; SINNBERG.** *Molecular Cancer,* 2014, vol. 13, 231
- **HUTCHINSON et al.** *JBC,* 2011, vol. 286 (10), 8688
- **JARAS et al.** *J. Exp. Med.,* 2014, vol. 211 (4), 605
- **BIDERE.** *Nature,* 05 March 2009, vol. 458
- Isotopic Compositions of the Elements 1997. *Pure Appl. Chem,* 1998, vol. 70 (1), 217-235
- **H. J. LEIS et al.** *Curr. Org. Chem.,* 1998, vol. 2, 131
- **ESAKI et al.** *Tetrahedron,* 2006, vol. 62, 10954
- **ESAKI et al.** *Chem. Eur. J.,* 2007, vol. 13, 4052
- **J. R. MORANDI et al.** *J. Org. Chem.,* 1969, vol. 34 (6), 1889
- **N. H. KHAN.** *J. Am. Chem. Soc.,* 1952, vol. 74 (12), 3018
- **S. CHANDRASEKHAR et al.** *Tetrahedron,* 2011, vol. 52, 3865
- **HANZLIK et al.** *J. Org. Chem.,* 1990, vol. 55, 3992-3997
- **R. P. HANZLIK et al.** *Biochem. Biophys. Res. Commun.,* 1989, vol. 160, 844
- **P. J. REIDER et al.** *J. Org. Chem.,* 1987, vol. 52, 3326-3334
- **M. JARMAN et al.** *Carcinogenesis,* 1993, vol. 16 (4), 683-688
- **J. ATZRODT et al.** *Angew. Chem., Int. Ed.,* 2007, vol. 46, 7744
- **K. MATOISHI et al.** *J. Chem. Soc, Chem. Commun.,* 2000, 1519-1520
- **A. STREITWIESER et al.** *J. Am. Chem. Soc.,* 1963, vol. 85, 2759
- **C. L. PERRIN et al.** *J. Am. Chem. Soc.,* 2007, vol. 129, 4490
- **C. L. PERRIN et al.** *J. Am. Chem. Soc.,* 2003, vol. 125, 15008
- **C. L. PERRIN.** *Advances in Physical Organic Chemistry,* vol. 44, 144
- **C. L. PERRIN et al.** *J. Am. Chem. Soc.,* 2005, vol. 127, 9641
- **B. TESTA et al.** *Int. J. Pharm.,* 1984, vol. 19 (3), 271
- **D. J. KUSHNER et al.** *Can. J. Physiol. Pharmacol.,* 1999, vol. 77, 79
- **A. E. MUTLIB et al.** *Toxicol. Appl. Pharmacol.,* 2000, vol. 169, 102
- **A. M. SHARMA et al.** *Chem. Res.Toxicol.,* 2013, vol. 26, 410
- **UETRECHT et al.** *Chemical Research in Toxicology,* 2008, vol. 21 (9), 1862
- **A. J. MORALES et al.** *The 15th North American Meeting of the International Society of Xenobiotics, San Diego, CA,* 12 October 2008
- **C. J. WENTHUR et al.** *J. Med. Chem.,* 2013, vol. 56, 5208
- **F. SCHNEIDER et al.** *Arzneim. Forsch. Drug. Res.,* 2006, vol. 56, 295
- **TELAPREVIR: F. MALTAIS et al.** *J. Med. Chem.,* 2009, vol. 52, 7993
- *Pure Appl Chem,* 1976, vol. 45, 11-30
- **S. M. BERGE et al.** Pharmaceutical Salts. *J. Pharm. Sci.,* 1977, vol. 66, 1-19
- **D. E. WORRALL.** *J. Am. Chem. Soc.,* 1940, vol. 62, 675
- **J. H. HUTCHINSON et al.** *J. Med. Chem.,* 1996, vol. 39, 4583-4591
- **N. L. SUBASINGHE et al.** *Bioorg. Med. Chem. Lett.,* 2013, vol. 23, 1063-1069
- **C. F. JONES et al.** *Synlett,* 2010, 654-658
- **M. NOGUCHI et al.** *Bull. Chem. Soc. Japan,* 1986, vol. 59, 1355-1362

- **S. MIWATASHI et al.** *J. Med. Chem.,* 2005, 5966-5979
- **J. ZHAO et al.** *Bioorg. Med. Chem. Lett,* 2014, vol. 24, 2802-2806
- **M. P. HAY et al.** *J. Med. Chem.,* 2010, vol. 53, 787-797
- **J. M. KEITH et al.** *Med. Chem. Lett,* 2012, vol. 3, 823-827
- **J. LIANG et al.** *Eur. J. Med. Chem.,* 2013, vol. 67, 175-187
- **T. W. GREENE.** Protective Groups in Organic Synthesis. John Wiley & Sons, 1999
- **P. KOCIENSKI.** Protecting Groups. Thieme Medical Publishers, 2000
- **T.W. GREENE ; P.G.M. WUTTS.** Protective Groups in Organic Synthesis. Wiley, 1999
- **CLARK et al.** *J. Heterocyclic Chem.,* 1993, vol. 30, 829-831
- **CLARK et al.** *J. Med. Chem.,* 1993, vol. 36, 2645-2657
- **SCHNELLER et al.** *J. Med. Chem.,* 1978, vol. 21, 990-993
- **SMITH et al.** *Bioorg. Med. Chem. Lett.,* 2007, vol. 17, 673-678
- **AIELLO, E. et al.** *J. Heterocyclic Chem.,* 1982, vol. 19, 977-979
- **DURANTI, A. et al.** *Bioorg. Med. Chem.,* 2003, vol. 11, 3965-3973
- Protecting Groups. Thieme Medical Publishers, 2000
- **AIELLO et al.** *New Engl. J. Med.,* 1994, vol. 331, 1480
- **PEER et al.** *Lab. Invest.,* 1995, vol. 72, 638
- **LOPEZ et al.** *Invest. Opththalmol. Vis. Sci.,* 1996, vol. 37, 855
- **POWELL, M.F. et al.** Compendium of Excipients for Parenteral Formulations. *PDA Journal of Pharmaceutical Science & Technology,* 1998, vol. 52 (5), 238-311
- **STRICKLEY, R.G.** Parenteral Formulations of Small Molecule Therapeutics Marketed in the United States (1999)-Part-1. *PDA Journal of Pharmaceutical Science & Technology,* 1999, vol. 53 (6), 324-349
- **NEMA, S. et al.** Excipients and Their Use in Injectable Products. *PDA Journal of Pharmaceutical Science & Technology,* 1997, vol. 51 (4), 166-171
- **GOODMAN ; GILMAN'S et al.** The Pharmacological Basis of Therapeutics. McGraw-Hill, 1996, 1225-1287
- *CHEMICAL ABSTRACTS,* 845267-80-3
- *CHEMICAL ABSTRACTS,* 115497-23-9
- *CHEMICAL ABSTRACTS,* 10416-59-8
- *CHEMICAL ABSTRACTS,* 105-56-6
- *CHEMICAL ABSTRACTS,* 4039-32-1
- *CHEMICAL ABSTRACTS,* 6226-25-1
- *CHEMICAL ABSTRACTS,* 37517-81-0
- *CHEMICAL ABSTRACTS,* 199296-51-0
- *CHEMICAL ABSTRACTS,* 1189-71-5
- *CHEMICAL ABSTRACTS,* 142-29-0
- *CHEMICAL ABSTRACTS,* 103-72-0
- *CHEMICAL ABSTRACTS,* 6674-22-2
- *CHEMICAL ABSTRACTS,* 1538957-14-0
- *CHEMICAL ABSTRACTS,* 128625-52-5
- *CHEMICAL ABSTRACTS,* 118263-97-1
- *CHEMICAL ABSTRACTS,* 7087-68-5
- *CHEMICAL ABSTRACTS,* 68384-70-3
- *CHEMICAL ABSTRACTS,* 1122-58-3
- *CHEMICAL ABSTRACTS,* 7647-01-0
- *CHEMICAL ABSTRACTS,* 7087-685
- *CHEMICAL ABSTRACTS,* 4403-70-7
- *CHEMICAL ABSTRACTS,* 84-58-2